# EUROPEAN PATENT APPLICATION

(11) **EP 1 288 296 A2**
(43) Date of publication of application: **05.03.2003**
(21) Application number: 02008657.5
(22) Date of filing: 29.04.1993
(51) Int. Cl.: C12N 15/11

(54) **Method and reagent for inhibiting HBV viral replication**

(30) Priority: 11.05.1992 US 882689; 14.05.1992 US 882712; 14.05.1992 US 882713; 14.05.1992 US 882714; 14.05.1992 US 882823; 14.05.1992 US 882824; 14.05.1992 US 882886; 14.05.1992 US 882888; 14.05.1992 US 882889; 14.05.1992 US 882921; 14.05.1992 US 882922; 14.05.1992 US 883823; 14.05.1992 US 883849; 14.05.1992 US 884073; 14.05.1992 US 884074; 14.05.1992 US 884333; 14.05.1992 US 884422; 14.05.1992 US 884431; 14.05.1992 US 884436; 14.05.1992 US 884521; 31.07.1992 US 923738; 26.08.1992 US 935854; 26.08.1992 US 936086; 18.09.1992 US 948359; 15.10.1992 US 963322; 07.12.1992 US 987129; 07.12.1992 US 987130; 07.12.1992 US 987133
(62) Divisional of application: 93910893.2
(71) Applicant: RIBOZYME PHARMACEUTICALS, INC., Boulder, CO 80301 (US)
(72) Inventor: Draper, Kenneth, G., Boulder, CO 80301 (US); McSwiggen, James A., Boulder, CO 80301 (US); Holecek, James J., Solon, OH 44139 (US); Dudycz, Lech W., Boulder, CO 80301 (US); Macejak, Dennis G., Denver, CO 80220 (US); Mamone, J. Anthony, Parma, OH 44134 (US)
(74) Representative: Viering, Jentschura & Partner

(57) **Abstract**

The invention relates to enzymatic RNA molecules which are active to specifically cleave genomic RNA, or mRNA, or hnRNA of or encoded by a hepatitis B Virus.

## Description

### Background of the Invention

This invention relates to reagents useful as inhibitors of viral replication, infection and gene expression.

### Summary of the Invention

The invention features novel enzymatic RNA molecules, or ribozymes, and methods for their use for inhibiting viral replication, e.g., the replication of picornaviruses (poliovirus, coxsackievirus, hepatitis A virus, echovirus, human rhinovirus, cardioviruses (e.g., mengovirus and encephalomyocarditis virus) and foot-and-mouth disease virus); immunodeficiency virus (e.g., HIV-1, HIV-2 and related viruses including FIV-1 and SIV-1); hepatitis B virus (HBV); papillomavirus; Epstein-Barr virus (EBV); T-cell leukemia virus, e.g., HTLV-I, HTLV-II and related viruses, including bovine leukemia virus (BLV) and simian T-cell leukemia virus (STLV-I); hepatitis C virus (HCV); cytomegalovirus (CMV); influenza virus; herpes simplex virus (HSV). Such ribozymes can be used in a method for treatment of diseases caused by these viruses in man and other animals, including other primates. Indeed one ribozyme may be designed for treatment of many of the diseases caused by these viruses.

Ribozymes are RNA molecules having an enzymatic activity which is able to repeatedly cleave other separate RNA molecules in a nucleotide base sequence specific manner. Such enzymatic RNA molecules can be targeted to virtually any RNA transcript, and efficient cleavage has been achieved *in vitro.* Kim et al., 84 Proc. Natl. Acad. Sci. USA 8788, 1987; Haseloff and Gerlach, 334 Nature 585, 1988; Cech, 260 JAMA 3030, 1988; and Jefferies et al., 17 Nucleic Acids Research 1371, 1989.

Ribozymes act by first binding to a target RNA. Such binding occurs through the target RNA binding portion of a ribozyme which is held in close proximity to an enzymatic portion of the RNA which acts to cleave the target RNA. Thus, the ribozyme first recognizes and then binds a target RNA through complementary base-pairing, and once bound to the correct site, acts enzymatically to cut the target RNA. Strategic cleavage of such a target RNA will destroy its ability to direct synthesis of an encoded protein. After a ribozyme has bound and cleaved its RNA target it is released from that RNA to search for another target and can repeatedly bind and cleave new targets.

The enzymatic nature of a ribozyme is advantageous over other technologies, such as antisense technology (where a nucleic acid molecule simply binds to a nucleic acid target to block its translation) since the effective concentration of ribozyme necessary to effect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base pairing. Thus, it is thought that the specificity of action of a ribozyme is greater than that of antisense oligonucleotide binding the same RNA site.

Ribozymes targeting any of the specific viral regions described in detail below should be able to cleave the RNAs in a manner which will inhibit the translation of the molecules.

Thus, in a first aspect, the invention features an enzymatic RNA molecule (or ribozyme) which specifically cleaves the mRNA, hnRNA, or gene of any one of the following viruses: picornavirus, HIV-1, HBV, papillomavirus, EBV, T-cell leukemia virus, e.g., HTLV-1, HCV, CMV, influenza virus, and HSV.

By "gene" is meant to refer to either the protein coding regions of the cognate mRNA, or any regulatory regions in the RNA which regulate synthesis of the protein or stability of the mRNA; the term also refers to those regions of an mRNA which encode the ORF of a cognate polypeptide product, and the proviral genome.

By "enzymatic RNA molecule" it is meant an RNA molecule which has complementarity in a substrate binding region to a specified gene target, and also has an enzymatic activity which is active to specifically cleave RNA in that target. That is, the enzymatic RNA molecule is able to intermolecularly cleave RNA and thereby inactivate a target RNA molecule. This complementarity functions to allow sufficient hybridization of the enzymatic RNA molecule to the target RNA to allow the cleavage to occur. One hundred percent complementarity is preferred, but complementarity as low as 50-75% may also be useful in this invention. By "equivalent" RNA to a virus is meant to include those naturally occurring viral encoded RNA molecules associated with viral caused diseases in various animals, including humans, cats, simians, and other primates. These viral or viral-encoded RNAs have similar structures and equivalent genes to each other.

In preferred embodiments, the enzymatic RNA molecule is formed in a hammerhead motif, but may also be formed in the motif of a hairpin, hepatitis delta virus, group I intron or RNaseP-like RNA (in association with an RNA guide sequence). Examples of such hammerhead motifs are described by Rossi et al., 8 Aids Research and Human Retroviruses 183, 1992; of hairpin motifs by Hampel and Tritz, 28 Biochemistry 4929, 1989 and Hampel et al., 18 Nucleic Acids Research 299, 1990; an example of the hepatitis delta virus motif is described by Perrotta and Been, 31 Biochemistry 16, 1992; of the RNaseP motif by Guerrier-Takada et al., 35 Cell 849, 1983; and of the group I intron by Cech et al., U.S. Patent 4,987,071. These specific motifs are not limiting in the invention and those skilled in the art will recognize that all that is important in an enzymatic RNA molecule of this invention is that it has a specific substrate binding site which is complementary to one or more of the target gene RNA regions, and that it have nucleotide sequences within or surrounding that substrate binding site which impart an RNA cleaving activity to the molecule.

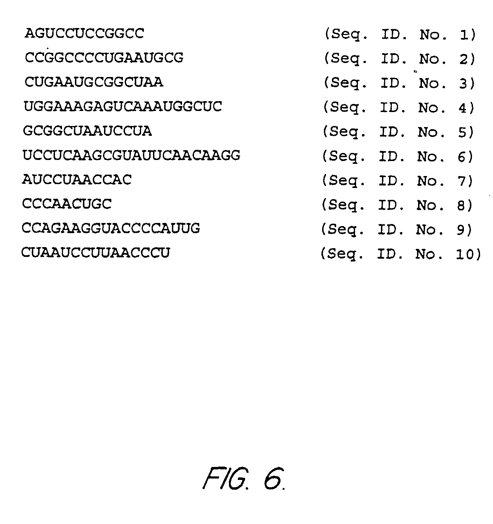

In particularly preferred embodiments, the RNA which is cleaved in viral RNA is selected from one or more of the sequences shown in Figs. 6 to 15.

In a second related aspect, the invention features a mammalian or vertebrate cell which includes an enzymatic RNA molecule as described above. Preferably, the cell is a human cell, for example, a T4 lymphocyte having a CD4 receptor molecule on its cell surface, or other primate cell.

In a third related aspect, the invention features an expression vector which includes nucleic acid encoding the enzymatic RNA molecules described above, located in the vector, e.g., in a manner which allows expression of that enzymatic RNA molecule within a mammalian or vertebrate cell. Such a vector may include the ribozyme under control of a virus promoter, e.g., an EBV promoter.

In a fourth related aspect, the invention features a method for treatment of a virus-caused disease by administering to a patient an enzymatic RNA molecule which cleaves virus or virus encoded nucleic acid.

In other related aspects, the invention features treatment of cats or simians with ribozymes of this invention. Such ribozymes may be identical to those able to cleave HIV-1 RNA, or may be modified to target analogous locations in FIV and SIV virus RNAs.

In other related aspects, the invention features treatment of cattle or simians with ribozymes of this invention. Such ribozymes may be identical to those able to cleave HTLV-I or II RNA, or may be modified to target analogous locations in BLV and STLV virus RNAs.

The invention provides a class of chemical cleaving agents which exhibit a high degree of specificity for the viral related RNA in virus-infected cells or virion particles. If desired, such ribozymes can be designed to target equivalent single-stranded DNAs by methods known in the art. The ribozyme molecule is preferably targeted to a highly conserved sequence region of a virus such that all types and strains of these viruses can be treated with a single ribozyme. Such enzymatic RNA molecules can be delivered exogenously or endogenously to infected cells. In the preferred hammerhead motif the small size (less than 40 nucleotides, preferably between 32 and 36 nucleotides in length) of the molecule allows the cost of treatment to be reduced compared to other ribozyme motifs.

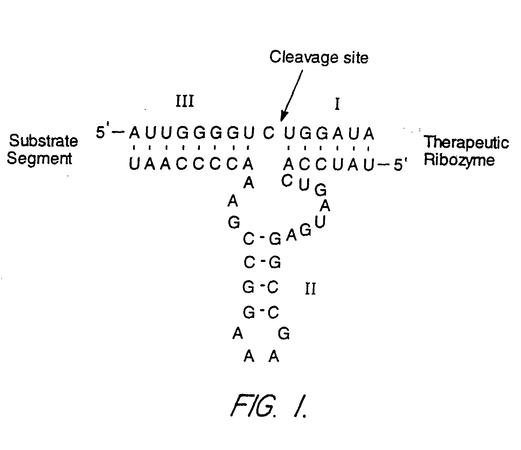

The smallest ribozyme delivered for treatment of HIV infection reported to date (by Rossi et al., 1992, *supra*) is an *in vitro* transcript having a length of 142 nucleotides. Synthesis of ribozymes greater than 100 nucleotides in length is very difficult using automated methods, and the therapeutic cost of such molecules is prohibitive. Delivery of ribozymes by expression vectors is primarily feasible using only *ex vivo* treatments. This limits the utility of this approach. In this invention, small ribozyme motifs (e.g., of the hammerhead structure, shown generally in Fig. 1) are used for exogenous delivery. The simple structure of these molecules also increases the ability of the ribozyme to invade targeted regions of the mRNA structure. Thus, unlike the situation when the hammerhead structure is included within longer transcripts, there are no non-ribozyme flanking sequences to interfere with correct folding of the ribozyme structure or with its complementary binding of the ribozyme to the mRNA target region.

The enzymatic RNA molecules of this invention can be used to treat virus infections including those caused by both HIV-1 and HIV-2, HTLV-I and HTLV-II. Such treatment can also be extended to other related viruses which infect non-human primates including the simian and feline immunodeficiency viruses and bovine leukemia viruses. Infected animals can be treated at the time of productive or latent infection. This timing of treatment will reduce viral loads and gene expression in infected cells and disable viral replication in any subsequent rounds of productive infection. This is possible because the preferred ribozymes disable those structures required for successful initiation of viral protein synthesis.

For treatment of transformed cervical epithelia or keratinocytes, the method of this invention will inhibit the expression of viral genes known to cause cell immortalization. For treatment of latent viral infections, this invention will inhibit gene expression required for the maintenance of the viral episomal genome. For treatment of transformed hepatocytes, the methods of this invention allow inhibition of the expression of viral genes thought to cause cell transformation.

The preferred targets of the present invention are advantageous over other targets since they act not only at the time of viral absorption or genomic replication (reverse transcription) during the productive infection, but also in latently infected cells and in virally transformed cells. In addition, viral particles which are released during a first round of infection in the presence of such ribozymes will still be immunogenic by virtue of having their capsids or virions intact. Thus, one method of this invention allows the creation of defective but immunogenic viral particles, and thus a continued possibility of initiation of an immune response in a treated animal.

In addition, the enzymatic RNA molecules of this invention can be used *in vitro* in a cell culture infected or transfected with viruses to produce defective viral particles, which may have intact capsids and defective genomic RNA or exhibit structurally intact but biologically deficient virions, i.e., the virion may be stable but deficient in a protein required for adsorption or penetration. These particles can then be used for instigation of immune responses in a prophylactic manner, or as a treatment of infected animals.

In yet another aspect, the invention features use of viruses as vectors for carrying an enzymatic RNA molecule to a cell infected with another virus. Such vectors can be formed by standard methodology.

The invention also features immunization preparations formed from defective viruses, e.g., HIV-1, EBV, or HTLV-I particles (or related particles), created by a method of this invention, and methods for immunization or vaccination using these defective particles, e.g., with DNA or vectors encoding a ribozyme of this invention under the control of a suitable promoter.

Ribozymes of this invention may be used as diagnostic tools to examine genetic drift and mutations within diseased cells. The close relationship between ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structure and function *in vitro,* as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple ribozymes targeted to different genes, ribozymes coupled with known small molecule inhibitors, or intermittent treatment with combinations of ribozymes and/or other chemical or biological molecules).

Other features and advantages of the invention will be apparent from the following description of the preferred embodiments thereof, and from the claims.

### Description of the Preferred Embodiments

The drawings will first briefly be described.

### Drawings

Fig. 1 is a diagrammatic representation of a hammerhead motif ribozyme showing stems I, II and III (marked (I), (II) and (III) respectively) interacting with a target region. The 5' and 3' ends of both ribozyme and target are shown. Dashes indicate base-paired nucleotides.
Figs. 2A and 2B are diagrammatic representations of the various genes and gene regions in HIV-1 and HIV-2.
Figs. 3A - 3C are diagrammatic representations of three ribozymes of this invention.
Figs. 4A - 4G are diagrammatic representations of chemically modified ribozymes of this invention (solid circles indicate modified bases).
Fig. 5 is a diagrammatic representation of a chemically modified ribozyme of this invention (circled bases are modified with 2'-O-methyl).
Figs. 6 - 15 are preferred targets for picornavirus, HIV, HBV, papilloma, EBV, HTLV, HCV, CMV, influenza, and HSV, respectively.
Figs. 16, 17 and 18 are diagrammatic representations of various vectors of the present invention.
Fig. 19 is a diagrammatic representation of a method for formation of a vector of this invention.

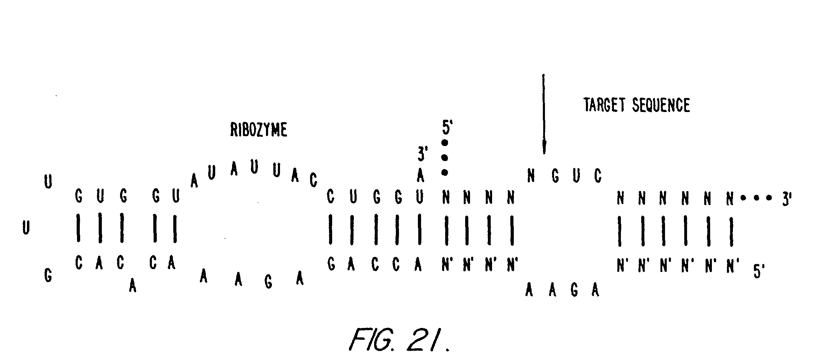

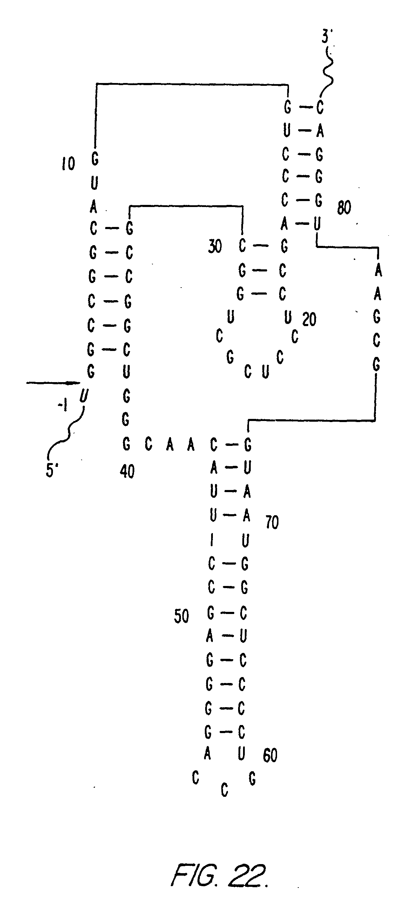

Figs. 20, 21 and 22 are diagrammatic representations of hammerhead, hairpin and HDV ribozymes, respectively.
Figs. 23 and 24 are copies of HPLC results showing purification of ribozyme from failure sequences (Fig. 23) and other contaminants (Fig. 24).
Fig. 25 is a diagrammatic representation of a hammerhead ribozyme in equilibrium between a tightly constrained structure and an undesirable loose structure.
Fig. 26 is a general scheme for generation of useful substrate sets.

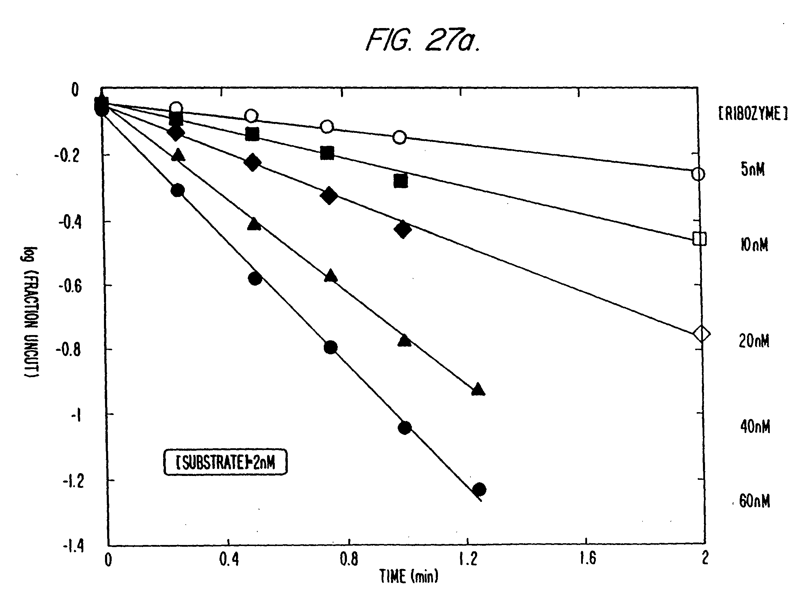

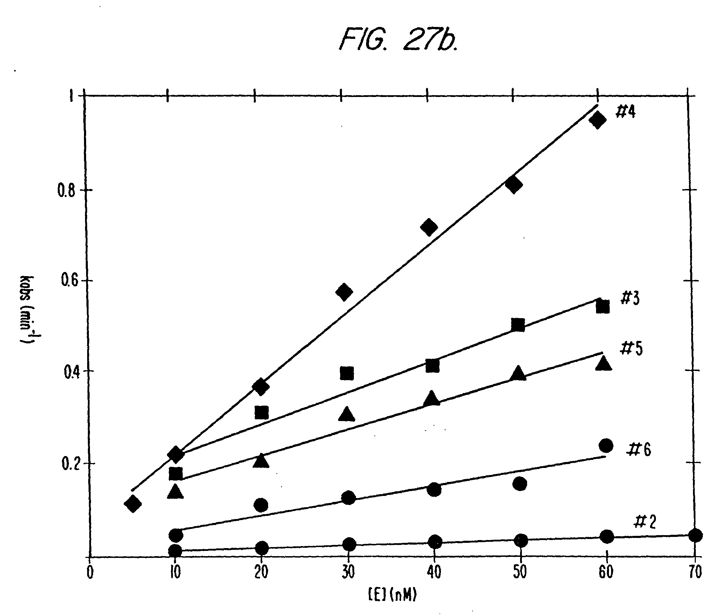

Fig. 27 shows an example of a standard kinetic analysis of ribozyme activity with enzyme concentration in excess over substrate concentration. 5'-[³²P]-labeled substrate (typically at a concentration of 1-2 nM) is incubated with ribozyme at varying concentrations (typically 5-100 nM) at 37°C in a buffer containing 75 mM Tris-HCl, pH 8.0, 10 mM MgCl₂. At various times, aliquots are removed and mixed into a standard formamide/EDTA/dyes gel-loading buffer to stop the reaction and prepare the samples for gel-electrophoresis. The products are separated by denaturing polyacrylamide gel electrophoresis and quantitated by a beta emission scanner (Ambis Systems, San Diego, CA).
   Specifically, Fig. 27A shows a plot of the log of the fraction of substrate molecules remaining uncleaved as a function of time. In enzyme (e) excess conditions, substrate cleavage follows the equation:${\text{S}}_{\text{t}} {\text{= S}}_{\text{o}} {\text{e}}^{\text{-kobs t}}$ where Sₜ is the concentration of substrate at time, t, Sₒ is the starting concentration of substrate, and kobs is the observed cleavage rate. Thus:${\text{log}}_{\text{c}} {\text{(S}}_{\text{t}} {\text{/ S}}_{\text{o}} {\text{) = log}}_{\text{c}} \text{(F) = -kobs t}$ where F = Sₜ / Sₒ is the fraction of substrate at time t. So the slope of the lines in Fig. 27A gives kobs for each ribozyme concentration shown at the right of the graph.
   Fig. 27B shows a plot of kobs vs. enzyme concentration for five different ribozymes. Under enzyme excess conditions (and low enzyme concentration) the curves follow the equation:$\text{kobs = (kcat / Km) [Enzyme]}$ so the slope of each line gives the (kcat / Km) value for that enzyme. Lines with steeper slopes have higher kcat/km values and indicate more active ribozymes. In this figure, ribozyme #4 is the most active and ribozyme #2 is the least active.

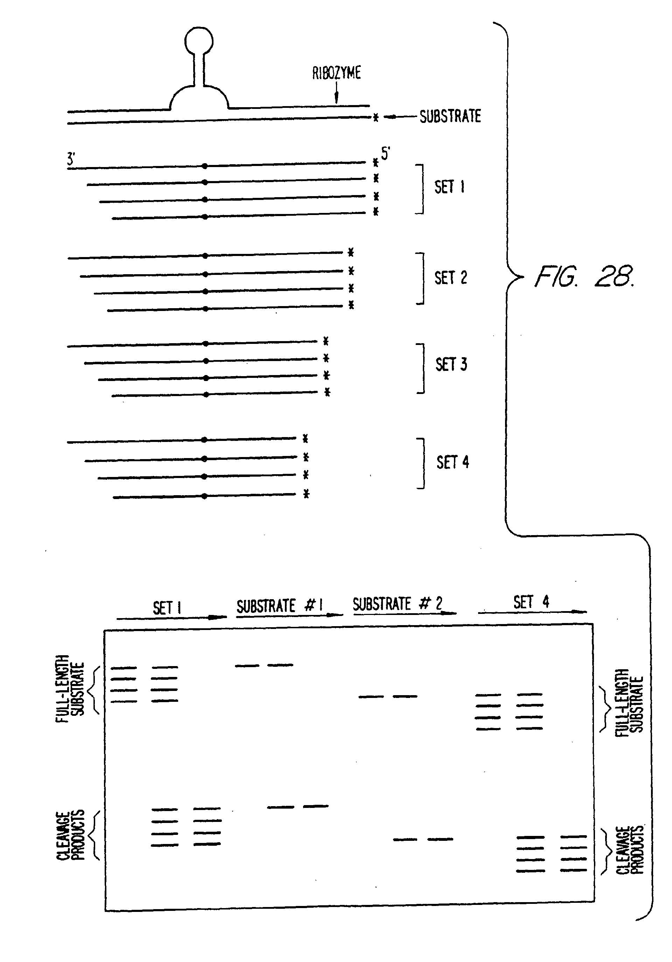

Fig. 28 is a general scheme for a method of this invention.

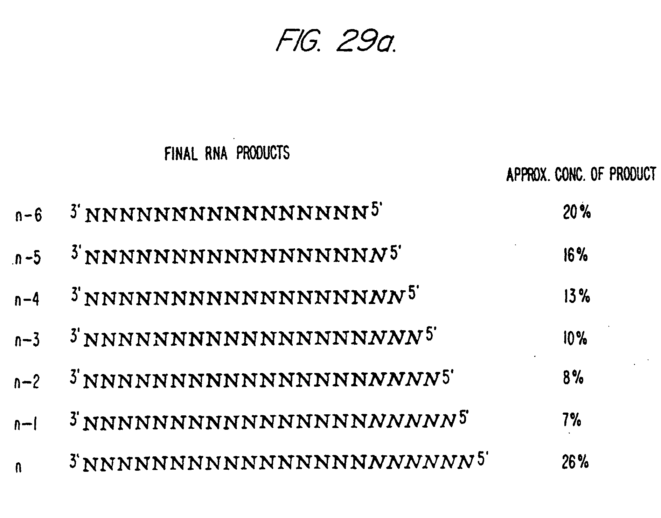

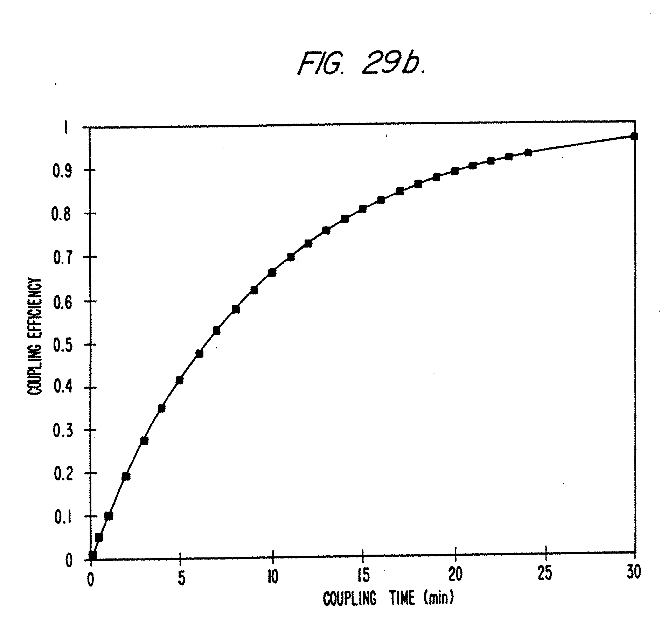

Fig. 29A is a diagrammatic representation of a method of the invention.
Fig. 29B is a graph showing expected coupling efficiencies vs. time.

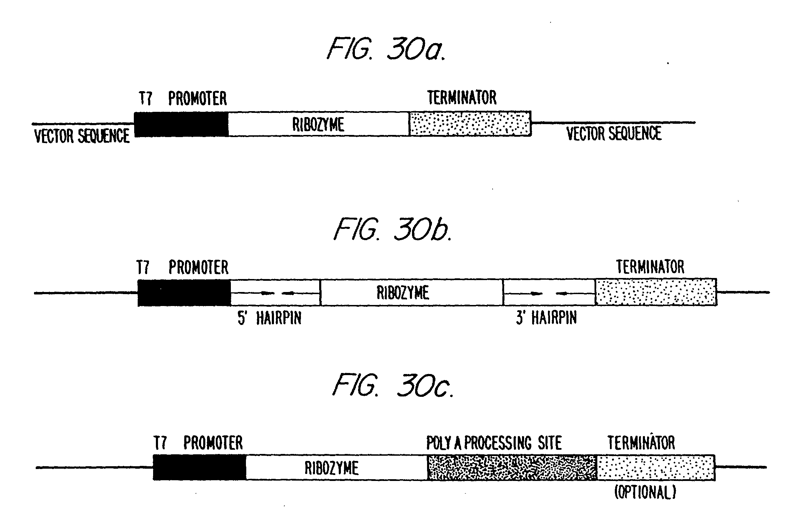

Figs. 30A, B, and C are diagrammatic representations of several vectors of this invention. The three vectors shown as general examples in Fig. 30 all contain a T7 promoter upstream from a region encoding a ribozyme. The vectors may also include a sequence known to encode a T7 terminator located downstream of the ribozyme-encoding sequence. The simplest of the vectors, shown in Fig. 30A, includes a T7 promoter, a ribozyme encoding sequence and a T7 terminator. As shown in Fig. 30B, additional sequence encoding hairpin structures may also be included on both the 5' and 3' sides of the ribozyme-encoding sequence. In Fig. 30C, a poly(A) polymerase processing site is included downstream of the ribozyme-encoding sequence and may or may not be followed by a T7 terminator sequence. The T7 promoter is not limiting to this invention. If other promoters are employed (e.g., HSV ICP4-responsive promoters, or the thymidine kinase gene promoter) then the T7 termination sequence may not be effective in terminating transcription. In that case, the poly(A) processing site would be valuable in ensuring proper termination of the transcript.

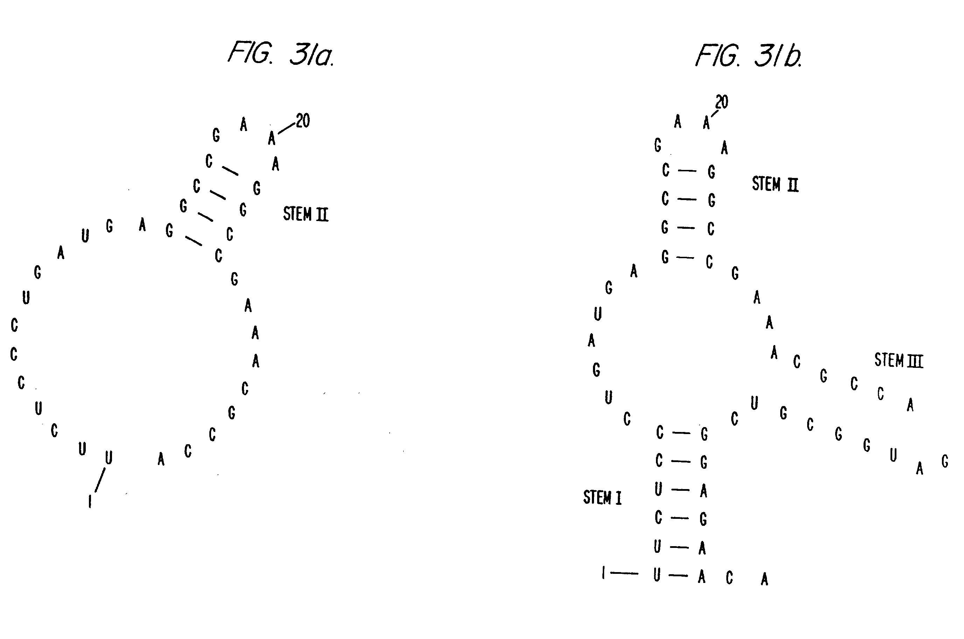

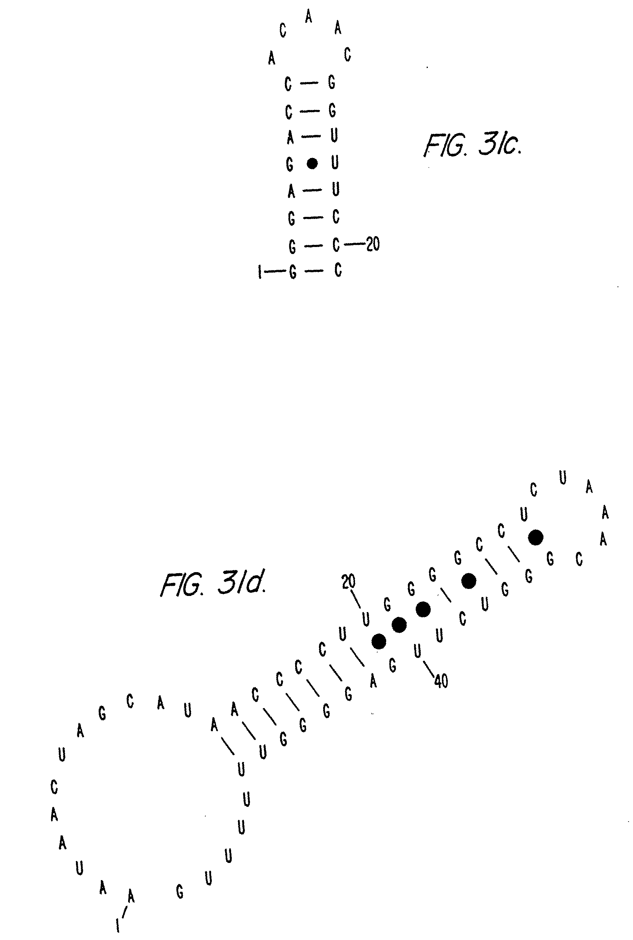

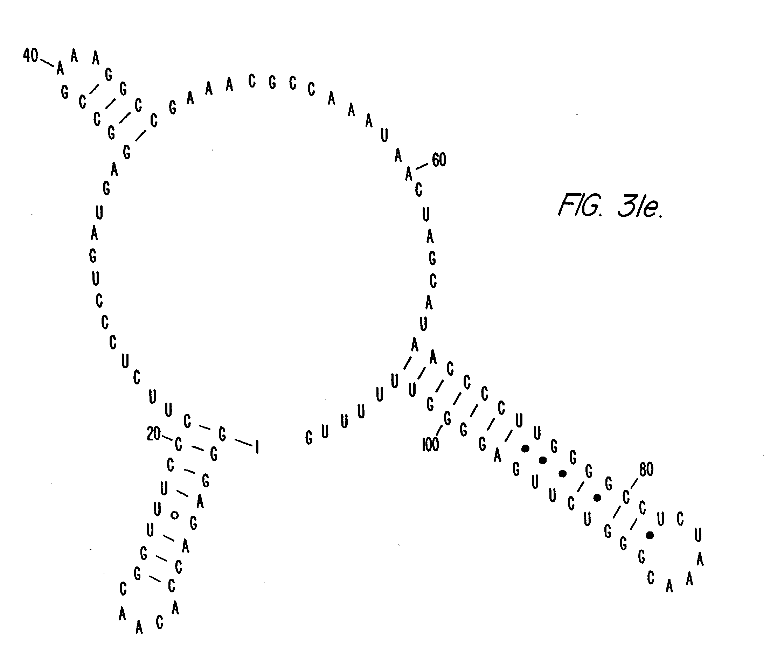

Figs. 31A-E show examples of ribozyme sequences with 5' and 3' flanking hairpin sequences. Specifically, Fig. 31A shows ribozyme alone, showing that the ribozyme forms only stem II and no other secondary structures in the absence of substrate. The ribozyme is targeted against a site (E) in the HSV ICP4 gene having the sequence UGGCGUCGGAGAA. The six nucleotides at each end of the ribozyme are complementary to the 6 nucleotides on each side of the central C in this sequence.
Fig. 31B shows the HSV ICP4 site E ribozyme of Fig. 30 bound to a small RNA substrate containing the target sequence. All three stems (I, II, and III) of the hammerhead ribozyme are now formed.
Fig. 31C shows a folded structure for one possible sequence for a 5' flanking hairpin. This sequence is compatible with transcription from a T7 promoter as it begins with the preferred sequence GGGAG. Other possible sequences would include, but are not limited to, GGGCGAAAGCCC, GGAGGAAACUCC, and GGGACGAAAGUCCC. For efficient T7 transcription the occurrence of U nucleotides should be avoided in the first 9 nucleotides as U promotes undesirable abortive transcription.
Fig. 31D shows a folded structure of the Tø termination sequence from phage T7 (Dunn and Studier, 166 J. Mol. Biol. 477, 1983) as one possible 3'-flanking hairpin. Sequences 5' to the hairpin might be dispensable, in order to reduce excess unstructured RNA sequences, without reducing termination efficiency of the RNA polymerase.
Fig. 31E shows the structure of the ribozyme shown in Fig. 31A coupled with the 5' and 3' hairpins shown in Figs. 31C and 31D.

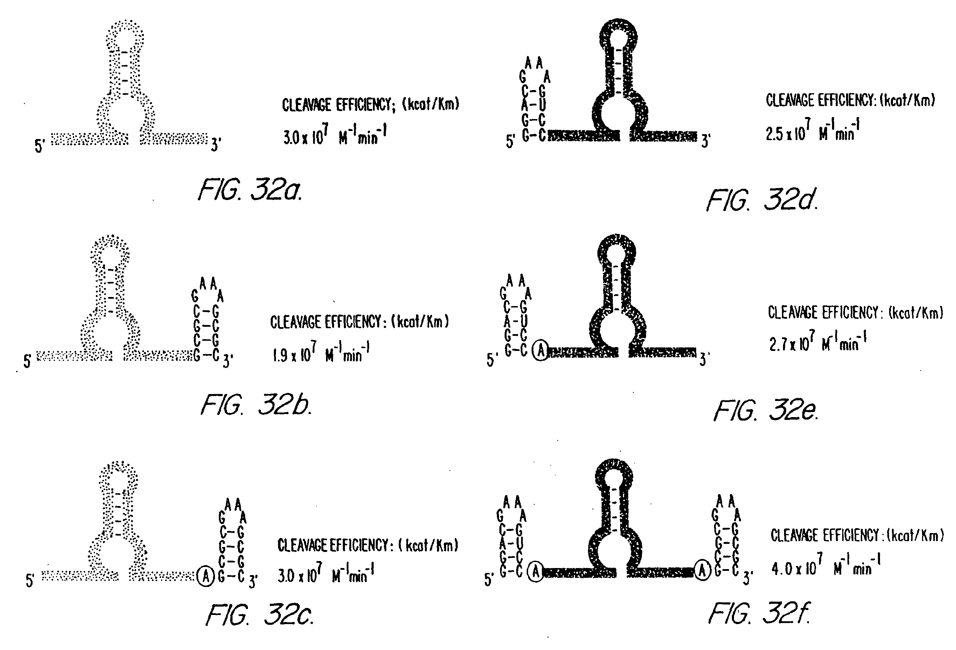

Figs. 32A-F are diagrammatic representations of a hammerhead having various hairpin sequence designs, with a summary of the cleavage activities obtained from said ribozymes.

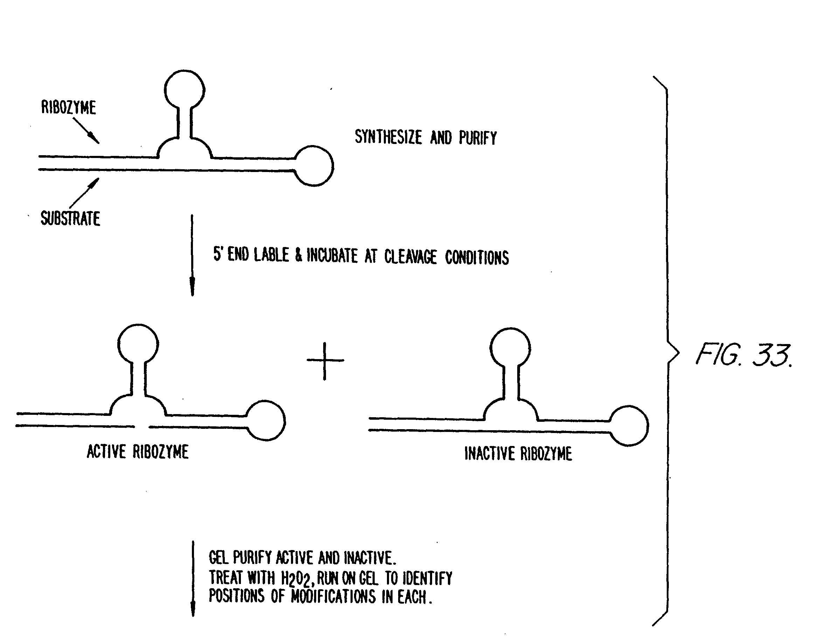

Fig. 33 is a diagrammatic representation showing a general method of this invention. Specifically, a ribozyme population is formed including analogues at various sites. This population is 5' end-labeled and incubated under cleavage conditions. The active and inactive ribozymes are gel purified by size, treated with an agent which allows determination of the site of the analog (e.g., H₂O₂), and then determination of those sites.

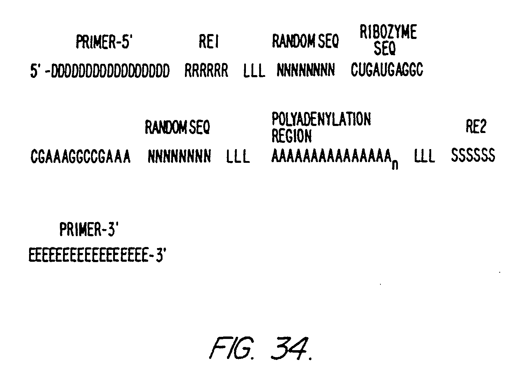

Fig. 34 is a diagrammatic representation of the ribozyme-coding strand of a DNA fragment prepared for insertion into an expression vector.

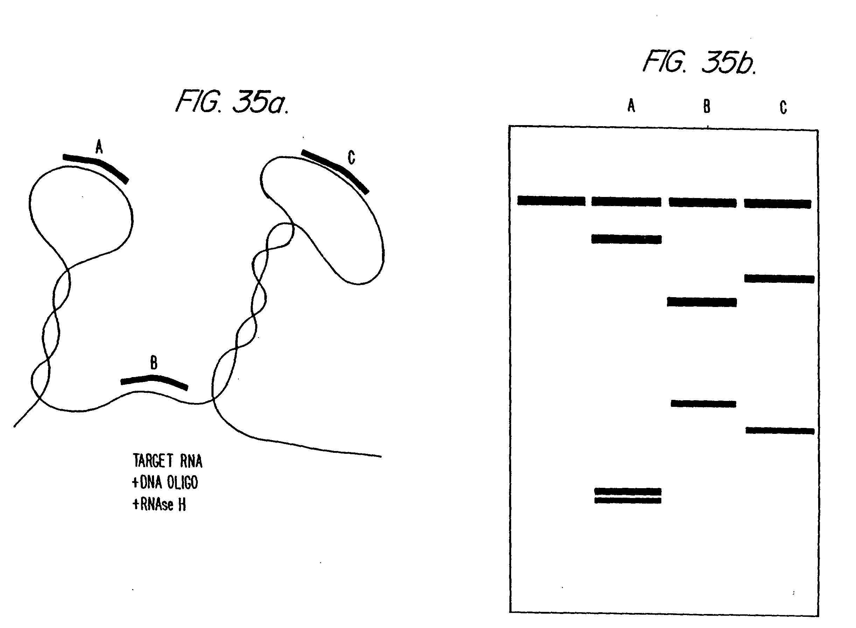

Figs. 35A and 35B are a schematic representation of an RNaseH accessibility assay. Specifically, Fig. 35A is a diagram of complementary DNA oligonucleotides bound to accessible sites on the target RNA. Complementary DNA oligonucleotides are represented by broad lines labeled A, B, and C. Target RNA is represented by the thin, twisted line. Fig. 35B is a schematic of a gel separation of uncut target RNA from a cleaved target RNA. Detection of target RNA is either by autoradiography of body-labeled target RNA, by northern blotting with labeled target cDNA or by RNase protection with labeled RNA. The bands in each lane represent uncut target RNA. The remaining bands in each lane represent the cleaved products.

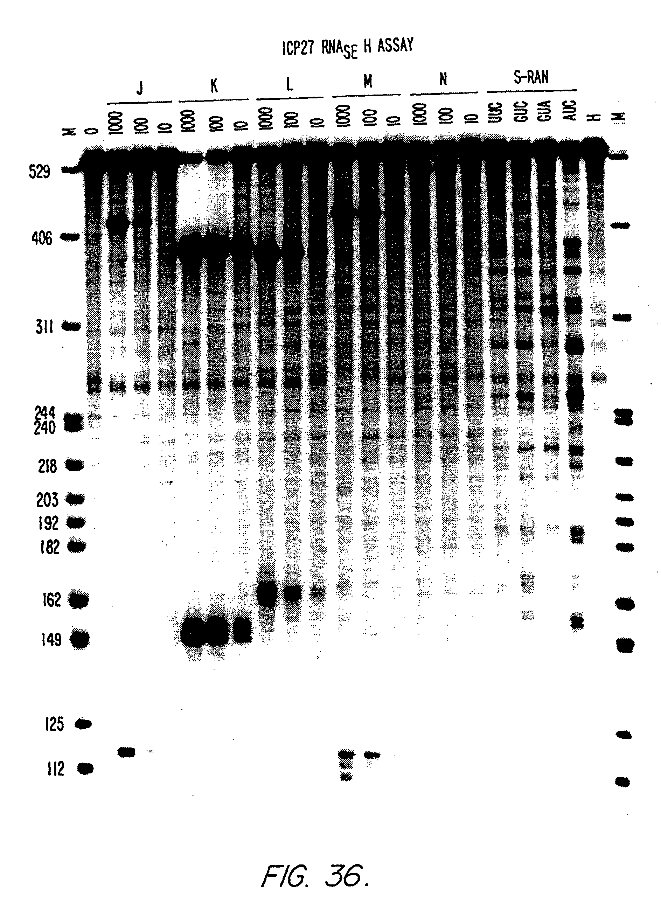

Fig. 36 is a copy of an autoradiogram of an RNaseH assay performed on *in vitro* transcribed target RNA using five probes to a portion of the HSV ICP27 mRNA. Probes labeled J, K, L, M, and N respectively represent five 13-mer DNA oligonucleotides complementary to five different sites on ICP27 (see Table 1). The end lanes labeled M are DNA size markers (DNA sizes are indicated at the left of the figure). Lanes labeled ⌀ and H represent target RNA immediately after transcription (⌀) or after incubation with RNaseH but without a DNA oligonucleotide (H). Lanes marked 1000, 100 and 10 represent digestion by RNaseH in the presence of 1000 nM, 100 nM and 10 nM respectively of DNA probe. Lanes marked S-RAN: UUC, GUC, GUA and AUC represent digestion of target RNA by RNaseH in the presence of 10 µM of the semi-random DNA oligonucleotides containing 12-nucleotides total with the middle three comprising sequences complementary to UUC, GUC, GUA and AUC, respectively. 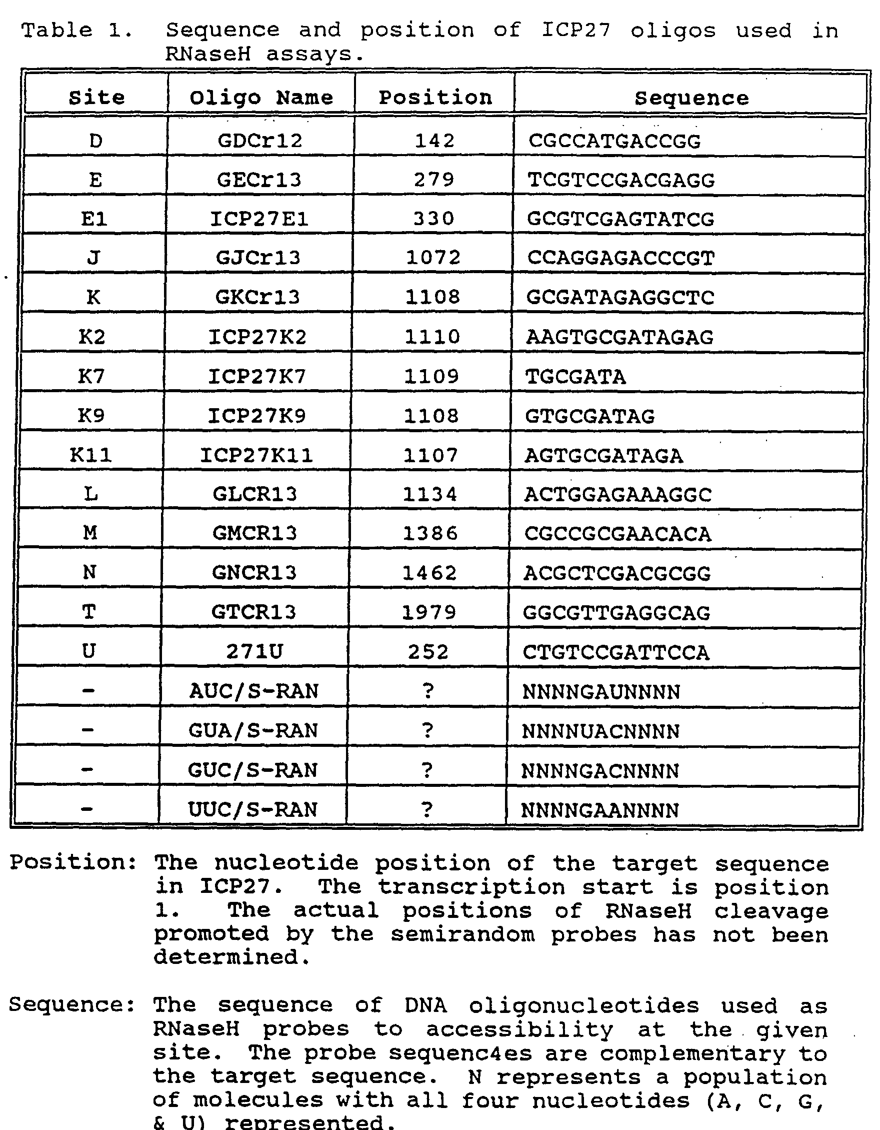

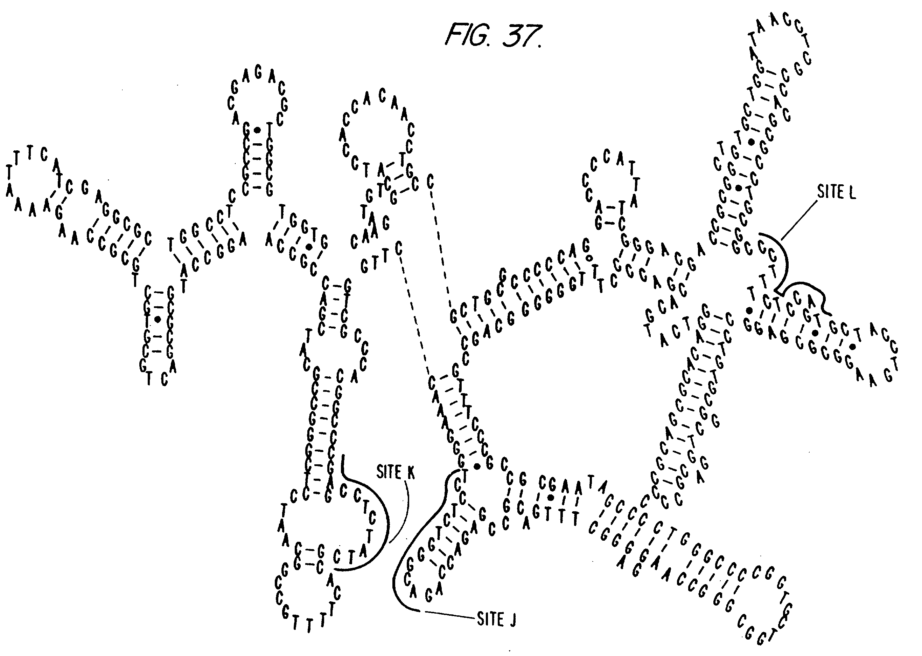

Fig. 37 is a computer generated folding pattern of the sequence of HSV ICP27 from nucleotides 952-1376. Three of the sites that were targeted for cleavage in Fig. 36 are indicated by broad lines and labels (sites J, K and L).

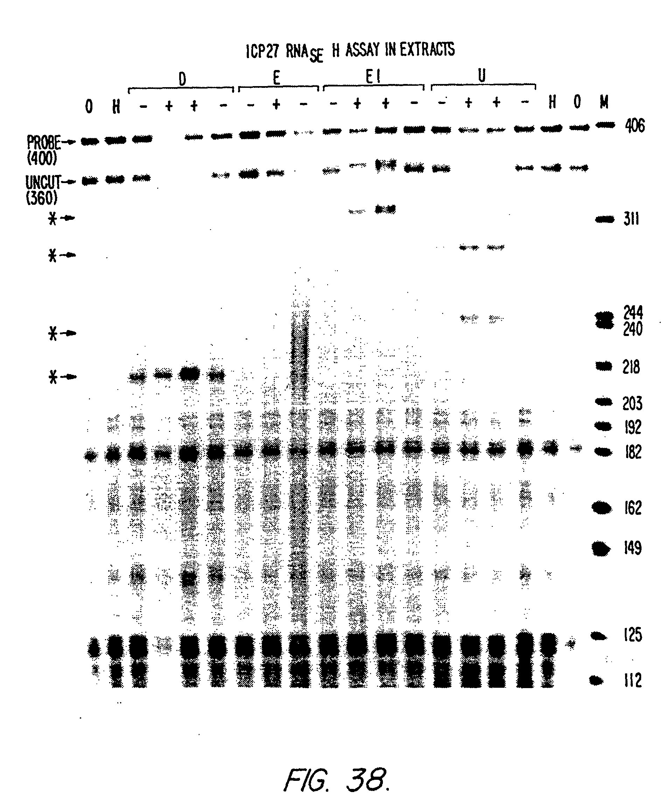

Fig. 38 is a copy of an autoradiogram of an RNaseH assay performed on HSV ICP27 mRNA contained in a cellular extract, using four DNA oligonucleotide probes. Target RNA was produced by infection of Vero cells with Herpes Simplex Virus (HSV). Cell extracts were separated into nuclear, cytoplasmic and membrane fractions. ICP27 RNA in the cytoplasmic fraction was probed for accessibility to RNaseH cleavage in the presence of 15 µM DNA oligonucleotides complementary to site D, E, E1 or U. Cleaved and full-length target RNA was detected by RNase protection assay using a 400 nucleotide antisense RNA. Full-length message was detected as a 360 nucleotide band, while cleaved message was detected as bands in the 200-330 nucleotide size range. Each site was probed twice without added RNaseH (labeled "-") and twice in the presence of RNaseH (labeled "+"). Cleavage products in the "-" lane for site D confirms the presence of endogenous RNaseH in the extracts.

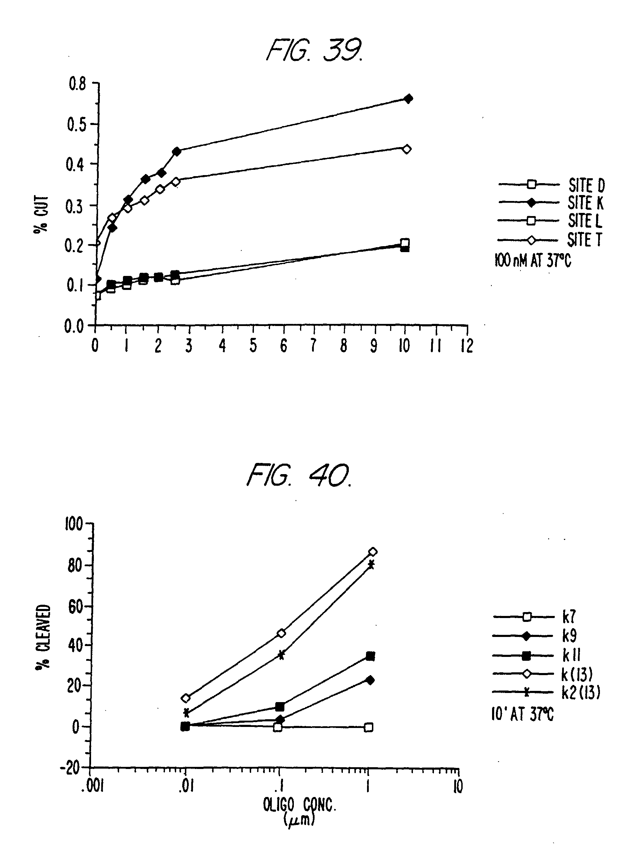

Fig. 39 is a graph showing quantitation of RNaseH cleavage rates at different sites on HSV ICP27. Sites K and T show efficient levels of cleavage at this DNA probe concentration; sites D and L show only low levels of cleavage.
Fig. 40 is a graph illustrating the effect of DNA probe length on RNaseH cleavage activity at an accessible site (K) in ICP27 target RNA. A plot of cleavage activity (% target RNA cleaved) as a function of DNA probe concentration for DNA probes of length 7, 9, 11 and 13 nucleotides is shown. The 7 nucleotide probe shows no activity even at 1 µm concentrations, suggesting that probe lengths greater than 7 nucleotides are preferred.

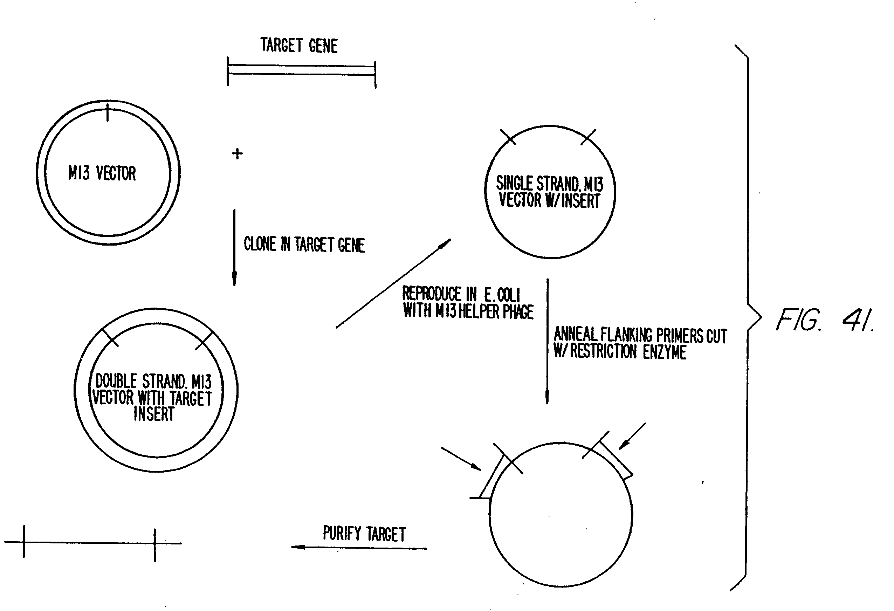

Fig. 41 is a diagrammatic representation of a method for producing target nucleic acid.

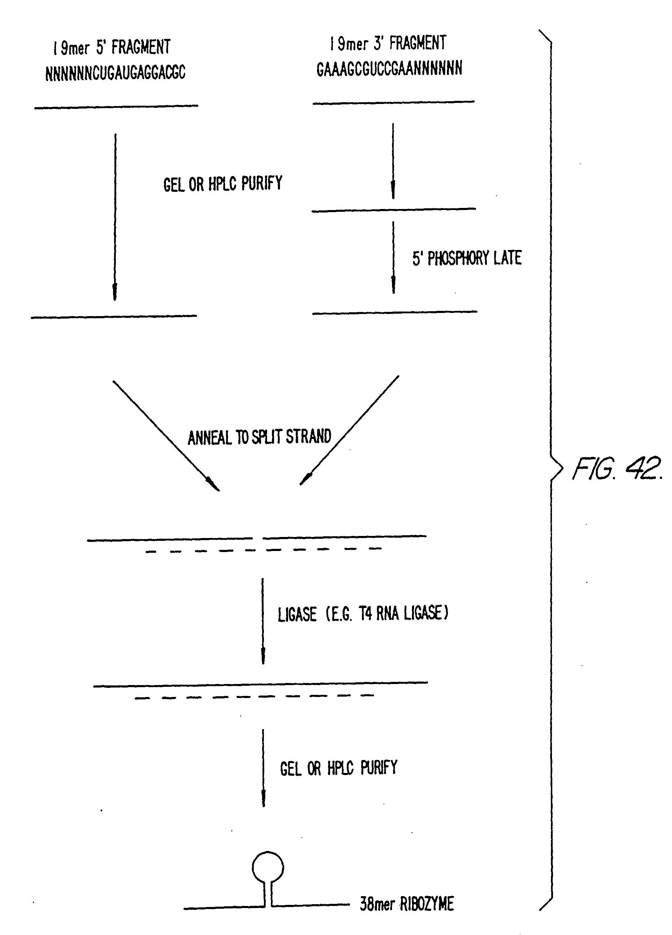

Fig. 42 is a schematic representation of a method of this invention; the dashed line represents a splint strand to aid joining and ligation of the two RNA molecules (solid lines).

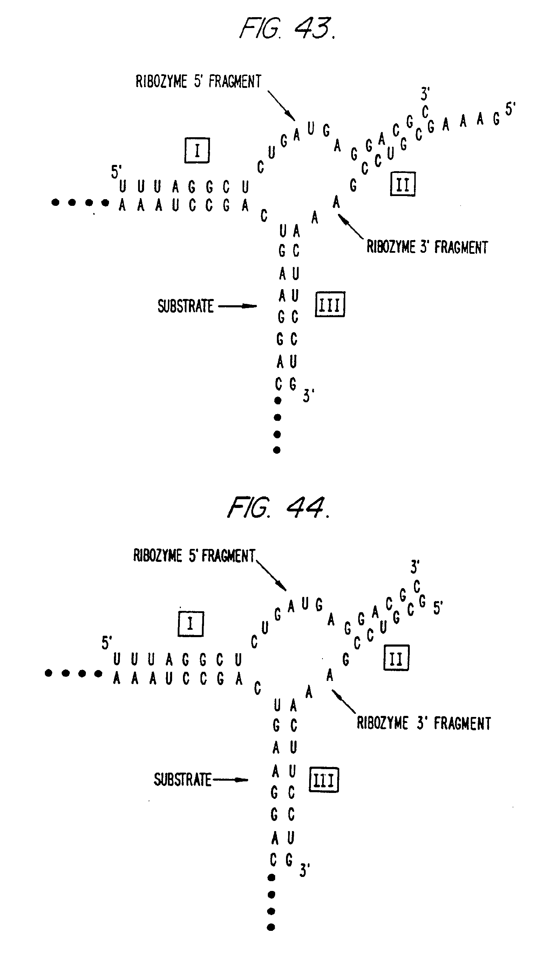

Figs. 43 and 44 are diagrammatic representations of ribozymes formed by a method of this invention showing the 5' and 3' fragments and the substrate.

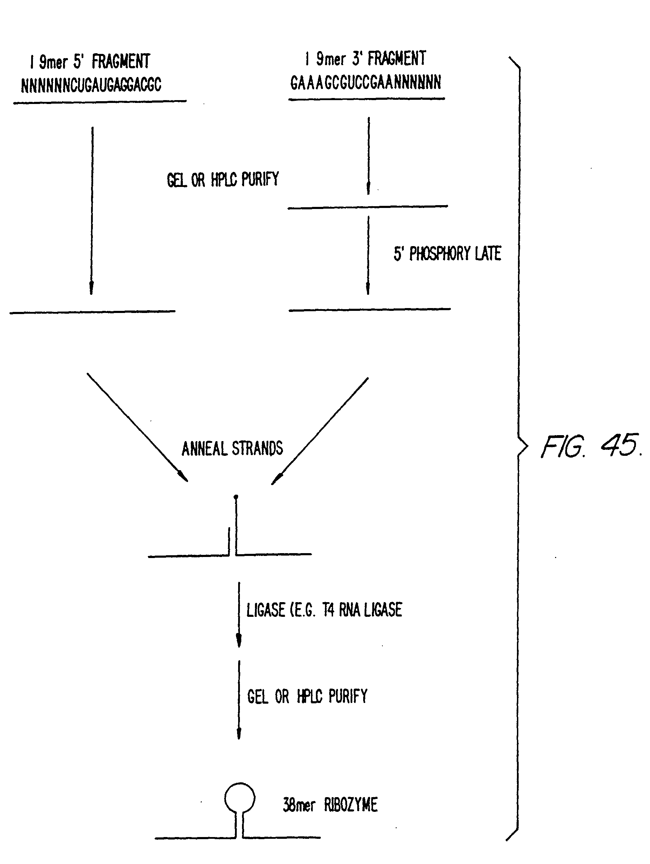

Fig. 45 is a schematic representation of an alternative method of this invention in which no splint strand is required for ligation but rather the two fragments hybridize together at stem II prior to ligation.

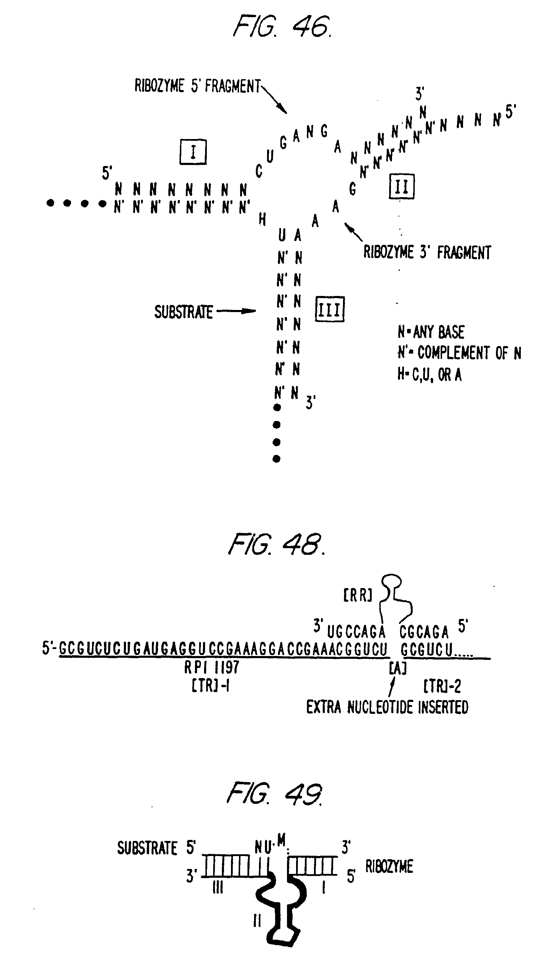

Fig. 46 is a diagrammatic representation of the preferred half ribozyme structure prior to ligation.

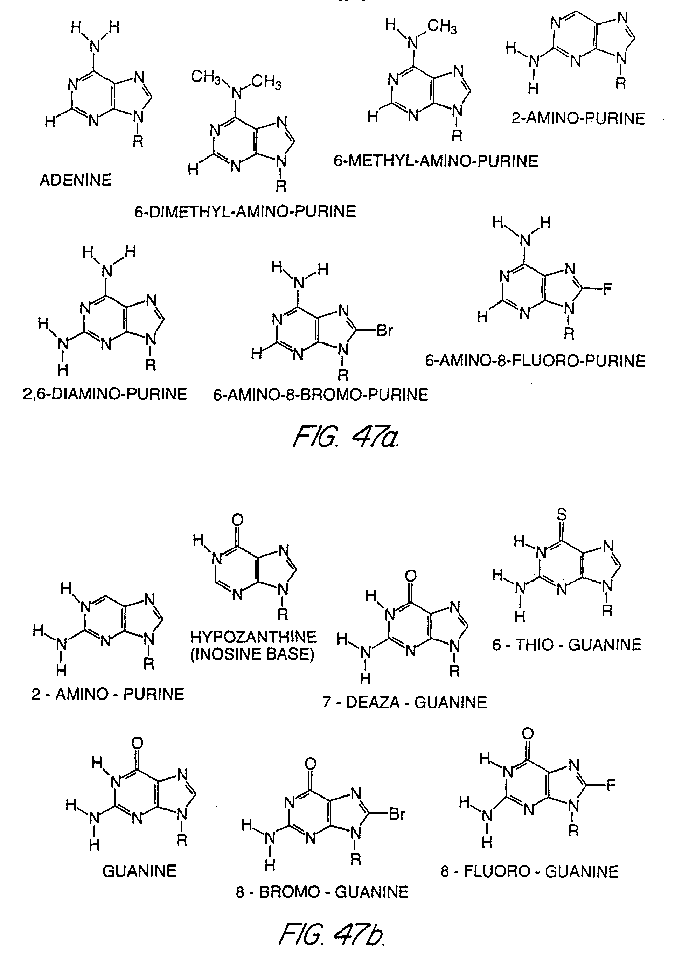

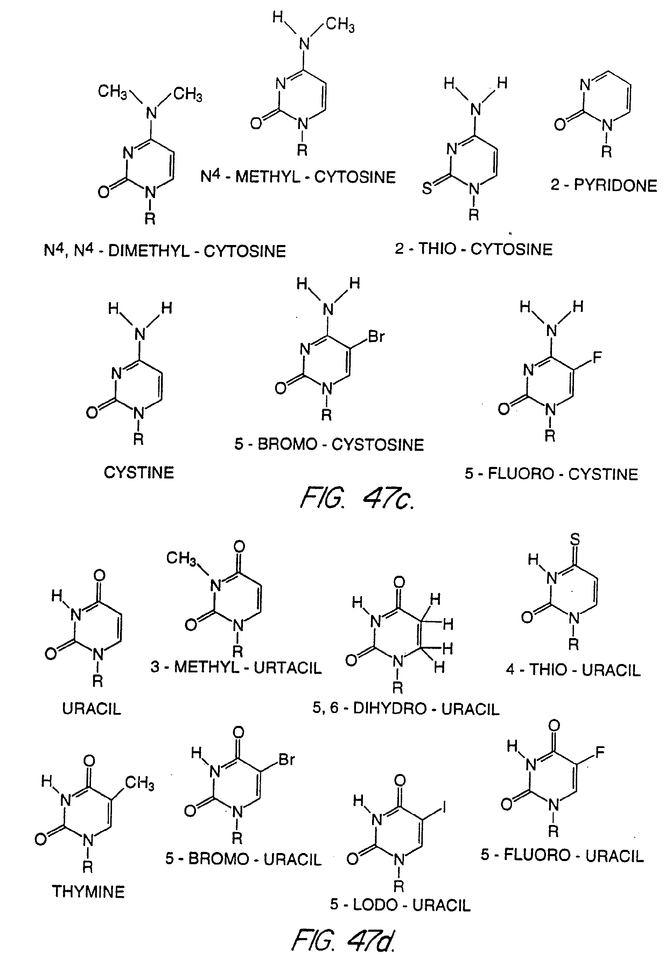

Figs. 47A-D are diagrammatic representations of standard base modifications for adenine, guanine, cytosine and uracil, respectively; modifications on the upper line of each figure represent structures with stronger base pairing ability compared to those on the lower lines.

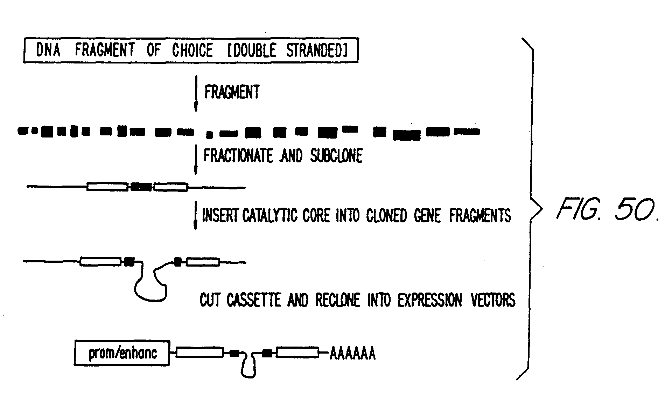

Fig. 48 is a diagram of the interaction between the releasing ribozyme (RR) and the therapeutic ribozyme (TR) cassette. (A) is an unpaired nucleotide between the TR concatamers which is required for effective RR cleaving activity. (TR)-1 and (TR)-2 are the first and second TR monomers released by RR cleavage.
Fig. 49 is a diagrammatic representation of hammerhead ribozyme:substrate complex. N = G, A or C. M = U, A, C. Vertical lines represent intramolecular paired nucleotides between ribozyme and substrate molecules.
Fig. 50 is a flow diagram for cloning of quasi-random ribozyme cassettes.

### Target Sites

Ribozymes targeting selected regions of a virus genome are preferably chosen to cleave the target nucleic acid in a manner which inhibits expression or translation of the nucleic acid. Genes are selected such that such inhibition of translation will occur or viral replication is inhibited, e.g., by inhibiting protein synthesis or genomic replication and packaging into virions. Such inhibition of translation will generally inhibit viral replication, e.g., by inhibiting protein synthesis. Selection of effective ribozymes to cleave target sites within these critical regions of viral nucleic acid entails testing the accessibility of the target nucleic acid to hybridization with various oligonucleotide probes. These studies can be performed using nucleic acid probes and assaying accessibility by cleaving the hybrid molecule with RNaseH (see below). Alternatively, such a study can use ribozyme probes designed from secondary structure predictions of the nucleic acids, and assaying cleavage products by polyacrylamide gel electrophoresis (PAGE), to detect the presence of cleaved and uncleaved molecules.

The following are examples of methods by which suitable target sites can be identified and are not limiting in this invention. Generally, the methods involve identifying potential cleavage sites for a hammerhead ribozyme, and then testing each of these sites to determine their suitability as targets by ensuring that secondary structure formation is minimal.

### Example 1: Picornavirus

Picornaviruses include those referred to as poliovirus, coxsackievirus, hepatitis A virus, human rhinovirus, ECHO virus, the cardioviruses, (e.g., mengovirus, encephalomyocarditis virus) and foot-and-mouth disease virus. These viruses cause many harmful diseases in humans and other animals. See for example, Pelletier and Sonenberg, 334 Nature 320, 1988.

The picornaviruses comprise one of the largest and most important families of human and agricultural pathogens. The family is currently divided into 4 genera: the enteroviruses, the cardioviruses, the rhinoviruses and the aphthoviruses (foot-and-mouth disease viruses). Sequence analysis has shown that these classifications may be too superficial. The rhinoviruses share many sequence similarities to the enteroviruses, while enterovirus 72 (hepatitis A virus) is markedly different and should be included in its own subgenus. Finally, the murine polioviruses (Thieler's viruses) which are classified as enteroviruses are more closely related to the cardioviruses.

The enteroviruses, so-called because they infect the alimentary tract, include three serotypes of poliovirus, 23 serotypes of coxsackievirus, 32 serotypes of echoviruses, hepatitis A virus and 37 serotypes of non-human enteric viruses. The human rhinoviruses are important etiologic agents of the cold and other nasopharyngeal infections. There are over 100 serotypes of rhinovirus. Aphthoviruses infect cloven-footed animals, especially cattle, goats, pigs, sheep, and rarely, even humans. Seven immune types of aphthoviruses have been isolated. The cardioviruses (Columbia SK virus, encephalomyocarditis [EMC] virus, ME virus, MM virus and mengovirus) all belong to a single serotype and are considered to be strains of EMC virus. These viruses are primarily murine, but will infect humans, pigs, elephants and squirrels. Some viruses such as the equine rhinovirus 1 and 2 and a collection of insect picornaviruses have not been assigned to genera. One of these, cricket paralysis virus cross-reacts serologically with EMC virus.

The picornaviral genome consists of a single strand of mRNA which can be extracted from the virions. Isolated RNA has a specific infectivity about one-millionth that of the intact virion. Two reasons for this drop in infectivity are: (i) the loss of the vial capsid eliminates receptor mediated uptake of the RNA and (ii) a single break in the RNA, whether free or inside the virus particle, is sufficient to destroy infectivity. Viral RNA isolated from the capsid is particularly susceptible to nicks and breaks.

The small protein VPg (virion protein, genome) is attached to the 5' terminal -pUpUp of the genomic RNA through a phosphodiester linkage to the phenolic hydroxyl group of a tyrosine residue. The length of VPg varies only slightly in different species of picornaviruses. VPg plays a important role in initiation of picornaviral RNA synthesis and possibly also in the packaging of the RNA into virions.

The 5' non-translated region (NTR) of the RNA is unusually long compared to the homologous region of cellular mRNAs; it ranges from 624 bases in HRV14 to 1200 in aphthovirus. Computer analyses predict the presence of stable stems and loops in the 5'-NTR and the folding patterns correlate with each virus group. For example, the first 600 bases of the rhinovirus 5' NTR align well with those of the enteroviruses but lack an additional 140 base loop which is found in the enteroviruses. The role of these structures in the control of internal initiation of translation has been shown for poliovirus and others.

Cardioviruses, aphthoviruses and Thieler's viruses each display distinctive folding patterns which probably play an important role in the translational control and/or tissue-trophic expression of those viruses. A single C to U base change in nucleotide 472 of the Sabin strain of poliovirus 3 results in increased neurovirulence.

The 3' NTR is relatively short, ranging from 47 bases in HRV14 to 126 for EMC virus. The function is unknown but an 8 base insertion in this region of poliovirus RNA produces a temperature sensitive phenotype. The poly(A) sequence at the 3' end of the RNA is heterogeneous in length and the mean length is genetically determined. The range for poly(A) length is from 36 bases in EMC virus to 100 in aphthovirus.

The RNA contains a single long ORF encoding a long polypeptide chain, called the polyprotein which is cleaved during translation. A cascade of cleavages, performed by three viral proteases, ultimately generates 11 viral protein products, 12 in the case of viruses encoding a leader (L) protein. Protease 3C, or its precursor 3CD, mediates most of the cleavages. Protein 3D is an enzyme capable of elongating nascent RNA chains from an RNA template. Protein 2C is also involved in RNA synthesis, perhaps in the initiation process. The 2C sequence is the most strongly conserved sequence in the polyprotein. Mutation analysis has shown that protein 2B and 2C are involved in RNA synthesis while protein 2A is involved in the shut-off of host protein synthesis.

Recombination takes place in RNA viruses, including the picornaviruses. It has been proposed that recombination occurs when viral RNA polymerase detaches from its original template and finishes synthesis of the nascent chain from a second template. Studies supporting this model suggest that strand switching occurs most frequently during synthesis of the minus strands. The error frequency of RNA synthesis is one substitution per genome while the rate of recombination may occur in as much as 10-20% of all viral RNA molecules. Thus, the recombination rate may partially correct for an unusually high error frequency during RNA synthesis.

In humans, poliovirus infects cells of the nasopharynx and gut although the paralytic form of poliovirus also has a high specificity for the anterior horn cells of the spinal cord. Coxsackieviruses also infect nasopharyngeal cells have a propensity for skeletal and heart muscle cells. The mechanisms underlying cell tropisms are not understood. The most plausible hypothesis for explaining cell tropism is that cells carry specific receptor molecules on their surfaces. For example, the ability of coxsackie A to produce myositis only in baby mice correlates with specific receptors present in differentiating myoblasts but not in other mouse tissues. The receptors for poliovirus, coxsackie B virus, echovirus and the major sero group of human rhinoviruses all map to chromosome 19 and those of poliovirus and rhinovirus have been identified as members of the immunoglobulin super-family of molecules. The major poliovirus receptor is a protein of 45 kd and the rhinovirus receptor has been identified as ICAM-1 (intercellular adhesion molecule-1).

Enteroviruses are associated with clinical manifestations which range from a mild febrile illness to permanent, sometimes fatal, paralysis. Among the infections associated with enteroviruses are: poliomyelitis, abortive poliomyelitis, meningitis, aseptic meningitis, paralytic poliomyelitis, epidemic myalgia, herpangina, hand-foot-and-mouth disease, various respiratory illnesses, eye disease, acute myocardiopathy, chronic cardiovascular disease, neonatal disease and fetal defects, gastrointestinal disease, hepatitis, post-viral fatigue syndrome and diabetes.

Treatment of picornaviral disease has concentrated on prophylactic vaccination, primarily with poliovirus. Vaccines are not available for the other picornaviruses due to an inability to target all of the multiple serotypes known to exist. Interferon treatment has been used with rhinovirus infections, but some people experienced more morbidity from the interferon treatment than from the rhinovirus infection. A number of candidate treatments for certain picornaviral diseases are currently in clinical trials. Most of these compounds work by binding the viral capsid and inhibiting the adsorption or penetration/uncoating of the viral particle. Other alternatives which do not depend upon the three dimensional structure of the viral capsid are preferable. Despite the availability of two effective polio vaccines, susceptible individuals still exist even in well-vaccinated populations. World-wide, there occur more than 200,000 cases of polio annually.

Many regions of the RNA associated with picornaviruses are appropriate targets for ribozyme attack. The untranslated regions of the genomic RNA, however, are of particular interest. The positive strand genomes of picornaviruses are uncapped and have untranslated regions with between 600 and 1300 bases. These regions contain extensive secondary structure which correlates well with the virus groupings in genera. Extensive regions of sequence homology exist within the 5'-NTR of the human picornaviruses. For example, see Table 2 which compares the RNA sequences of 8 human picornaviruses in a region known to influence initiation of translation and poliovirus virulence. Thus, this region is a good ribozyme target for all human picornaviruses. A ribozyme targeted to this region in these picornaviruses may be useful for targeting an equivalent region in related viruses whose sequences are unknown.

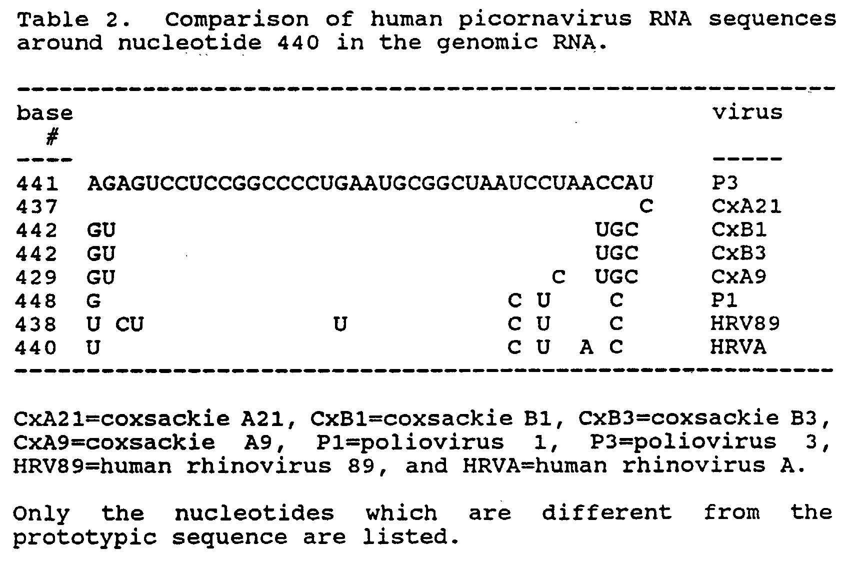

There is a high degree of nucleotide sequence homology within the secondary structures of the human and non-human picornavirus 5' non-translated regions. Particularly useful regions of homology for the cardioviruses is between nucleotides 450-834, and for the human viruses are regions between nucleotides 125-164, 236-538 and 564-828 of poliovirus, and their homologous positions in other genomes.

Preferred cleavage for picornaviruses is at regions required for viral replication (e.g., protein synthesis, such as a 5' non-translated region), the poliovirus 2A or 3C proteases, and the 3'-NTR and their equivalent in other viruses. Alternative regions may also be used as targets for ribozyme-mediated cleavage of these viral genomes. Each target can be chosen as described below, e.g., by a study of the secondary structure of the RNA, and the individual role of such RNA in the replication of the virus. If the targets are contained within the open reading frame of the polyprotein in regions which encode proteins essential to the replication of the virus, then these other targets are preferred candidates for cleavage by ribozymes, and subsequent inhibition of viral replication. The regions encoding the poliovirus 2A or 3C proteases and their homologous proteins in the other viruses are examples of such preferred targets.

The genome of picornaviruses may be subject to rapid genetic drift by virtue of its RNA content and the nature of errors in genomic replication. Those regions (genes) of the genome which are essential for virus replication, however, are expected to maintain a constant sequence (i.e., are conserved) over extensive periods of time. These regions are preferred target sites in this invention since they are more likely to be conserved between different types or strains of picornaviruses, and thus only one ribozyme is needed to destroy all such viruses. Thus, one ribozyme may be used to target all picornavirus. We have selected several such RNA regions of these viruses, and examined their nucleotide sequences for the presence of conserved areas which may be cleaved by ribozymes targeted to those regions. Three regions analyzed in detail are the 5' non-translated region, the 3' non-translated region and the 3A and 2C protease genes; other genes can be analyzed in a manner similar to that described below.

Predictions of effective target sites in the viral genes, based upon computer generated sequence comparisons, were obtained and identified as SEQ. ID. NOS. 1-10 in Fig. 6.

### Example 2: HIV

Acquired immunodeficiency syndrome (AIDS) is thought to be caused by infection with the virus HIV-1. At present, it is treated by administration of the drug azidothymidine (AZT), which is thought to slow the progress of, but not cure, the disease. AZT resistant strains of HIV-1 are found to develop after a year of treatment. In some patients AZT has limited efficacy and may be found intolerable. More recently, drugs such as dideoxyinosine (DDI) and dideoxycytidine (DDC) have been tested as treatments for AIDS. None of these compounds reduce the viral load in patients, but they do treat the disease symptoms.

Rossi et al., 8 Aids Research and Human Retroviruses 183, 1992, provide a review of the use of ribozymes as anti-HIV-1 therapeutic agents. They state:
An emerging strategy in the treatment of viral infections is the use of antisense DNA or RNA to pair with, and block expression of viral transcripts. RNA, in addition to being an informational molecule, can also possess enzymatic activity. Thus, by combining anti-sense and enzymatic functions into a single transcript, it is now possible to design catalytic RNAs, or ribozymes, which can specifically pair with virtually any viral RNA, and cleave the phosphodiester backbone at a specified location, thereby functionally inactivating the viral RNA. In carrying out this cleavage, the ribozyme is not itself altered, and is thus capable of recycling and cleaving other molecules, making it a true enzyme. There are several different catalytic motifs which possess enzymatic activity, and each one of these can be incorporated into an enzymatic antisense with site-specific cleavage capabilities.

Rossi et al. also state that studies have demonstrated that a hammerhead ribozyme targeted to the *gag* gene RNA in the vicinity of the translational initiation codon is capable of specifically cleaving that target in a complex milieux of total cellular RNA. With reference to identification of ribozyme targets in HIV-1 they state that mRNAs for the two regulatory proteins tat and rev are clearly targets of choice, and that they are examining potential ribozyme cleavage sites in the tat mRNA, as well as in the exon shared by *tat* and *rev.* In addition, they state:
A rational approach to the problem of target selection involves the following criteria. First, one should select a functionally important target, such as tat, rev, int, psi (packaging site), or the tRNA^{lys} priming site. Once a gene or target region has been decided upon, the nucleotide sequence should be assessed for strong conservation of sequence among the various isolates. Within these conserved regions, the potential cleavage sites, preferably GUC or GUA (others will suffice, but appear to be less efficiently cleaved) should be chosen. The region should be examined for potential secondary structures, and then the most promising sites chosen. Finally, before testing the ribozyme in cell culture, it is advisable to carry out a series of in vitro cleavage reactions (preferably kinetic analyses) using long (at least 100 nucleotides in length) substrates to verify that the chosen sites are truly structurally favorable for cleavage. [Citation omitted.]

Rossi et al. further state that a target which deserves further consideration and testing as a potential ribozyme cleavage site is the viral packaging signal or psi sequence.

Sioud and Drlica, 88 Proc. Natl. Acad. Sci. USA 7303, 1991, describe ribozymes designed to cleave the integrase gene of HIV. They state that when the ribozyme is transcribed from a plasmid in *E. coli* it leads to destruction of the integrase RNA and complete blockage of integrase protein synthesis. They state that the HIV-1 integrase gene may be a useful target for therapeutic ribozymes.

Heidenreich and Eckstein, 267 J. Biol. Chem. 1904, 1992, describe three ribozymes targeted to different sites on the long terminal repeat (LTR) RNA of HIV-1. They also describe the influence of chemical modifications within the ribozyme on the cleavage of the LTR RNA, including 2'-Fluorocytidine substitutions and phosphorothioate internucleotidic linkages.

Weerasinghe et al., 65 J. Virology 5531, 1991, describe ribozymes designed against a conserved region within the 5' leader sequence of HIV-1 RNA.

Chang et al., 2 Clinical Biotechnology 23, 1990, describe ribozymes designed to target two different sites in the HIV-1 *gag* gene, and a single site in the viral 5'-LTR region.

Lorentzen et al., 5 Virus Genes 17, 1991, describe a ribozyme targeted to the virion infectivity factor (*vif*) of HIV-1.

Sarver et al., 247 Science 1222, 1990, describe ribozymes in the hammerhead family targeted to HIV-1 *gag* transcripts. They state that cells challenged with HIV-1 showed a substantial reduction in the level of HIV-1 *gag* RNA relative to that in nonribozyme-expressing cells, and that the reduction in *gag* RNA was reflected by a reduction in antigen p24 levels. They state that the results suggest the feasibility of developing ribozymes as therapeutic agents against human pathogens such as HIV-1.

Hampel et al., "RNA Catalyst for Cleaving Specific RNA Sequences", filed September 20, 1989, which is a continuation-in-part of U.S. Serial No. 07/247,100, filed September 20, 1988, describe hairpin ribozymes, and provides an example of such a ribozyme apparently specific to the *gag* gene of HIV-1. Hampel and Tritz, 28 Biochemistry 4929, 1989 and Hampel, et al., 18 Nucleic Acids Research 299, 1990, also describe hairpin catalytic RNA models and state that one target site is the *tat* gene in HIV-1.

Goldberg et al., WO 91/04319, and Robertson and Goldberg, WO 91/04324, describe ribozyme expressed within a hepatitis delta vector and state that the genome of the delta virus may carry a ribozyme against the *env* or *gag* mRNA of HIV. Rossi et al., WO 91/03162, describe chimeric DNA-RNA catalytic sequences used to cleave HIV-1 *gag* transcript or the 5' LTR splice site.

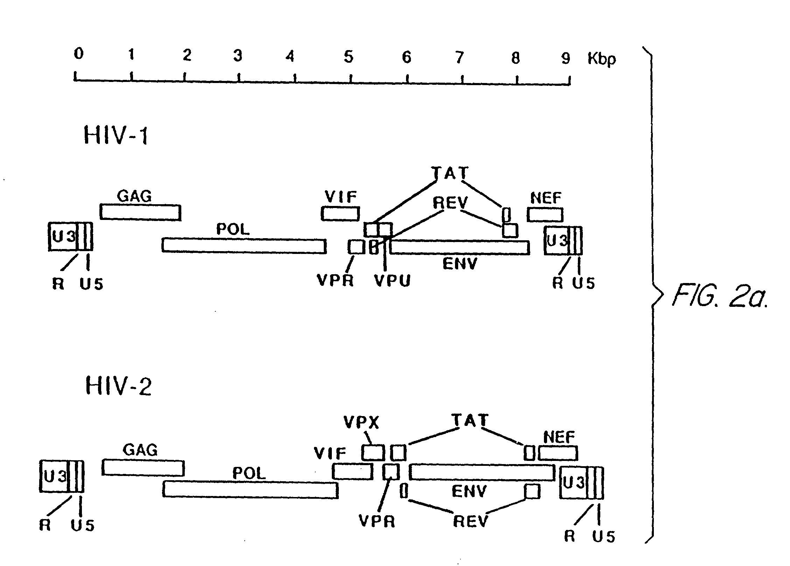

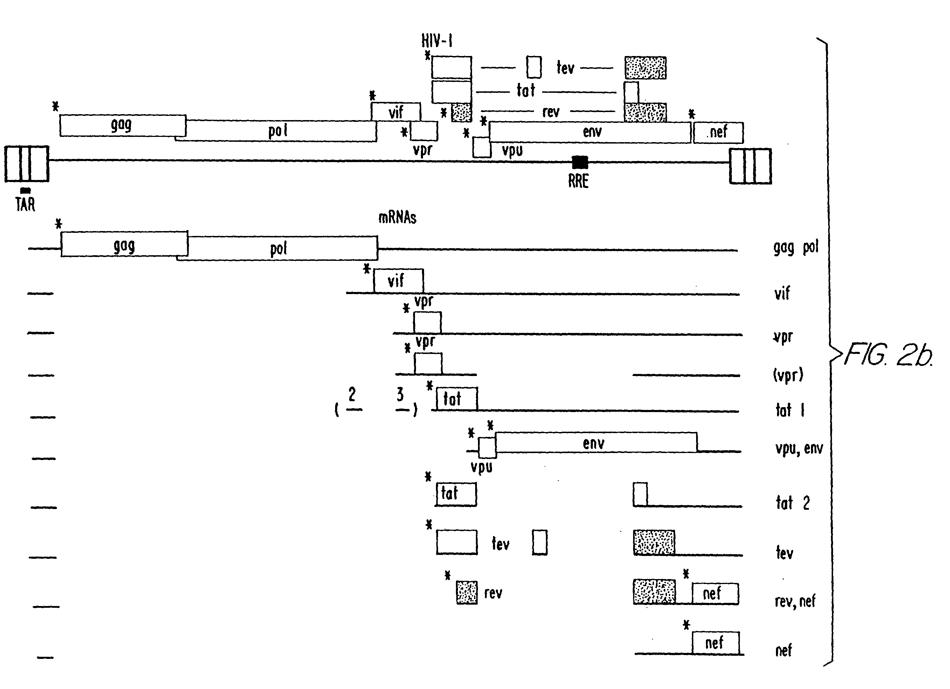

The invention features cleavage of the RNA of these viruses by use of ribozymes. In particular, the ribozyme molecules described are targeted to the *nef*, *vif, vpr* and *rev* viral genes. These genes are known in the art, see, e.g., Matsukura et al., 86 Proc. Natl. Acad. Sci. USA 4244, 1989, Cheng-Mayer et al., 246 Science 1629, 1989, Viscidi et al., 246 Science 1606, 1989, Malim et al., 86 Proc. Natl. Acad. Sci. USA 8222, 1989, Terwilliger et al., 88 Proc. Natl. Acad. Sci. USA 10971, 1991, and Bartel et al., 67 Cell 529, 1991 and Figs. 2A and 2B.

Preferred cleavage for HIV is at regions required for viral replication (e.g., protein synthesis), e.g., the *vif, nef, vpr* or *rev* gene regions, or at structures known to regulate viral *gene* expression, e.g., *tar, rre* or 3'-LTR regions.

As above, the genome of HIV-1 is subject to rapid genetic drift by virtue of its RNA content and the nature of errors in reverse transcription. Those regions (genes) of the genome which are essential for virus replication, however, are expected to maintain a constant sequence (i.e., are conserved) over extensive periods of time. These regions are preferred target sites in this invention since they are more likely to be conserved between different types or strains of immunodeficiency viruses, and thus only one ribozyme is needed to destroy all such viruses. Thus, one ribozyme may be used to target all HIV-1 virus, as well as all HIV-2, SIV and FIV viruses. We have selected several such genes of HIV-1, and examined their nucleotide sequences for the presence of conserved regions which may be cleaved by ribozymes targeted to those regions. The 2.5 kb subgenomic mRNA was analyzed in detail especially in the regions encoding the *vif* and *nef* proteins; the *tat*, *vpr, vpu* and *rev* proteins could also be synthesized from this mRNA. Nucleotide sequences were acquired from the Los Alamos HIV gene bank.

The HIV-1 genomic sequences of the Los Alamos data bank were compared in the regions encoding the *vif* and nsf genes. Fifteen putative ribozyme cleavage sites were found to be highly conserved between the 11 strains of virus sequence. These sites represent the preferable sites for hammerhead ribozyme cleavage within these two target RNAs. Two of the *nef* gene sites overlap regions within the 3'-LTR of the HIV-1 genome, which represents another target of potential therapeutic value. All of the *nef* targets are present in all known HIV-1 mRNAs and may represent targets for cleavage which would disrupt 3' terminal control regions of the mRNA which may be required required for efficient translation or export of the RNAs. In a In a similar manner, a number of the *vif* target sites are present in the *pol, tat* and *vpr* mRNAs (see Fig. 2).

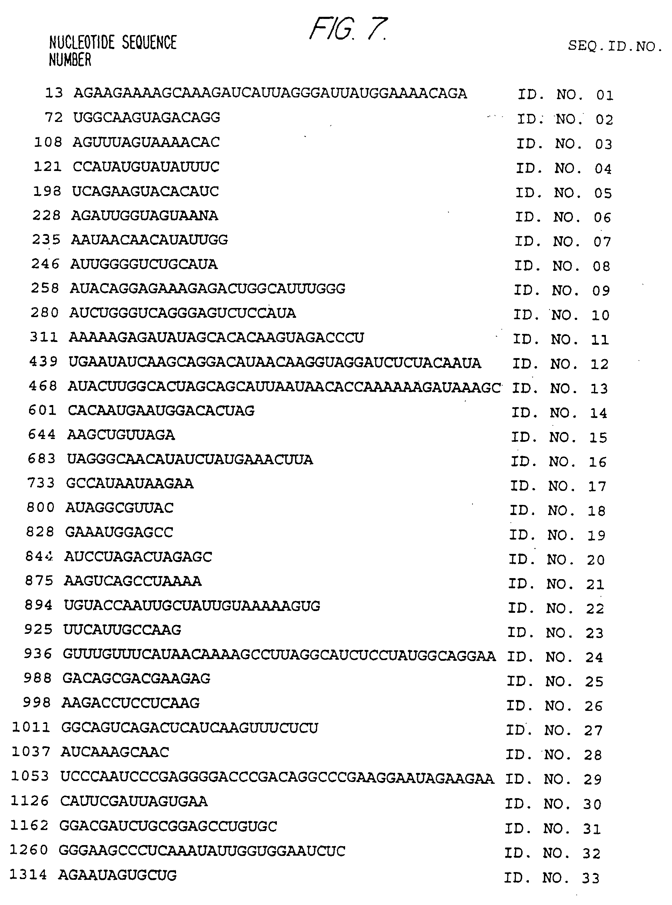

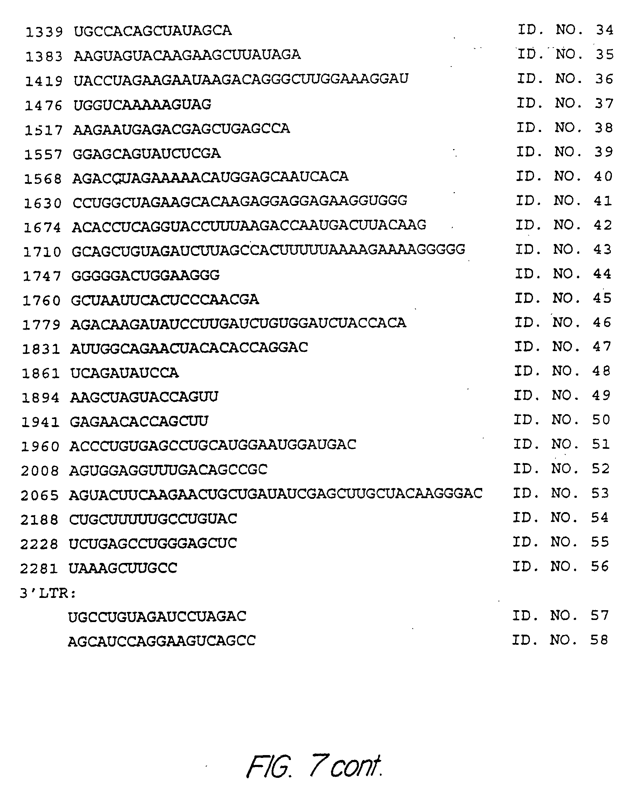

Using the comparative sequence analyses and an analysis of the predicted RNA structure across this region, the following predictions of effective target sites in the 2.5 kb subgenomic mRNA of HIV were obtained (and identified as SEQ. ID. NOS. 1-58 in Fig. 7). The HIVPCV12 sequence was used as a prototypic strain for analysis. The nucleotide numbers given in the table correspond to the 5' residue within the target sequence in in the 2.3 kb mRNA sequence of Arya and Gallo, 83 Proc. Natl. Natl. Acad. Sci. USA 2209, 1986.

### Example 3: HBV

The following is a discussion of relevant art, none of which is admitted to be prior art to the pending claims. Examples of art discussed below include Kato et al., 266 J. Biol. Chem. 22071, 1991; Price et al., 86 Proc. Natl. Acad. Sci. USA 8541, 1989; and Nassal et al., 63 Cell 1357, 1990.

Although acute hepatitis following perfusion exposure to human serum or blood was recognized more than 100 years ago, a causative agent was not identified until the 1960s when hepatitis B surface antigen (HBsAg) was discovered and subsequently shown to be a viral antigen. Many liver infections are asymptomatic, but the virus is hepatotrophic and may persist for years. Persistent infections are often associated with minimal liver damage, but the risk of cirrhosis or hepatocellular carcinoma in patients carrying HBV can be more than 200 times greater than in non-carriers.

The Hepatitis B virus (HBV) is the prototypic member of the family of viruses known as the hepadnaviridae. The 3.2 kb genome is characterized by extensively overlapping reading frames which express different proteins through the use of internal initiator codons (AUGs). Three major transcripts are synthesized from the HBV genome, but the major pregenomic mRNA (3200 nucleotides in length) is probably the template for DNA synthesis and the translation of the C and P gene products. The transcript from which the X gene is translated is unclear. In principle, any of the three transcripts can function as a polycistronic mRNA and be utilized for the translation of proteins, including the X protein. Although all RNAs contain an 85 nucleotide encapsidation signal near their 5' ends, only the shortest mRNA is encapsidated into core viral particles and used for reverse transcription into viral genomes. Recently, it was demonstrated that the progression of the 80s ribosome through the region containing the encapsidation signal would preclude the functioning of the signal and prevent the encapsidation of an RNA into the core particles. Thus, disruption of this signal and the prevention of ribosomal progression could be utilized to alter protein expression and viral maturation.

Virions of HBV are spherical particles which are surrounded by a lipid envelope and contain the small circular DNA genome of approximately 3200 nucleotides. The virion also contains a DNA polymerase (reverse transcriptase) activity and the viral core protein (HBcAg). The HBsAg protein is found in the viral envelope and may exist as three different species. The antigenic complexity of the HBsAg is much greater than expected from the three species synthesized. Greater than eight subtypes of HBsAg can be found in clinical isolates. The P gene (polymerase) shares sequence homology with sequences in many retrovirus *pol* genes. The X gene encodes a polypeptide of unknown function during viral replication, but which can serve as a transactivator of transcription, and thus is a candidate oncoprotein in the morphogenetic transformation of hepatocytes infected with HBV.

Much of the hepatocellular injury which is associated with mild cases of hepatitis is thought to be caused by immunologic defenses, particularly cytotoxic T-cell responses. It has additionally been shown that chronic carriers have impaired ability to induce interferon protection in infected cells. The role of HBV in progression to hepatocellular carcinoma (HCC) is supported epidemiologically, but no gene product has been demonstrated to be essential. In a number of cases the X gene has been found in an integrated form, often with deletions, but the duck HBV contains no X gene and thus the role of X in duck HCC is not supported.

For some disease syndromes there is evidence that HBsAg-anti-HBs complexes play a role in the pathogenesis, and usually these extrahepatic manifestations occur in the form of skin rash, glomerulitis, arthritis, or necrotizing vasculitis. One third of the patients with biopsy proven polyarteritis nodosa have persistent HBV infection. A fraction of the membranous glomerulonephritis cases are associated with chronic acute HBV and persistent HBV infections. Immune complex deposits can be found along the subepithelial surfaces of the glomerular basement membrane. Pathogenesis appears to be tissue damage mediated by immune complexes.

In general, treatment of chronic hepatitis B has been relatively unsuccessful. Corticosteroids and adenine arabinoside have not been beneficial in the treatment of chronic hepatitis B although some patients do respond to treatment with steroids. After cessation of treatment, these patients show a rebound in serum aminotransferase levels due to an immune response to the increased levels of Hepatitis B which accumulate in the blood during steroid treatment.

Interferons have been used to treat chronic Hepatitis B. Alpha-interferon appears to be more effective than beta- or gamma-interferons although its effectiveness as a single drug has yet to be determined.

Although vaccines for HBV exist, attempts to recruit individuals at risk and institute pre-exposure prophylaxis have been unsuccessful. Adequate control of the disease will require vaccination of newborn infants with a subunit or killed virus vaccine. Approval of such a vaccination regimen is distant. Post-exposure prophylaxis using vaccine or vaccine and Hepatitis B immunoglobulin is sufficient to prevent HBV infection following percutaneous inoculation, oral ingestion or direct mucous membrane contact with HBsAG positive material. The timing of the initial dose of immune globulin is crucial to the success of the treatment. There is no precedent for recommending immunoprophylaxis if exposure to HBV has occurred more than 7 days earlier. Thus a need exists for effective treatment of this disease which utilizes antiviral inhibitors which work by mechanisms other than those currently utilized in the treatment of both acute and chronic hepatitis B infections.

Ribozymes of this invention exhibit a high degree of specificity for only the viral mRNA in infected cells. Ribozyme molecules targeted to highly conserved sequence regions allow the treatment of many strains of human HBV with a single compound. No treatment presently exists which specifically attacks expression of the viral gene(s) which is responsible for transformation of hepatocytes by HBV.

The methods of this invention can be used to treat human hepatitis B virus infections, which include productive virus infection, latent or persistent virus infection and HBV-induced hepatocyte transformation. The utility can be extended to other species of HBV which infect non-human animals where such infections are of veterinary importance.

Preferred cleavage sites are genes required for viral replication, e.g., protein synthesis, such as the 5' most 1500 nucleotides of the HBV pregenomic mRNAs. For sequence references, see Renbao et al., 30 Sci. Sin. 507, 1987. This region controls the translational expression of the core protein (C), X protein (X) and DNA polymerase (P) genes and plays a role in the replication of the viral DNA by serving as a template for reverse transcriptase. Disruption of this region in the RNA results in deficient protein synthesis as well as incomplete DNA synthesis (and inhibition of transcription from the defective genomes). Cleavage 5' of the encapsidation site will result in the inclusion of the cleaved 3' RNA within the core virion structure and cleavage 3' of the encapsidation site will result in the reduction in protein expression from both the 3' and 5' fragments.

Alternative regions outside of the 5' most 1500 nucleotides of the pregenomic mRNA also make suitable targets of ribozyme-mediated inhibition of HBV replication. Such targets include the mRNA regions which encode the viral S gene. Selection of particular target regions will depend upon the secondary structure of the pregenomic mRNA. Targets in the minor mRNAs can also be used, especially when folding or accessibility assays in these other RNAs reveal additional target sequences which are unavailable in the pregenomic mRNA species.

As with other viruses, the genome of HBV may be subject to rapid genetic drift by virtue of its RNA content and the nature of errors in reverse transcription. Those regions (genes) of the genome which are essential for virus replication, however, are expected to maintain a constant sequence (i.e., are conserved) over extensive periods of time. These regions are preferred target sites in this invention since they are more likely to be conserved between different types or strains of HBV viruses, and thus only one ribozyme is needed to destroy all such viruses. Thus, one ribozyme may be used to target all HBV viruses. We have selected several such genes of these viruses, and examined their nucleotide sequences for the presence of regions which may be cleaved by ribozymes targeted to those regions. One region analyzed in detail is the 5' 1500 base region; other genes can be analyzed in a manner similar to that described below.

The mRNA sequences of the virus are folded using RNAfold computer analyses. The regions of the mRNA, in this case the 5' most 1500 nucleotides of the HBV pregenomic mRNA, which are predicted to have weak or non-base paired nucleotides are searched for putative ribozyme recognition motifs. These sites represent the preferred sites for hammerhead or other ribozyme cleavage within these target RNAs.

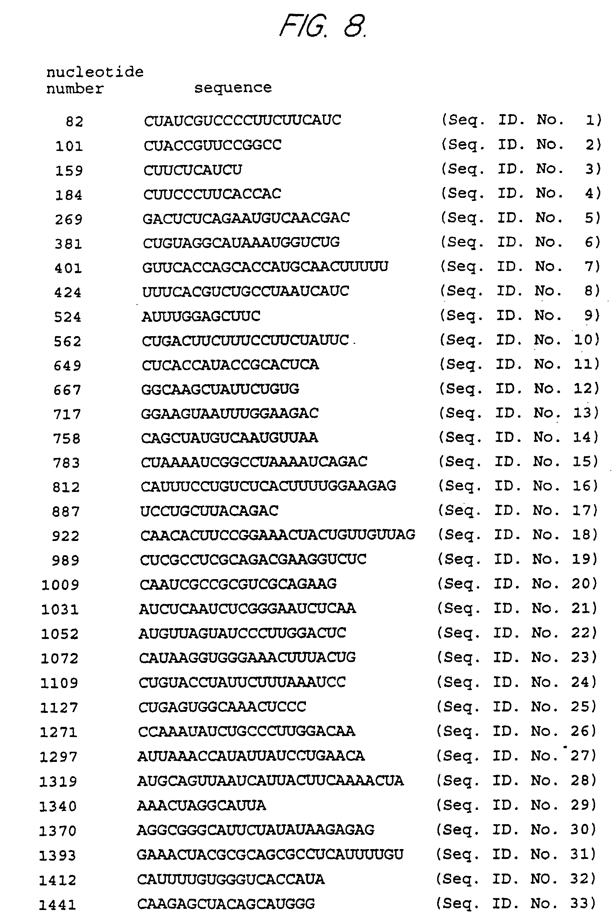

Using such analyses, predictions of effective target sites in the genes, based upon computer generated RNA structure analysis, were obtained (see SEQ. ID. NOS. 1-33 in Fig. 8). The target nucleotides are noted as the pregenomic RNA nucleotide numbers (in the HBV sub-type adr-1 pregenomic mRNA) given to the left of the sequence. Flanking nucleotides are given for reference. These regions are selected from genes encoding polymerase, core protein, and X protein.

### Example 4: Papilloma

Examples of art discussed below include Scheffner et al., 63 Cell 1129, 1990, Hausen, 184 Virology 9, 1991, Jones et al., 267 J. Biol. Chem. 908, 1992, McCance et al., 85 Proc. Natl. Acad. Sci. USA 7169, 1988, Storey et al., 19 Nucleic Acids Research 4109, 1991, Greenfield et al., 88 Proc. Natl. Acad. Sci. USA 11217, 1991.

The papillomaviruses are small viruses containing double-stranded DNA genomes, whose gene expression has been etiologically implicated in some malignant tumors. The link between papillomavirus replication and some benign squamous epithelial cell tumors was the first recognized example of virally induced cellular transformation. There are over 60 different human papillomaviruses associated with a variety of clinical lesions and over 20 of these have been closely linked to lesions of the anogenital tract. Some of these viruses are associated only with benign proliferative lesions (e.g., HPV-6 and HPV-11) while others are considered to be associated with a high risk of progression from preneoplastic lesions to carcinomas of the anogenital region (e.g., HPV-16, HPV-18, HPV-31, HPV-33, HPV-35).

The papillomaviruses are highly species specific and have a specific tropism for squamous cells. Viral replication is restricted to the most terminally differentiated keratinocytes of the epithelium, although viral DNA can be found within basal and parabasal cells of a papilloma. Viral transcripts have been found in the basal cells of the epidermis, but late gene expression occurs only in the terminally differentiated cells. Thus, viral replication is latent in basal cells and vegetative (productive) in the more terminally differentiated cells within the upper layers of the epithelium.

Papillomaviruses encode trans-acting factors that modulate viral transcription. The first of these factors to be described was the E2 protein. The E2 ORF contains domains that are relatively well-conserved among the papillomaviruses. E2 proteins are DNA-binding proteins which consist of two highly conserved domains joined by a more degenerate central region which is considered to be a linker region. Limited homology of E2 protein with the cellular oncogene c-mos has been noted. The E7 genes of HPV-16 and HPV-18 and the E6 gene of HPV-18 have been reported to exhibit transactivational properties.

The E5 ORF of bovine papillomavirus (BPV) encodes a 44 amino acid protein which is composed of two domains: (i) a strongly hydrophobic domain at its amino terminus and (ii) a hydrophilic carboxy-terminal domain that has the capacity to induce cellular DNA replication in quiescent cells. The E5 ORF is highly conserved among the papillomaviruses which induce fibropapillomas and has structural analogues in many other papillomaviruses, including HPV-16.

Expression of the HPV E6 and E7 genes is key to the development of neoplastic lesions. The E6 protein binds to the cellular p53 protein, which is a native tumor suppressor, and causes the degradation of the p53 protein. This degradation results in the loss of tumor suppression which is mediated by p53. Elimination of E6 expression may result not only in loss of viral replicative ability, but also in the maintenance or renewal of the normal cellular inhibition of tumor induction in premalignant cells.

Expression of the HPV E7 protein results in the binding and methylation of the retinoblastoma growth suppressor protein (RB). Because the RB protein is also implicated in the control or regulation of cellular growth, this interaction may be responsible for some aspect of the transformation in HPV infected epithelial cells. As was the case above with the E7/p53 interaction, reduction of E7 expression will inhibit an important function of HPV gene expression and result in increased levels of RB protein control of cell growth. It is currently believed that the expression of E6 protein is a prerequisite for E7 expression. The expression of both E6 and E7 may be required for the two step changes required to transform a normal epithelial cell to a cell which is capable of causing metastasizing tumors after the introduction into appropriate animal systems.

The development of a model system for the study of HPV-induced neoplasia has been hampered by the absence of both a permissive tissue culture system for viral replication and an easy method for growing normal stratified epithelium *in vitro.* The implantation of HPV-infected tissue under the renal capsules of nude mice results in features of HPV infection *in vivo,* but the technique is not amenable to the genetic manipulation of the virus. Most *in vitro* transformation studies have been done in rodent cells and do not accurately mimic the transformation process in differentiating epithelial cells.

An *in vitro* system has been developed which allows stratification and production of differentiation-specific keratins. Kopan et al., 105 J. Cell Biol. 427, 1987. This system uses human epithelial cells containing transfected copies of HPV-16 DNA and the cells retain the ability to stratify and express differentiation-specific keratins but lose their ability to differentiate morphologically. The histopathological lesions produced by transfection of HPV DNA in these keratinocytes is typical of intraepithelial neoplasia.

For the most part warts are benign, self-limiting proliferative lesions. All warts consist of localized epithelial hyperplasia with a well-defined boundary and an intact basement membrane. All of the normal layers of the skin are represented in a wart. Since only the basal layers of the epithelium have the potential for multiplication, a wart must result from the infection, transformation and multiplication of a basal cell. Studies of such clonal expansion in genital warts have suggested other scenarios. Infection probably occurs as a result of exposure of basal cells to infectious virus particles after mild trauma to the epithelium (e.g., trauma such as that which occurs during sexual intercourse, skin abrasions or passage through the birth canal). The virus stimulates the proliferation of basal cells, initiating the formation of a wart.

The HPVs fall naturally into two classes, cutaneous HPVs (about 33 types) and mucosal HPVs (about 25 types). About 17 of the cutaneous types have been recovered from patients suffering from epidermodsplasia verruciformis (EV). The genital tract is the main reservoir for the mucosal HPVs, although HPV-6 and HPV-11 will infect the respiratory tract, the oral cavity and the conjunctiva. HPV-13 and HPV-32 exclusively infect the oral cavity. The clinical significance of warts is determined primarily by their location, the viral type and host factors such as immune competence. Skin warts are often transient and self-limiting. A wart on the vocal cords can be life-threatening.

There is no "one-time" treatment that will cure all warts. Effectiveness of therapy is difficult to assess because on the one hand spontaneous regression can be observed with recurrence on the other hand. The various kinds of therapy include application of caustic agents (e.g., podophyllin), cryotherapy, use of DNA replication inhibitors (e.g., 5-fluorouracil) and surgical therapy. Immunotherapy has been unsuccessful. Interferon treatment has given mixed results depending upon the type of wart and the immune status of the patient. There is no immediate hope for effective immunization. Elimination of the latent viral state is not possible using current non-invasive therapies.

These ribozymes exhibit a high degree of specificity for only the virally encoded mRNA in infected cells. Ribozyme molecules targeted to highly conserved sequence regions will allow the treatment of many species of human papilloma virus with a single compound. There is no acceptable treatment which will give a broad spectrum of activity with no toxic side effects. No treatment exists which specifically attacks viral gene expression which is responsible for the transformation of epithelial cells by HPV, for the maintenance of the episomal genome in latently infected cells or for the vegetative replication of the virus in permissive cells.

The methods of this invention can be used to treat human papilloma virus infections, which include warts and epithelial cell transformation. The utility can be extended to other papilloma viruses which infect non-human primates where such infections are of veterinary importance.

Preferred cleavage sites are at genes required for viral replication, e.g., protein synthesis, such as in the HPV 16, E6 and E7 mRNAs.

Alternative regions outside of the E6 and E7 genes make suitable targets of ribozyme-mediated inhibition of HPV replication. Such targets include the viral E2 transactivator gene and the E5 gene which has been shown to be important in some animal virus systems.

As above, those regions (genes) of the genome which are essential for papilloma virus replication are expected to maintain a constant sequence (i.e., are conserved) over extensive periods of time. These regions are preferred target sites in this invention since they are more likely to be conserved between different types or strains of papilloma viruses, and thus only one ribozyme is needed to destroy all viral RNA. Thus, one ribozyme may be used to target all papilloma virus encoded RNA. We have selected several such genes of these viruses, and analyzed the structure of the encoded mRNAs using RNAfold computer analyses. We have identified open structures in the RNAs which may be cleaved by ribozymes targeted to those regions. Two regions analyzed in detail are the HPV-16, E6 and E7 mRNAs; other genes can be analyzed in a manner similar to that described below.

The mRNA sequences of the viruses are analyzed throughout the regions discussed above. Putative ribozyme cleavage sites are found to be weak or non-base paired regions of the virus mRNA. These sites represent the preferred sites for hammerhead or other ribozyme cleavage within these target RNAs.

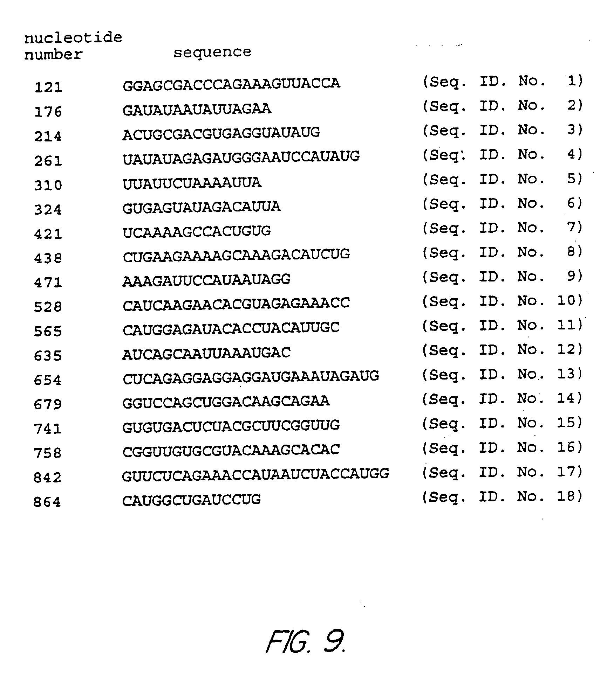

Using such analyses, predictions of effective target sites in the viral mRNAs, based upon computer generated sequence comparisons, were obtained (see SEQ. ID. NOS. 1-18 in Fig. 9). The target nucleotide is listed with the genomic nucleotide number (in the HPV-16 genome) noted as base number. Flanking nucleotides are given for reference.

### Example 5: EBV

Epstein-Barr virus belongs to the subfamily gammaherpesvirinae. These viruses are distinguished by their *in vitro* host range restriction to B-lymphocytes, their ability to establish latent infections, and their propensity to oncogenicity. Relatives of EBV can be found only in Old World primates. The details of gene expression in the other lymphocryptoviruses is not well understood although some proteins of these other species are cross-reactive with EBV-specific antibodies. At least two EBV types have been identified in most human populations. These are called EBV-1 and EBV-2 to parallel the nomenclature of HSV-1 and HSV-2, although the EBV types are more closely related in terms of sequence homology and restriction enzyme profiles.

EBV usually initiates infection in the oropharyngeal epithelium, which is permissive for viral replication. A persistent active infection ensues which continues for many years. Early in the course of the primary infection EBV infects B-lymphocytes, which pass close to the epithelial basement membrane of the oropharynx. EBV does not replicate in B lymphocytes but sets up a latent infection. Events associated with the establishment of this latent infection transform the lymphocytes and prevent the normal course of cell death or apoptosis. It is the study of these latently infected B lymphocyte populations which has provided much of the *in vitro* experimentation into understanding the molecular bases of EBV gene expression.

At least nine EBV genes are expressed during the latent infection. Two encode small, non-polyadenylated RNAs (EBER-1 and EBER-2). Six genes encode nuclear proteins (EBNA-1, -2, -3A, -3B, -3C or -LP). One encodes an integral membrane protein (LMP-1) which is known to interact with the cellular gene bcl-2 in a way which leads to the immortalization of cells. While the EBNA and LMP genes are transcribed by cellular polymerase II, the EBER genes are transcribed by cellular polymerase III even though the EBER promoter regions also contain elements known to be recognized by the polymerase II molecules. The EBNA-3 and EBNA-1 mRNAs are less abundant than the EBNA-2 or EBNA-LP mRNAs. All of the EBNA regions share the 5' leader sequence. While the EBNA-LP is encoded by the 5'-most ORF, the other proteins are encoded by ORFs which are located 3' to this region. EBNA-2 is translated more efficiently from a mRNA which is lacking the 5' leader sequence, suggesting that EBNA-2 mRNA with the long leader sequence can moderate internal initiation of translation. EBNA-1 and EBNA-3 proteins are also translated from a 3'-ORF and contain glycine-alanine repeat motifs of variable lengths within their ORFs.

Although most of the EBNA proteins bind to DNA cellulose, only EBNA-1 binds with high affinity to specific sites within the EBV genome. The cognate sequences are located at three sites within the genome, two of which comprise the oriP domain. EBNA binding to the oriP domain allows viral DNA to replicate and persist as an episome in latently infected cells. In latently infected cell nuclei, EBNA-1 is associated with chromatin and may interact with cell proteins to ensure that EBV episomes are partitioned to progeny nuclei. The abundance of EBNA-1 in nuclei is disproportionately high considering the low abundance of the EBNA-1 mRNA, suggesting that there is a translational up-regulation of EBNA-1 synthesis.

Little is known of the EBNA-2 role in latent infection although there is evidence that the EBNA-2 proteins are direct or indirect transactivators of latent infection and viral gene expression.

Despite having weaker promoters, the LMP gene produces almost 10 times as much mRNA as the EBNA gene. LMP mRNA has two introns and encodes an integral membrane protein. LMP is phosphorylated and associates with the cytoskeleton. Nascent LMP has a half-life of between 2-5 hours and represents the conversion time to the form of LMP associated with the cytoskeleton. Once in the cytoskeleton, LMP has a half-life of 16-20 hours. LMP appears to interact with itself or other cell membrane components to form large, non-covalently linked, membrane complexes. More recently, LMP was shown to regulate the expression of the cellular oncogene bcl-2 which in turn is responsible for the loss of apoptosis in transformed cells.

The 1 kb BZLF1 mRNA and the 2.8 kb BRLF1 mRNA are key immediate-early type transactivators of early EBV gene expression in the replicative phase of the virus gene expression. A unique fusion mRNA, the RAZ ORF mRNA which is composed of regions from both the BZLF1 and BRLF1 ORFs as well as the BZLF1/BZLF2 intergenic region, encodes a unique tyrosine kinase protein which may also be an important regulator of EBV gene expression. In transient transfection studies, both the BZLF1 and the BRLF1 proteins transactivate the strong EBV early promoters. The BZLF1 mRNA includes an exon which has homology to the c-fos/c-jun family of transcriptional activators. Like c-fos, BZLF1 will bind directly to AP1-like DNA segments.

DNA replication of EBV is catalyzed by a viral DNA polymerase which resembles the situation observed for replication of the other herpesvirus DNAs. The BALF2 ORF encodes the 110 kd DNA polymerase protein. The EBV polymerase is unlike the HSV polymerase in its salt sensitivity and resistance to amphidicolin. This basic difference between the HSV and EBV polymerases may account for the relative differences in the viral sensitivities to acyclovir and other inhibitors of HSV DNA polymerase activity. A number of other EBV genes are known to encode viral proteins which are accessory factors in the viral DNA replication.

Infectious mononucleosis (IM) is a polyclonal transformation of B cells which is initiated by EBV. Autoantibodies are the hallmark of IM.EBV infection. IM results in polyclonal B-cell activation, including the activation of many cells which are not even infected with EBV. It is likely that many of the clinical manifestations of IM are due to immune responses to the proliferating B-cells.

A number of recent reports have described the occurrence of diffuse lymphoproliferative disease with features of malignant lymphoma occurring in the wake of infectious mononucleosis. The infected patients are a heterogeneous group, including both girls and boys, but half of the cases are males with the X-linked Lymphoproliferative Syndrome (XLPS). The lymphomas contain EBV DNA and express EBNA. Boys with XLPS are not globally immunodeficient although they do express a deficiency in natural killer cell activity.

Although the association of EBV with Burkitts Lymphoma (BL) has been known since the discovery of the virus, the exact role which the virus plays in the etiology and pathogenesis of BL is still controversial. More than 90% of the tumors contain EBV DNA and express EBNA. In lymphoblastoid cell lines there is expression of all identified latent nuclear products and LMP. However, in BL cells there is repression of EBNA-2 and LMP, although the cells still produce EBNA-1. This could mean that the same genes which are required for immortalization are not required in BL cells, which may be immortalized by some other mechanism, perhaps induction of c-myc activity by the chromosomal translocations which are known to take place in BL cells.

The association of EBV with nasopharyngeal carcinoma (NPC) is strong. EBV DNA is regularly found in all cases of undifferentiated NPC and in a number of cases of differentiated NPC. Expression of EBV genes in NPC differs from that observed in BL and IM. The two most notable features are the limited expression of the latent proteins to EBNA-1 and LMP, and expression of several genes which are thought to be part of the early-antigen complex.

EBV induces fatal lymphoproliferative disease, sometimes with the features of frank lymphoma. There are thought to be several components of these progressive lymphoproliferative diseases in immunodeficient patients. The tumors which arise are often polyclonal initially, which implies that the tumors arise from several independent transformation events. With time the tumors evolve to an oligoclonal or even monoclonal state. Most EBV-associated lymphoproliferative lesions do not exhibit the same chromosomal translocations observed with BL. Patients which exhibit these types of tumors include post-transplant recipients and AIDS patients. AIDS patients develop three types of EBV-associated lesions: (i) diffuse polyclonal lymphomas, often at extranodal sites such as the gut and central nervous system, (ii) lymphocytic interstitial pneumonitis (LIP), and (iii) hairy cell leukoplakia of the tongue. LIP occurs mainly in children with AIDS and is not a progressive lymphoproliferative disease. Hairy leukoplakia is associated with chronic production of EBV in epithelial cells of the tongue. In the basal layers, the BZLF1 gene product is found; in the upper layers, early and late viral gene products are expressed.

The invention features the use of ribozyme inhibitors of EBV replication, especially the productive infection of epithelial cells or the establishment of latent EBV infections in B-lymphocytes. This invention may also be used to reverse the immortalization of B-lymphocytes or latent EBV infections.

The cleavage of EBV mRNA or hnRNA using this new class of chemical inhibitors, ribozymes, allows specific interruption of viral replication or maintenance of EBV latency by cleavage of the viral RNA. This class of chemical cleavers will exhibit a high degree of specificity for only the viral RNA in infected cells. A ribozyme molecule targeted to a highly conserved sequence region will allow the treatment of all strains of EBV with a single compound. No current treatment can impact either the latent state or the transformed phenotype associated with EBV infections. All treatments with nucleoside analogues are virustatic and the effects are reversed upon withdrawal of the drug. Ribozymes offer a non-reversible treatment for EBV infections, and thus are virucidal.

This invention allows treatment of Epstein-Barr virus infections in humans. Different ribozyme preparations can be used in treating productive, persistent and latent EBV infections. Moreover, ribozymes targeted to the LMP mRNA are useful in reversing the immortalization event in lymphocytes and thus in allowing apoptosis in affected cells. Ribozymes targeted to the EBNA-1 and RAZ mRNAs can be used to disrupt the latent and productive stages of EBV infection, respectively.

Preferred cleavage sites are at genes or regions required for viral replication, e.g., protein synthesis, e.g., those genes and regions described above, e.g., EBNA-1 and RAZ.

Ribozymes targeting any of the above regions of these mRNAs can be designed to cleave the mRNAs in a manner which will inhibit translation of the molecules. Alternative regions outside of the EBNA-1 and RAZ regions also make suitable targets for ribozyme-mediated inhibition of EBV gene expression. In particular, other targets include mRNAs encoding the EBNA-2 proteins, LMP protein, the viral DNA polymerase and the BZLF1, BRAF1 and BMRF1 proteins. Secondary structure analyses of the mRNAs or hnRNAs encoding these proteins can be used to determine ribozyme-targeting regions, to aid selection of these other regions of viral RNA which are good candidates for cleavage by ribozymes and subsequent inhibition of viral replication, disruption of latent viral infection, or termination of B cell immortalization.

As above, those genes of the EBV genome which are essential for virus replication are expected to maintain a constant sequence (i.e., are conserved) over extensive periods of time. These regions are preferred target sites in this invention since they are more likely to be conserved between different types or strains of EBV, and thus only one ribozyme is needed to destroy all such viruses. We have selected several such genes of EBV, and examined their nucleotide sequences for the presence of weak or non-base paired regions in the mRNA which may be cleaved by ribozymes targeted to those regions. Two genes analyzed in detail are the EBNA1 and RAZ genes; the other genes noted above can be analyzed in a manner similar to that described below.

The EBV mRNA sequences are analyzed using PC fold in the regions encoding the EBNA1 and RAZ genes. Putative ribozyme cleavage sites are found which are weak or non-base paired. These sites represent the preferable sites for hammerhead ribozyme cleavage within these two target RNAs.

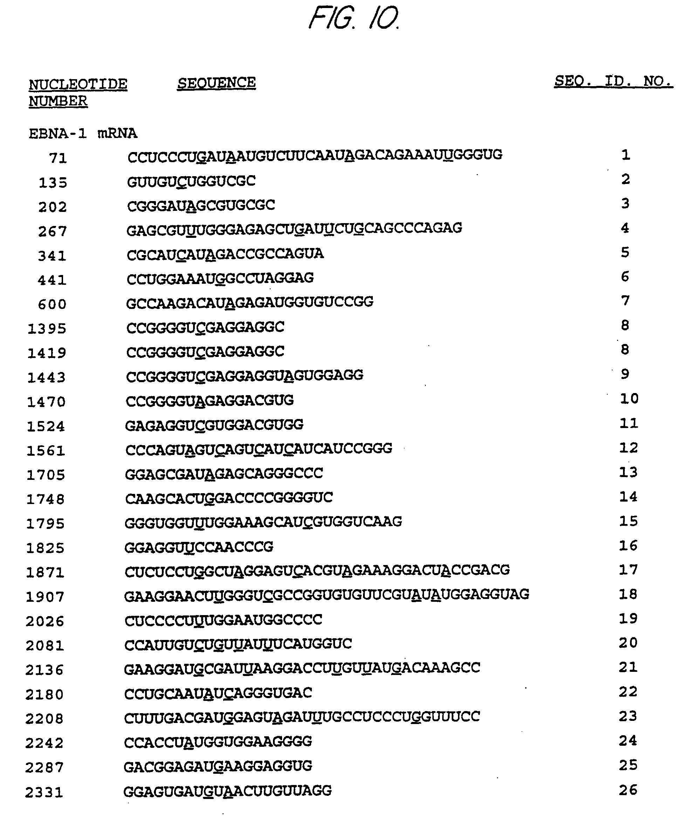

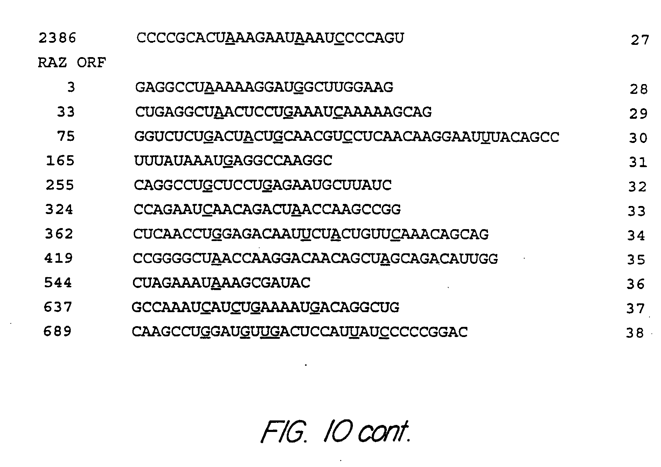

Using the above analyses, the following predictions of effective target sites in the chosen mRNA of the EBV genome, based upon computer generated sequence comparisons, are obtained (see Fig. 10). The target sequence is listed first with the 5'-most nucleotide number, for reference.

Preliminary predictions of effective target sites in the EBNA-1 and RAZ genes, based upon computer generated RNA structure, are listed.

### Example 6: T-Cell Leukemia

Adult T-cell leukemia (ATL) was first described by workers in Japan. Takatsuki et al., 9 Jpn. J. Clin. Oncol. 312, 1979. Since then, the disease has been found in other areas of the Orient, the Caribbean basin, South America and central Africa. The epidemiology has shown a clear association with the presence of a retrovirus known as human T-cell leukemia virus type I (HTLV-I). HTLV-I infection of lymphocytes *in vitro* results in immortalization of the same cell type as tumor cells, and HTLV-I has proven to be oncogenic in animal model systems, e.g., the rabbit.

HTLV-I was first identified in the HUT 102 cell line (Gallo et al., 43 Cancer Res. 3892, 1983), which was established from a patient diagnosed with cutaneous T-cell lymphoma. Yoshida et al., 79 Proc. Natl. Acad. Sci. USA 2031, 1982, demonstrated that another cell line, known as MT-1, derived from a patient with ATL, also harbored a C-type retrovirus which was later named HTLV-I.

Since the initial description of ATL and discovery of HTLV-I, the virus has been shown to be associated with other human diseases. The most notable of these is a neurologic disorder known as tropical spastic parapesis (TSP), or HTLV-I associated myelopathy (HAM).

HTLV-II was first described in a T-cell line derived from a patient diagnosed with hairy-cell leukemia. The cell line (Mo-T) was derived from splenic tissue and later shown to harbor a virus which was related to (but distinct from) HTLV-I, and subsequently named HTLV-II. Like HTLV-I, HTLV-II will immortalize lymphocytes. However, limited numbers of HTLV-II associations with disease has precluded epidemiologic demonstration of an etiologic role of the virus in human malignancy.

HTLV is a member of the class of retroviruses that includes bovine leukemia virus (BLV) and simian T-cell leukemia virus (STLV-I). The viruses are grouped within the oncovirus subfamily and are distinct from the other human pathogenic retroviruses, e.g., human immunodeficiency virus (HIV), which belong to the subfamily of lentiviruses. Although the leukemia viruses are oncogenic, they do not harbor oncogenic sequences derived from cellular genomes and their replication is regulated by at least three other genes, known as *tax*, *rex,* and *pro.*

Three mRNA species have been identified for HTLV. The full length RNA, transcribed from the U3-R junction in the 5'LTR and terminating in the R-U5 region of the 3'LTR, is used for the synthesis of *gag* and *pol* gene products, and serves as the genomic RNA packaged into virions. A subgenomic RNA with one intron removed serves as the template for env protein synthesis, and a second subgenomic RNA with a second intron removed serves to encode the tax and rex products. The *tax* initiator codon is the same as that of the env protein and is encoded within the second exon. The *rex* initiator codon is also within the second exon but is located 59 nucleotides 5' to that of the *tax* and *env.*

Unlike the protease genes of the other retroviruses, the HTLV protease gene is encoded by a reading frame which spans the 3' part of the *gag* region and the 5' part of the *pol* region, but is distinct from both of the other ORFs. In some of the clones of HTLV-I, mutations in the protease region may be responsible for the lack of infectivity of the clones. The protease is responsible for processing mature gag products and autocatalyzed self-cleavage to produce the mature protease molecule.

Two unique genes are found in HTLV (*tax* and *rex*) which encode nonvirion proteins translated from a single, sliced mRNA with three exons. Deletion of sequences within these two genes render infectious clones of HTLV-II noninfectious, providing evidence that the expression of these genes is critical for viral replication.

The HTLV-I and HTLV-II *tax* genes encode proteins of 40 and 37 kd, respectively. The tax proteins are *trans*-acting transcriptional activators which increase the rate of transcription initiation from the proviral 5'LTR promoter. Using co-transfection techniques, it has been shown that the tax protein is capable of *trans*-activating heterologous promoters, including the promoters for IL-2 and GM-CSF, *c-fos* and *c-sis*.

The rex gene of HTLV-I/HTLV-II encodes proteins of 27 and 21 kd for HTLV-I, and 26 and 24 kd for HTLV-II. The relationship between the two protein species is uncertain. Evidence suggests that the smaller rex protein is synthesized from an internal initiation codon within the same open reading frame. Both rex proteins are phosphorylated and localized in the nucleus of infected cells.

Like the product of the tax gene, the rex protein is essential to the replication of HTLV. Unlike the tax protein, the rex product appears to act post-transcriptionally to regulate viral gene expression. For HTLV-I, rex has been shown to increase the ratio of non-spliced RNA to the fully spliced mRNA which encodes tax and rex. In HTLV-II, rex protein increases the overall level of *trans*-activation in concert with tax protein. Additionally, in HTLV-II rex has a negative regulatory role which decreases viral mRNA levels. The ultimate effect of rex upon HTLV-I and HTLV-II infected cells is to regulate the levels of expression of genes encoding virion components, thereby determining the production of infectious virions.

The HTLV R and U5 regions are unusually long in comparison to other retroviruses. These regions encode the non-translated leader sequences at the 5' ends of all viral mRNAs, exhibit extensive secondary structure and probably play a role in the control of translation of those mRNAs.

HTLV-I and HTLV-II share about 65% overall sequence homology at the nucleotide level. The homology is lowest in the LTR and non-translated regions and is highest in the *tax* and *rex* genes. Among different HTLV-I isolates, there is 96-99% homology in these two regions. Sequence homology is equally high among the HTLV-II isolates.

Direct cell-to-cell contact appears to be required for efficient HTLV infection. *In vitro* infection is usually accomplished by co-cultivation of target cells with X-ray-irradiated or mitomycin C-treated virus producing cells. Infection by HTLV of susceptible cells is inefficient, with a slow course, as compared to other retrovirus infections. Infection of human cord or peripheral blood lymphocytes by HTLV results in virus production, and eventual immortalization of the cells. Only T-cells have been shown to be transformed by HTLV. Viral replication can occur in other cell types under certain circumstances, such as EBV-transformed B-cells. Productive infection has been observed in a few cells of non-lymphoid origin, most notably human endothelial cells and diploid human fibroblasts.

Infection of cells with HTLV *in vitro* can usually be accomplished only by co-cultivation of the cells to be transformed with virus-infected cells. Proliferating transformed cells predominate in the cultures after 4 or more weeks. Transformed lymphocytes are mostly of the CD4⁺ phenotype which reflects the observation that tumors in ATL patients are almost invariably of CD4⁺ type. However, occasional CD8⁺tumors have been reported and HTLV can transform CD8⁺ cells *in vitro.* Cells transformed *in vitro* transcribe RNA and produce virions, and these cells can be used to transform other cells. The lack of HTLV gene expression in ATL tumor cells suggests that *in vitro* transformation does not directly parallel the formation of a leukemic clone *in vivo.* These differences may reflect differences in selection pressure between *in vitro* and *in vivo* transformed clones.

Virion preparations from supernatants of HTLV-infected cells are mitogenic for quiescent human T-cells. Activated T-cells are more easily transformed by HTLV than are quiescent cells. This mitogenic activity of HTLV may be an important evolutionary adaptation because 95% of the T-cells in the body are generally in a quiescent state.

The mitogenic activity of virions suggests a mechanism for HTLV transformation whereby continual stimulation of T-cells via a T-cell receptor results in continuous cell proliferation. The corequisite of tax protein expression resembles the situation observed in transformation of primary rodent fibroblasts *in vitro,* where co-expression of two oncogenes, one acting in the nucleus and the other at the cell membrane, are required to effect transformation of the cell. Thus, unlike with other retroviruses, it appears that T-cell transformation is a normal course of events for infection with HTLV.

Several categories have been described as stages in ATL. These stages include: (i) asymptomatic carriers; (ii) preleukemic state (pre-ATL); (iii) chronic/smoldering ATL; and (iv) acute ATL. In some cases, these stages may be temporally related. The only significant prognostic factor in acute ATL is the presence of ascites, which is associated with shorter survival. Despite aggressive chemotherapy, the median survival in acute ATL is measured in months.

The form of disease in some ATL patients is that of a T-cell lymphoma rather than a leukemia. The differential diagnosis of ATL includes other T-cell malignancies such as non-Hodgkins lymphoma, mycosis fungoides, Sezary's syndrome and T-cell chronic lymphocytic leukemia (CLL). One malignancy distinct from ATL in which HTLV-I may play a role is B-cell chronic lymphocytic leukemia (B-cell CLL). There is some evidence for functional impairment of the immune response in ATL patients, as well as some HTLV-I carriers. The role of HTLV-II in the few cases of leukemia associated with viral infection is unclear, since insufficient cases are available for study.

ATL is a highly malignant disease where survival of subacute or acute disease is measured in months. Since only about 1% of patients progress from asymptomatic to acute disease, treatment is reserved for more advanced disease. Standard combination therapies which are used for other aggressive lymphomas and leukemias have not worked for ATL. Other agents such as deoxycoformycin, beta interferon, gamma interferon and anti-Tac antiboby have not been of value in treating ATL.

The invention also features cleavage of the RNA of these viruses by use of ribozymes. In particular, the ribozyme molecules described are targeted to the *nef, vif, tat* and *rev* viral genes. These genes are known in the art, see, e.g., Matsukura et al., 86 Proc. Natl. Acad. Sci. USA 4244, 1989, Cheng-Mayer et al., 246 Science 1629, 1989, Viscidi et al., 246 Science 1606, 1989, Malim et al., 86 Proc. Natl. Acad. Sci. USA 8222, 1989, Terwilliger et al., 88 Proc. Natl. Acad. Sci. USA 10971, 1991, and Bartel et al., 67 Cell 529, 1991.

Preferred cleavage sites are in regions required for viral replication, e.g., protein synthesis, e.g., the *tax, rex,* or *pro* gene regions, or at structures known to regulate viral gene expression, e.g., the 5'-LTR or 3'-LTR regions.

The genome of HTLV-I is subject to genetic drift by virtue of its RNA content and the nature of errors in reverse transcription. Those regions (genes) of the genome which are essential for virus replication, however, are expected to maintain a constant sequence (i.e., are conserved) over extensive periods of time. These regions are preferred target sites in this invention since they are more likely to be conserved between different types or strains of immunodeficiency viruses, and thus only one ribozyme is needed to destroy all such viruses. Thus, one ribozyme may be used to target all HTLV-I viruses, as well as all HTLV-II, BLV and STLV viruses. We have selected two genes of HTLV-I which are known to be highly conserved, and examined their nucleotide sequences for the presence of regions which may be cleaved by ribozymes targeted to those regions. Two genes analyzed in detail are the *tax* and *rex* genes; the *pro* gene, the 5'-LTR and other genes noted above can be analyzed in a manner similar to that described below.

The HTLV-I mRNA sequence of the HS-35 clone was analyzed in the regions encoding the *tax* and *rex* proteins. Twenty-three putative ribozyme cleavage sites were found to be present in open (weakly base-paired) regions of the mRNA. These sites represent the preferable sites for hammerhead ribozyme cleavage within these two target RNAs.

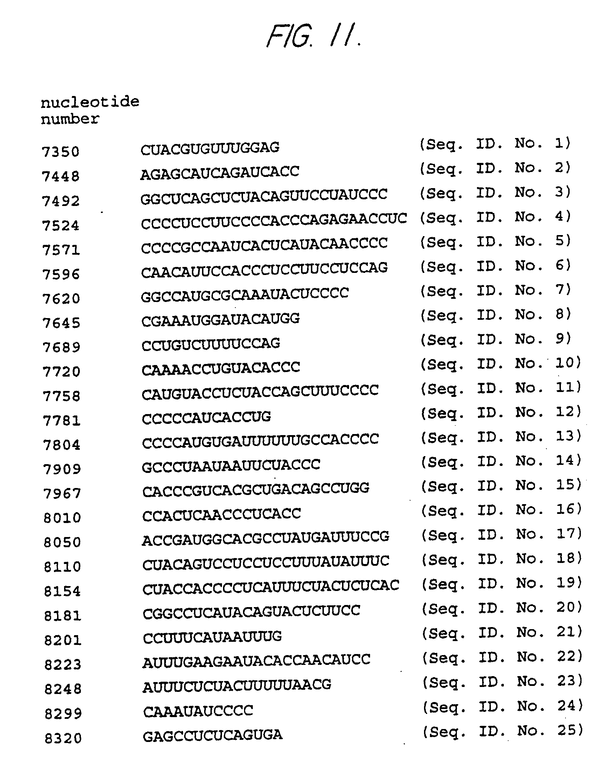

Using such analyses, the following predictions of effective target sites in the *tax* and *rex* genes of the HTLV-I genome, based upon computer generated sequence comparisons, were obtained (see Fig. 11). The target sequence is listed first with the 5'-most nucleotide number, for reference.

### Example 7: HCV

The Hepatitis C virus group is responsible for most cases of non-A, non-B hepatitis. Although non-A, non-B hepatitis may be caused by many different viruses, the principal cause is HCV. The infection, is transmitted through blood transfusions (25-50% of hemophiliacs have chronic hepatitis), percutaneous infusion from illicit drug usage (25-50% of all drug users have non-A, non-B hepatitis), renal organ transplant (where most late mortality is due to non-A, non-B hepatitis), and maternal-neonatal transfer. Up to 20% of sporadic outbreaks of hepatitis are caused by HCV.

The viral particles are 30-60 nM in diameter, enveloped, and consist of a linear RNA genome which is plus polarity and contains approximately 10 kb of coding information. Not much is known about the virus family in terms of the gene expression or encoded proteins. The viral genome is a positive-stranded RNA molecule which resembles that of the Flavivirus group in composition of open reading frames and overall size. There is no nucleotide homology between the HCV genome and that of the other Flaviviruses or Togaviruses (another positive stranded RNA virus). It is believed that the RNA is translated from a single open reading frame and then the polypeptide precursor is processed into the individual proteins required for viral replication. Aside from this superficial resemblance to the Flaviviruses, the HCV family also resembles the picornavirus family of RNA viruses. As in the picornaviruses, the 5' non-translated region of the HCV genome controls the translational expression of the viral RNA.

The replication of HCV in hepatocytes is integrally intertwined with the replication of other hepatitis viruses. Although hepatitis delta virus (HDV) is quite distinct from HCV, the life cycles of the two viruses can be interdependent. Normally HDV depends upon the replication of Hepatitis B Virus (HBV) to supply capsid proteins and other functions necessary for the assembly of infectious HDV particles. Because HCV inhibits HBV and Hepatitis A virus (HAV) growth in co-infected cells, the replication of HCV can also inhibit the maturation of HDV. The use of HDV as a gene vector for treatment of HCV infections would not be influenced by this interference because gene expression of the HDV genome is apparently uninhibited by HCV.

This class of chemical cleavers will exhibit a high degree of specificity for only the viral mRNA in infected cells. A ribozyme molecule targeted to a highly conserved sequence region will allow the treatment of many strains of human HCV with a single compound. No treatment exists which specifically attacks expression of the viral gene(s) of HCV. Current treatment protocols (interferon and ribavirin) consist of treating the symptoms of the disease, not the viral replication. Upon cessation of current treatment protocols, a rebound of disease ensues because the treatment does not impact the latency of the virus or actual viral replication. The cleavage of viral RNA offers a novel treatment which will reduce virus replication and establishment of latency in newly infected cells.

The methods of this invention can be used to treat human hepatitis C virus infections, which include both acute and chronic virus infection and HCV-induced hepatocyte transformation. The utility can be extended to other species of HCV which infect non-human animals, where such infections are of veterinary importance.

Treatment will occur at the time of active viral infection and will reduce virus loads in the infected cells and also disable viral replication. Ribozyme treatment may also be used as a means of creating defective genomes which can be used in vaccines.

Preferred cleavage sites are within regions required for viral replication, e.g., protein synthesis, such as the 5' non-translated region of the HCV genome. This region is believed to control the translation of viral proteins in a manner which is reminiscent of the cap independent translational control of picornaviruses. Disruption of this region in the RNA results in deficient protein synthesis as well as incomplete DNA synthesis (and inhibition of transcription from the defective genomes).

Alternative regions outside of the 5' non-translated region also make suitable targets of ribozyme-mediated inhibition of HCV replication. Such targets include the mRNA regions which encode the viral structural proteins. Selection of particular target regions will depend upon the secondary structure of the genome. Targets in the minor mRNAs can also be used, especially when folding or accessibility assays in these other RNAs reveal additional target sequences which are unavailable in the genome. Two or more different ribozymes can be used in this invention for therapeutic treatment.

As above, the genome of HCV may be subject to rapid genetic drift by virtue of its RNA content and the nature of errors in genomic replication. Those regions (genes) of the genome which are essential for virus replication, however, are expected to maintain a constant sequence (i.e., are conserved) over extensive periods of time. These regions are preferred target sites in this invention since they are more likely to be conserved between different types or strains of HCV viruses, and thus only one ribozyme is needed to destroy all such viruses. Thus, one ribozyme may be used to target all HCV viruses. We have selected several such genes of these viruses, and examined their nucleotide sequences for the presence of regions which may be cleaved by ribozymes targeted to those regions. One region analyzed in detail is the 5' non-translated region; other genes can be analyzed in a manner similar to that described below.

The mRNA sequences of the virus are folded using RNAfold computer analyses. The regions of the mRNA, in this case the 5' nucleotides of the HCV genome, which are predicted to have weak or non-base paired nucleotides are searched for putative ribozyme recognition motifs. These sites represent the preferred sites for hammerhead or other ribozyme cleavage within these target RNAs.

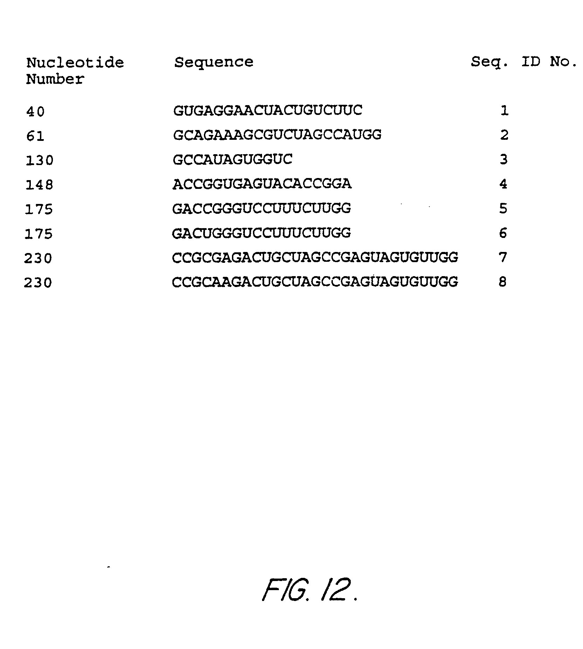

Using such analyses, the following predictions of effective target sites in the HCV genomic RNA, based upon computer generated RNA structure analysis, were obtained (see Fig. 12). The target nucleotide is listed with the genomic nucleotide number (in the HCV genome) given to the left of the sequence. Flanking nucleotides are given for reference.

### Example 8: CMV

The following is provided as a general outline of relevant CMV biology.

Cytomegaloviruses (CMVs) belong to the family of betaherpesvirus and are species-specific viruses for replication and pathogenesis. Even in the natural host, not all cells are equally susceptible to productive viral infection. In the case of human CMV (HCMV) the abortive infections of lymphocytes may even contribute to the invasion of the host immune surveillance. CMV infections proceed much more slowly than those of alphaherpesviruses (e.g., HSV and VZV) both in cell culture and in the animal host. Infectious virus may persist for an extended period of time, after which the virus may establish a latent infection. Although these viruses have at least 50% more sequence complexity than the alphaherpesviruses, there is still a dependence on and close association with the host cell. These include a growth requirement for differentiated cells *in vitro* and a stimulation of host cell macromolecular synthesis.

Although both families of the herpesviruses enter the host and express their first set of genes very rapidly (1-3 hr), the betaherpesviruses require 12-24 hours to initiate viral DNA synthesis while the alphaherpesviruses initiate viral DNA synthesis within 4-6 hours. Similarly, the alphaherpesviruses form plaques in cell monolayers at 24-48 hour postinfection while the betaherpesviruses require between 7-14 days to form detectable plaques. As these results suggest, the control of genetic expression is quite different in the CMVs when compared to that of HSV or VZV.

Although CMVs replicate in many of the same cell types as the other herpesviruses in their natural hosts, this group of viruses is restricted *in vitro* to replication in fibroblasts. The reason for this restriction is unknown.

Because the CMV genome can remain in host cells in a productive, persistent or latent state, there must be a number of features which regulate the expression of viral genes. The expression of the immediate early genes of the CMVs plays an important role in the replication of the virus because the products of these genes are known to influence the expression of other viral genes, their own genes and even cellular genes. The ie1 gene spans approximately 2.8 kb between 0.739 and 0.751 map units and codes for a 1.95 kb RNA which consists of a 5' leader exon of 121 nucleotides, followed by three exons of 88, 185, and 1,341 nucleotides. The open reading frame (ORF) of the mRNA begins in the second exon and encodes a 491 aa polypeptide. The size of the ie1 protein varies somewhat between strains of HCMV yet some ie1 nucleotide sequence homology is conserved even between CMVs of different species.

By an alternate splicing pattern, exons 1, 2, and 3 of the ie1 region can be ligated onto the ie2 mRNA resulting in two mRNAs of 2.25 and 1.70 kb. The proteins encoded by these mRNAs contain 85 amino acids derived from the exons 2 and 3 of ie1 but the majority of the protein is derived from region ie2a. The 2.25 kb and 1.70 kb mRNAs differ by a small splice in ie region 2a and they are predicted to encode proteins of /1 and 53 kd, respectively. A smaller mRNA of 1.4 kb also originates from ie region 2a. This mRNA is transcribed under independent promoter control only late in the infectious cycle.

The HCMV DNA polymerase is physiologically distinct from host cell polymerases. The viral polymerase is a 140-kd protein which is associated with a 51-58 kd polypeptide, referred to as the polymerase associated DNA binding protein. The polymerase exhibits regions of homology with other herpesvirus polymerases but also has regions which are unique to the CMV polymerases. Most antiviral agents are targeted to the inhibition of the CMV polymerase.

Another viral protein (UL97 gene product) has been found to be linked genetically with the CMV polymerase in antiviral resistance studies. The UL97 gene has been observed to be mutated in a number of viable strains which exhibit simultaneous mutations in the viral DNA polymerase which produce a gancyclovir resistant phenotype. The interactions of these two proteins in the replication of CMV genomic DNA is uncertain, but homology of the UL97 gene with other herpesvirus genes (e.g., the UL13 gene of HSV-1) and the general conservation of the transcription unit which encodes the mRNA among herpesviruses suggests that this gene may be important to the efficiency of viral replication. Numerous protein kinase motifs exist within the putative ORF of the UL97 gene, and the HSV UL13 protein is known to possess protein phosphorylating activity. Thus, it may be assumed that the UL97 gene of HCMV helps to control gene expression by phosphorylation of some CMV protein, possibly the DNA polymerase.

Many of the structural proteins of CMV are expressed at late times after infection. Although all of the structural proteins are assumed to play a role in the formation or integrity of the virion particle, the most relevant among these are the viral glycoproteins. The gB homolog has homology to the gB genes of other herpesviruses such as HSV, VZV, EBV and other animal CMVs. This viral protein is encoded by an unspliced mRNA of approximately 3.9 kb that codes for a protein 906 aa in length. The amino acid sequence contains a typical eucaryotic signal sequence, and as many as 17 potential N-linked glycosylation sites and transmembrane domains near the carboxy terminus. It has been proposed that the HSV gB functions to facilitate membrane fusion during viral infection. Since the gB genes are so highly conserved among the herpesviruses, it is reasonable to assume that the CMV gB protein may also participate in membrane fusion events during infection. As in the case for HSV, other glycoproteins of HCMV may be involved in the viral infection, including the gH homolog, gcI, gcII, gcIII and the HXLF family of glycoproteins.

Humans are believed to be the only reservoir for HCMV and transmission occurs by direct or indirect person-to-person contact. Sources of virus include nasopharyngeal secretions, urine, cervical and vaginal excretions, spermatic fluids, breast milk, tears, feces and blood. Virus excretion persists for years after congenitally, perinatally and early postnatally acquired infections. Prolonged replication of virus can follow infection in adolescents and young adults, and recurrences are generally associated with periods of intermittent shedding in adults.

Oral and respiratory spread appear to be the dominant routes of transmission during childhood and adult life. Blood transfusions are associated with increased risk of both primary and recurrent CMV infection. Transfusion of leukocyte-enriched fractions further increases the risk. CMV infections are even more markedly increased in patients which are immunosuppressed, such as those undergoing chemotherapy for cancer or preparing for organ transplants. Perhaps the most striking risk group are those patients suffering from AIDS. The occurrence of CMV infection is so great among AIDS patients that HCMV was once considered as the etiologic agent.

Studies on the pathology of CMV infections has focused upon describing the distribution of cytomegalic inclusion bodies in various infected tissues. With severe disseminated disease, evidence of CMV infection can be found in virtually all organ systems. In the human, CMV primarily infects the ductal epithelial cells and seldom involves fibroblasts, unlike the growth in cell culture. Viruria is a common feature of CMV infection in all age groups and results from renal involvement. The cytomegalic inclusion bodies are most often pronounced in the proximal tubules near cortical areas.

With the possible exception of renal transplant patients, CMV renal infections seldom result in kidney dysfunction. In the renal transplant patients however, CMV may play a role in dysfunction and even graft rejection. In adults, CMV hepatitis is manifest by the cytomegalic bodies in hepatocytes. In lung infections, the bodies are most concentrated in the alveolar and broncheolar epithelium. The mucous membranes of the trachea are less often involved and most lung infections occur in older immunosuppressed patients. CMV pneumonia is one of the leading causes of death in AIDS patients.

In the past, CMV infection of the CNS was rare except in fetal infections that resulted in cytomegalic inclusion disease (CID). In recent years, CNS involvement has increased in frequency in adults with AIDS. Permanent, usually severe generalized brain damage is characteristic of CID of the newborn. In both children and adults glial and neuronal cells may be either productively or abortively infected. Inclusion-bearing cells in capillary epithelium suggests blood-born spread in most cases.

Gastrointestinal involvement is also on the increase. CMV may coinfect with other pathologic agents associated with AIDS and may be present with other lesions, such as Kaposi's sarcoma. Necrotizing lesions carry a poor prognosis.

Sensineural hearing loss is a feature of both congenital and neonatal infections. Cytomegalic cells have been seen in a number of cells including epithelial cells of the cochlea and semicircular canal. Other organs with a reasonable degree of occurrence include adrenals, ovaries, bones, pancreas and skin.

CMV shares properties with other oncogenic viruses, such as the ability to stimulate host cell macromolecular synthesis, and to stimulate the synthesis of ornithine decarboxylase. Genetic sequences of HCMV with transforming capabilities have been described. In addition, CMV or its products have been found in human tumors, including prostatic carcinoma, adenocarcinoma of the colon, cervical carcinoma and Kaposi's sarcoma.

The invention also features cleavage of the mRNA or hnRNA encoded by these viruses by use of ribozymes. In particular, the ribozyme molecules described are targeted to the ie1 and ie2 viral genes. These genes are known in the art, see, e.g., Stenberg et al., 56 J. Virol. 665, 1985, Alerigg et al., 2 Virus Res. 107, 1985, Stenberg et al., 63 J. Virol. 2699, 1989, Hermiston et al., 61 J. Virol. 3214, 1987.

Preferred cleavage sites are at regions required for viral replication, e.g., protein synthesis, e.g., regions in the intron/exon regions of the region encoding the immediate early (ie) 1 and 2 mRNAs. This region controls the subsequent timing of viral gene expression and therefore the successful initiation of viral replication. Disruption of this region in the mRNA results in deficient protein synthesis as well as incomplete DNA synthesis.

Alternative regions outside of the HCMV ie region also make useful targets for ribozyme-mediated inhibition of HCMV replication. Other targets which are preferred in this invention include the viral DNA polymerase, gB homolog and UL97 genes. Targets in the other HCMV genes can also be designed, and accessibility assays (see below) in these other mRNAs and hnRNA will reveal additional target sequences which are not predicted to be accessible from RNA folding programs. Those genes which encode RNA essential for viral replication in humans can be targeted for ribozyme cleavage.

The use of ribozyme inhibitors of cytomegalovirus replication, especially human CMV (HCMV), allows ribozyme specific inhibition of gene expression which causes CMV-induced cellular transformation, CMV-induced immunodeficiency, and maintenance of latent CMV infections.

The invention provides a novel method of inhibiting human cytomegalovirus replication and provides methods for the treatment of diseases caused by these viruses in both man and other animal species.

These ribozymes exhibit a high degree of specificity for only the viral RNA in infected cells. A ribozyme molecule targeted to a highly conserved sequence region allows the treatment of many strains of HCMV with a single compound. No treatment exists which specifically attacks expression of the viral gene(s) other than those involved in the replication of viral genomic DNA. The drugs currently used for clinical treatment of HCMV infections present serious side effects which preclude usage in all patients. For example, Gancyclovir is known to cause non-reversible gonadal toxicity, and phosphonoformic acid (PFA) causes neutropenia and renal disfunction. Both of these latter effects are significant problems in organ transplant patients.

This invention can be used to treat human cytomegalovirus infections, which include both productive virus infection and latent infections or HCMV-induced cellular transformation. The utility can be extended to other species of cytomegalovirus which infect non-human animals, where such infections are of veterinary importance.

Treatment will occur at the time of active, latent or persistent viral infection and will reduce virus loads in the infected cells and also disable viral replication. For treatment of transformed epithelial cells, the use of this invention will inhibit the expression of viral genes or related RNAs thought to cause cell transformation.

The genome of CMV is subject to rapid genetic drift by virtue of errors in DNA replication. Those genes which are essential for virus replication, however, are expected to maintain a constant sequence (i.e., are conserved) over extensive periods of time. These regions are preferred target sites in this invention since they are more likely to be conserved between different types or strains of CMV, and thus only one ribozyme is needed to destroy all such viruses. Thus, one ribozyme may be used to target all CMVs. We have selected several such genes of CMV and examined their nucleotide sequences for the presence of open mRNA regions which may be cleaved by ribozymes targeted to those regions. Two genes analyzed in detail are the ie1 and ie2 genes; other genes noted above can be analyzed in a manner similar to that described herein.

The CMV mRNA sequences were analyzed for weak or non-base paired regions in the sequences encoding the ie1 and ie2 proteins. Putative ribozyme cleavage sites were found which represent the preferable sites for hammerhead ribozyme cleavage within these two target RNAs.

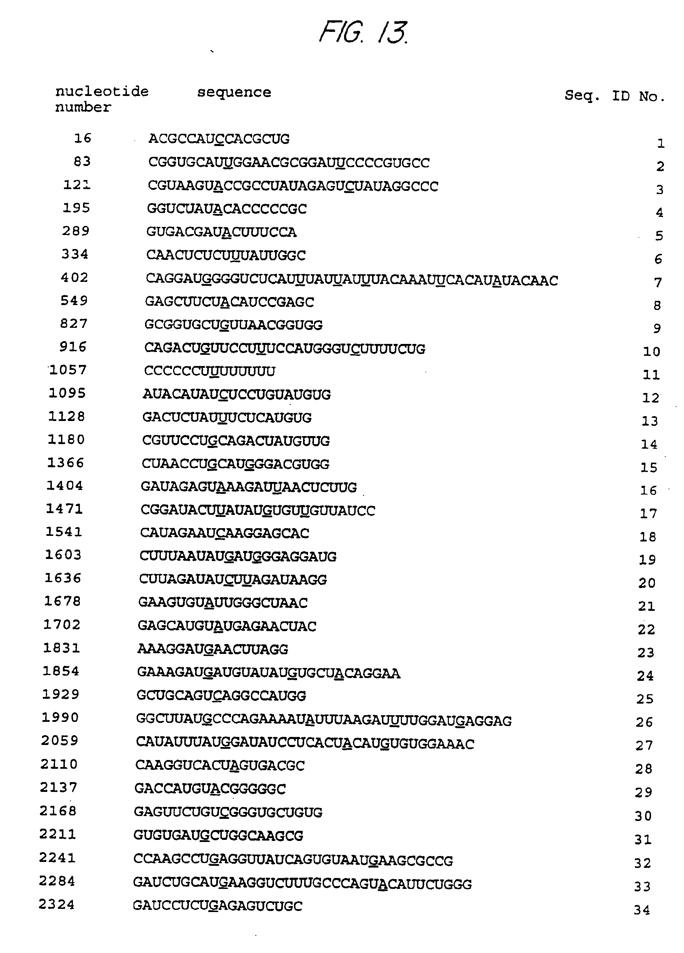

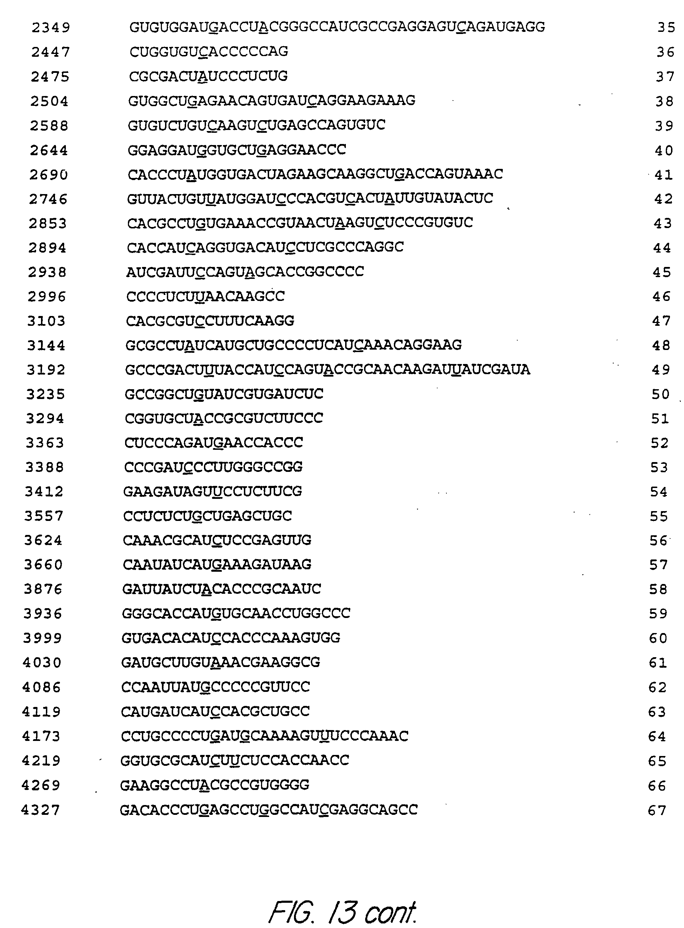

Using such analyses, the following predictions of effective target sites in the ie1 and ie2 genes of the CMV genome, based upon computer generated sequence comparisons, were obtained (see Fig. 13). The target sequence is listed first with the 5'-most nucleotide number, for reference. The target nucleotide is underlined with reference nucleotide numbers given at the beginning of each line. Flanking nucleotides which will constitute all or part of the region complementary to the ribozyme binding arms are shown. The sequences were taken from the DNA sequence of the AD169 strain of HCMV.

### Example 9: Influenza Virus

Three types of influenza viruses (A, B, and C) are distinguishable by antigenic reactivities of their internal antigens. There are other biological properties which characterize the three types: (a) Influenza A viruses have been isolated from many animal species in addition to humans while influenza B and C viruses are mainly human pathogens; (b) the surface glycoproteins of influenza A exhibit much greater variability than their homologues in the B and C viruses; (c) morphological and molecular features of C viruses are distinctive from those of the A and B viruses.

The morphological characteristics of influenza viruses are a genetic trait, but spherical morphology dominates after passage in chicken embryos or tissue culture. The genes that specify morphology are uncertain, but segregate separately from the hemagglutinin (HA) and neuraminidase (NA) envelope surface proteins. Within the lipid envelope lies the matrix protein (M), which plays a structural function. Within the matrix shell are eight single-stranded RNA molecules of negative sense associated with the nucleoprotein (NP) and three large proteins (PB1, PB2, and PA) required for RNA replication and transcription. At least three viral encoded nonstructural proteins (NS1, NS2 and M2) are formed in infected cells.

The organization of the eight RNA segments within the virion has not been completely resolved. Although each segment may exist *in vivo* as a nucleoprotein complex, electron microscopic studies have shown that the internal component from disrupted virions is a single large helix. The virion particle does not seem to be a tight protective coat around the RNA because ribonuclease digestion of virion reduces the RNA segments to nucleotide. Genomic RNAs of influenza virus are held in a circular conformation in a virion and in infected cells by a terminal panhandle that plays a role in viral replication. The panhandle structure is present in all segments of genomic RNA.

The HA accounts for 25% of viral protein and is distributed evenly on the virion surface. It is responsible for attachment of the virus to cells and penetration of virus into cells early in infection. The HA monomer is encoded by the fourth largest RNA segment and is synthesized as a single polypeptide chain which undergoes posttranslational cleavage at a minimum of three sites. Cleavage of the HA polypeptide into HA1 and HA2 is necessary for virus particle infectivity. A sequence of 25-32 hydrophobic amino acids at the C-terminus of HA2 saves to anchor HA in the virus membrane. In spite of functional domain conservation in HA, the amino acid or nucleotide sequences of the proteins vary considerably between isolates of different subtypes.

The NA is the second subtype-specific glycoprotein of the virion and is composed of a single polypeptide chain. The NA is not evenly distributed on the surface of the virion but is found in patches. The role of the NA in the life cycle of the virus is unclear. No posttranslational cleavage of the NA polypeptide occurs. The nucleotide sequences of different NA gene isolates varies considerably between subtypes (e.g., A and B virus amino acid homology is 26-29%). The NA gene of influenza B encodes two proteins, NA and NB. The NA is thought to be structurally and functionally similar to the type A NA. The NB protein is a glycoprotein of unknown function which is 100 amino acids in length.

The nucleoprotein (NP) is one of the type-specific antigens of influenza viruses that distinguishes among the influenza type A, B, and C viruses. The NP is a multifunctional protein having a structural role in forming the nucleoprotein complex and a putative role in transcription and replication. Genetic analysis of a large number of influenza strains has revealed that the NP genes can be placed into one of five different groupings. All avian strains fall within two groups, equine strains fall within two more groups and all human and swine strains form the final group. The restriction of certain species strains to these groups suggests that the NP gene may influence species-specificity or host range.

RNA segment 7 encodes the two M proteins (M1 and M2). The mRNA encoding M1 is colinear with RNA segment 7, whereas M2 is encoded by a spliced mRNA. The two proteins share the same initiation codon for protein synthesis and the eight amino acid residues before the 5' splice junction of the M2 mRNA. The remaining 88 amino acids of M2 are encoded in the +1 reading frame from nucleotides 740-1104. This organization of RNA segment 7 is present in all influenza A and B viruses sequenced.

The M1 protein is a virion structural protein that is intimately associated with the lipid bilayer in close proximity to both glycoproteins and the ribonucleoprotein complex. It is also believed to have a role in the down-regulation of the virion transcriptase activity. Passively transferred monoclonal antibodies to this protein do not confer resistance to infection by influenza virus.

The M2 protein of influenza A is an integral membrane protein that is expressed at the surface of infected cells. The M2 protein may be a virion associated protein with between 14 and 68 molecules per virion. Amantadine-resistant mutants of influenza virus contain mutations in the transmembrane domain of the M2 protein. Because amantadine alters viral penetration into cells, it is likely that M2 is in the virion.

Comparison of RNA segment 7 sequences of the H3N2 (Udorn) and H1N1 (PR8) strains show that the M protein coding sequences of these viruses (isolated 38 years apart) are highly conserved. Lamb, "The genes and proteins of influenza viruses," in. Krug. ed. The Influenza Viruses NY, Plenum. 1989. Comparison of 230 nucleotides of RNA segment 7 from 5 human H1N1, H2N2 and H3N2 strains isolated over a 43 year period suggests that the same segment 7 was retained throughout the antigenic shifts of HA and NA. Hall and Air, 38 J. Virol. 1, 1981.

Studies have shown that RNA segment 8 of influenza A and B encodes two nonstructural proteins which are translated from separate mRNAs. NS1 and NS2 polypeptides of influenza A share 9 amino acids at their N termini, after which the NS2 mRNA has a 423 nucleotide deletion; then, the NS2 mRNA rejoins the NS1 3' region in the +1 reading frame. NS1 is synthesized in large amounts early in infection. NS2 is made only late in infection. Both proteins share a nuclear localization signal and can be found in the nuclei of infected cells. Large deletions occur in the carboxyl termini of the NS1 proteins of field isolates from humans or birds, which indicates that a high degree of variation can be tolerated in this polypeptide without affecting its function.

The three largest proteins of the virion (PB1, PB2 and PA) are found associated with NP and virion RNA and carry the polymerase activity which transcribes invading viral RNA. The PB1 and PB2 proteins form a complex when expressed in the absence of other virion proteins or RNA and are probably required for complementary RNA synthesis. PA and NP are required for virion RNA synthesis. The PB1 gene of influenza B virus shows 61% homology with that of the influenza A virus.

Influenza virus produces an acute febrile infection of the respiratory tract characterized by abrupt onset prominent myalgias, headache and cough. Pneumonia is the most frequent complication; it may be primary viral (due to invasion of lung parenchyma), secondary bacterial, or mixed viral and bacterial pneumonia. It may be severe and progressive or mild and segmental. Other complications which occur with less frequency include Reye's syndrome, myocarditis, pericarditis, myositis, encephalopathy and transverse myelitis. It has been estimated that the direct costs of influenza exceed $1 billion per year and may reach $3 to $5 billion. Total costs may be two to three times higher.

Two types of vaccines are available for influenza. The "split" vaccines are chemically treated to reduce pyrogenic components and are the only type given to children under 13 years of age. The "whole" vaccine is generally given to adults. Protective antibody titers are present in more than 90% of normal subjects after vaccination with influenza A antigens, but there is much less response to influenza B antigens. Additionally, elderly subjects and patients with renal failures or immunosuppression are at much greater risk to infection even with vaccination. The 70-80% efficacy of the vaccine is only observed when strain matches are good. Lower efficacy is observed when the match is not close, and when patients are immunocompromised, or in institutional situations in which virus is readily transmitted.

Two drugs, amantadine and rimantadine, are as effective as influenza vaccine in preventing influenza A infections. Unfortunately, they are not as active against influenza B, which is responsible for 20% of all influenza epidemics and in a given year may be the only virus circulating. Amantadine is approved in the United States; rimantadine is not. Both drugs appear to impair the uncoating of viral RNA in infected cells by blocking the acidification process required to open the viral particles.

Resistance to amantadine and rimantadine is easily produced in the laboratory by serial passage of strains of influenza A virus in low concentrations of the drug, and such isolates are cross-resistant to both drugs. Drug resistant strains of influenza virus are able to initiate infection of cells as effectively as their wild type progenitors. Resistance is associated with the presence of point mutations in the RNA sequence coding for the M2 protein. This occurs most frequently at amino acid 31, but may also occur at positions 27 to 34, which encompass the transmembrane domain of the protein. It has been hypothesized that M2 protein may act as an ion channel to facilitate the acidification of the virus particle, and that amantadine and rimantadine block this viral-envelope pore.

Many regions of the RNAs associated with influenza viruses are appropriate targets for ribozyme attack. A ribozyme targeted to these regions in influenza viruses may be useful for targeting an equivalent region in related viruses whose sequences are unknown.

There is a high degree of nucleotide sequence homology within the sequences of the human and non-human influenza virus genomes and mRNAs. Particularly useful regions include sequences in the 5' and 3' regions of the genomic RNA segments and complementary regions at the 3' ends of all influenza A and B virus mRNAs and their homologous positions in other genomes. In addition, the 3' sequence which contributes to the panhandle (CCUGCUUUUGCU) is highly conserved among all influenza virus isolates and contains two putative ribozyme cleavage sites in a relatively accessible region of the RNA. The panhandle sequence (AGUAGAAACAAGG) at the 5' ends of the RNAs also contains a suitable ribozyme target site. The complementary sequence (CCUUGUUUCUACU) of the 5' panhandle is present at the 3' terminus in all mRNAs and contains an additional 4 ribozyme target sites which occur within accessible regions of these molecules (i.e., the loosely base-paired terminal regions). Detailed information of the sequence of the eight RNA segments of influenza (seven in influenza C strains) and the molecular weight of the encoded proteins is known.

Preferred cleavage is at regions required for viral replication (e.g., protein synthesis, such as the regions in RNA segment 7 which regulate or encode the M proteins) the conserved 5' and 3' regions of the genomic RNA segments which are involved in the panhandle structures, as well as the regions at the 3' ends of all mRNAs which are complementary to the 5' panhandle structure and their equivalent in other viruses. Alternative regions may also be used as targets for ribozyme-mediated cleavage of these viral genomes. Each target can be chosen as described below, e.g., by a study of the secondary structure of the RNA, and the individual role of such RNA in the replication of the virus. If the targets are contained within the open reading frames of regions which encode proteins essential to the replication of the virus, then these other targets are preferred candidates for cleavage by ribozymes, and subsequent inhibition of viral replication. The regions encoding the influenza A virus PB1, PB2 and PA proteins and their homologous proteins in the other viruses are examples of such preferred targets.

The genome of influenza viruses may be subject to rapid genetic drift by virtue of its RNA content and the nature of errors in genomic replication. Those regions (genes) of the genome which are essential for virus replication, however, are expected to maintain a constant sequence (i.e., are conserved) over extensive periods of time. These regions are preferred target sites in this invention since they are more likely to be conserved between different types or strains of influenza viruses, and thus only one ribozyme is needed to destroy all such viruses. Thus, one ribozyme may be used to target all influenza viruses. We have selected several such RNA regions of these viruses, and examined their nucleotide sequences for the presence of conserved areas which may be cleaved by ribozymes targeted to those regions. Three regions analyzed in detail are the 5' and 3' panhandle regions, the complementary 3' regions present in all mRNAs, and the mRNAs encoding the M proteins; other genes can be analyzed in a manner similar to that described below.

The genomic sequences of the viruses are compared in their potential target regions. Putative ribozyme cleavage sites are found to be highly conserved between strains and species of virus sequence. These sites represent the preferred sites for hammerhead or other ribozyme cleavage within these target RNAs.

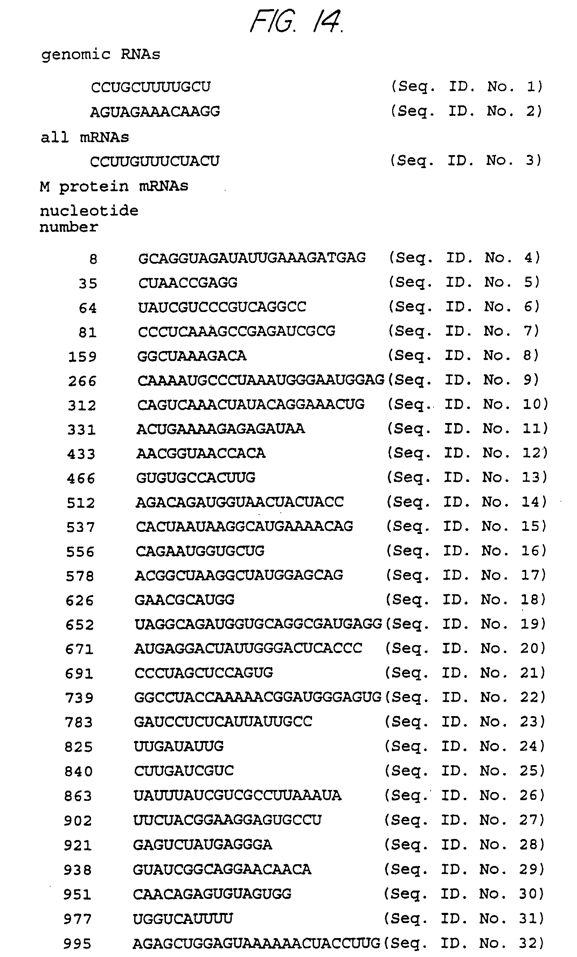

Using such analyses, predictions of effective target sites in the viral genes, based upon computer generated sequence comparisons, were obtained. These are identified as SEQ. ID. NOS. 1-32, shown in Fig. 14.

### Example 10: HSV

Human herpesviruses cause a wide variety of diseases which result in significant levels of morbidity and mortality worldwide. The HSV group accounts for about one million new cases of infection each year in the United States. These infections are maintained for the lifetime of the patient as latent viral infections, which can be stimulated to reactivate by a variety of factors. The manifestations of HSV infection range from mild infections of herpes labialis to more serious infections such as herpes encephalitis.

HSV contains a double-stranded DNA genome within its central core, has a molecular weight of approximately 100 million, and a genome encoding at least 70 polypeptides. The DNA core is surrounded by a capsid constructed from capsomers arranged in icosapentahedral symmetry. Tightly adherent to the capsid is the tegument, which appears to consist of amorphous material. Loosely surrounding the capsid and tegument is a lipid bilayer envelope containing polyamines, lipids, and the viral glycoproteins. These glycoproteins confer distinctive properties to the virus and provide unique antigens to which the host is capable of responding. Glycoprotein G (gG), for example, confers antigenic specificity to HSV, and therefore results in an antibody response that can be used to distinguish HSV-1 (gG-1) from HSV-2 (gG-2).

Replication of HSV is a multi-step process. Following the onset of infection, DNA is uncoated and transported to the nucleus of the host cell. Transcription of immediate-early genes encoding various regulatory proteins follows. Expression of immediate-early gene products is then followed by the expression of proteins encoded by early and then late genes, including structural proteins as well as proteins necessary for viral replication. Assembly of the viral core and capsid takes place within the nucleus. This is followed by envelopment at the nuclear membrane and transport out of the nucleus through the endoplasmic reticulum and the Golgi apparatus, where viral envelope proteins are glycosylated. Mature virons are transported to the outer membrane of the host cell, and release of progeny virus is accompanied by cell death. Replication for all herpesviruses is considered inefficient, with a high ratio of noninfectious to infectious viral particles.

The complete sequence of the HSV-1 genome is known. McGeoch et al., 69 J. Gen. Virol. 1531, 1988; McGeoch et al., 14 Nucleic Acids Research 1727, 1986; and the elucidation of the HSV-2 genome sequence is underway in laboratories worldwide. The two subtypes of HSV, HSV-1 and HSV-2, are 60-80% homologous at the DNA level, but intragenic variation, where known, is less.

Antiviral drugs including acyclovir have been used to effectively treat HSV infections, although with limited success. For example, chronic treatment with acyclovir has resulted in the development of acyclovir-resistant strains. Nucleoside analogues, such as acycloguanosine and tri-fluorothymidine are currently used for treatment of mucosal and ocular HSV infections, but these compounds have little if any effect upon recurrent or secondary infections (which are becoming more prevalent as the number of HIV-immunosuppressed patients rises). In addition, nucleoside analogues are poorly soluble in aqueous solutions, are rapidly catabolized intracellularly, and can be extremely toxic.

Ribozymes of this invention exhibit a high degree of specificity for only the virally encoded mRNA in infected cells. Ribozyme molecules targeted to highly conserved sequence regions will allow the treatment of many species or subtypes of HSV with a single compound. There is no acceptable treatment which will give a broad spectrum of activity with no toxic side effects. No treatment exists which specifically attacks viral gene expression which is responsible for the transformation of epithelial cells by HSV, for the maintenance of the episomal genome in latently infected cells or for the vegetative replication of the virus in permissive cells.

The methods of this invention can be used to treat HSV infections, which includes these diseases noted above. The utility can be extended to other HSV-like virus which infect non-human primates where such infections are of veterinary importance.

The ribozymes of the invention are capable of specifically cleaving particular viral mRNA targets, resulting in the destruction of mRNA transcript integrity required for translation, and therefore preventing the synthesis of the encoded protein. More specifically, the ribozymes of the invention are targeted to and prevent the translation of mRNAs encoding proteins required for viral genomic replication, virion structure, and viral infectivity, maintenance of the latent state, etc., and therefore interfere with critical events required for viral survival. Thus, diseases caused by HSV may be effectively treated by ribozyme-mediated interruption of the viral life-cycle.

Preferred cleavage sites are at genes required for viral replication, e.g., protein synthesis, such as in the immediate early genes (ICP0, ICP4, ICP22 and ICP27), genes required for nucleic acid metabolism (UL13, 39, 40, 50), host shut-off (UL41), control of late viral protein synthesis (γ 34.5), DNA replication (ULS, 8, 9, 29, 30, 42, 53) and structural protein encoding genes (gB and gC).

Alternative regions make suitable targets of ribozyme-mediated inhibition of HSV replication. Most preferred targets include ICP4(IE3), ICP27(UL54), UL39, UL40, ULS, γ 34.5 and UL27(gB) genes. Below are provided examples of ribozymes targeted to the ICP4 gene but other such ribozymes are expected to have utility at these other genes.

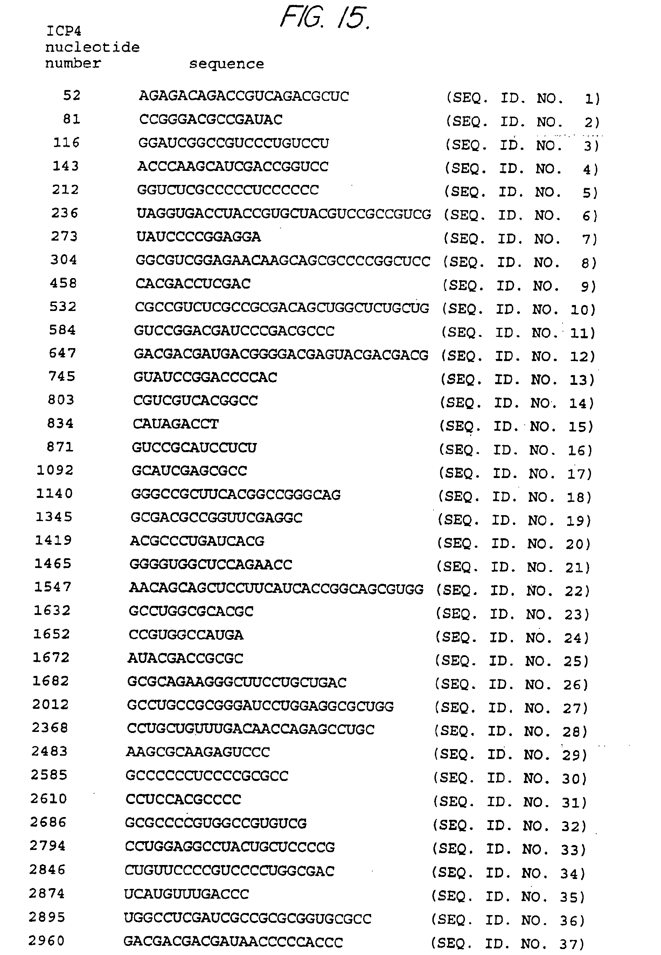

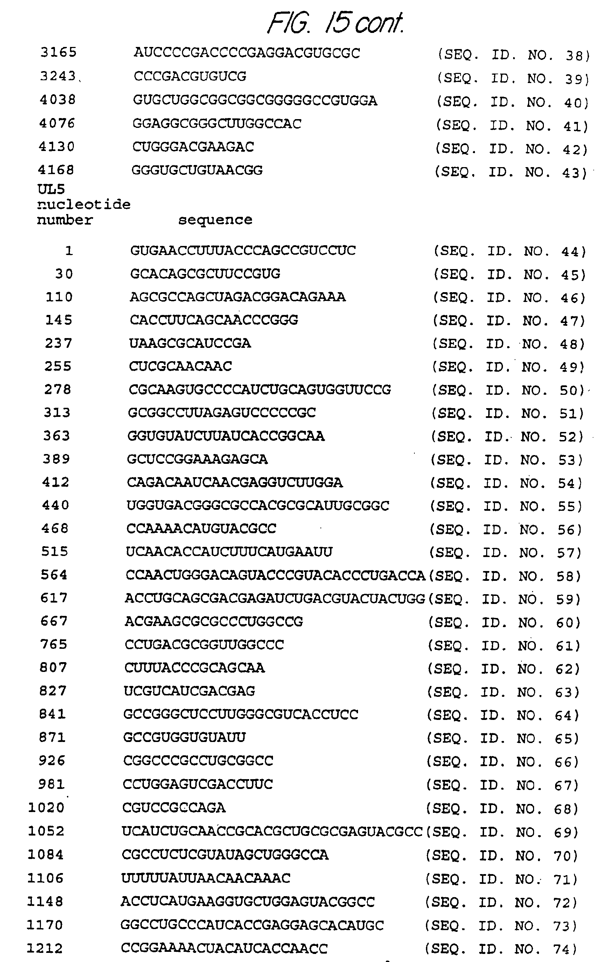

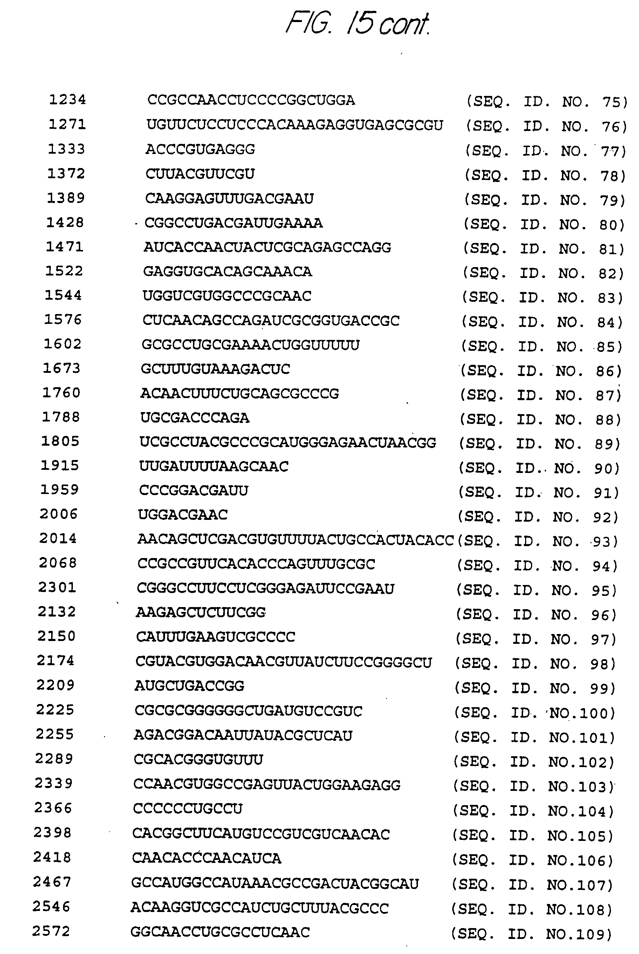

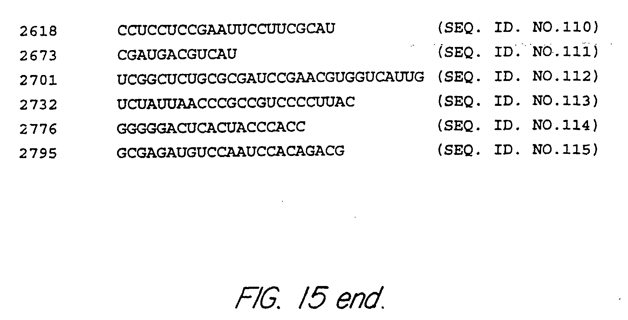

In particularly preferred embodiments, the RNA which is cleaved is HSV ICP4 or UL5 mRNA regions (for ULS, nucleotide #1 is defined by preliminary mapping of the cap site in our laboratory and nucleotide 2800 is the site of translation initiation for the UL4 protein whose open reading frame is also included in the UL5 mRNA) selected from one or more of the sequences shown in Fig. 15.

Those regions (genes) of the genome which are essential for HSV replication are expected to maintain a constant sequence (i.e., are conserved) over extensive periods of time. These regions are preferred target sites in this invention since they are more likely to be conserved between different types or strains of HSV, and thus only one ribozyme is needed to destroy all viral RNA. Thus, one ribozyme may be used to target all HSV-encoded RNA. We have selected several such genes of these viruses, and analyzed the structure of the encoded mRNAs using RNA-fold computer analyses. We have identified open structures in the RNAs which may be cleaved by ribozymes targeted to those regions. Two regions analyzed in detail . are the HSV-1 ICP4 and UL5 mRNAs; other genes can be analyzed in a manner similar to that described below.

HSV specific ribozymes may be designed to also inhibit other herpesviruses, particularly varicella zoster virus (VZV), which exhibits significant intragenic nucleotide homology with a number of analogous HSV genes. For example, the VZV immediate-early protein IE62 exhibits considerable amino acid homology to the HSV immediate-early regulatory protein ICP4/IE3 and is a major component of VZV particles (Kinchington et al., 66 J. Virol. 359, 1992). Also, some transcription units and their encoded proteins are conserved within the genomes of evolutionarily divergent human herpesviruses, and ribozyme targets within these genetic units may therefore provide the basis for designing ribozymes with broad therapeutic applications.

The mRNA sequences of the viruses are analyzed throughout the regions discussed above (using the nucleotide sequence of the HSV-1 ICP4 gene (EMBL No. HEHSV1G3, nucleotides 1176-5435) and UL5 gene). Putative ribozyme cleavage sites are found to be weak or non-base paired regions of the virus mRNA. These sites represent the preferred sites for hammerhead or other ribozyme cleavage within these target RNAs.

Using such analyses, predictions of effective target sites in the viral mRNAs, based upon computer generated structural analyses, were obtained (see SEQ. ID. NOS. 1-115). The target region is listed with the nucleotide number (in the HSV-1 ICP4 or UL5 mRNA) noted as base number.

In each of the above examples, short RNA substrates corresponding to each of the gene sites are designed. Each substrate is composed of two to three nucleotides at the 5' and 3' ends that will not base pair with a corresponding ribozyme recognition region. The unpaired regions flank a central region of 12-14 nucleotides to which complementary arms in the ribozyme are designed.

The structure of each substrate sequence is predicted using a standard commercially available PC fold computer program. Sequences which give a positive free energy of binding are accepted. Sequences which give a negative free energy are modified by trimming one or two bases from each of the ends. If the modified sequences are still predicted to have a strong secondary structure, they are rejected.

After substrates are chosen, ribozymes are designed to each of the RNA substrates. Ribozyme folding is also analyzed using PC fold.

Ribozyme molecules are sought which form hammerhead motif stem II (see Fig. 1) regions and contain flanking arms which are devoid of intramolecular base pairing. Often the ribozymes are modified by trimming a base from the ends of the ribozyme, or by introducing additional base pairs in stem II to achieve the desired fold. Ribozymes with incorrect folding are rejected. After substrate/ribozyme pairs are found to contain correct intramolecular structures, the molecules are folded together to predict intermolecular interactions. A schematic representation of a ribozyme with its coordinate base pairing to its cognate target sequence is shown in Fig. 1.

Those targets thought to be useful as ribozyme targets can be tested to determine accessibility to nucleic acid probes in a ribonuclease H assay (see below). This assay provides a quick test of the use of the target site without requiring synthesis of a ribozyme. It can be used to screen for sites most suited for ribozyme attack.

### Synthesis of Ribozymes

Ribozymes useful in this invention can be produced by gene transcription as described by Cech, *supra,* or by chemical synthesis. Chemical synthesis of RNA is similar to that for DNA synthesis. The additional 2'-OH group in RNA, however, requires a different protecting group strategy to deal with selective 3'-5' internucleotide bond formation, and with RNA susceptibility to degradation in the presence of bases. The recently developed method of RNA synthesis utilizing the t-butyldimethylsilyl group for the protection of the 2' hydroxyl is the most reliable method for synthesis of ribozymes. The method reproducibly yields RNA with the correct 3'-5' internucleotide linkages, with average coupling yields in excess of 99%, and requires only a two-step deprotection of the polymer.

A method based on H-phosphonate chemistry exhibits a relatively lower coupling efficiency than a method based upon the phosphoramidite chemistry. This is a problem for synthesis of DNA as well. A promising approach to scale-up of automatic oligonucleotide synthesis has been described recently for the H-phosphonates. A combination of a proper coupling time and additional capping of "failure" sequences gave high yields in the synthesis of oligodeoxynucleotides in scales in the range of 14 µmoles with as little as 2 equivalents of a monomer in the coupling step. Another alternative approach is to use soluble polymeric supports (e.g., polyethylene glycols), instead of the conventional solid supports. This method can yield short oligonucleotides in hundred milligram quantities per batch utilizing about 3 equivalents of a monomer in a coupling step.

Various modifications to ribozyme structure can be made to enhance the utility of ribozymes. Such modifications will enhance shelf-life, half-life *in vitro,* stability, and ease of introduction of such ribozymes to the target site, e.g., to enhance penetration of cellular membranes, and confer the ability to recognize and bind to targeted cells.

Exogenous delivery of ribozymes benefits from chemical modification of the backbone, e.g., by the overall negative charge of the ribozyme molecule being reduced to facilitate diffusion across the cell membrane. The present strategies for reducing the oligonucleotide charge include: modification of internucleotide linkages by ethylphosphonates or methylphosphonates, use of phosphoramidites, linking oligonucleotides to positively charged molecules, and creating complex packages composed of oligonucleotides, lipids and specific receptors or effectors for targeted cells. Examples of such modifications include sulfur-containing ribozymes containing phosphorothioates and phosphorodithioates as internucleotide linkages in RNA. Synthesis of such sulfur-modified ribozymes is achieved by use of the sulfur-transfer reagent, ³H-1,2-benzenedithiol-3-one 1,1-dioxide. Ribozymes may also contain ribose modified ribonucleotides. Pyrimidine analogues are prepared from uridine using a procedure employing diethylamino sulphur trifluoride (DAST) as a starting reagent. Ribozymes can also be either electrostatically or covalently attached to polymeric cations for the purpose of reducing charge. The polymer can be attached to the ribozyme by simply converting the 3'-end to a ribonucleoside dialdehyde which is obtained by a periodate cleavage of the terminal 2',3'-cis diol system. Depending on the specific requirements for delivery systems, other possible modifications may include different linker arms containing carboxyl, amino or thiol functionalities. Yet further examples include use of methylphosphonates and 2'-O-methylribose and 5' or 3' capping or blocking with m₇GpppG or m₃^{2,2,7}GpppG.

For example, a kinased ribozyme is contacted with guanosine triphosphate and guanyltransferase to add a m³G cap to the ribozyme. After such synthesis, the ribozyme can be gel purified using standard procedure. To ensure that the ribozyme has the desired activity, it may be tested with and without the 5' cap using standard procedures to assay both its enzymatic activity and its stability.

Synthetic ribozymes, including those containing various modifiers, can be purified by high pressure liquid chromatography (HPLC). Other liquid chromatography techniques, employing reverse phase columns and anion exchangers on silica and polymeric supports may also be used.

### Expression Vector

While synthetic ribozymes are preferred in this invention, those produced by expression vectors can also be used. In designing a suitable ribozyme expression vector the following factors are important to consider. The final ribozyme must be kept as small as possible to minimize unwanted secondary structure within the ribozyme. A promoter (e.g., the human cytomegalovirus immediate early region promoter or keratin promoters from human keratin genes) should be chosen to be a relatively strong promoter, and expressible both *in vitro* and *in vivo.* Such a promoter should express the ribozyme at a level suitable to effect production of enough ribozyme to destroy a target RNA, but not at too high a level to prevent other cellular activities from occurring (unless cell death itself is desired).

A hairpin at the 5' end of the ribozyme is useful to protect the ribozyme from 5'-3' exonucleases. A selected hairpin at the 3' end of the ribozyme is useful since it acts as a protection from 3'-5' exonucleases. The T7 RNA polymerase termination signal is about 30 nucleotides in length (Fig. 31D) and may serve as a good hairpin structure to stabilize ribozymes from exonuclease digestion, although other 3' hairpins of shorter length can be used to provide RNA stability. Such hairpins can be inserted within the vector sequences to allow standard ribozymes to be placed in an appropriate orientation and expressed with such sequences attached.

Poly(A) tails are also useful to protect the 3' end of the ribozyme. These can be provided by either including a poly(A) signal site in the expression vector (to signal a cell to add the poly(A) tail *in vivo*), or by introducing a poly(A) sequence directly into the expression vector. In the first approach, the signal must be located to prevent unwanted secondary structure formation with other parts of the ribozyme. In the second approach, the poly(A) stretch may reduce in size over time when expressed *in vivo,* and thus the vector may need to be checked over time. Care must be taken in addition of a poly(A) tail which binds poly(A) binding proteins which prevent the ribozyme from acting upon their target sequence.

### High Yield Ribozyme Production

There follows a description of a method for expression of a ribozyme in a bacterial or eucaryotic cell, and for production of large amounts of such a ribozyme. In general, the method involves preparing multi-copy cassettes encoding a defined ribozyme structure for production of a ribozyme at a decreased cost. A vector is produced which encodes a plurality of ribozymes which are cleaved from an RNA transcript produced from the vector by another ribozyme. The system is useful for scaling up production of a ribozyme, which may be either modified or unmodified, *in situ* or *in vitro.* Such vector systems can be used to express a desired ribozyme in a specific cell, or can be used in an *in vitro* system to allow production of large amounts of a desired ribozyme. The vectors allow a higher yield synthesis of a ribozyme in the form of an RNA transcript which is cleaved *in situ* or *in vitro* before or after transcript isolation.

The stability of the ribozyme produced in this method can be enhanced by provision of sequences at the termini of the ribozymes as described herein.

The method is advantageous since it provides high yield synthesis of ribozymes by use of low cost transcription-based protocols, compared to existing chemical ribozyme synthesis, and can use isolation techniques currently used to purify chemically synthesized oligonucleotides. Thus, the method allows synthesis of ribozymes in high yield at low cost for analytical, diagnostic, or therapeutic applications.

The method is also useful for synthesis of ribozymes *in vitro* for ribozyme structural studies, enzymatic studies, target RNA accessibility studies, transcription inhibition studies and nuclease protection studies, much is described herein.

The method can also be used to produce ribozymes *in situ* either to increase the intracellular concentration of a desired therapeutic ribozyme, or to produce a concatameric transcript for subsequent *in vitro* isolation of unit length ribozyme. The desired ribozyme can be used to inhibit gene expression in molecular genetic analyses or in infectious cell systems, and to test the efficacy of a therapeutic molecule or treat afflicted cells.

Thus, in general, the vector includes a bacterial, viral or eucaryotic promoter within a plasmid, cosmid, phagmid, virus, viroid or phage vector. Other vectors are equally suitable and include double-stranded, or partially double-stranded DNA, formed by an amplification method such as the polymerase chain reaction, or double-stranded, partially double-stranded or single-stranded RNA, formed by site-directed homologous recombination into viral or viroid RNA genomes. Such vectors need not be circular. Transcriptionally linked to the promoter region is a first ribozyme-encoding region, and nucleotide sequences encoding a ribozyme cleavage sequence which is placed on either side of a region encoding a therapeutic or otherwise desired second ribozyme. Suitable restriction endonuclease sites can be provided to ease construction of this vector in DNA vectors or in requisite DNA vectors of an RNA expression system. The desired second ribozyme may be any desired type of ribozyme, such as a hammerhead, hairpin or other catalytic center, and can include group I and group II introns, as discussed above. The first ribozyme is chosen to cleave the encoded cleavage sequence, and may also be any desired ribozyme, for example, a hepatitis delta virus sequence, a *Tetrahymena* derived ribozyme, which may, for example, include an imbedded restriction endonuclease site in the center of a self-recognition sequence to aid in vector construction. This endonuclease site is useful for construction of the vector, and subsequent analysis of the vector.

When the promoter of such a vector is activated an RNA transcript is produced which includes the first and second ribozyme sequences. The first ribozyme sequence is able to act, under appropriate conditions, to cause cleavage at the cleavage sites to release the second ribozyme sequences. These second ribozyme sequences can then act at their target RNA sites, or can be isolated for later use or analysis.

Thus, the vector includes a first nucleic acid sequence (encoding a first ribozyme having intramolecular cleaving activity), and a second nucleic acid sequence (encoding a second ribozyme having intermolecular cleaving enzymatic activity) flanked by nucleic acid sequences encoding RNA which is cleaved by the first ribozyme to release the second ribozyme from the RNA transcript encoded by the vector. If desired, the first ribozyme may be encoded on a separate vector and may have intermolecular cleaving activity.

As discussed above, the first ribozyme can be chosen to be any self-cleaving ribozyme, and the second ribozyme may be chosen to be any desired ribozyme. The flanking sequences are chosen to include sequences recognized by the first ribozyme. When the vector is caused to express RNA from these nucleic acid sequences, that RNA has the ability under appropriate conditions to cleave each of the flanking regions and thereby release one or more copies of the second ribozyme. If desired, several different second ribozymes can be produced by the same vector, or several different vectors can be placed in the same vessel or cell to produce different ribozymes.

Preferably, the vector includes a plurality of the nucleic acid sequences encoding the second ribozyme, each flanked by nucleic acid sequences recognized by the first ribozyme. Most preferably, such a plurality includes at least six to nine nucleic acid sequences. In other preferred embodiments, the vector includes a promoter which regulates expression of the nucleic acid encoding the ribozymes from the vector; and the vector is chosen from a plasmid, cosmid, phagmid, virus, viroid or phage. More preferably, the plurality of nucleic acid sequences are identical and are arranged in sequential order such that each has an identical end nearest to the promoter. If desired, a poly(A) sequence adjacent to the sequence encoding the first or second ribozyme may be provided to increase stability of the RNA produced by the vector; and a restriction endonuclease site adjacent to the nucleic acid encoding the first ribozyme is provided to allow insertion of nucleic acid encoding the second ribozyme during construction of the vector.

A method for formation of a ribozyme expression vector includes providing a vector including nucleic acid encoding a first ribozyme, as discussed above, and providing a single-stranded DNA encoding a second ribozyme, as discussed above. The single-stranded DNA is then allowed to anneal to form a partial duplex DNA which can be filled in by a treatment with an appropriate enzyme, such as a DNA polymerase in the presence of dNTPs, to form a duplex DNA which can then be ligated to the vector. Large vectors resulting from this method can then be selected to insure that a high copy number of the single-stranded DNA encoding the second ribozyme is incorporated into the vector.

A method for production of ribozymes involves providing a vector as described above, expressing RNA from that vector, and allowing cleavage by the first ribozyme to release the second ribozyme.

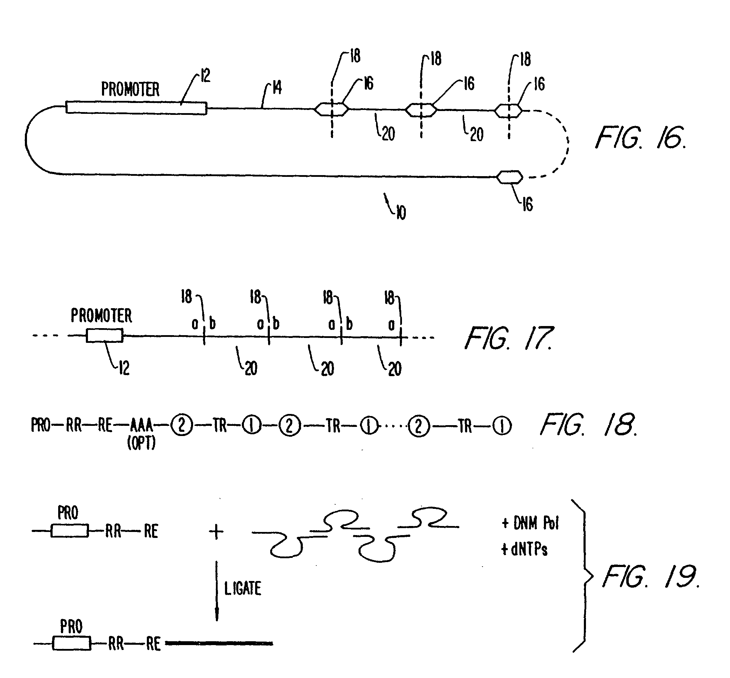

Specifically, referring to Fig. 16, a vector 10 is provided with a promoter 12 which regulates expression of a downstream nucleic acid region 14 encoding a first ribozyme, followed by DNA encoding an RNA cleavage sequence 16 which is recognized by the first ribozyme and can be cleaved by that ribozyme in the corresponding RNA at the location shown by the dashed line 18. Several such sequences 16 are provided in the vector, each of which can be cleaved at the line marked 18. Cleavage at line 18 causes release of a second ribozyme encoded by DNA 20 provided between each of the repeated sequences 16.

Referring to Fig. 17, in a preferred embodiment the repeated regions 20 are provided with identical ends, each of which has the same end (shown as b in the figure) nearest to the promoter 12. In this way, each unit has a sequence a/b, the corresponding RNA of which can be recognized and cleaved at line 18 by the first ribozyme.

Referring to Fig. 18, the vector may be provided with a promoter (PRO) followed by a first releasing ribozyme (RR) and then a restriction endonuclease site (RE). Optionally, there may be provided a poly(A) sequence (AAA) which will provide stability to the releasing ribozyme. Repeating structures are then provided with flanking regions (1 and 2) surrounding a second therapeutic ribozyme (TR).

The actual sequence of the desired second ribozyme unit will depend upon the target it will recognize and cleave. This in turn will have some impact on the acceptable sequence of the cleavage site 18 chosen, as well as the restriction endonuclease site (RE) chosen. In all cases, minimization of any secondary interactions between regions of the RNA transcript encoded by vector 10 will be sought, and other intermediate sequences may be required to space the active components at critical distances to allow the necessary folding of the RNA. An alternative approach includes provision of a second transcription site within the vector, or within another vector, to synthesize other RNA with ribozyme activity which can cause appropriate cleavage of the transcript of a vector of this invention.

The actual cleavage of RNA to free the active desired therapeutic ribozyme may occur within a bacterial or eucaryotic cell in which the vector is grown, or can be accomplished after isolation of the transcript RNAs by addition of critical salt and/or other requisite cofactors. Isolation of unit length therapeutic ribozyme from the RNA mixture can be achieved by a sequence specific affinity chromatography, or charge or other components of the ribozyme may be used in other suitable isolation techniques.

### Example 11:

A preferred arrangement of sequences in the vector of this invention is to have the cleaving ribozyme-encoding region 5' in the vector, and the therapeutic second ribozyme-encoding region 3' in the vector (as shown in Figs. 16-18). Each sequence encoding a second ribozyme is surrounded by flanking sequences encoding cleavage sites. Any number of such flanking sequences can be added. Other flanking structures, such as the poly(A) sequence shown in Fig. 18, can be used and flexibility may be increased by provision of useful restriction endonuclease sites, such as AU rich restriction endonuclease sites, which would give regions convenient for cloning that would not have extensive secondary structure at physiological temperatures in cells or *in vitro* extracts. The minimization of extensive RNA structure in regions flanking the therapeutic ribozyme are essential to optimizing the catalytic activity of the ribozyme. It is possible, for example, to provide the desired second ribozyme encoding sequence flanked by a chosen restriction endonuclease site. DNA encoding such a sequence can then be readily ligated to other such sequences to provide the plurality of repeated sequences desired in a DNA construct. In this sort of procedure, flanking inserts with two different restriction endonuclease sites make the construction of the vector easier.

For example, *Hin*dIII/*Sal*I fragments encoding a releasing ribozyme for a desired ribozyme (VAHR36) are cloned into a plasmid pTZ19U (equivalent vectors are known in the art). The sequences of these fragments are: 5'-CCCAAGCTTGTCGCCTGATGAGGTCCGAAAGGA    Primer RRHS 3'-CTCCAGGCTTTCCTGGCTTTCTGGCAGCTGCCC     RRSH These will give a clone fragment after restriction endonuclease treatment, and insertion into the vector, having the following sequence: 
AGCTTGTCGCCTGATGAGGTCCGAAAGGACCGAAAGACCGTCGA
HindIII                                    SalI
\_______________________________________/
releasing ribozyme
 This segment encodes a releasing first ribozyme surrounded by *Hin*dIII/*Sal*I restriction endonuclease sites. This releasing ribozyme will cleave flanking sequences in a concatamer having the following sequence: 
ACGGUCUCGCGUCU
↑cleaved base
 The desired second ribozyme, VAHR36, has the following sequence: 
GCGUCUCUGAUGAGGUCCGAAAGGACCGAAACGGUCU [C]
Flanking                                Flanking ↑
Region                                  Region    extra base
                                                  after concatamer
                                                  cleavage
 which has the two flanking sequences GCGUCU and ACGGUCU. Two primers used to create a DNA insert encoding this sequence are synthesized having the following sequences: 
ACGGTCTCGCGTCTCTCATGAGGTCCGAAAGGACCGAAACGGTCT
(primer TRS (Sense))
 
[G]AGACCGTTTCGGTCCTTTCGGTCCTCATCAGAGACGCGAGACCGT
(primer TRC (Complement))
 These two primers can be used for concatamer formation as shown in Fig. 18. Hybridization of the primers, synthesis of the second DNA strand to make double-stranded inserts and insertion of these fragments into a suitable vector can be accomplished by methods known to others in the art. If desired, the sequence GGGGTCG can be added to the 5' end of the TRS primer to allow cloning into the *Sal*I site, and GGGGGTACC added to the 5' end of the TRC primer to allow cloning into a *Bam*HI site of pTZ19U or other suitable vector. These primers can be combined as discussed above to allow synthesis of the desired ribozyme vector. Similar constructs can be obtained for RNA vectors by utilizing these methods to make expression vectors, which will be used to synthesize the appropriate RNA or DNA molecules for homologous recombination of ribozyme sequences into the viral, viroid or phage vectors.

If delivery of the constructed vector to a eucaryotic cell is contemplated, cellular splicing machinery within the eucaryotic cell can be incorporated to cleave out the multimeric therapeutic second ribozyme by encoding recognition sequences for the ribozyme segments within the flanking sequences of the expressed transcript. Multiple copies of the releasing first ribozyme may be provided to enhance release of the desired or therapeutic second ribozyme if turnover rate is slower than the degradation rate of the therapeutic second ribozyme. Thus, for these vectors cytoplasmic signals or poly(A) tails may be provided to enhance transport to an appropriate cellular compartment. In bacterial cells, *in vitro* modifications and certain cell modifications can be enhanced by provision of appropriate sequences within the vector, and are useful to enhance ribozyme turnover rate, cleavage activity, and nuclease stability.

### Example 12:

Referring to Fig. 19, a preferred method for formation of a vector of the present invention is to provide a promoter sequence linked to a releasing first ribozyme (RR) with an appropriate restriction endonuclease site (RE). A single-stranded oligonucleotide is then provided which encodes the two flanking regions and a therapeutic second ribozyme. Oligonucleotides such as those designed in Example 1 are allowed to form partial duplexes by hybridization at the flanking regions. The single-stranded regions are then filled in, using a DNA polymerase and dNTPs, to form a double-stranded DNA which can then be ligated to the restriction endonuclease site to form the desired vector.

The preferred method of isolating therapeutic ribozyme is by a chromatographic technique. The HPLC purification methods and reverse HPLC purification methods described herein can be used. Alternatively, the attachment of complementary oligonucleotides to cellulose or other chromatography columns allows isolation of the therapeutic second ribozyme, for example, by hybridization to the region between the flanking arms and the enzymatic RNA. This hybridization will select against the short flanking sequences without the desired enzymatic RNA, and against the releasing first ribozyme. The hybridization can be accomplished in the presence of a chaotropic agent to prevent nuclease degradation. The oligonucleotides on the matrix can be modified to minimize nuclease activity, for example, by provision of 2'-O-methyl RNA oligonucleotides. Such modifications of the oligonucleotide attached to the column matrix will allow the multiple use of the column with minimal oligo degradation. Many such modifications are known in the art, but a chemically stable non-reducible modification is preferred. For example, phosphorothioate modifications can also be used.

The expressed ribozyme RNA can be isolated from bacterial or eucaryotic cells by routine procedures such as lysis followed by guanidine isothiocyanate isolation.

### Example 13:

The current known self-cleaving site of *Tetrahymena* can be used in an alternative vector of this invention. If desired, the full-length *Tetrahymena* sequence may be used, or a shorter sequence may be used. It is preferred that, in order to decrease the superfluous sequences in the self-cleaving site at the 5' cleavage end, the hairpin normally present in the *Tetrahymena* ribozyme should contain the therapeutic second ribozyme 3' sequence and its complement. That is, the first releasing ribozyme-encoding DNA is provided in two portions, separated by DNA encoding the desired second ribozyme. For example, if the therapeutic second ribozyme recognition sequence is CGGACGA/CGAGGA, then CGAGGA is provided in the self-cleaving site loop such that it is in a stem structure recognized by the *Tetrahymena* ribozyme. The loop of the stem may include a restriction endonuclease site into which the desired second ribozyme-encoding DNA is placed.

If desired, the vector may be used in a therapeutic protocol by use of the systems described by Lechner, "Regulation of Nucleic Acid Translation", International Patent Publication WO 92/13070, to allow a timed expression of the therapeutic second ribozyme, as well as an appropriate shut off of cell or gene function. Thus, the vector will include a promoter which appropriately expresses enzymatically active RNA only in the presence of an RNA or another molecule which indicates the presence of an undesired organism or state. Such enzymatically active RNA will then kill or harm the cell in which it exists, as described by Lechner *id.,* or act to cause reduced expression of a desired protein product.

A number of suitable RNA vectors may also be used in this invention. The vectors include plant viroids, plant viruses which contain single or double-stranded RNA genomes and animal viruses which contain RNA genomes, such as the picornaviruses, myxoviruses, paramyxoviruses, hepatitis A virus, reovirus and retroviruses. In many instances cited, use of these viral vectors also results in tissue specific delivery of the ribozymes.

### Cloning of a Concatameric Ribozyme Construct

The following is one specific example showing the cloning of multiple therapeutic ribozymes (TRs) within a transcription unit. The release of the TRs from the transcript necessitates the cloning of an additional releasing ribozyme (RR) which can cleave between TR units in the nascent transcripts.

To test the concatameric ribozyme vector construction, we chose a ribozyme targeted to the HSV-1 ICP4 mRNA. This ribozyme (RPI 1197, same as VAHR36) was chosen because it has been extensively characterized enzymatically using both short and long RNA substrates (K_{cat}/Kₘ = 4 x 10⁷ and 3 x 10⁴ mole⁻¹/min⁻¹, respectively) and has been used in tissue culture studies to inhibit the replication of herpes simplex virus. These data afford us important catalytic and therapeutic values to which we can compare ribozyme activities observed with a concatameric vector.

After choosing the TR, RR must be designed which is capable of cleaving tail-to-head concatamers of the TR. As shown in Fig. 48, the sequence of RPI 1197 made design of a RR very easy because insertion of one nucleotide (A) between the concatamers gave a good ribozyme target site. We have found that extension of the arms of hammerhead ribozymes by one or two nucleotides does not reduce the catalytic activity significantly (usually <30%). This allows some flexibility in designing the TR monomers to accommodate extra nucleotides which optimize the sequence of the RR cleavage site. If the sequence cannot be manipulated using nucleotides present in the gene, it is preferable to place a UM sequence at the 3' end of the TR, where M = C, U or A. This modification of the sequence will give a UM trinucleotide of unpaired bases when the TR hybridizes to target RNA. Unpaired nucleotides at the 3' termini of hammerhead ribozymes usually reduce catalytic activity only when they force the ribozyme into altered structures which are more stable than the enzyme-substrate complex. A preliminary check to detect such unfavorable structures can be performed using computer-based RNA folding algorithms.

If additional sequences such as cytoplasmic localization signals or polyadenylation signals are desired in the TR, these sequences should be considered when designing the RR. Our cloning protocol may be used to incorporate these structures at either terminus of the TR, but we have found that many additional sequences interfere with proper folding of the TR. Exceptions to this observation are the sequences which have strong internal base-pairing, such as hairpin loops or tRNA motifs.

After deciding upon the sequence of the RR motif, it is preferable but not necessary to determine if the RR will cleave the tail-to-head concatameric TR junction. This may be accomplished by synthesizing substrate and ribozyme RNA, as described previously, then performing cleavage reactions in the presence of 10 mM Mg⁺². After verifying the catalytic activity of the RR, the RR motif is cloned into an appropriate vector which contains multiple cloning sites 3' of a phage promoter. For this cloning step, we chose to use PTZ19R (USB). By using a directional cloning protocol, the screening required consists of restriction digests of the isolated plasmid DNA. Without a directional protocol, the small size of the RR inserts would necessitate sequencing of the plasmid DNA to choose correct clones, except in the rare cases where the stem I or stem III regions (see Fig. 48) contain a convenient, diagnostic restriction endonuclease recognition site.

Cloning of the RR fragment into PTZ19R used the two oligonucleotides, 5'-CCCAAGCTTGTCGCCTGATGAGGTCCGAAAGGA-3' and 5'-CCCGTCGACGGTCTTTCGGTCCTTTCGGACCTC-3', which will create the RR coding sequence flanked by *Hin*dIII and *Sal*I restriction endonuclease sites (bold faced type) at the 5' and 3' termini, respectively. Four microgram samples of each oligonucleotide were added to a reaction mixture containing 4 µl of 10x SEQUENASE buffer, 4 µl of 2.5 mM dNTP, 4 µl of 0.1 M DTT, and 1.5 µl of SEQUENASE polymerase (20U, USB) in a total volume of 40 µl. After the mixture was incubated at 37°C for 1 hour, a 5 µl aliquot was removed and added to 5 µl of loading buffer prior to electrophoresis in 10% polyacrylamide gels. Bands were visualized by staining with Stains-all (USB). The remainder of the sample was frozen at -20°C. After verification that the appropriate fragment was generated, 5 µl of the frozen mixture was added to a 15 µl mixture containing 10 U each of *Sal*I and *Hin*dIII restriction endonucleases, 1.3 x restriction endonuclease buffer, and 10 U of shrimp alkaline phosphatase (USB) and the final mixture was incubated at 37°C for 1 hour before phenol/chloroform/isoamyl alcohol extraction and precipitation of the DNA with ethanol. The pellet was dissolved in water and 30 ng of the insert DNA fragment was added to 300 ng of PTZ19R DNA fragment which had been digested with *Hin*dIII and *Sal*I enzymes then gel isolated in low melting point agarose. This plasmid (PTZ19R):insert concentration gave an approximate molar ratio of 1:10, respectively. The DNA samples were added to ligation buffer and ligated with T4 DNA ligase (USB) at room temperature for 2 hours, then at 4°C for 18 hours. One-half of the ligation mixture (8 µl) was transfected into 100 µl of competent DH5α-*E. coli* cells (GIBCO/BRL) and plated onto LB agar plates containing ampicillin. Six clones were picked from the plates and grown in 10 ml overnight preparations for the extraction and restriction enzyme digestion of the plasmid DNA. The insertion of the RR DNA was verified by the increase in plasmid DNA size when digested with either *Hin*dIII or *Sal*I enzymes and by the disappearance of the *Pst*I site which was replaced in the PTZ19R vector with the RR coding DNA. A 250 ml preparation of bacteria transformed with the new RR-containing plasmid was grown and the plasmid DNA isolated was digested with *Sal*I and *Bam*HI restriction enzymes prior to use as the vector for subsequent cloning of the concatameric TR inserts.

Four oligonucleotides were synthesized for use in the TR concatamer cloning. The oligos were: TRS 5'GCGTCTCTGATGAGGTCCGAAAGGACCGAAACGGTCTA TRC 5'CATCAGAGACGCTAGACCGTTTCGG TRSS 5'GGGTCGACGCGTCTCTGATGAGGTCCGAAAGGACCGAAACGGTCTA TRCB 5'GGGGATCCCATCAGAGACGCTAGACCGTTTCGG where the bold faced sequences in TRSS and TRCB are the restriction endonuclease recognition sequences for the *Sal*I and *Bam*HI enzymes, respectively. The oligonucleotides, in a 5:1:0.1:0.1 molar ratio of TRS (5 µg):TRC:TRSS:TRCB, respectively, were added to a mixture containing 15 U of SEQUENASE polymerase and 1x SEQUENASE buffer in a total volume of 15 µl and incubated at 37°C for 2 hours. To optimize the fill-in reaction, another 10 U of SEQUENASE polymerase was added after the first hour of incubation. The mixture was diluted to 150 µl and desalted by spin chromatography through G-50 SEPHADEX columns and reconcentrated to a volume of approximately 15 µl by lyophilization in a Speed-Vac. Nine microliters of the DNA mixture was transferred to another tube containing 1 µl of 10x ligase buffer and 2 U of T4 DNA ligase and incubated at 37°C for 2 hours to ligate gapped DNA pieces. Two microliters of 10x *Sal*I digestion buffer, 1 µl (5U) each of *Sal*I and *Bam*HI enzyme and 4 µl of water were added and the mixture was incubated at 37°C for 3 hours. After the restriction digestion, the mixture was subjected to electrophoresis in 0.8% agarose gel. The electrophoresis was performed until the bromphenol blue marker was 1.5 inches into the gel. This separated the cleaved end fragments from the concatameric DNA fragments. After electrophoresis the gel lane containing the DNA was cut from the area just above the bromphenol blue dye band to the area approximately 1 mm below the bottom of the gel loading area. The DNA was electroeluted from the lane and concentrated by precipitation with ethanol. This protocol gives a variety of concatameric sizes which can also be size selected from the agarose gel, if desired. Alternatively, the insert size can be selected after the concatamers are cloned into the RR vector DNA.

Concatameric DNA was cloned into the RR-containing vector described above by mixing the eluted concatameric DNA with a gel-purified fragment of the RR vector which had been digested previously with BamHI and *Sal*I restriction enzymes. The molar ratio of the vector DNA to insert DNA (as calculated from the original TR concentrations) used in our experiments was 1:100. This gives approximately a 1:2 ratio if the concatamer size averages 2.3 kb. We have found this ratio to give multiple inserts of concatamer and recommend that the ratio be increased to contain at least a 3:1 ratio of vector:insert. We have used this protocol to clone fragments of concatamer which are between 1200 and 1500 nucleotides in length, as judged by restriction analysis and transcription of *Bam*HI linearized DNA fragments.

After the concatameric cassette is developed, the "self"-cleavage of the TR concatamer by the RR can be checked by incubation of ³²P-labeled, in vitro transcribed ribozyme in the presence of 10 mM Mg⁺² as described previously. After appropriate times, aliquots should be analyzed by gel electrophoresis. A successful digestion reaction is exemplified by the disappearance of full-length transcript and accumulation of monomer or multimer length ribozymes over time. After verification of RR activity in vitro the cassette can be excised from the plasmid vector and inserted into a eucaryotic expression vector for intracellular analyses of ribozyme transcription and stability and the cleavage activities of the RR and TR can be assessed.

We have found it preferable to limit the size of the TRC and TRCB oligonucleotides because longer annealing arms decrease the extent of concatamer formation. This cloning protocol may be modified for use with any ribozyme motif, but use of larger inserts limits the number of ribozymes which may be encoded by any transcript because of limitations upon the size of the encoding DNA which may be effectively introduced into cells. If one desires to use multiple ribozymes in the cassettes, then separate mini-cassettes containing the TR concatamer (or monomer) and a corresponding RR should be prepared for incorporation into an expression vector. Use of different 5' and 3' restriction endonuclease sites flanking the individual cassettes would facilitate the cloning into vectors. Because the efficiency of ribozyme release within a cell will depend upon the intracellular salt concentrations, it is preferable to use the same ribozyme motif for the RR as is used for the TR. In this manner, both ribozymes will be active in the same cell types. A preliminary assay of ribozyme activity in cellular extracts is recommended to identify the best cleaving motif for a given target cell type.

### Other Vectors

In designing suitable ribozyme expression vectors the following factors are important to consider.

The final ribozyme must be kept as small as possible to minimize unwanted secondary structure within the ribozyme and to prevent binding of protein factors which may bind to longer RNAs. A promoter (e.g., the CMV immediate early promoter) should be chosen to be a relatively strong promoter to which RNA polymerase binds with a high binding constant (K_{B} > 10⁸ M⁻¹) and rapidly enters the active mode for transcription (rate > 10⁻² sec⁻¹). Such a promoter should express the ribozyme at a level suitable to effect production of enough ribozyme to destroy a target RNA, but not at too high a level to prevent other cellular activities from occurring (unless cell death itself is desired).

A hairpin (i.e., an RNA molecule which base pairs with itself) at the 5' end of the ribozyme is useful to protect the ribozyme from 5'-3' exonucleases. A selected hairpin at the 3' end of the ribozyme is useful to protect from digestion by 3'-5' exonucleases. Such hairpins can be inserted within the vector sequences to allow standard ribozymes to be placed in an appropriate orientation and expressed with such sequences attached.

Poly(A) tails are also useful to protect the 3' end of the ribozyme. This can be done by either including a poly(A) signal site in the expression vector (to signal a cell to add the poly(A) tail *in vivo*), or by introducing a poly(A)-coding sequence directly into the expression vector. In the first approach the signal must be located to prevent unwanted secondary structure formation with other parts of the ribozyme. For polyadenylation, the sequence AAUAA must be present in the correct context at/near the 3' end of the transcript in order to be cleaved by a specific eucaryotic endonuclease. Poly(A) polymerase then adds A residues (about 250) onto the 3' end of the transcript. In the second approach, the poly(A) stretch may reduce in size over time when expressed *in vivo,* and thus the vector may need to be checked over time. Care must be taken in addition of a poly(A) tail which binds poly(A) binding proteins which prevent the ribozyme from acting, or which transport the ribozyme from the nucleus. Such selected transport may be advantageous for certain ribozyme applications.

The correct combination of 5' and 3' protecting groups can be determined without actually making expression vectors of this invention. This is advantageous because it takes time to produce such vectors. Thus, the hairpins can be added to the ribozyme by either chemical RNA synthesis or by transcription using the system described by Milligan et al., 15 Nucleic Acids Research 8783, 1988. Poly(A) tails can be added directly to the ribozyme using poly(A) polymerase or polynucleotide phosphorylase.

### Preparation of Purified Ribozymes

The following is a method for preparation of pure enzymatically active ribozymes (of size between 28 and 70 nucleotide bases) in sodium, potassium or magnesium salt form by a two step purification method. Generally the method is applicable to both synthetically and enzymatically produced ribozymes, and entails use of high performance liquid chromatography (HPLC) techniques on reverse phase columns. Unlike gel purification, HPLC purification as described in this application can be applied to virtually unlimited amounts of purified material. This allows generation of kilogram quantities of ribozymes in each purification batch.

Thus, the method involves purification of an enzymatic RNA molecule of 28-70 nucleotide bases by passing that enzymatic RNA molecule over a high pressure liquid chromatography column. Surprisingly, applicant has discovered that enzymatically active ribozymes can be purified in the desired salt form by the described HPLC methodology.

Preferably, the method includes passing the enzymatically active RNA molecule over a reverse phase HPLC column; the enzymatically active RNA molecule is produced in a synthetic chemical method and not by an enzymatic process; and the enzymatic RNA molecule is partially blocked, and the partially blocked enzymatically active RNA molecule is passed over a reverse phase HPLC column to separate it from other RNA molecules.

More preferably, the enzymatically active RNA molecule, after passage over the reverse phase HPLC column is deblocked and passed over a second reverse phase HPLC column (which may be the same as the reverse phase HPLC column), to remove the enzymatic RNA molecule from other components. In addition, the column is a silica or organic polymer-based C4 or C8 column having a porosity of at least 125 Å, preferably 300 Å, and a particle size of at least 2 µm, preferably 5 µm.

Pure ribozyme in a Na⁺, K⁺, or Mg²⁺ salt form is produced. By "pure" is meant that the ribozyme is preferably provided free of other contaminants, and is at least 85% in the desired salt form.

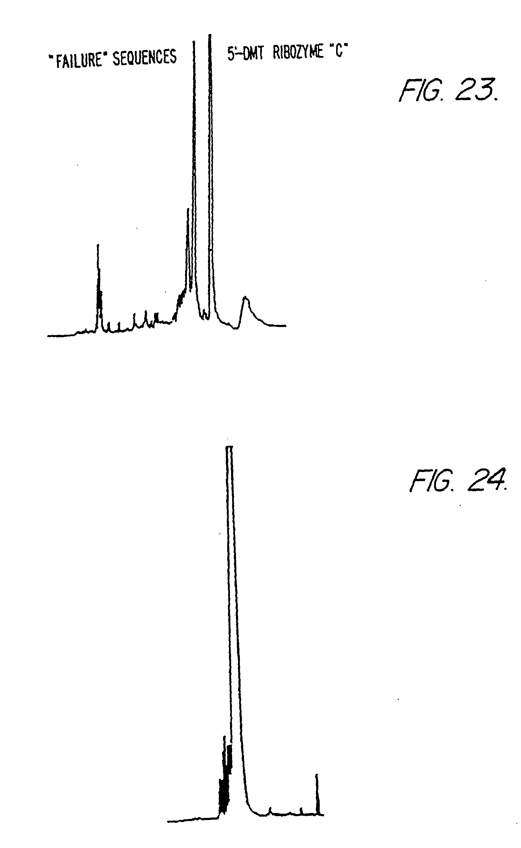

The method generally features HPLC purification of ribozymes. An example of such purification is provided below in which a synthetic ribozyme produced on a solid phase is blocked. This material is then released from the solid phase by a treatment with methanolic ammonia, subsequently treated with tetrabutylammonium fluoride, and purified on reverse phase HPLC to remove partially blocked ribozyme from "failure" sequences (Fig. 23). Such "failure" sequences are RNA molecules which have a nucleotide base sequence shorter to that of the desired enzymatic RNA molecule by one or more of the desired bases in a random manner, and possess free terminal 5'-hydroxyl group. This terminal 5'-hydroxyl in a ribozyme with the correct sequence is still blocked by lipophilic dimethoxytrityl group. After such partially blocked enzymatic RNA is purified, it is deblocked by a standard procedure, and passed over the same or a similar HPLC reverse phase column to remove other contaminating components, such as other RNA molecules or nucleotides or other molecules produced in the deblocking and synthetic procedures (Fig. 24). The resulting molecule is the native enzymatically active ribozyme in a highly purified form.

Below is provided one example of such a method. This example can be readily scaled up to allow production and purification of gram or even kilogram quantities of ribozymes.

### Example 14:

In this example solid phase phosphoramidite chemistry was employed for synthesis of a ribozyme. Monomers used were 2'-*tert*-butyl-dimethylsilyl cyanoethylphosphoramidites of uridine, N-benzoyl-cytosine, N-phenoxyacetyl adenosine, and guanosine (Glen Research, Sterling, VA).

Solid phase synthesis was carried out on either an ABI 394 or 380B DNA/RNA synthesizer using the standard protocol provided with each machine. The only exception was that the coupling step was increased from 10 to 12 minutes. The phosphoramidite concentration was 0.1 M. Synthesis was done on a 1 mmole scale using a 1 mmole RNA reaction column (Glen Research). The average coupling efficiencies were between 97% and 98% for the 394 model and between 97% and 99% for the 380B model, as determined by a calorimetric measurement of the released trityl cation.

After synthesis, the blocked ribozymes were cleaved from the CPG support, and the bases and diphosphoester moiety deprotected in a sterile vial by incubation in dry ethanolic ammonia (2 mL) at 55°C for 16 hours. The reaction mixture was cooled on dry ice. Later, the cold liquid was transferred into a sterile screw cap vial and lyophilized.

To remove the 2'-*tert*-butyl-dimethylsilyl groups from the ribozyme the obtained residue was suspended in 1 M tetra-n-butylammonium fluoride in dry THF (TBAF), using a 20-fold excess of the reagent for every silyl group, for 16 hours at ambient temperature. The reaction was quenched by adding an equal volume of a sterile 1 M triethylamine acetate, pH 6.5. The sample was cooled and concentrated on a SpeedVac to half the initial volume.

The ribozymes were purified in two steps by HPLC on a C4 300 Å 5 µm DeltaPak column in an acetonitrile gradient.

The first step, or "trityl on" step, was a separation of 5'-DMT-protected ribozyme(s) from failure sequences lacking a 5'-DMT group. Solvents used for this step were: A (0.1 M triethylammonium acetate, pH 6.8) and B (acetonitrile). The elution profile was: 20% B for 10 minutes, followed by a linear gradient of 20% B to 50% B over 50 minutes, 50% B for 10 minutes, a linear gradient of 50% B to 100% B over 10 minutes, and a linear gradient of 100% B to 0% B over 10 minutes.

The second step was a purification of a completely deblocked ribozyme by a treatment of 2% trifluoroacetic acid or 80% acetic acid on a C4 300 Å 5 µm DeltaPak column in an acetonitrile gradient. Solvents used for this second step were: A (0.1 M Triethylammonium acetate, pH 6.8) and B (80% acetonitrile, 0.1 M triethylammonium acetate, pH 6.8). The elution profile was: 5% B for 5 minutes, a linear gradient of 5% B to 15% B over 60 minutes, 15% B for 10 minutes, and a linear gradient of 15% B to 0% B over 10 minutes.

The fraction containing ribozyme, which is in the triethylammonium salt form, was cooled and lyophilized on a SpeedVac. Solid residue was dissolved in a minimum amount of ethanol and ribozyme in sodium salt form was precipitated by addition of sodium perchlorate in acetone. (K⁺ or Mg²⁺ salts can be produced in an equivalent manner.) The ribozyme was collected by centrifugation, washed three times with acetone, and lyophilized.

### Preferred Targets

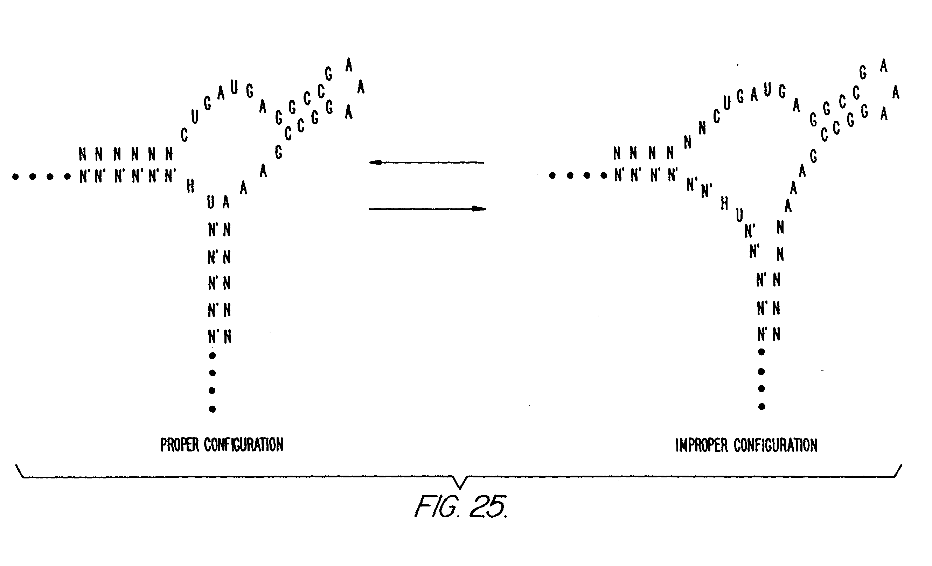

An improved method for designing active hammerhead ribozymes is provided herein. Such ribozymes are chosen to include sequences which form "strong" base-pairing interactions at the positions closest to the internal loop formed by the ribozyme-substrate complex (parameters for sequence-specific duplex stability are found in Freier *et al.*, "Improved Parameters for Predictions of RNA Duplex Stability", 83 Proc. Natl. Acad. Sci. USA 9373, 1986). Thus, such ribozymes have a substrate sequence XXUH/XX (where XX are "strong" duplex forming bases, H is U, A or C, and the slash represents the cleavage site). "Strong" duplex forming bases are chosen to be those which have dinucleotide free energies of helix propagation of -2.0 kcal/mole or less (i.e., GA, UC, GU, AC, CG, GC, GG, CC; see, Freier *et al.*). Such ribozymes are more active than other ribozymes having "weak" duplex forming bases at the positions XX. These ribozymes are more efficient at cleaving the substrate RNA molecules since the ribozyme is able to bind to the substrate tightly enough to allow the catalytic components to be positioned for cleavage, but is still weakly enough bound to allow the reaction to be driven to completion by dissociation of the product RNA. Provision of strong ribozyme-substrate interactions at these positions allows for maximal cleavage activity by forming a rigid structure close to the central loop of the ribozyme. This prevents the interior portions of stems I and III of the ribozyme from separating prematurely due to local thermal fluctuations. Fig. 25 is a diagrammatic representation of the equilibrium between a tightly constrained structure ("Proper configuration") and an undesirable loose structure ("Improper configuration"). The method of this invention is to select ribozymes and substrates which maximize the proper configuration.

Stem III is limited in this regard since the innermost base-pair must be AU with the U in the substrate. Stem I, however, has no such constraints and ribozymes can be chosen which include "strong" duplex forming bases immediately adjacent to the internal loop.

Thus, the method for providing a more active hammerhead ribozyme includes modifying the ribozyme to have "strong" duplex forming bases at the variable positions closest to the internal loop.

In addition, a method for selecting a substrate or target site at which a hammerhead ribozyme will be more active is provided. The method includes the step of selecting a substrate site having the sequence XXUH/XX (where XX are "strong" duplex forming bases, H is U, A or C, and the slash represents the cleavage site).

### Example 15: HIV-1 and HSV Targeted Ribozymes

The following is data obtained from various ribozymes demonstrating the desirability of ribozymes having strong duplex forming bases at the variable positions closest to the internal loop.

Tables 3 and 4 contain sequence and activity information for ribozymes designed against targets found in the HIV-1 and HSV-1 genomes. Activities (as kcat/k_{M}) were determined by incubating 2-100 nM ribozyme with approximately 1 nM radioactively labeled, substrate for different times in 75 mM Tris-HCl pH 8.0, 10 mM MgCl₂. The products are separated by denaturing polyacrylamide electrophoresis and quantitated by a beta emission scanner (Ambis Systems, San Diego, CA). The data are treated per Michaelis-Menton for excess enzyme, specifically, the negative log of the fraction substrate remaining is plotted as a function of time. The resulting slope is then plotted as a function of ribozyme concentration. The slope of this line is then kcat/k_{M}.

Ribozymes which meet the strong base-pair criteria for stem I are marked with a "I" in the left margin. The average activity of these ribozymes is 43 x 10⁶/M·min, which is above the average for ribozymes not meeting this criteria (23 x 10⁶/M·min). Ribozymes which meet the strong base-pair criteria for stem III are marked with a "III" in the left margin. The average activity of these ribozymes is 28 x 10⁶/M·min, and for those not meeting this criteria is 8 x 10⁶/M·min. Six ribozymes have the appropriate strong inner bases in both stems. Their average activity is 37 x 10⁶/M·min.

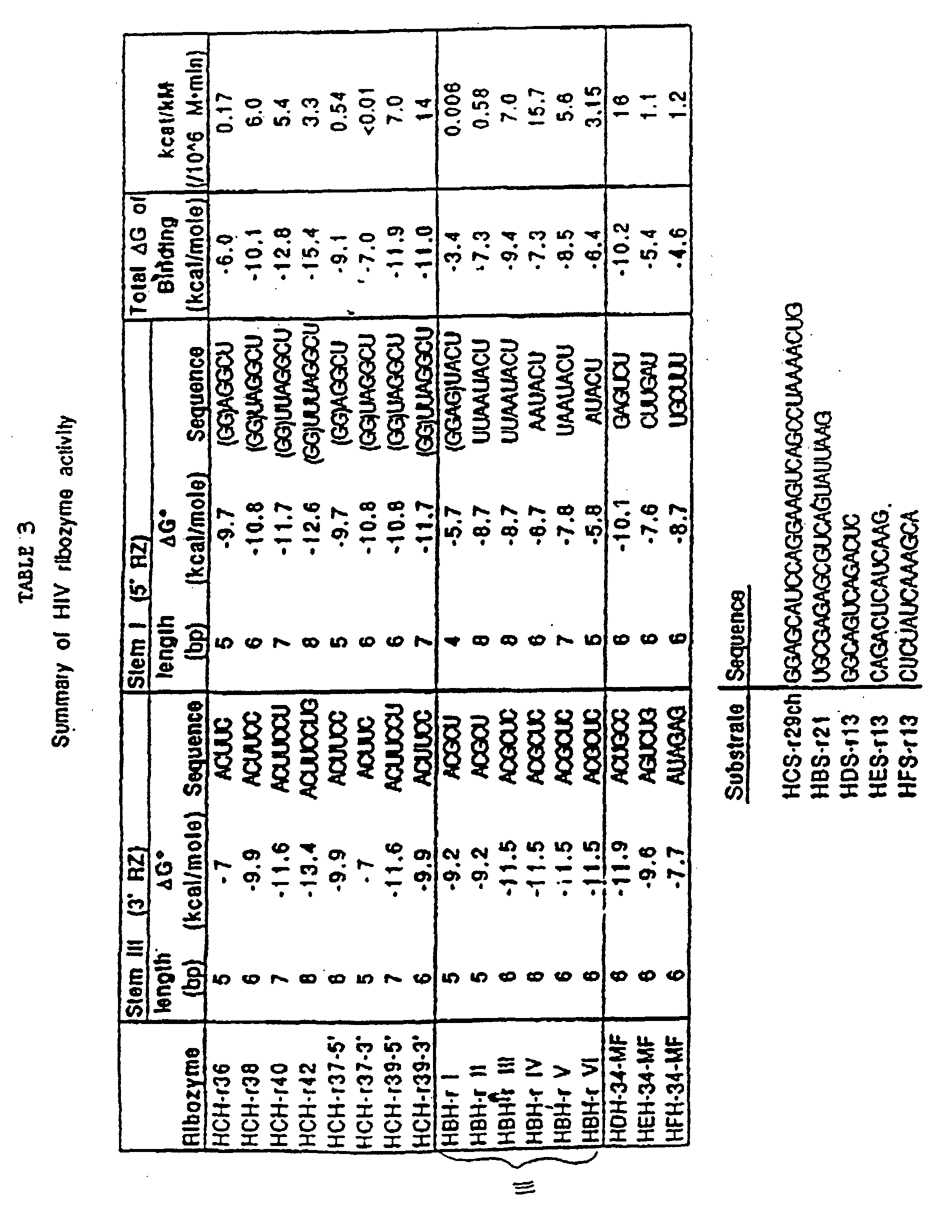

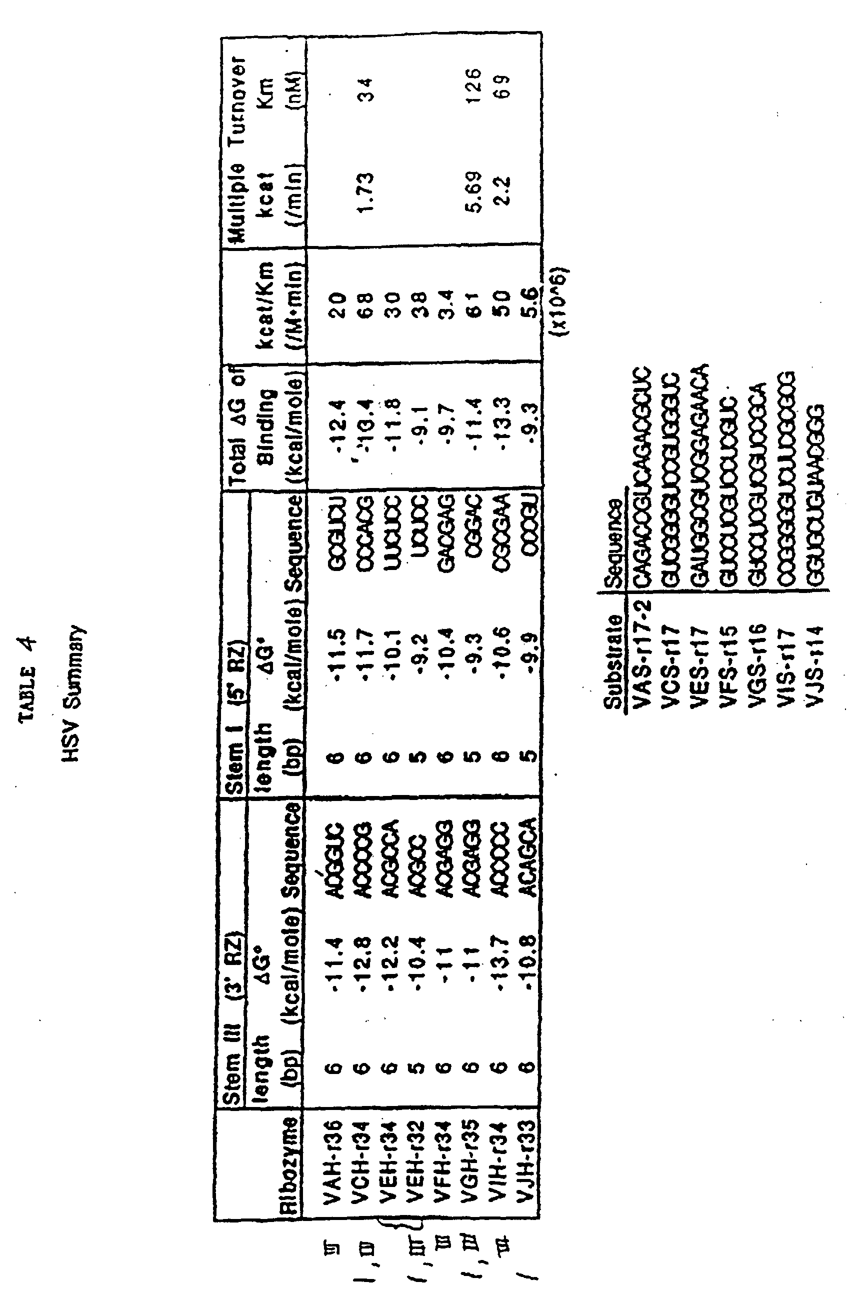

### Arm Lengths for Optimal Enzyme Activity

A rapid assay for determining optimum length of ribozyme/substrate interaction regions is provided. Applicant has determined that it is important to know the optimum length of the interaction regions, e.g., stem I & stem III, of a hammerhead ribozyme targeted against a specific RNA sequence. Similarly, it is important to determine such parameters for other ribozymes, such as hairpin ribozymes, and those based upon the hepatitis delta virus structure.

Generally, a series of ribozymes that vary in the length of these regions has been made, the ribozymes purified, and kinetic assays performed on such ribozymes to determine the best configuration. Synthesis and testing of three ribozymes takes about two weeks. Thus, testing many different target sites with 6-8 ribozymes per site becomes a lengthy and expensive proposition.

Applicant has discovered that a more rapid determination of optimum arm lengths can be achieved by evaluation of many different arm-length combinations in one set of experiments. It is difficult to assay multiple ribozymes (targeted to the same substrate) in a single experiment because it is hard to distinguish which ribozyme is responsible for each cleavage event. However, it is possible to make a nested set of substrate RNAs that differ in the number of base-pairs that are made with a single ribozyme. This reverse experiment provides approximately the same results as the desired experiment (changing the length of the ribozyme) as long as the ribozyme used is long enough to interact with the longest substrate. Preferably, neither the ribozyme nor the longest substrate form alternative structures by themselves. Applicant recognizes that the interaction free energies between a long ribozyme and a truncated substrate will not be identical to the interaction free energies for long substrates and truncated ribozymes, even if the base-pairs formed are the same and there are no competing secondary structures. This is because the terminal stacks contribute from 0 to -1.7 kcal/mole to the interaction free energy, and the difference in interaction free energies can be as much as 2.0 kcal/mole. Thus, it may be necessary to make and test a few ribozymes with arms of varying lengths to ensure that these differences are not overlooked. The number of ribozymes that will need to be made and tested, however, will be significantly less than that number when each different ribozyme must be synthesized rather than each substrate.

Thus, a method is provided for assaying optimal arm lengths in an enzymatic RNA molecule by contacting the molecule with a plurality of substrate RNAs. Each substrate RNA differs from another in the length or number of base pairs which each substrate RNA forms with the molecule. The method is performed under RNA substrate cleaving conditions, and the rate of cleavage of the substrate RNAs is determined.

Preferably, the ribozyme is a hammerhead, hairpin or hepatitis delta virus ribozyme; each substrate RNA has an identical 5' or 3' end; and the kinetics of cleavage of each substrate is determined.

By "kinetic assays" or "kinetics of cleavage" is meant an experiment in which the rate of cleavage of target RNA is determined. Often a series of assays are performed in which the concentrations of either ribozyme or substrate are varied from one assay to the next in order to determine the influence of that parameter on the rate of cleavage.

By "rate of cleavage" is meant a measure of the amount of target RNA cleaved as a function of time.

By "nested set of RNA" is meant a set of RNA molecules having a common sequence but varying in length through truncation of the sequence from either the 3' or 5' ends (or both). The set of sequences AU, AUG, AUGC, AUGCG, AUGCGA defines a nested set RNA sequences having common 5' ends, while AUGCGA, UGCGA, GCGA, CGA, GA defines a nested set of RNA sequences having common 3' ends.

By "ribozyme/substrate interaction regions" is meant sequences within a ribozyme which are intended to bring ribozyme and target RNA together through complementary base-pairing interactions; e.g., ribozyme sequences within stems I and III of the hammerhead ribozyme make up the ribozyme/substrate interaction regions (see Fig. 29).

A method is also provided for production of a set of RNA substrates. This involves synthesizing an RNA molecule with a limiting level of phosphoramidite or using shortened coupling times under conditions in which RNA sequences are capped when no nucleotide base is added at a given step.

### Example 16:

In one example, a set of substrates is synthesized by separate chemical synthesis of each individual substrate. Because test substrates are shorter in length than ribozymes, this approach uses less reagent and time than synthesis of an equivalent set of ribozymes and is consequently less expensive. Synthesis of shorter RNA molecules is also more efficient and the resulting products are easier to purify.

### Example 17:

A time and cost efficient way to generate a nested set of substrates is to use standard chemical synthesis with high coupling efficiency for the initial portion of a synthesis, and then switch to low efficiency coupling for synthesis of the last few (5-9) nucleotides of the substrate RNA. Low efficiency coupling can be achieved by lowering the concentration of phosphoramidites or by decreasing the time allowed in the coupling reaction. This method is outlined in McSwiggen, *supra.* The end result is a set of substrates sharing a common 3' end but that differ in length at the 5' end.

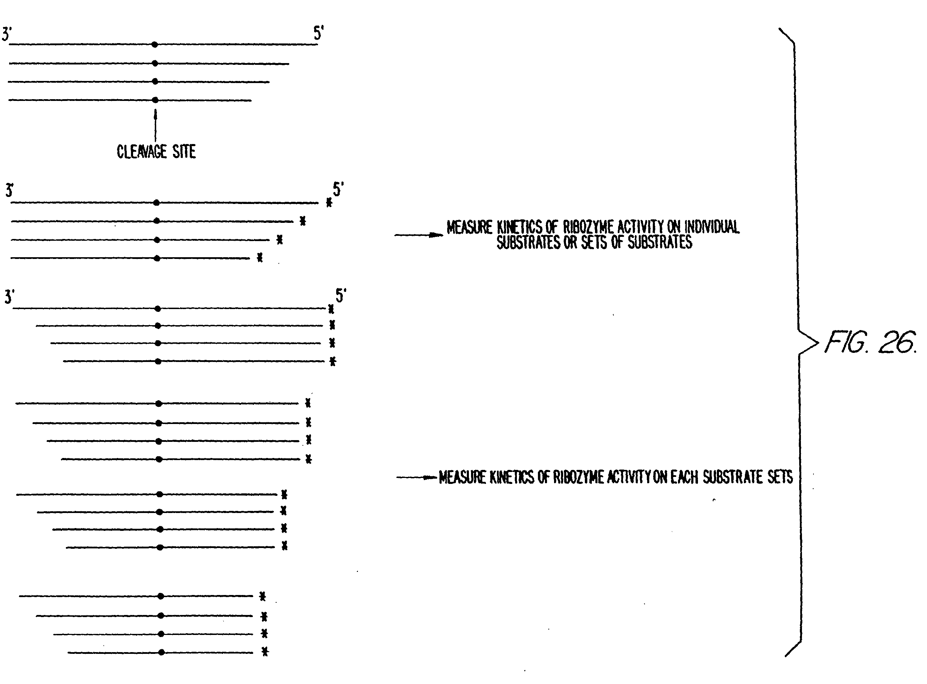

After chemical synthesis of the set of RNAs, individual RNAs of the set can be isolated (e.g., by gel-purification). The isolated RNAs are then 5' end-labeled with [γ-³²P]ATP and T4 polynucleotide kinase. To generate molecules in which the 3' end is also truncated, the individual 5' end-labeled substrates are subjected to limited base hydrolysis or exonuclease treatment and those substrates in which 0 to 5 nucleotides have been removed from the 3' end are gel-purified. This procedure yields sets of RNAs that have a common 5' end, but are shortened on their 3' end. This general scheme is outlined in Fig. 26.

Together these two procedures, chemical synthesis with low efficiency coupling during 5' end synthesis and subsequent base hydrolysis or exonuclease treatment of isolated RNAs, yields a full set of RNA substrates with a common central sequence but with all possible combinations of truncations in the last 5-9 nucleotides at both ends. Since only 'one' chemical synthesis is performed, this method of generating a full set of substrates is greatly advantageous over chemical synthesis of each individual substrate. Less time and reagent is required to generate the full set of substrates to be tested.

### Example 18:

The substrates are tested in a standard kinetic assay to determine which one provides the best substrate for a particular ribozyme (see Fig. 27). Each substrate is mixed with a constant amount of ribozyme and incubated over a particular time course. The resulting products are electrophoresed on a denaturing polyacrylamide gel and the extent of cleavage determined for each time point. Kinetic analysis reveals on which substrate the ribozyme is most active.

### Example 19:

The individual substrates need not be separated from each other in order to be tested. Rather, a set of substrates that, for example, differ only in length at their 5' end can be 5' end-labeled with [γ-³²P]ATP and T4 polynucleotide kinase. Incubation of this set of 5' labeled substrates with a ribozyme allows the same kinetic analysis as incubation with a single substrate. The cleavage products generated also vary at their 5' end, and thus will be distinguishable from each other and the full-length molecule on a denaturing polyacrylamide gel (see Fig. 28). This method of kinetic analysis of entire sets of substrates also works with substrates that have a common 5' end, but are different at their 3' end. The latter can be 3' end-labeled with [³²P]-cordycepin and poly(A) polymerase or [³²P]-labeled pCp and T4 RNA ligase using standard methods.

### Example 20:

The method includes determination of the maximum allowable length for the ribozyme substrate interaction. The candidate is folded using a standard computer program, e.g., one described by Zucher, 244 Science 48, 1989, along with the full-length ribozymes and substrate oligonucleotides, and examined for secondary structures which might affect activity. The length of the ribozyme/substrate interaction is adjusted to avoid such secondary structures. Full-length ribozyme is made and purified substrate oligonucleotides containing 5' truncations are synthesized, e.g., on an ABI synthesizer. A nested set of substrate RNAs containing the same 3' end but differing 5' ends can be produced by reducing the phosphoramidite concentration and ensuring that failure sequences get fully capped (see McSwiggen, *supra*). The 5' truncated substrates are labeled at their 5' end with [γ-³²P]ATP and T4 polynucleotide kinase and gel purified. Excess ATP is used to ensure complete 5' phosphorylation so that the resulting RNA does not contain unlabeled n+1 substrate, where N is the desired length. Alternatively, the set of RNA molecules may be gel purified and then 5' end labeled with [γ-³²P]ATP.

The 5' truncated substrates are checked by RNA sequencing to ensure that the expected RNA has been obtained. 3' truncated substrates are generated for each 5'-truncated substrate by limited base-hydrolysis or 3' nuclease digestion (e.g., Nuclease P). The base-hydrolysis reaction is neutralized and used directly in kinetic reactions, or 3' truncated substrates are gel purified for more detailed analysis.

Kinetics are then analyzed in terms of fraction of initial substrate remaining vs. time, since in this case the product band will be the same for a given set of 3' truncated substrates that are ³²P-end-labeled at their common 5' end.

### Synthesizing Sets of RNA Molecules

Applicant has recognized that a 1 µmole scale standard chemical synthesis of a ribozyme produces a lot of ribozyme compared to the amount needed to carry out initial activity tests. Thus, applicant has devised a method by which such syntheses can be optimized to produce multiple ribozymes or other RNA molecules in a single synthesis. This allows tremendous savings of both material costs and purification time.

Test sets of ribozymes are routinely made that are targeted to the same target sequence but which contain varying lengths of substrate-binding arms. Using the method, sets of ribozymes containing variable-length 5' substrate-binding arms can be chemically synthesized. In one embodiment, this is achieved by simply reducing the concentration of phosphoramidites used during the last few steps of RNA synthesis (i.e., near to the 5' end) in order to promote production of completed sequences of varying lengths at each synthetic step.

Thus, a method for synthesis of a set of enzymatic RNA molecules each member of the set differing in length is provided. In the method, synthesis of the molecules entails use of a first concentration of reagent (preferably in excess) and employment of an incubation time sufficient to allow at least 95% coupling efficiency per nucleotide position. After a few synthesis steps (e.g., during synthesizing of the 5' substrate binding region) a second concentration of reagent is used which is insufficient to allow the high coupling efficiency. Alternatively, the time allotted to each coupling step is reduced such that incomplete coupling is achieved.

Preferably, the limiting reagent is phosphoramidite or time is made limiting; the limiting concentration or time is sufficient to allow between 80-90%, or even 70-80% coupling efficiency; at least 3 (most preferably 5-9) different RNA molecules are synthesized which differ in length by at least one nucleotide base each, and those RNA molecules have different 5' ends (see Fig. 29).

### Example 21:

Referring to Fig. 29, the following is one example of this invention. Those in the art will recognize that equivalent strategies can be used to attain an equivalent result.

Two RNA synthesizers (or two sets of reagents on the same synthesizer) are set up, one contains the standard concentrations of phosphoramidites (e.g., 0.1 M), while the other contains phosphoramidites at a lower concentration designed to give only 80% coupling efficiency under standard synthesis conditions. Such a concentration can be determined empirically for each synthesizer or set of reactants.

RNA synthesis is started with the standard concentrations of phosphoramidites and continued until the RNA sequence position at the 5' end where failure sequences are desired.

The RNA synthesis columns are transferred at this point to the second synthesizer and synthesis continued for up to seven more positions at 80% coupling efficiency per position. A set of ribozymes containing n, n-1, n-2, n-3, n-4, n-5, n-6 nucleotides at the 5' end will be generated in concentrations of 26%, 7%, 8%, 10%, 13%, 16% and 20% of the total, respectively. Capping is done by standard procedure (ABI Bulletin 53:1-7 (1988)).

The RNA in each column is deprotected and desalted in the usual manner. Small failure sequences can be discarded if desired by gel purification, but n, n-1, n-2, n-3, n-4, n-5, and n-6 RNAs are not separated from each other.

A base composition analysis is run on the desalted RNAs (one base-composition analysis will suffice for up to 7 RNAs). Individual RNAs are purified using either ion exchange HPLC or gel purification. In some instances, purification of individual RNAs may not be necessary as the entire set can be utilized together. If separation of n from n-1 is difficult, synthesis can be extended one more nucleotide so that the relatively large amount of full-length RNA (26% in this case) is more easily separated from the largest desired failure sequence (at 7% in this case).

Purified ribozymes are then labeled at the 5' end with [γ-³²P] ATP using T4 polynucleotide kinase. The ³²P-labeled ribozyme is then checked for purity and sequenced to ensure that the desired sequence has been obtained.

### Example 22:

A more uniform distribution of n, n-1, n-2, n-3, n-4, etc. RNAs can be obtained if the coupling efficiency is adjusted at each of the n-4 through n coupling steps. The most direct way of varying coupling efficiency at will is to change the coupling time while keeping the phosphoramidite concentration constant. The standard coupling reaction employs 0.1 M phosphoramidites and a coupling time of 12-15 minutes to obtain 97-99% coupling efficiency. By reducing the phosphoramidite concentration, a longer time will be required to obtain the same level of coupling (0.1 M phosphoramidite is about 20-fold molar excess over growing RNA chains in the standard reaction; it is important that the phosphoramidite concentration not be dropped below a 1:1 molar ratio with the growing RNA chains). Fig. 30 shows a hypothetical situation in which the phosphoramidite concentration is reduced sufficiently to require 43 minutes to obtain 99% coupling efficiency. The coupling efficiency is assumed to follow a curve of the form${\text{Coupling Efficiency = 1-3}}^{\text{-k(coupline time)}}$

In such a case, the coupling efficiency would be 50% at 6.5 minutes, 66.7% at 10.3 minutes, 75% at 12.9 minutes, and 80% at 15 minutes. If 5 RNAs are desired in equal concentrations then the coupling times and yields could be achieved as shown below:

| RNA Length | Coupling Time(min) | Coupling Efficiency | RNA Available For Extension(%) | RNA Not Extended (i.e.capped & terminated) |
|---|---|---|---|---|
| n-4 | - | - | 100% | 20% |
| n-3 | 15 | 80% | 80% | 20% |
| n-2 | 12.9 | 75% | 60% | 20% |
| n-1 | 10.3 | 66.7% | 40% | 20% |
| n | 6.5 | 50% | 20% | 20% |

### Improved Ribozymes

One or more stabilizing RNA hairpins may be provided to a hammerhead or hairpin ribozyme, to provide an improved ribozyme. These RNA hairpins provide protection from RNases within a cell with minimal increases in the toxicity of the RNA molecule. These additions are readily added to existing or designed ribozymes, since they can simply be appended to the RNA sequence making up a ribozyme. Such hairpins may be added either by chemical synthesis, by gene transcription, or by RNA ligation procedures. A 5' hairpin protects the ribozyme from 5'-3' exonucleases and a 3' hairpin can be chosen to protect from digestion by 3'-5' exonucleases.

Thus, the hammerhead or hairpin ribozyme may have a 5' or 3' RNA hairpin which does not significantly reduce the enzymatic activity of the ribozyme.

By "hairpin" is meant a nucleotide base sequence of RNA that may be ligated or otherwise attached to one end of a hammerhead ribozyme. This RNA will base pair with itself to form a folded structure, commonly referred to as a hairpin. This structure may also fold onto nearby ribozyme sequences so long as such folding does not reduce the activity of the ribozyme significantly. Such a hairpin may be spaced from one end of the ribozyme by provision of one, two or more non-pairing nucleotide bases.

Preferred hairpins include GC rich stems with stable tetranucleotide loops (e.g., GAAA, UUCG, GNRA or UNCG where N = A, U, C or G and R = A or G); most preferred are hairpins including a sequence selected from GGCCGAAAGGCC, GCGCUUCGGCGC, and GGAGGAAACUCC, with the hairpin consisting of between a 4 and 20 base pair stem and between a 4 and 7 nucleotide loop.

Hairpins can be readily added to 5' or 3' ends of a hammerhead ribozyme. Any particular hammerhead or hairpin ribozyme may be chosen and its stability enhanced by provision of such a hairpin. The optimum hairpin for any particular ribozyme may differ, but enhanced stability can be provided by use of one or more universal hairpins. Such universal hairpins can be chosen based upon an analysis of the kinetics of enzymatic activity, and the stability of a ribozyme using standard assays.

Initially, in choosing an optimal hairpin, computer folding of various ribozyme-hairpin combinations will indicate whether a chosen hairpin will promote misfolding of the ribozyme. Such hairpins should be avoided. Design of the hairpins which do not cause misfolding is readily performed. Steric hindrance of the folding of the ribozyme should be avoided as well, or the enzymatic activity of the ribozyme may be reduced.

In a model system, the length of the hairpin and the length of non-pairing spacer sequences between the ribozyme substrate binding site or sequence and the hairpin may be varied to determine which hairpin or non-pairing spacer sequence provides optimum stability, without significantly reducing enzymatic activity. Hairpins which promote *in vivo* binding to cellular factors should be avoided since these may lead to a reduction of ribozyme activity. Such a possibility may be assayed using a routine assay in which a cellular extract is added to an assay mixture to determine ribozyme activity in the presence of such a cellular extract. Thus, for example, recognition sequences, such as the tat recognition sequence, should be avoided in the design of such hairpins.

After selection of a hairpin which does not significantly diminish the enzymatic activity of the ribozyme when provided at the 3' or 5' end, the stability of the ribozyme can be tested by addition of specific RNases using a standard assay or by testing in cell extracts. Larger hairpins are more effective at stabilizing ribozymes against exonuclease attack, but shorter hairpins are more readily synthesized and are less likely to reduce ribozyme activity. Thus, the optimum size of such a hairpin will need to be discovered empirically for any chosen ribozyme.

There follows examples of a procedure for assaying for a suitable 3' or 5' hairpin. These examples are not limiting in the invention and those of ordinary skill in the art will recognize that many equivalents to these examples exist.

### Example 23:

A ribozyme having strong enzymatic activity such as one termed HSV, ICP4 site E (shown in Fig. 31A) was selected as a model ribozyme. Referring to Figs. 32A-F, the core ribozyme was synthesized, and other modifications of the ribozyme synthesized using standard procedures. These included the core ribozyme (Fig. 32A), core ribozyme including a minimal 3' hairpin (e.g., GCGCGAAAGCGG, Fig. 32B), a core ribozyme having the same minimal hairpin except including a one nucleotide non-pairing spacer between the ribozyme sequence and the hairpin (Fig. 32C), a core ribozyme including a minimal 5' hairpin which includes the T7 transcription start sequence (but avoiding uracil nucleotides in the first nine nucleotides to minimize abortive initiation, e.g., GGACGAAAGUCC, Fig. 32D), the core ribozyme with a 5' hairpin which includes the T7 transcription start sequence but with a one nucleotide spacer between the hairpin sequence and the ribozyme (Fig. 32E), and a core ribozyme flanked on its 5' side with the hairpin which includes the T7 transcription start site, a one nucleotide spacer and on its 3' side with the 3' hairpin and a one nucleotide spacer. Other hairpins, or any combination of the above variations which- might be readily synthesized can also be tested and used. All of these ribozymes can then be tested for their enzymatic activity *in vitro* (shown in Figs. 32A-F) and then *in vivo,* and then tested for their stability using routine assays such as those provided below.

### Example 24: Enzymatic Assay

To test the effect on activity *in vitro* small stem-loops to the termini of a ribozyme, the ribozymes shown diagrammatically in Figs. 32A-F were made and tested for cleavage activity on a short (17 mer) synthetic substrate.

Activities (as kcat/k_{M}) were determined by incubating 2-100 nM ribozyme with approximately 1 nM radioactively labeled substrate for different times in 75 mM Tris-HCl pH 8.0, 10 mM MgCl₂. The products were separated by denaturing polyacrylamide electrophoresis and quantitated by a beta emission scanner (Ambis Systems, San Diego, CA). The data were treated per Michaelis-Menton for excess enzyme, specifically, the negative log of the fraction substrate remaining was plotted as a function of time. The resulting slope was then plotted as a function of ribozyme concentration. The slope of this line is kcat/k_{M}.

These ribozyme constructs all have activities within a factor of 2. This demonstrates that the addition of terminal stem-loops is not inhibitory to the activity of this ribozyme.

These ribozymes were further tested for activity *in vitro* against a long (496 nucleotide) T7 transcript containing the target site. In this experiment, 4 µM of each ribozyme was incubated with 47 nM body-labeled transcript in the above-mentioned buffer for various times. The products were separated by a 5% denaturing gel and visualized by autoradiography. The result showed that cleavage by each ribozyme (except that in Fig. 32C which was shown to be degraded prior to the experiment) occurred at virtually identical rates.

### Example 25: Stability Assay

Ribozyme constructs such as those described in Example 23 and Figs. 32A-F can be tested for stability against ribonuclease degradation in cellular extracts. The ribozyme construct is first labeled (e.g., with ³²P). This can be accomplished by incubation with [γ-³²P]ATP and T4 polynucleotide kinase to label the 5' end, incubation with ³²P-cordycepin and poly(A) polymerase to label the 3' end, or by inclusion of a [³²P]NTP during the transcription reaction if the construct is such that it can be made by *in vitro* transcription. Various concentrations of ³²P-labeled ribozyme are then incubated with cellular extracts. Aliquots are removed at various times, mixed with standard stop buffer containing EDTA and formamide, and loaded onto a denaturing polyacrylamide gel. Full-length ribozymes are distinguished from smaller degradation products by size. Alternatively, complete degradation is followed as a disappearance of ³²P-labeled full-length ribozyme.

The modified ribozymes of this invention may be formed using a DNA template which is transcribed or chemically synthesized using standard procedures. Such ribozymes can be synthesized for RNase experiments in which the ribozymes are labeled and tested in stability assays using cell extracts.

### Method for Forming Active Ribozymes

Once a ribozyme or enzymatic RNA molecule has been designed to a particular target RNA site, it is often useful to modify various nucleotide bases in the ribozyme to determine those bases which can be modified without loss of enzymatic activity, and those bases which can be modified to enhance enzymatic activity. Individual modification of each base position in a ribozyme is a slow method for determining those bases in the RNA molecule which are suited for such modifications. This invention features a method by which rapid screening of various modifications can be achieved in a random or non-random manner.

In this method, the ribozyme is synthesized such that it contains chemical modifications at a given base or linkage in such a manner that that chemical modification can be later detected. The ribozyme is synthesized so that it is linked to or contains a substrate portion, such that the ribozyme may act in an intramolecular manner to cleave the substrate in *cis,* to generate at least two RNA fragments. In this way, the activity of each modified ribozyme can be tested by incubating the modified ribozymes *in vitro* under conditions which allow intramolecular cleavage to occur. Any modification which prevents the ribozyme from causing such cleavage will result in the substrate remaining bound to that ribozyme molecule during such an incubation. Such bound substrate molecules are readily separated from those in which cleavage has occurred, and the position of the modified base or bases in the cleaved or uncleaved RNA molecules can be determined using standard technology.

Thus, the method involves synthesis of an enzymatic RNA molecule in which the ribozyme molecule is formed with a modified nucleotide base or a non-nucleotide base which can be detectably located relative to unmodified nucleotide bases. The RNA molecule is formed with a substrate portion which the enzymatic portion of the RNA molecule can cleave in an enzymatic manner.

By "unmodified nucleotide" is meant one of the bases adenine, cytosine, guanosine, uracil joined to the 1' carbon of β-D-ribo-furanose. The sugar also has a phosphate bound to the 5' carbon. These nucleotides are bound by a phosphodiester between the 3' carbon of one nucleotide and the 5' carbon of the next nucleotide to form RNA.

By "modified nucleotide/RNA" is meant to include nucleotides or RNA which have modifications of: (1) the base moiety (e.g., 2-Bromo-uracil); (2) the sugar moiety (e.g., 2'-O-methyl); or (3) the phosphate moiety (e.g., thiophosphates).

Preferably, the method involves synthesis of a hammerhead, hairpin or hepatitis delta virus (HDV) ribozyme with the substrate attached to the 3' or 5' end (for example, in a manner that allows end-labeling of the products of the enzymatic reaction), in which intramolecular cleaving of the ribozyme and substrate is achieved. For example, the HDV may include an extraneous sequence (not normally associated with the HDV ribozyme) on the 5' side of the cleavage site so that active or inactive ribozymes can be separated from each other dependent on size.

More preferably, the chemical modification involves use of thiophosphate. A modified base linkage formed in this manner can be detected by preferential cleavage with hydrogen peroxide or periodate cleavage, by protection from digestion with snake venom phosphodiesterase, as well as some base specific RNases. Such cleavage allows location of the modified base(s) relative to non-modified bases.

The bases may be modified by use of a methyl phosphonate, and can be cleaved and thereby detected by use of base specific RNases; or the bases are modified by use of 2'-O-methyl, 2'-fluoro, 2'-amino nucleotides or 2'-deoxynucleotides. The position of these modifications can be detected by resistance to base hydrolysis.

In addition, a method is provided for screening for nucleotide positions in a ribozyme which can be modified without eliminating ribozyme activity. The method involves providing a chemically synthesized ribozyme having a substrate attached in a manner that allows intramolecular cleaving of the substrate by the enzymatic portion of the ribozyme, and synthesizing the ribozyme such that a modification is made to one or more bases in the ribozyme. That ribozyme is then incubated under intramolecular cleaving conditions, and the location of those modifications which eliminate or have no effect on the intramolecular cleaving reaction determined.

Preferably, the method further features synthesizing further modified ribozymes which may include modifications at those nucleotide positions which were previously found not to affect the intramolecular cleaving activity. This procedure may be repeated until all those positions that can be modified have been identified.

Finally, the modified ribozymes having intramolecular cleaving activity can be tested with or without an attached substrate and compared in their stability and kinetic activity with an unmodified or parental ribozyme molecule. In this way, not only may modification sites be determined which may later be modified in the ribozyme, but also ribozymes which have increased activity due to such modifications can be determined.

### Example 26:

Referring to Fig. 33, an unmodified hammerhead ribozyme having a substrate attached at the 3' end is synthesized and purified using standard procedures (ABI User Bulletins 1988, 47:1-9; 1989, 53:1-7). This synthesis is then repeated in a manner that causes about 3% of the ribozyme bases to be chemically modified during synthesis (to give about one modification per 35 nucleotide residues). Such chemical modification may include the use of thiophosphate or methylphosphonate among other reagents. Thiophosphates may be introduced by limited treatment with 3H-1,2-Benzodithiol-3-one-1,1-dioxide at specific bases during automated synthesis (Iyer et al., 11 JACS 121, 1990).

The ribozyme-substrate population of molecules is then 5' end labeled and incubated under RNA cleavage conditions and both the products and the starting material are gel purified to isolate intramolecularly-cleaved ribozymes from non-cleaved ribozymes. Base positions which prevent intramolecular cleaving are determined by comparing the reacted and unreacted ribozyme populations by using a technique that is sensitive to the presence of the chemical modification, for example, for thiophosphate, periodate, snake venom phosphodiesterase, or base specific RNases can be utilized. For methyl phosphonate, a base specific RNase can be used. For 2'-O-methyl, 2'-fluoro, 2'-amino, or 2'-deoxy modifications base hydrolysis can be used.

The above procedure can be repeated by synthesizing a ribozyme in such a manner that each ribozyme position has about a 6 to 20% chance of the bases being chemically modified (i.e., between 2 to 7 modifications per 35 nucleotide ribozyme) and those positions which were found in the previous step to inhibit intramolecular cleaving are not modified. The positions of chemically modified nucleotides which prevent intramolecular cleaving can then be determined as described above. This procedure can be repeated as many times as desired.

The method of making and testing ribozymes with increased levels of modifications at allowed sites, while not modifying at the disallowed sites, will ultimately provide a population of active ribozymes that are fully modified at all allowed sites and completely unmodified at the disallowed sites. These ribozymes can be made and tested with or without substrate attached using standard assays to determine their activity in kinetic experiments as well as their stability *in vitro* and *in vivo*. See, McSwiggen, "Improved Ribozymes", U.S. Serial No. 07/884,521, filed May 14, 1992 and assigned to the same assignee as the present application, the whole of which (including drawings) is hereby incorporated by reference herein.

Catalytic activities of enzymatically active modified ribozymes can next be determined by testing the specific ribozyme with a separate substrate such that the ribozyme can cleave in the separate substrate. Specifically, the modified ribozyme of interest and its substrate are synthesized and purified separately. Single turnover activities are determined by incubating 2-100 nM ribozyme with approximately 1 nM radioactively labeled substrate for different times in 75 mM Tris-HCl pH 8.0, 10 mM MgCl₂. The products are separated by denaturing polyacrylamide electrophoresis and quantitated by a beta emission scanner (Ambis Systems, San Diego, CA). The data are treated per Michaelis-Menton for excess enzyme; specifically, the negative log of the fraction substrate remaining is plotted as a function of time. The resulting slope is then plotted as a function of ribozyme concentration. The slope of this line is then kcat/k_{M}.

These ribozymes can also be tested for stability against endogenous cellular nucleases by incubating end labeled ribozyme in cell extracts. Alternatively, the labeled ribozyme can be delivered to cells in culture or to animals to determine stability. Those ribozymes which are enzymatically active and stable are most useful in therapeutic and other applications.

This section also relates to production of enzymatic RNA molecules or ribozymes having enhanced or reduced binding affinity and enhanced enzymatic activity for their target nucleic acid substrate by inclusion of one or more modified nucleotides in the substrate binding portion of a ribozyme such as a hammerhead, hairpin or hepatitis delta virus derived ribozyme. Applicant has recognized that only small changes in the extent of base pairing or hydrogen bonding between the ribozyme and substrate can have significant effect on the enzymatic activity of the ribozyme on that substrate. Thus, applicant has recognized that a subtle alteration in the extent of hydrogen bonding along a substrate binding arm of a ribozyme can be used to improve the ribozyme activity compared to an unaltered ribozyme containing no such altered nucleotide. Thus, for example, a guanosine base may be replaced with an inosine to produce a weaker interaction between a ribozyme and its substrate, or a uracil may be replaced with a bromouracil (BrU) to increase the hydrogen bonding interaction with an adenosine. Other examples of alterations of the four standard ribonucleotide bases are shown in Figs. 47A-D with weaker or stronger hydrogen bonding abilities shown in each figure.

Thus, a modified ribozyme is provided having one or more substrate binding arms including one or more modified nucleotide bases; and in a related aspect, the invention features a method for production of a more active modified ribozyme (compared to an unmodified ribozyme) by inclusion of one or more of such modified nucleotide bases in a substrate binding arm.

The invention provides ribozymes having increased enzymatic activity *in vitro* and *in vivo* as can be measured by standard assays. Thus, the kinetic features of the ribozyme are enhanced by selection of appropriate modified bases in the substrate binding arms. Applicant recognizes that while strong binding to a substrate by a ribozyme enhances specificity, it may also prevent separation of the ribozyme from the cleaved substrate. Thus, applicant provides means by which optimization of the base pairing can be achieved. Specifically, the invention features ribozymes with modified bases with enzymatic activity at least 1.5 fold (preferably 2 or 3 fold) or greater than the unmodified corresponding ribozyme. The invention also features a method for optimizing the kinetic activity of a ribozyme by introduction of modified bases into a ribozyme and screening for those with higher enzymatic activity. Such selection may be *in vitro* or *in vivo.*

By "enzymatic portion" is meant that part of the ribozyme essential for cleavage of an RNA substrate.

By "substrate binding arm" is meant that portion of a ribozyme which is complementary to (i.e., able to base-pair with) a portion of its substrate. Generally, such complementarity is 100%, but can be less if desired. For example, as few as 10 bases out of 14 may be base-paired. These arms contain sequences within a ribozyme which are intended to bring ribozyme and target RNA together through complementary base-pairing interactions; e.g., ribozyme sequences within stems I and III of a standard hammerhead ribozyme make up the substrate-binding domain (see Fig. 1).

Preferably, the modified ribozyme is a hammerhead, hairpin or hepatitis delta virus derived ribozyme, and the hammerhead ribozyme includes between 32 and 40 nucleotide bases. The selection of modified bases is most preferably chosen to enhance the enzymatic activity (as observed in standard kinetic assays designed to measure the kinetics of cleavage) of the selected ribozyme, i.e., to enhance the rate or extent of cleavage of a substrate by the ribozyme, compared to a ribozyme having an identical nucleotide base sequence without any modified base.

There is a narrow range of binding free-energies between a ribozyme and its substrate that will produce maximal ribozyme activity. Such binding energy can be optimized by making ribozymes with G to I and U to BrU substitutions (or equivalent substitutions) in the substrate-binding arms. This allows manipulation of the binding free-energy without actually changing the target recognition sequence, the length of the two substrate-binding arms, or the enzymatic portion of the ribozyme. The shape of the free-energy vs. ribozyme activity curve can be readily determined using data from experiments in which each base (or several bases) is modified or unmodified, and without the complication of changing the size of the ribozyme/substrate interaction.

Such experiments will indicate the most active ribozyme structure. It is likely that only one or two modifications are necessary since a very small change in binding free energy (even one base-pair interaction) can dramatically affect ribozyme activity; the use of modified bases thus permits "fine tuning" of the binding free energy to assure maximal ribozyme activity. In addition, replacement of such bases, e.g., I for G, may permit a higher level of substrate specificity when cleavage of non-target RNA is a problem.

### Method

Modified substrate binding arms can be synthesized using standard methodology. For example, phosphoramidites of inosine and 5-bromouracil can be used. Generally, a target site that has been optimized for stem I and III lengths (in a hammerhead ribozyme -- other ribozymes can be treated in a similar manner), and that has G and/or U in the ribozyme portion of stem I and III, is selected. Modified ribozymes are made by replacing various G and U residues with I and BrU, respectively, during synthesis of the ribozyme. The modified ribozymes are then tested to determine kinetic parameters using standard procedures. The binding affinities for the ribozymes can also be determined by standard procedures, e.g., by T-melt, gel-binding, or by competition kinetics assays. By comparison of binding affinity and ribozyme activity the optimum binding affinity of a ribozyme can then be found. Other combinations of G, I, U BrU, and other bases can then be tested with nearly identical binding free energy, but different base sequence, to determine whether factors other than simple binding free-energy play a role.

It is preferred to perform routine experiments of this type to select a desired ribozyme substrate binding sequence by use of an unmodified ribozyme with a modified substrate (which contains the modified bases). That is, the reverse experiment to that described above is performed. Such an experiment is more readily performed since the substrate is generally shorter than the ribozyme, and can be readily synthesized without concern about its secondary structure. Thus, a single ribozyme can be tested against a plurality of modified substrates in order to define which of the substrates provides better kinetic results. Once a preferred substrate is identified, the ribozyme can then be modified in a way which mirrors the selected substrate, and then tested against an unmodified substrate.

Such experiments will define useful ribozymes of this invention in which one or more modified bases are provided in the substrate binding arms with greater enzymatic activity *in vitro* and *in vivo* than comparable unmodified ribozymes. Such modifications may also be advantageous if they increase the resistance of a ribozyme to enzymatic degradation *in vivo.*

### In Vivo Selection of Ribozyme Targets

This method is for constructing and selecting ribozymes, and in particular, amplifying vectors which express ribozymes in tissue culture systems. These ribozymes are selected for their ability to cleave a given target nucleic acid (e.g., RNA), and to inhibit the biological function of that molecule or any protein encoded by it. It is not necessary to know either the mRNA sequence of the target mRNA, or the intracellular localization of the ribozyme in order to select for active ribozyme constructs in this cellular system. This *in vivo* screening protocol (i.e., one which takes place inside a cell) offers many advantages over extracellular systems, because the synthesis of large quantities of RNA by enzymatic or chemical methods prior to assessing the efficacy of the ribozyme is not necessary.

Applicant provides an *in vivo* system for selection of ribozymes targeted to a defined RNA target. The system allows many steps in a selection process for desired ribozymes to be bypassed. In this system, a population of ribozymes having different substrate binding arms (and thus active at different RNA sequences) is introduced into a population of cells including a target RNA molecule. The cells are designed such that only those cells which include a useful ribozyme will survive, or only those cells including a useful ribozyme will provide a detectable signal. In this way, a large population of randomly or non-randomly formed ribozyme molecules may be tested in an environment which is close to the true environment in which the ribozyme might be utilized as a therapeutic agent.

Thus, the method is for *in vivo* selection of a ribozyme active at a specific RNA target. In this method, a first population of ribozymes with differing substrate binding arms is introduced into a second population of cells which includes or is later caused to include the RNA target, and those cells which contain a ribozyme active at the target RNA identified.

Preferably, the ribozymes are encoded by an expression vector, and the method includes causing those ribozymes to be expressed from the vector in the cells. More preferably, the identified ribozyme is isolated and tested to determine activity *in vivo;* the RNA target is a viral RNA and the identifying step includes determining cell survival after infection by the viral RNA; the RNA target is a mammalian RNA and the cells are mammalian cells; the vector includes sequences encoding a 5' or 3', hairpin or a poly(A) tail for the ribozymes; the ribozyme is a hammerhead ribozyme with at least one substrate binding arm having between five and eight nucleotides; and the identifying step includes detecting expression of a reporter RNA attached to or regulated by the RNA target.

More preferably, a ribozyme active at the RNA target is recloned into an amphitrophic retrovirus vector, and a retrovirus vector having or encoding the ribozyme is selected for ribozyme activity active at the RNA target.

Also provided is a population of nucleic acid molecules of the formula 5' NCNA 3', wherein each N is, or encodes, a ribozyme substrate binding arm, C is, or encodes, an enzymatic portion of a ribozyme, and A is a polyadenylation sequence; wherein at least one of the Ns in the population differs in each vector.

Preferably, the formula is 5' RNCNAS 3', wherein R and S are restriction endonuclease sites; the formula is 5' RLNCNLALS 3', wherein each L is nothing, or an insertion region which is or encodes a nuclear processing signal, an RNA stability signal, a splicing signal, or another nucleotide signal which effects transport or stability of the ribozyme molecule; the formula is 5' DRLCNLALSE 3', wherein D and E are defined nucleotide sequences which can be used to prime amplification of the nucleic acid molecule, or the complement of which can be used to prime amplification of the molecule.

Also provided is a population of expression vectors each having one member of the population of nucleic acid molecules described above located within each vector, and a population of cells each having one member of the population of expression vectors present within each cell; and a ribozyme expression vector encoding a ribozyme and a hepatitis delta virus ribozyme motif, wherein the motif is able to cleave RNA including the ribozyme.

A method is provided for construction of expression vectors for ribozymes for use in their selection in eucaryotic cells. These vectors are selected for their ability to express inhibitory ribozymes; a pluripotent cell line which is preselected for the maintenance of DNA containing ribozyme expression vectors; a method for the amplification of desired ribozyme-expressing regions, and the use of these regions or their equivalent for the therapeutic, diagnostic or prophylactic administration of ribozymes; and a method for targeting accessible sites within an mRNA which can be treated with either chemically synthesized ribozymes or ribozyme-containing transcripts.

### Ribozyme Population

In general, to practice a method of this invention, it is necessary to generate a population of RNAs that have a high probability of containing the active ribozyme that is sought. It is also necessary to provide a method for selecting such an active ribozyme out of that population of RNAs. If desired, the population of RNAs can be formed with stabilizing structures, such as 3' and 5' hairpins, to protect the RNA molecules from nucleases. In addition, the RNA may be capped or a poly(A) tail provided. The ribozyme to be tested should be as small as possible to ensure that the possibility of misfolding of the ribozyme, or secondary structure formation deleterious to enzymatic activity, is reduced. Thus, the ribozyme should be formed with as little flanking sequence as possible, and any such flanking sequences should be provided in a form that ensures that it forms a hairpin at the end of the ribozyme sequence.

The length of a substrate binding arm of the ribozyme should be optimized so that it is not so short that an optimum ribozyme can never be found, and not so long that any extra sequence might fold upon itself and prevent binding. Long substrate binding arms are also less useful because they are more laborious to randomize. A shorter binding arm permits use of a suitable size population of ribozymes to be formed, screened, and tested. Substrate binding arms for a hammerhead ribozyme having between six and eight nucleotides in length is optimal; thus, for a hammerhead ribozyme if the substrate binding arm is eight nucleotides in length, with one invariant nucleotide at the 3' end of stem I, then only 15 positions need to be randomized to provide about 10⁹ ribozyme permutations.

### Vector System

Once the generic structure of the ribozyme is chosen the position and identity of nucleotide variations can be selected by standard procedure. Generally, such ribozymes will be provided in a plasmid or other vector system. For example, such a vector may be a plasmid having the human cytomegalovirus immediate early promoter region to give strong constitutive expression or an inducible promoter to allow control of expression of the ribozyme. The promoter may be followed by a hairpin structure, a ribozyme-encoding DNA sequence and a minimal T7 terminator hairpin to provide stability at the 3' end of the encoded ribozyme transcript. The 3' termination sequences or a hepatitis delta virus "cis-cleaving" ribozyme may be inserted after the 3' terminal hairpin structure to create the 3' terminus of the ribozyme transcript. Such a plasmid may be provided with a suitable restriction endonuclease site(s) to allow ready insertion of a population of random or otherwise synthesized ribozymes at the appropriate location. Care must be taken to ensure that such sequences do not interfere with ribozyme activity, which can be accomplished by including such sequences in a defined hairpin structure. Alternatively, the restriction endonuclease sequences can be omitted and cloning can be achieved by standard PCR techniques.

In order to aid in generation of a population of ribozymes, it may be preferable to make a series of libraries that differ in the length of the substrate binding arms, and then mix those libraries together prior to transformation of a test cell. For example, 16 different libraries can be formed which have randomized substrate binding arms from 4 to 8 nucleotides on each side.

### Selection

The selection strategy may take many forms. Preferably, the RNA that is targeted for cleavage should be in a configuration that is as close to "natural" as possible, so that the natural state of the target site will be accessible. If possible, selection should be carried out against full-length target RNA with no foreign flanking sequence. The target RNA also preferably is expressed in a cell that is its natural environment. For example, if the RNA is a mammalian RNA, a bacterial expression cell should be avoided since it is unlikely to contain the splicing factors and nuclear transport factors that bind to natural target eukaryotic mRNA. The selection system may have to be specifically chosen for each specific RNA target.

If the RNA target is a viral RNA, then the selection system can be devised to select for survival of a host cell after infection by that viral RNA. That is, a population of ribozymes are introduced into a host cell and those cells infected by the virus. Any cell which contains a ribozyme able to cleave the viral RNA will allow that cell to survive and grow. Thus, any growing cell potentially contains a desired ribozyme. It may be difficult to identify which viral gene is being cleaved by the selected ribozyme. While it may be important at some point to identify the target gene, it does not really matter which gene is targeted as long as the ribozyme actually works.

For other target RNAs it is possible to attach a reporter RNA to the end of the target RNA and to select for reduction in reporter-gene activity after ribozyme introduction. That is, a reporter DNA is ligated to DNA encoding the RNA target so that the two RNAs are produced as one RNA molecule. Cleavage of the RNA target can be designed to either activate or inactivate expression of the reporter RNA. If it is activated then a signal is provided in the cell (e.g., an enzyme activity such as β-galactosidase); if it is inactivated then the signal may be cell survival when the RNA encodes a toxic cell product (e.g., cholera toxin). This method permits ribozyme selection for one particular target RNA.

Any selected ribozymes will need to be screened to ensure that the reporter RNA is not the target of the ribozyme attack. In addition, they will need to be screened to ensure that the presence of any reporter RNA did not alter accessibility to the target RNA. That possibility can be readily checked in a subsequent screen involving the target RNA not attached to the reporter RNA.

If the RNA encoded by a regulatory gene is the RNA target, it is possible to place a reporter gene under the control of the target gene product. In that way a ribozyme can be selected against one particular RNA without worry about reporter RNA disrupting target RNA accessibility.

There follows examples useful in this invention by way of illustration. These examples are not limiting in the invention.

### Example 27: Synthesis and Construction of Random Ribozyme Coding Insert

The random ribozyme coding insert can be constructed by any number of methods which are appreciated by those skilled in the art. The following is but one method of synthesis which illustrates various essential and non-essential features of the insert.

The DNA fragments used for the insert are synthesized on an ABI 380B synthesizer according to the protocols supplied by the manufacturer. For the insertion of random nucleotides, a bottle which contain equimolar mixtures of monomers is sequentially sampled and the coupling and deprotection times are increased. These precautions will ensure the addition of the correct numbers of bases at these critical points in the construct. After synthesis, the DNA sequence of the mixture is end-labeled with ³²P and analyzed by Maxam and Gilbert analysis, or primed with radiolabeled complementary fragments which are extended and sequenced using a dideoxy sequencing method.

Referring to Fig. 1, there is shown one example of a ribozyme of the general hammerhead structure. The ribozyme population desired to select a useful ribozyme, however, has a randomized sequence in the regions which base pair with the target, i.e., the substrate binding arms.

The DNA sequence of the random ribozyme coding strand is synthesized first. One example is shown in Fig. 34, where D₁₇ is any defined nucleotide sequence which can be used to give effective priming for amplification of the sense strand of the DNA fragment; E₁₇ is the inverse complement of a defined nucleotide sequence (F₁₇) which can also be used to prime synthesis, but this time primes synthesis of the antisense strand of the fragment; R₆ and S₆ are restriction endonuclease recognition sequences for RE1 and RE2 enzymes, respectively; CUGAUGAGGCCGAAAGGCCGAAA is just one example of a core hammerhead ribozyme sequence for a hammerhead motif ribozyme; N₈ is a random substrate binding arm sequence of 8 nucleotides in length; AAAAAAAAAAAAAAAₙ is a polyadenylation region of undefined length; and each LLL is an optional insertion region which may be used to add nuclear processing signals, RNA stability signals, splicing signals or other such nucleotide signals which may effect the transport or stability of an expressed ribozyme molecule. Terminal sequences are chosen to have minimal homology between the 3' end of D₁₇ and the 5' end of E₁₇. RE1 and RE2 are any restriction endonuclease enzymes required for subsequent cloning procedures. The exact lengths of any of these regions may vary in different constructs, but the general structure and arrangement of the various regions is useful in this invention.

Because the terminal 3' nucleotide of the oligonucleotide is attached to the support for synthesis, it would be preferable to begin with an A-containing CpG bead and then extend the next 8 nucleotides of the 3' random binding arms, proceed with the core ribozyme (enzymatic) sequence, the 8 nucleotides of the 5' random binding arms, a restriction enzyme (RE1) recognition sequence (R₆), and end with a primer sequence (D₁₇) at the 5' terminus. Capping, uncoupling and deprotection would proceed by standard procedures.

The 3' termini of the random ribozyme coding sequences can be polyadenylated using terminal deoxynucleotidyltransferase according to standard protocols. After the addition of the poly(A) tails at the 3' termini of the DNA fragments, the fragments can be partially purified by extraction, heat denatured and annealed to a nucleotide primer composed of the F₁₇ sequence at the 5' end, followed by a predetermined 6 nucleotide RE2 recognition sequence (S₆) and terminating in a (dT)₂₅ sequence.

Second strand synthesis is achieved by the addition of Taq DNA polymerase, appropriate salts and nucleotide substrates, and incubation for 16 minutes at 65°C. The resultant mixture contains double-stranded random ribozyme inserts which are composed of blunt-ended termini. The coding strands of these fragments contain the D₁₇ primer sequence, followed by the RE1 site, the random ribozyme coding sequence, a variable length poly(A) region, the RE2 site and an annealing region at the 3' terminus for the F₁₇ primer. These double-stranded fragments are amplified using Taq polymerase and appropriate DNA primer fragments (D₁₇ and F₁₇) prior to cloning into a desired vector.

Before ligation into the expression vector, the DNA mixture is digested with the restriction enzymes to create fragments containing defined 5' and 3' termini to allow cloning into the vector in a directional manner. The mixture is phenol extracted and purified by column chromatography to desalt and remove undesirable nucleotide fragments which are cleaved from the termini. Alternatively, the termini may contain a blunt end and subsequent cloning into an expression vector can be achieved after treating the DNA fragments with Klenow polymerase according to established protocols.

If desired, the polyadenylation sequence may be omitted, and the restriction enzyme primer or blunt end primer-containing regions complementary to the 3' regions of the random ribozyme coding strands can be used to initiate the synthesis of the second DNA strand. If either of the latter approaches are preferred, then the initial randomized ribozyme coding sequences should contain a common DNA sequence of 15-25 nucleotides at the 3' termini to facilitate second strand synthesis. This common DNA sequence may be complementary to a subset of the random sequences and may limit the utility of the described invention by base pairing with the ribozyme binding sequences and preventing the proper formation of ribozyme/substrate interactions.

It may be appreciated that the generation of random sequences in the binding regions of the ribozymes will also generate restriction endonuclease cleavage sites used to clone the DNAs into the vectors. Cleavage with the cognate restriction endonuclease may therefore result in the loss of any ribozyme constructs containing the given restriction enzyme recognition site. For this reason, each generation of construct libraries uses at least two dissimilar restriction enzyme sequences at the 5' and 3' termini (e.g., recognition sequences for *Sal*I and *Pvu*II are GTCGAC and CTGCAG, respectively). A series of fragments containing *Eco*RI sites at the 5' ends and either *Pvu*II or *Sal*I sites at the 3' ends, as well as fragments containing *Eco*RI sites at the 3' ends and either *Sal*I or *Pvu*II sites at the 5' ends creates ribozyme coding fragments with enough degeneracy in the coding regions to represent all possible nucleotide sequences in the expression vectors.

### Example 28: Construction of Ribozyme Expression Vectors

A number of suitable vectors for the expression of mRNA constructs in tissue culture and animals have been described in the literature, and may be used as effective vectors for the therapeutic expression: of ribozymes. Stably transformed cell lines present the best test system for establishing parameters of expression which will be necessary for the development of optimal ribozyme-expressing vectors. Fragments of DNA can be easily introduced into these types of cells (e.g., HeLa cells) and selected for the maintenance of expression from the new fragments of DNA (e.g., by means of using the co-expression of a second gene which lends a selective advantage to the survival of the cell). Thus, for the effective construction of ribozyme expression vectors, a number of features may be built into the initial plasmid DNA construct; these include an eucaryotic transcriptional enhancer/promoter complex located immediately 5' to the ribozyme coding sequence, the presence of a self-cleaving (e.g., hepatitis delta virus) ribozyme motif located immediately 3' of the ribozyme coding sequence, the presence of a second coding sequence whose transcription is controlled by eucaryotic promoter and polyadenylation signal sequences, a bacterial origin of replication and an antibiotic resistance gene. The bacterial origin of replication and the antibiotic resistance genes are present to aid in growing the plasmid constructs prior to introduction into cell lines.

Many human transformed cell types express transacting factors which are capable of activating the SV40 early promoter region. For this reason, it is preferable to use an expression vector which contains the selection gene under transcriptional control of the SV40 early promoter. In cell types where the appropriate transcriptional activators are not ordinarily present, certain chemicals can be added exogenously to activate this promoter. Another preferred promoter is the metallothionein promoter which can be induced to activity by the addition of either glucocorticoids or heavy metal salts to the cells. Additional promoters which can be induced by endogenous or extracellular substances which are readily available to the cells are considered as alternative promoters in this invention.. The ribozyme coding sequences may also be induced using the same promoter sequences as the selective gene, but the ribozyme construct can also utilize any promoter which is activated by a desired target disease condition (e.g., ribozymes targeted to inhibit specific viral gene expression, such as the herpes simplex virus ICP27 gene may utilize the same promoter as the viral gene, e.g., the ICP27 promoter). The preferable situation would involve a ribozyme gene operating under the promoter control of a very strong constitutive promoter such as the human cytomegalovirus immediate early region promoter (CMV ie1 promoter) or any of a number of housekeeping gene promoters (e.g., beta actin promoter). It is appreciated that many preferred promoters contain enhancer sequences within the DNA sequences required for optimal promoter activity. It is preferable to utilize promoters which contain natural enhancer sequences because the addition of certain enhancer-like regions upstream of some promoter regions may suppress the promoter activity.

Because extra nucleotides outside of the catalytic region in the ribozyme may cause the formation of secondary structures which would inactivate the enzymatic function of the molecule, the vector construct does not always contain intramolecular signaling sequences which are necessary for the correct polyadenylation of the transcript or termination of transcription. The poly(A) sequence which was introduced into the ribozyme construct will substitute for the polyadenylation signal, but the termination signal is an undefined sequence which is thought to be no less than 25 nucleotides in length. Instead of the termination signal sequence, each vector construct can have a self-processing (e.g., hepatitis delta virus) ribozyme sequence incorporated immediately 3' of the restriction enzyme cloning site at the 3' terminus of the ribozyme coding fragment described previously. This sequence is able to cleave the ribozyme transcript as transcription is proceeding past the region and will function as an artificial transcription terminator for the ribozyme transcript. The hepatitis delta ribozyme cleavage will leave one extra nucleotide 3' of the restriction enzyme insertion site (RE2). With certain restriction enzyme recognition sequences, it is possible to engineer the hepatitis delta ribozyme to cleave within the restriction site or at the 3'-terminus of the poly(A) stretch. The appropriate hepatitis delta virus (or hairpin or other ribozyme) ribozyme can be engineered into the expression vector immediately 3' of the RE2 site by standard insertion mutagenesis techniques.

After the appropriate expression vector is constructed and the random ribozyme insert is in place, the constructs must be stably introduced into cell lines for expression. The stable integration and expression is driven by the selective pressure imposed upon the cells by treatment with toxic chemicals, such as G418 (gentamicin) or hypoxanthine/puromycin/thymidine (HPT) mixtures. The expression of neomycin resistance or thymidine kinase, respectively, will allow the transformed, expressing cells to survive and simultaneously afford the surviving cells the DNA needed to express transcripts from the random ribozyme genes introduced during transformation.

### Example 29: Stable Introduction of Ribozyme Expression Vectors Into Cell Lines

A number of methods to stably introduce DNA into cells are known in the literature, these include but are not limited by Ca₂PO₄ precipitation, DEAE dextran precipitation and electroporation. Generally, one microgram of DNA is applied to a 100 mm tissue culture dish containing subconfluent cultures of cells (approximately 3-4 million cells). Initially, at least 10 dishes will be transfected for each ribozyme DNA preparation. After an appropriate time is given for DNA uptake, the cells are rinsed in buffered medium and then refed with supplemented medium. In some cases, it may be preferable to include a glycerol shock step or the addition of inducing chemicals (e.g., glucocorticoid hormones or heavy metals to induce metallothionein promoter expression) to initiate the synthesis of selective gene products (e.g., thymidine kinase or neomycin resistance protein).

After 10-18 hours, the cells are trypsinized and replated in selective medium containing the toxic chemicals used for selection (e.g., 250-350 µM G418 in HeLa cells) against viable non-transfected cells. This medium may also contain chemicals required for the continued expression of any selective gene products necessary for cell survival. The continued presence of selective gene induction in the surviving cells may be required to maintain the ribozyme-containing plasmid because the transfected DNA is usually inherited as a unit which is maintained under the selective pressure used in the protocol. Often, when the selective pressure is removed, the foreign DNA is lost or deregulated in transformed cells. Because the cells will be maintained in the presence of chemical agents, care should be taken to avoid the use of agents which will interfere with subsequent ribozyme activity or introduction of infectious agents into the cell system.

Selected cells will be grown with medium changes every three days until confluent cultures are obtained. As the cells fill the tissue culture vessels, they will be trypsinized and transferred to larger vessels until cultures of between 10⁸⁻⁹ cells can be harvested for the preparation of frozen stocks. It is estimated that if each cell contains 1 nanogram of DNA, this will be 500 different ribozyme constructs if the average size of the plasmids is 7000 nucleotides. If the random ribozyme arms contain eight flanking nucleotides at each end for hybridization, this will give 4¹⁶ or 4.29 x 10¹⁰ unique molecules. A total representation of these molecules should easily be found in 10⁸ cells if no more than 100 ribozyme constructs per cell is realized during the transfection/selection procedures. The expression of 4¹⁶ ribozymes within this cell culture should give sequences capable of hybridizing and cleaving virtually any unique mRNA sequence which can be targeted. It has been estimated that there are only 4¹² unique RNA sequences expressed from the human genome. Clearly, any infectious agent would express a great deal less unique RNA sequences than can be targeted with these pluripotent ribozyme-expressing lines.

### Example 30: Selection of Therapeutic Ribozyme-Expressing Cells

There are many ways in which cells expressing therapeutically active ribozymes can be selected. In every case, the cells which express the desired ribozymes must be screened, cloned, and expanded for subcloning of the constructs into new vectors which allow the introduction of single copies of vectors into new cell lines. An example of an expression/selection protocol is given.

For this example, a ribozyme vector which is transcriptionally controlled by the HCMV ie1 promoter was used. This promoter is a very strong constitutively acting promoter which is controlled by transcriptional factors which are present in all cells. By using this promoter or any similar promoter, preinduction of transcription by hormones or chemicals is generally not required. Additionally, the HCMV ie1 promoter is transcriptionally activated by many factors which control genetic expression during infection of cells with heterologous viruses (e.g., the ie1 promoter is activated by the ICP4 and ICPO proteins which control expression of herpes simplex virus [HSV] genes). Selective pressure which will kill all but the ribozyme expressing cells is presented as a viral infection (e.g., HSV infection of HeLa cells). This infection should be performed at a tissue culture infectious dose which will kill approximately 50% of the cells in one replicative cycle. The cells are treated with virus for three infectious cycles of the virus (three days for HSV-1) and then the RNA is harvested from a portion of the surviving cells to verify the expression of ribozyme molecules from the transcription vectors. Surviving cells are expanded for the preparation of frozen stocks. Stocks are prepared by standard methods. A culture of the resistant cells is grown for the preparation of DNA and a verification that the cells are resistant to infection with the target virus (e.g., verification of resistance will be determined by reinfecting the survivors with HSV-1).

### Example 31: Recloning of Ribozyme-Expressing Vector DNA

Resistant ribozyme-expressing cells selected by protocols described in the previous section will be expanded for the purpose of extracting the ribozyme cassettes which are expressed in these cells. Total cellular DNA will be isolated and purified from the cells by standard methods. The DNA preparation will be subjected to restriction enzyme digestion using enzymes capable of recognizing sites present in the expression vectors which are at least 50 nucleotides 5' and 3' of the ribozyme cassettes. By using this approach, the DNA sequences present upstream and downstream of the ribozyme cassettes are used to hybridize primer DNA fragments. After primer hybridization to the restricted DNA preparation, the ribozyme cassettes can be amplified by incubation with appropriate salts, nucleotide precursors and Taq polymerase (United States Biochemical Corp.) and incubation at 65°C. The resultant amplified DNA fragments can be electrophoretically separated from the other DNA fragments (unwanted cellular DNAs) in 2% agarose or other suitable matrix. The DNA cassettes are released from the amplified DNAs by digestion with restriction endonucleases which recognize the RE1 and RE2 sites used in the original cloning. A second agarose gel is run to purify the desired cassettes from the unwanted ends of the amplified DNA which correspond to expression vector sequences. The ribozyme cassettes are then cloned into appropriate amphitrophic retrovirus vectors using standard protocols.

These vectors should contain an inducible/selectable gene such as neomycin resistance and restriction enzyme cloning sites RE1 and RE2 which are downstream of an inducible or strong constitutive promoter. A protocol for packaging the recombinant DNAs into amphitrophic virus particles and transfection into cells is well known. Infection and selection of cells with these vectors will result in the incorporation of only one ribozyme vector per cell.

### Example 32: Reselection for Therapeutic Ribozyme-Expressing Cassettes

After cell lines are prepared which contain ribozyme expression cassettes within retrovirus vectors, the cells are again selected for the expression of ribozymes which protect the cells from infectious agents, or prevent expression of an undesirable genetic trait. The selection protocol is similar to that described for the first selection (i.e., infection with HSV-1 at appropriate concentrations of virus, growth for three viral replicative cycles, and clonal expansion of any surviving cells). After stock cells are prepared, the cells are rechecked for viral resistance and the retroviral vector is induced to replicate by standard methods (e.g., chemical treatment). Viral particles are isolated and the sequences of the therapeutic ribozymes are determined by direct sequencing of the viral genomes. Molecular methods of inducing retroviral replication, isolation of particles and sequencing of the genomes are known to those in the art.

Once the sequence of a therapeutic ribozyme has been determined by the methods described herein, the ribozyme can either be incorporated into an expression vector for *in vitro* synthesis, or genetic therapy, or the ribozyme can be synthesized chemically for use with exogenous delivery techniques. Descriptions of useful systems are provided in the art cited above, all of which is hereby incorporated by reference herein.

### Quasi random methods

The following are examples methods of cloning quasi-random ribozymes into expression vectors, *in vitro* assays for optimizing ribozyme structural features which yield favorable catalytic activity, and methods of cloning concatameric ribozymes into an expression vector.

The techniques described below may be used to find effective therapeutic ribozymes to any desired target RNA even if the exact location of the transcript within a gene is uncertain. Methods are described whereby the selected ribozyme may be optimized for catalytic activity using cellular extracts for *in vitro* assays. Four vectors are described which allow the cloning of quasi-random ribozymes into plasmids and facilitate the excision of selected ribozyme cassettes for cloning into a eucaryotic expression vector for intracellular assays of ribozyme cleavage activity. A method is described which allows for the cloning of concatameric ribozyme cassettes into expression vectors and thereby results in a more effective ribozyme-expressing vector for use in cellular applications of genetic or therapeutic interest.

### Cloning of Ouasi-random Ribozymes

Four novel cloning vehicles were constructed for the generation of quasi-random ribozymes. The herpes simplex virus thymidine kinase (TK; Wagner et al, 78 Proc. Natl. Acad. Sci. USA 1441, 1981) gene was used as a prototype gene target for the cloning. Methods for the generation of the cloning vehicles, insertion of the TK DNA fragments and incorporation of a hammerhead ribozyme motif into the constructs follow. Use of this method allows one to clone target-specific ribozymes from any fragment of DNA, even when the primary DNA sequence or direction and dimensions of mRNA coding regions are uncertain. The subsequent use of biological selection and amplification of the desired clones will generate ribozyme constructs which have potentially advantageous genetic or therapeutic activities.

The basic requirement of the cloning vector in this protocol is that the restriction endonuclease site located immediately 5' of the cloned target DNA sequences be recognized by a restriction enzyme which cleaves the target DNA at a distance (x nucleotides) 3' of the recognition sequence. The distance (x) between the recognition and cleavage sites (along with the size of DNA fragments selected for cloning) will dictate the length of the ribozyme binding arms in the quasi-random constructs. The cloning of hammerhead ribozyme catalytic sequences into the inserted DNA fragments is facilitated greatly by using restriction enzymes that cleave to yield overhanging 3' ends which are 2 nucleotides in length. The cloning protocol deletes these two bases and inserts a ribozyme catalytic core at the site of the restriction enzyme cleavage. Use of restriction sites which give 5' overhangs, blunt ended fragments or 1 nucleotide 3' overhanging ends result in the generation of flanking sequences which no longer reflect the original DNA sequence or ribozymes which do not allow for the unpaired base (M) in the RNA substrate which is required for effective hammerhead ribozyme cleavage. See Figs. 49 and 50.

Cloning sites were constructed so that the ribozyme expression cassettes could be excised from the original plasmid vectors and inserted into eucaryotic expression vectors. Because restriction endonuclease sites will exist in the random fragments of target DNA, multiple releasing and core insertion cleavage sites should be available for each series of constructs. By using constructs with different releasing and core insertion restriction endonuclease sites, one can be relatively confident of acquiring all random ribozyme recognition sites with only four cloning vehicles. Two cloning inserts were designed for use in PGEM4 and pSP72 (Promega Corp; Madison,WI) vectors. For the PGEM4 vectors, *Bpm*I/*Sma*I and *Bsg*I/*Sma*I cloning sequences were flanked at the 5' and 3' ends by *Hin*dIII and *Eco*RI restriction endonuclease sites, respectively. For the pSP72 vectors, the 5' and 3' flanking restriction endonuclease sites were *Bgl*II and *Xho*I, respectively. Each of these vectors contains a T7 and SP6 promoter region at the 5' and 3' terminus of the cloning sites, respectively. The phage RNA polymerase promoters allow *in vitro* transcription of the ribozyme inserts and testing of ribozyme activity prior to the insertion into eucaryotic expression vectors.

The four pairs of DNA oligonucleotides (Midland certified Reagent Co.; Midland, TX) used to clone into the PGEM4 and pSP72 vectors were: 
(A) 5′ AGCTTCTGGAGCCCGGG 3′
3′ AGACCTCGGGCCCTTAA 5′
 
(B) 5′ GATCTCTGGAGCCCGGGC 3′
3′ AGACCTCGGGCCCGAGCT 5′
 
(C) 5′ AGCTTGTGCAGCCCGGG 3′
3′ ACACGTCGGGCCCTTAA 5′
 
(D) 5′ GATCTGTGCAGCCCGGGC 3′
3′ ACACGTCGGGCCCGAGCT 5′
 Pairs A and C were cloned into PGEM4 and pairs B and D were cloned into pSP72. To incorporate the cloning inserts into the plasmids, 10 µg of each oligonucleotide was combined with its paired oligonucleotide in a 1:1 ratio and incubated in TMN solution (50 mM Tris-HCl, pH 8; 10 mM MgCl₂, and 100 mM NaCl) at 90°C for 2 minutes, then slowly cooled to 25°C. One-tenth (2 µg) of the annealed mixture was ligated to the appropriate linearized vector fragments. Prior to addition to the ligation reaction, vector DNA was digested with the appropriate restriction enzymes (*Hin*dIII/*Eco*RI for PGEM4 and *Bgl*II/*Xho*I for pSP72) and the linearized fragments were isolated from low melting point agarose as described by Maniatis et al, 1982, Molecular Cloning: A Laboratory Manual. Cold Spring Harbor Laboratory, Cold Spring Harbor, NY. Ligations were performed at 14°C for 18-24 hours in 20 µl total volume. Buffer was as suggested by the manufacturer (United States Biochemical Corporation (USB), Cleveland, OH). After ligation of inserts into the vectors, bacteria (HB101) were transformed at 37°C for 20 minutes with approximately 100 ng of vector DNA (one-tenth of the reaction volume, 2 µl). Colonies of bacteria were selected on agarose plates containing ampicillin and then amplified in 3 ml cultures for identification of appropriate cloning vehicles. The presence of cloning inserts into PGEM4 and pSP72 vectors was analyzed by restriction endonuclease digestion of the plasmid DNA preparations. Samples of appropriately transformed bacteria were identified and 250 ml cultures of the bacteria were grown. Plasmid DNA was purified from the 250 ml cultures using QIAGEN columns (QIAGEN, Inc.; Chatsworth, CA) and protocols suggested by the manufacturer. Each of the four plasmid vectors generated in this manner were used in a similar protocol to clone random DNA inserts of the herpes simplex virus thymidine kinase (TK) gene.

For cloning of the TK gene fragments, 50 µg of the TK DNA (2.7 kb *Eco*RI fragment N) was excised by *Eco*RI/*Sca*I digestion of plasmid DNA containing this fragment. The *Sca*I digestion was incorporated to remove the plasmid bands of pSP72 which is also 2.7 kb in length. *Eco*RI fragment N was isolated by electrophoresis in low melting point agarose and subsequent elution by standard protocols (Maniatis et al., 1982). After isolation and concentration by ethanol/salt precipitation, the *Eco*RI fragment N DNA was fragmented by limited digestion with DNaseI. The digestion was performed by combining 20 µg of TK DNA and 2 units of DNaseI (USB) in 200 µl of total volume containing 50 mM Tris-HCl, pH 7.5; 10 mM MgCl₂, and 10 µg bovine serum albumin (BSA). The mixture was incubated at 37°C and 50 µl aliquots were removed and combined with 5 µl of 0.5 M EDTA after 1, 2.5, 5 and 10 minutes. All reaction aliquots were electrophoresed in 8% polyacrylamide gels to size fractionate the pieces. Fragments 20-40 nucleotides in length were cut from the gels and eluted according to the methods of Maniatis et al., 1982. The digestion protocol is dependent upon the base composition of the DNA and should be optimized for each fragment which is to be digested. We found that the optimum digestion time for the HSV TK gene was approximately 2.5 minutes. The termini of the eluted DNA fragments were repaired with Klenow fragment of DNA polymerase to generate blunt ended fragments for cloning into the plasmid cloning vehicles described above. The repair reactions were performed in 20 µl volumes at room temperature for 1 hour according to protocols described by the manufacturer (USB).

After repair of the termini, the DNA fragments were dephosphorylated by incubation with calf intestinal phosphatase enzyme (CIP, United States Biochemical Corp.) and the fragments were prepared for insertion into cloning vectors by extraction in phenol/chloroform/isoamyl alcohol mixtures (24:24:1) and ethanol precipitation. Ten micrograms of each cloning vehicle was linearized by digestion with *Sma*I restriction endonuclease and the linearized fragments were electrophoresed in 1% low melting point agarose gels to remove the fraction of undigested vectors. Elution from the agarose was as described by manufacturer. one microgram of vector was combined with one microgram of DNA fragment preparation (1:100 molar ratio of vector to insert, respectively) and then fragments were ligated in a total volume of 20 µl at 25°C for 18-24 hour. Prior to transformation of bacteria, the ligation mixture was digested with *Xma*I restriction endonuclease (5 units of enzyme in 20 µl volume for 3 hours) to relinearize vector pieces which had closed during ligation. (*Xma*1 is an isoschizimer of *Sma*I.) The digested preparations were then transformed into HB101 cells as described earlier. The resultant populations of ampicillin resistant bacteria were grown in a batch culture to recover all plasmid vehicles for the insertion of the ribozyme catalytic core sequences into the TK DNA fragments.

Prior to cloning the ribozyme catalytic core into the TK DNA fragments, plasmids were linearized by digestion with appropriate enzymes (*Bpm*I or *Bsg*I; New England Biolabs; Beverly, MA) and the linearized plasmids were isolated after electrophoresis in low melting point agarose gels. The linearized preparations were digested with T4 DNA polymerase to generate blunt ends and the DNAs were dephosphorylated by treatment with CIP enzyme. After phenol/chloroform/isoamyl alcohol extraction, the DNA fragments were precipitated with ethanol and resuspended in TE buffer (10 mM Tris-HCl, pH 7.5; 1 mM EDTA). The catalytic core sequence was inserted into the linearized plasmid DNA by annealing the following oligonucleotides as described above: 5′ CTGATGAGGCCGAAAGGCCGAAA 3′ 3′ GACTAACTCCGGCTTTCCGGCTTT 5′ After annealing, 0.1 µg of the oligonucleotide mixture was added to 1 µg of the blunt-ended, linearized plasmid preparation (gives a 10:1 molar ratio of insert:vector sequences) in ligation buffer to give a final reaction volume of 20 µl. The mixture was incubated overnight at 16°C and then one-tenth of the total volume (2 µl) was added to 100 µl of transformation solution containing HB101 cells. After transformation of the cells, 10 ml preparations of the bacteria were grown and plasmid DNA was isolated from 5 ml of the preparation as described in a previous section. The remaining 5 ml of bacteria was divided among three tubes, combined with an equal volume of 40% glycerol and frozen at -80°C for stock solutions.

Use of the quasi-random ribozyme cloning generates gene fragments and catalytic cores which are present in both sense and antisense orientations. Because ribozymes will be created which cleave transcripts from both strands of the DNA fragment, this mixture of inserts is advantageous when cell selection of ribozyme activity is sought and the direction of transcription within the target DNA is unknown. The random insertion method should result in a 1:1 mixture of sense strand and complementary strand cleaving ribozymes. The presence of RNA polymerase promoters at both ends of the DNA insertions allows versatility in the choice of transcription protocols (because roughly equal distributions of ribozyme structures and specificities should be obtained with either series of transcripts) and compensates for non-random directional insertions into the vector.

For a cursory examination of catalytic activity within the quasi-random ribozyme populations, *in vitro* transcripts were synthesized from the plasmid DNA templates prepared above. The plasmid DNA preparation was split into two fractions (the yield of DNA was approximately 10 µg in each fraction) for digestion with restriction endonucleases and *in vitro* transcription with either T7 or SP6 RNA polymerase. The PGEM4 based plasmids were cleaved with EcoRI for T7 transcriptions and with *Hin*dIII for the SP6 transcriptions. The pSP72 based plasmids were cleaved with *Xho*I for T7 transcriptions and with *Bgl*II for the SP6 transcriptions. One microgram of linearized DNA template was added to a transcription mixture containing 20 µl of 5x buffer (200 mM Tris-HCl, pH 7.5; 50 mM MgCl₂; 10 mM spermidine and 50 mM NaCl), 10 µl of 100 mM dithiothreitol (DTT); 5 µl of RNAsin (20,000 U/ml; GIBCO/BRL, Gaithersburg, MD); 5 µl of NTP mixture (20 mM each of ATP, GTP, CTP and UTP) and enough water to bring the final volume to 95 µl. 100 units of T7 or SP6 RNA polymerase (USB) was added and the mixture was incubated at 37°C for 1 hour, after which RNase-free DNaseI was added and the reaction was incubated an additional 15 minutes at 37°C. The solution was brought to a volume of 150 µl, extracted once with phenol/chloroform/isoamyl alcohol and subjected to spin chromatography through a 1 ml bed volume of G-50 Sephadex (Pharmacia, Upsalla). A typical transcription yields 10-15 µg of RNA per µg of DNA template.

For the synthesis of ³²P-labeled TK substrate RNA, template DNA was prepared by PCR amplification using primers 5′ TAATACGACTCACTATAGGGACCGAGCGACCCTGCAG 3′ and 5′ GCCGTCATAGCGCGGGT which resulted in the generation of double-stranded DNA template containing a T7 RNA polymerase promoter region located immediately 5' of the TK mRNA coding sequences (1262 nucleotides). The transcription reaction was essentially as described for ribozyme synthesis except that the total reaction volume was decreased to 20 µl, contained only 20 units of T7 polymerase and the 1 µl of 20 mM NTPs was substituted with 1 µl of 20 mM ATP, GTP and UTP, 1 µl of 2 mM CTP and 2 µl of α-³²P-CTP (80 µCi; 800 Ci/mmol; Dupont/NEN; Boston, MA). TK RNA was purified by spin chromatography through G-50 Sephadex columns. When ill-defined target DNA is used as the source of insert fragments, template for the transcription of target RNA may be prepared by blunt-end ligation of the DNA into pSP72, PGEM4 or other suitable transcription vectors.

For analysis of ribozyme cleavage of the TK transcript, the *in vitro* transcripts were mixed with 50,000 cpm of *in vitro* transcribed TK RNA and incubated at 37°C in a total volume of 50 µl containing 5 µl of 10x ribozyme cleavage buffer (750 mM Tris, pH 7.5, and 100 mM MgCl₂) and a final ribozyme concentration varying between 0.1 and 10 µM. Control reactions contained 10 ng of yeast tRNA instead of the ribozyme mixture. At given time intervals, aliquots were removed and quenched by addition to a solution containing (20 mM EDTA, 95% formamide and 0.1% Bromphenol Blue and Xylene Cyanol). Samples were heated at 90°C for 10 minutes, snap cooled in ice and cleavage products of the TK RNA were identified by electrophoresis of the samples in 3% polyacrylamide gels and subsequent visualization of cleaved RNA fragments by autoradiography. DNA preparations whose transcripts exhibited ribozyme activities specific for the TK RNA were amplified by growing 250 ml cultures of the parent bacterial stocks. The plasmid DNA isolated from these stocks was digested with either *Hin*dIII/*Eco*RI or *Bgl*II/*Xho*I restriction enzyme combinations and the released ribozyme coding sequences were isolated by gel electrophoresis and cloned into appropriate eucaryotic expression vectors for use in tissue culture experiments.

The screening and identification of ribozyme vectors which cleave target RNA intracellularly is tedious and depends upon the choice of target mRNA whose expression lends a negative selective advantage to the cells (e.g., HSV TK expression will render cells sensitive to treatment with acycloguanosine [ACV]). By growing TK-expressing cells in the presence of ACV and ribozyme-expression vectors, one may select and expand only the cells containing inhibitory ribozymes. The DNA encoding effective ribozymes may be extracted and PCR amplified by standard methods because the flanking regions within the eucaryotic expression vectors will be known. After identification of effective ribozyme sequences, the ribozyme structures may be optimized by the following *in vitro* methods prior to insertion into appropriate therapeutic expression vectors.

### In Vitro Ribozyme Optimization

An adjunct approach to cell-based selection of effective quasi-random ribozyme constructs uses a modification of the *in vitro* ribozyme activity assay described in the previous section. When uniformly labeled transcript is used to check ribozyme activity, multiple cleavages within the transcript prevent the identification of susceptible RNA structures. For any given quasi-random population of ribozymes, the number of possible ribozyme molecules is approximately 2(x-y)/4, where x is the length of one strand of DNA and y is the average length of the DNA fragments inserted into the vectors. Because the length of ribozyme binding arms is small compared to the sizes of most genes, the number of ribozymes screened will be approximately one-half the number of nucleotides in the coding strand of the DNA. For the 2.7 kb piece of TK DNA, one expects approximately 1350 ribozymes (approximately 632 ribozymes nucleotides in the TK coding region). Many of these ribozymes will target inaccessible structures within the target mRNA or possess incompatible secondary structures which will reduce their catalytic activity.

To determine the limited sites within the RNA which are accessible to active ribozymes, it is preferable to assay ribozyme activity using substrates with uniquely labeled sites (5' or 3' end-labeled). Analysis of the cleaved substrate fragment size will give an approximate location within the substrate where ribozyme cleavage occurs. By using different sizes of substrates spanning the target RNA, one may localize most of the preferred ribozyme target sites to defined distances from the 3' or 5' ends of the target RNA. Sequencing of the DNA template in these regions will reveal the probable ribozyme recognition sequence, which can be verified by synthesizing 20-40 mers of the substrate RNA and testing for cleavage in the presence of the quasi-random ribozyme preparation. Synthesis of RNA molecules in this size range may be accomplished enzymatically or chemically by established protocols.

After the target region has been identified, ribozymes to the target sequence may be synthesized which contain sequence modifications or additions which increase catalytic rates or nuclease stability in cellular extracts and/or alter intracellular localization when synthesized in transient assays from eucaryotic expression vectors. A reproducible, simple technique for assaying ribozyme cleavage of target RNAs in cellular extracts is described below.

DNA templates for the synthesis of antisense RNA probes spanning the TK gene were amplified from 10 ng of HSV-1 (KOS strain) DNA, using the following primers: PRIMER A 5′ TAATACGACTCACTATAGGGCTTCTTGCTGCCGGCGA 3′ PRIMER B  5′ CATCCCCGTGGCCCGTT  3′ Primer A contains a T7 RNA polymerase promoter. Probes with a specific activity of 1.8 x 10⁶ dpm/ng were synthesized directly from PCR-amplified template DNA using T7 RNA polymerase (USB) as described by the manufacturer, in the presence of ³²P-labeled CTP (Dupont/NEN). Cytoplasmic extracts (40 µl) derived from HSV-infected Vero cells (MOI =1, HSV-1, strain KOS) were combined with 5 µl of 10x ribozyme cleavage buffer (750 mM Tris, pH 7.5, and 100 mM MgCl₂) and ribozyme (diluted in 5 µl water, final ribozyme concentration varied from 0.1 to 10 µM), and incubated at 37°C for varying amounts of time. Following ribozyme cleavage, extracts were diluted to 400 µl with 4 M guanidine thiocyanate lysis buffer and RNA was extracted as described (Chomszynski and Sacchi, 162 Anal. Biochem. 156, 1987). RNA was resuspended in 45 µl lysis buffer, antisense TK probe (1-5 x 10⁵ cpm in 5 µl lysis buffer) spanning the cleavage site was added, then hybridization reactions were overlaid with 30 µl of mineral oil and incubated at 55°C for 18-20 hours. After hybridization, 500 µl of RNase solution (0.4 M NaCl, 20 U/ml RNaseA, 2 U/ml RNaseT1 and 10mM Tris/HCl pH 7.5) was added, and reactions were incubated at 37°C for 30 minutes. Following nuclease digestion, 10 µl of 20% SDS and 10 µl of 20 mg/ml proteinase K were added, the reactions were heated an additional 30 minutes at 37°C, and hybrids were precipitated with 500 µl of isopropanol. Pellets were resuspended in 10 µl of solution containing 95% formamide, 10 mM EDTA, 0.1% bromophenol blue and 0.1% xylene cylanol (USB), heated to 95°C for 4 minutes, then loaded onto 5% polyacrylamide/7 M urea gels and electrophoresed at 65 W constant power. Gels were dried and the radioactivity corresponding to protected RNA fragments was visualized by autoradiography and quantified using an AMBIS detector.

### Cloning of a Concatameric Ribozyme Construct

After ribozymes have been selected for effective cleavage of target RNA in cells or extracts and optimized for catalytic rate, intracellular stability and subcellular localization, the final step in designing efficient ribozyme-expression vectors involves the cloning of multiple therapeutic ribozymes (TRs) within the transcription unit. The release of the TRs from the transcript necessitates the cloning of an additional releasing ribozyme (RR) which can cleave between TR units in the nascent transcripts. This protocol has been described in a previous section.

### Assay for Ribozyme Target Site

Applicant has devised a method by which the accessibility of any particular target site for a ribozyme can be determined *in vitro* or *in vivo.* This assay is important to ensure that time and energy is not expended on developing highly active ribozymes which are unable to locate or bind to a target sequence *in vivo* because it is later found that the target sequence is inaccessible to the ribozyme. Such inaccessibility may be caused by the target RNA secondary structure, or by protein factors which bind to the target sequence. The assay for such target site accessibility can be performed without the need to form many ribozymes against each potential target site.

In general, the method involves use of an enzyme which is able to recognize a DNA-RNA duplex, and cleave the RNA in that duplex. For example, RNaseH can be used to cleave RNA bound to a DNA molecule. Applicant recognizes that the target site for a ribozyme is an RNA substrate molecule and that an RNA-DNA duplex can be formed with such a target site by provision of suitable complementary DNA. The method involves providing one or more such DNA oligonucleotides or molecules (which may be synthesized quickly and cheaply on a DNA synthesizer), allowing those DNA molecules to contact the target RNA, and determining whether the RNA target is rendered susceptible to cleavage by RNaseH. Such cleavage may be measured by gel electrophoresis of labeled target RNA, or by northern blotting, as well as by other methodology well known in the art.

The method of this invention allows DNA probes of various sizes to be chosen such that they approximate the size and shape of the ribozyme binding site so that accessibility of the entire ribozyme structure can be evaluated. In addition, since many eukaryotic cells contain endogenous RNaseH, it is possible to use the method to evaluate target site accessibility in whole living cells by delivering the DNA oligonucleotides to those cells under conditions in which endogenous RNaseH will cleave any RNA-DNA duplexes formed in the cell.

Thus, the invention features a method for determining RNA target site accessibility to a ribozyme by providing a DNA oligonucleotide complementary to the RNA target site and contacting that DNA oligonucleotide with the RNA target in the presence of a DNA-RNA specific cleaving agent, such as RNaseH, so that the RNaseH will cleave the RNA target when the DNA and RNA form a duplex. The occurrence of cleavage is detected by any standard methodology, e.g., northern blot or gel electrophoresis. Those DNA oligonucleotides which provide a detectable level of cleavage indicate that the complementary RNA target site is accessible to a ribozyme. Ribozymes to these target sites can then be synthesized, using standard methodology, and checked to determined that the RNA target site is truly accessible to a ribozyme.

By "target site" is meant a sequence within a target RNA that is "targeted" for cleavage: (i) by a ribozyme which contains sequences within its substrate-binding domain that are complementary to the target sequence, or (ii) by a DNA/RNA cleaving agent, such as RNaseH, following the binding of a complementary DNA oligonucleotide to the target sequence.

By "substrate-binding domain" is meant sequences within a ribozyme which are intended to bring ribozyme and target RNA together through complementary base-pairing interactions; e.g., ribozyme sequences within stems I and III of a standard hammerhead ribozyme make up the substrate-binding domain (see Fig. 35).

By "DNA oligonucleotide" is meant a short sequence of DNA; in this application, the sequence is in the range of 7-20 nucleotides, and preferably in the range of 9-13 nucleotides.

By "RNaseH" is meant an endoribonuclease that degrades the RNA portion of RNA-DNA hybrid duplexes. RNaseH is found in many organisms; *E. coli* RNaseH is available from many commercial sources.

By "detectable level of cleavage" is meant cleavage of target RNA (and formation of cleaved product RNAs) to an extent sufficient to discern cleavage products above the background of RNAs produced by random degradation of the target RNA. Production of cleavage products from 1-5% of the target RNA is sufficient to detect above the background for most methods of detection.

Preferably, the DNA oligonucleotides are between 9-15 bases in length, and completely complementary to the target site.

More preferably, the DNA oligonucleotides are of the form NGAN, NUAN or NXYZN where each N is any nucleotide sequence from 0-12 nucleotides and XYZ is selected from the group consisting of GAA, GAC, GAG, GAU, UAA, UAC, UAG and UAU; the DNA oligonucleotides are single-stranded fragments of a gene encoding the target RNA; and the DNA oligonucleotides are obtained by asymmetric amplification followed by fragmentation by use of a procedure selected from: (a) thiophosphate incorporation followed by peroxide degradation, (b) chemical cleavage, and (c) DNaseI digestion.

### Method

The method of the invention is generally described above. Examples of methods of the invention are provided below, and in Figs. 35 - 40 discussed above, which are not limiting to this invention. Those of ordinary skill in the art will recognize that many equivalents to such methods can be used.

The assay of this invention does not provide a final test of target site accessibility, but rather provides a preliminary test. A number of factors may cause the test to distort the picture of which sites are accessible and which are not. For example, the enzyme used, e.g., RNaseH, may cut the target RNA at a site that is only partially bound by a DNA oligonucleotide. Such a site may thus be only partially susceptible to RNaseH cleavage, and not accessible at all to a ribozyme. The occurrence of such an erroneous false positive result can be reduced by testing each RNA target site with a set of overlapping DNA oligonucleotides. In addition, a DNA oligonucleotide does not have exactly the same binding affinity as an RNA oligonucleotide, so that a site that is inaccessible to a DNA probe still may be accessible to an RNA probe, or *vice versa.* For the most part such differences are small, and false negative results are rare. Again, misguidance by the occurrence of such false results can be avoided by use of several different DNA oligonucleotides for each test site.

The DNA oligonucleotide used may completely span the ribozyme binding site in just one helix (i.e., with no base mismatches, bulges or loops). In contrast, an equivalent ribozyme binding at the same site may have two binding regions split by a core sequence, e.g., this is the case for a hammerhead ribozyme. The presence of such a central core may reduce the binding affinity of the ribozyme compared to a DNA oligonucleotide by as much as 7.0 kcal/mole; thus, a DNA oligonucleotide may bind many times more strongly than the equivalent ribozyme. The possibility of this discrepancy can be reduced by use of short DNA oligonucleotides (9-13 bases). Since short oligonucleotides will not necessarily bind to the same site as the ribozyme, a number of shorter overlapping DNA should be used in each set of experiments.

In general, the strategy involves the formation of DNA oligonucleotides complementary to any number of selected RNA target sites. The target RNA can be transcribed *in vitro* using labeled nucleotide triphosphates, and added directly to an RNaseH assay mixture. The target RNA is preferably not purified in order to keep its configuration as close as possible to that in a natural *in vivo* environment. The DNA oligonucleotides are mixed in various concentrations with an excess of RNaseH and the labeled target RNA and incubated at 37°C. Samples are removed at various times and loaded onto sequencing gels. The most useful RNA target sites are those that show the largest extent of cleavage in the shortest amount of time.

The RNaseH experiments can also be performed as described above, except that the target RNA need not be labeled, and can be provided within a cell extract. Northern blotting (or other means, e.g., RNase protection) of the RNA can then be used to detect cleavage of the RNA.

In one type of experiment, *in vitro* transcribed, body-labeled target RNA is digested by addition of high concentrations of RNaseH in the presence of varying concentrations of 9-15 mer DNA oligonucleotides complementary to specific sites on the target. The high concentration of RNaseH reduces the probability that cleavage by RNaseH will be the rate-limiting step in the reaction. Thus, differences in RNA cleavage rates (and extents) are interpreted to represent differences in rates (and extents) of association between the DNA oligonucleotides and their respective sites on the target RNA. Sites which are accessible to cleavage by RNaseH are more likely to be targetable by ribozymes.

In a further experiment, we have employed semi-random DNA oligonucleotides as RNaseH probes of target RNA accessibility. The semi-random probes are of the form, NXYZN, where N represents a randomized position (i.e., A, C, G, or T; or each N is individually between 0 and 12 nucleotides, and the oligonucleotide contains at least 8 - 12 nucleotides) and XYZ represents a specific sequence complementary to one of the preferred ribozyme cleavage sequences (e.g., GUC, GUA, CUC, CUA). Probing with fully randomized DNA provides unsatisfactory results; cleavage of the target RNA occurs only at high probe concentrations and without the formation of discernable bands. The semi-random probes described above permit a large number of potential target sites to be evaluated from a single reaction. The number of different sequences represented in the semi-random probe, however, is only a fraction (4⁻³ or 1.6%) of sequences present in a completely random probe. This has the advantage of increasing the concentration of any given sequence while the number of bands to be interpreted is kept to a manageable number.

### Example 33: RNaseH in vitro

The following reagents were used:
5X buffer:
   100 mM Tris pH 7.9
   500 mM KCl
   50 mM MgCl₂
   0.5 mM EDTA
   0.5 mM DTT
RNaseH:
   Obtained from Bethesda Research Labs. (BRL) 2.5 U/µl
Formamide dye stop:
   95% Formamide
   20 mM EDTA
   0.1% bromophenol blue
   0.1% xylene cyanol
DNA oligonucleotide (typically 13 nucleotide long) at a 10X concentration of 10, 1, and 0.1 µM.

The method involved the following steps:
1. Dilute a standard transcription reaction (including the target RNA formed by transcription in a cell lysate or in a test tube) to the appropriate counts per minute (cpm) per µl to a 10X concentration;
2. Set up the reaction mix: (typical reaction)

| | |
|---|---|
| 5x Buffer | 2.0 µl |
| Body labeled Target RNA 50,000 (cpm/λ) | 1.0 µl |
| RNaseH (2.5 u/λ) | 0.32 µl |
| H₂O | 5.68 µl |
| | 9.0 µl |

3. Prewarm reaction mix to 37°C, then add 1 µl of DNA oligonucleotide. Allow to incubate at 37°C for 10 minutes, then add 5 µl of formamide stop solution. Brief spin to remove condensation from the tubes wall, then heat at a minimum of 90°C for 3' before loading onto a denaturing acrylamide gel.

### Example 34: RNaseH in Cell Extracts

The following reagents were used:
5X buffer:
   100 mM Tris pH 7.9
   500 mM KCl
   50 mM MgCl₂
   0.5 mM EDTA
   0.5 mM DTT
RNaseH:
   BRL 2.5 U/µl
Cell lysis buffer:
   4 M guanidine thiocyanate
   0.5% sarcosyl
   25 mM sodium citrate pH 7.4
Formamide dye stop:
   95% Formamide
   20 mM EDTA
   0.1% bromophenol blue
   0.1% xylene cyanol
RNase Solution:
   0.4 M NaCl
   10 mM MgCl₂
   20 U/ml RNaseA (United States Biochemical Co. Ohio; USB)
   2 U/ml RNase T1 (USB)
   4 U/ml DNase 1, RNase free (USB)
20% SDS
Proteinase K: 20 mg/ml
Phenol: chloroform: isoamyl alcohol 25:25:1 Isopropanol
tRNA: 2 µg/µl

DNA oligonucleotide (typically 13 nucleotide long) at a 10X concentration of 150 µM.

After preparing a cell lysate (from a cell including a target RNA) using standard procedures the method involved the following steps:
1. In a 0.5 ml tube add:

| | |
|---|---|
| 5x buffer | 3 µl |
| cell lysate | 10 µl |
| RNaseH | 0.3 µl |
| H₂O | 1.68 µl |
| | 14 µl |

2. Prewarm to 37°C and add 1 µl of 10X stock of DNA oligonucleotide, incubate at 37°C for 10 minutes and stop the reaction by adding 40 µl of lysis buffer.
3. Add 5 µl of labeled RNase protection probe RNA (200,000 cpm/λ), overlay with 25 µl of mineral oil and hybridize overnight at 55°C.
4. Transfer to a 1.5 ml tube containing 500 µl of RNase solution and incubate at 37°C for 30 minutes.
5. Add 10 µl of 20% SDS and 10 µl of 20 mg/ml proteinase K, incubate at 37°C for 30 minutes.
6. Extract once with 500 µl of 25:25:1 mixture of phenol: chloroform: isoamyl alcohol, brief vortex and transfer the aqueous phase to a fresh 1.5 ml tube containing 500 µl of isopropyl alcohol with tRNA carrier.
7. Pellet at room temperature (about 20°C) for 8 minutes.
8. Resuspend in 10 µl of formamide dye stop.
9. Heat above 90°C for over 3 minutes and load onto a denaturing acrylamide gel.

Results of the above two examples are shown in the figures. These results were obtained using the standard procedures described above and other standard protocols well known in the art.

As noted above, it is important in this invention to provide a plurality of DNA oligonucleotides. Selection of appropriate oligonucleotides is important to the outcome of the experiment. Several examples of such selections are provided below. Each selection is useful dependent upon the data required, e.g., whether a target site in a genome is required, or a target site in a gene.

### Example 35:

Thus, in one example completely randomized oligonucleotides can be made on a DNA synthesizer. This is the easiest approach to try since it takes only one synthesis and one test to select a target site. Random 6-and 11-mers have been tried without success. The lack of success, however, may be due to too much overlap between the various oligonucleotides, or because the oligonucleotides lightly coat the RNA and make all sites slightly susceptible to cleavage.

### Example 36:

An alternative approach involves making many different DNA oligonucleotides, and mixing them together prior to testing in the assay. The approach saves time over testing each oligonucleotide separately. Oligonucleotides are best selected so that they are not so close together that it is difficult to determine which site is accessible.

### Example 37:

Another approach is to cut up a portion of a chosen target gene and use that mixture as DNA oligonucleotide material in the assay. This approach offers a complete set of oligonucleotides that match the target RNA with a minimum of unwanted sequence. The DNA strands first need to be separated so that only the complementary strand is used to make DNA oligonucleotide (since that other strand will act as a competitor for that DNA oligonucleotide). It is also best to cut the DNA relatively uniformly to produce a distribution of fragments in a narrow size range. Asymmetric polymerase chain reaction (PCR) is the best way of making single-stranded DNA. Cleavage of the DNA can be accomplished by: (i) incorporation of thiophosphate nucleotides into the DNA followed by H₂O₂ degradation, (ii) chemical cleavage of the DNA using DMS (at G) or DEPC (at A) or Hydrazine (at U or C), or (iii) digestion by DNaseI.

Asymmetric PCR may not yield sufficient quantities of single-stranded DNA complementary to the target sequence. An alternative is to clone the target gene or sequence into an M13 vector and then isolate large quantities of single-stranded DNA vector containing the target gene (M13 is a single-stranded DNA phage which has both a single-stranded and a double-stranded component to its life-cycle). Removal of the extra vector sequence could be achieved (if necessary) by annealing two complementary DNA oligonucleotides to regions flanking the target gene or sequence and then cutting the DNA within these duplex regions using restriction enzymes with sites located within those duplex regions (see Fig. 41).

### Example 38:

Another method for producing suitable DNA oligonucleotides is to make a partially-random oligomer. The strategy is based on the fact that it is preferred to study accessibility of ribozymes only at those sites that may be cleavable by such ribozymes. That is, sites that contain a UC or a UA in the sequence. Thus a series of oligonucleotides can be made of the form 5'-NGAN or 5'-NUAN (these oligonucleotides are complementary to the UC or UA sites and where each N is any nucleotide sequence from 0-12 nucleotides) to be used in the assay. Even greater specificity can be achieved if many different syntheses are performed with the central three nucleotides being exactly specified (e.g., GAA, GAC, GAG, GAU, UAA, UAC, UAG, UAU, see below). Each of the oligonucleotide probes can be tested in separate assays. The advantages of this method are: (i) the oligonucleotides can be made on the DNA synthesizer, (ii) three fewer sites are randomized so that each oligonucleotide sequence is present at a 64 fold (4³) higher concentration, (iii) possible problems with overlapping oligonucleotides are minimized since each central sequence appears at random only about every 64 (4³) nucleotides, and (iv) identification of the accessible sites is easier since part of the DNA oligonucleotide sequence will be known and the position of cleavage thus identifiable within about 64 nucleotides.

The assay of this invention does not provide a final test of target site accessibility, but rather provides a preliminary test. A number of factors may cause the test to distort the picture of which sites are accessible and which are not. For example, the enzyme used, e.g., RNaseH, may cut the target RNA at a site that is only partially bound by a DNA oligonucleotide. Such a site may thus be only partially susceptible to RNaseH cleavage, and not accessible at all to a ribozyme. The occurrence of such an erroneous false positive result can be reduced by testing each RNA target site with a set of overlapping DNA oligonucleotides. In addition, a DNA oligonucleotide does not have exactly the same binding affinity as an RNA oligonucleotide, so that a site that is inaccessible to a DNA probe still may be accessible to an RNA probe, or *vice versa.* For the most part such differences are small, and false negative results are rare. Again, misguidance by the occurrence of such false results can be avoided by use of several different DNA oligonucleotides for each test site.

The DNA oligonucleotides used may completely span the ribozyme binding sites in just one helix (i.e., with no base mismatches, bulges or loops). In contrast, an equivalent ribozyme binding at the same site may have two binding regions split by a core sequence, e.g., this is the case for a hammerhead ribozyme. The presence of such a central core may reduce the binding affinity of the ribozyme compared to a DNA oligonucleotide by as much as 7.0 kcal/mole; thus, a DNA oligonucleotide may bind many times more strongly than the equivalent ribozyme. The possibility of this discrepancy can be reduced by use of short DNA oligonucleotides (9-13 bases), or by reducing the concentration of DNA probes in the accessibility assay.

In general, the strategy involves the formation of DNA oligonucleotides complementary to any number of selected RNA target sites. The target RNA can be transcribed *in vitro* using labeled nucleotide triphosphates, and added directly to an RNaseH assay mixture. The target RNA is preferably not purified in order to keep its configuration as close as possible to that in a natural *in vivo* environment. The DNA oligonucleotides are mixed in various concentrations with an excess of RNaseH and the labeled target RNA and incubated at 37°C. Samples are removed at various times and loaded onto sequencing gels. The most useful RNA target sites are those that show the largest extent of cleavage in the shortest amount of time.

The RNaseH experiments can also be performed as described above, except that the target RNA need not be labeled, and can be provided within a cell extract. Northern blotting or RNase protection of the RNA can then be used to detect cleavage of the RNA.

In one type of experiment, *in vitro* transcribed, body-labeled target RNA is digested by addition of high concentrations of RNaseH in the presence of varying concentrations of 9-13 mer DNA oligonucleotides complementary to specific sites on the target. The high concentration of RNaseH reduces the probability that cleavage by RNaseH will be the rate-limiting step in the reaction. Thus, differences in RNA cleavage rates (and extents) are interpreted to represent differences in rates (and extents) of association between the DNA oligonucleotides and their respective sites on the target RNA. Sites which are accessible to cleavage by RNaseH are more likely to be targetable by ribozymes.

### Example 39:

In a further experiment, we have employed semi-random DNA oligonucleotides as RNaseH probes of target RNA accessibility. The semi-random probes are of the form, NXYZN, where N represents a randomized sequence position as above and XYZ represents a specific sequence complementary to one of the preferred ribozyme cleavage sequences (e.g., GUC, GUA, CUC, CUA). Probing with fully randomized DNA provides unsatisfactory results; cleavage of the target RNA occurs only at high probe concentrations and without the formation of discernable bands. The semi-random probes described above permit a large number of potential target sites to be evaluated from a single reaction. The number of different sequences represented in the semi-random probe, however, is only a fraction (4⁻³ or 1.6%) of sequences present in a completely random probe. This has the advantage of increasing the concentration of any given sequence while the number of bands to be interpreted is kept to a manageable number.

### Ribozyme Synthesis

A method is provided for automated chemical synthesis of a ribozyme by separately synthesizing two or more portions of the ribozyme and then ligating them to form an enzymatically active ribozyme. This method increases the yield of the final ribozyme over other methods, and makes manufacture of such ribozymes cheaper. The prior inefficient synthesis of long RNA molecules is reduced or obviated in this method. One or more ribozyme portions may be used as one portion of several different ribozymes, and combined with other synthetic portions. In addition, each ribozyme fragment is more likely to be free of secondary structure and therefore can be purified by HPLC with greater resolution. The full length ribozyme, in contrast, may elute as multiple peaks. Thus, purity of the ribozyme resulting from two fragments can be greater than that when the ribozyme is formed as a single oligonucleotide.

Specifically, the invention features automated chemical synthesis to make ribozymes as two "half ribozymes" that can be joined by RNA ligase (e.g., one derived from the bacteriophage T4) to produce a "full length" enzymatically active ribozyme molecule. This method will minimize any impact of inefficient synthesis of long RNA on final yield. This scheme can also be used to mix and match different half ribozyme units allowing the characterization of many half ribozyme combinations without necessitating additional costly syntheses.

Thus, a method is provided for synthesis of an enzymatically active ribozyme by providing two or more portions of a ribozyme and ligating those portions together to form the active ribozyme. The ribozyme may be formed from RNA only, RNA and DNA, or may include modified nucleotide bases.

By the phrase "portions of a ribozyme" is meant an RNA (or modified RNA) molecule which can be used to form an enzymatically active RNA molecule by providing at least one other RNA molecule to which it is ligated or otherwise juxtaposed. For example, a portion is not enzymatically active until joined with such another RNA molecule. Preferably, only two or three such portions are required to form a fully enzymatically active ribozyme. Such enzymatically active ribozymes are active to cleave specific target RNAs or to cleave intramolecularly.

Preferably, the ribozyme is a hammerhead ribozyme having between 34 and 40 nucleotides and each portion includes 15 or more nucleotides; the portions are hybridized to a DNA strand complementary to each portion and are juxtaposed by that hybridization; the juxtaposed portions are ligated together by an RNA ligase, e.g., T4 RNA ligase; one portion is phosphorylated; the ligated portions are purified after ligation; and more than two portions are provided and a plurality of different ribozymes simultaneously formed in the method.

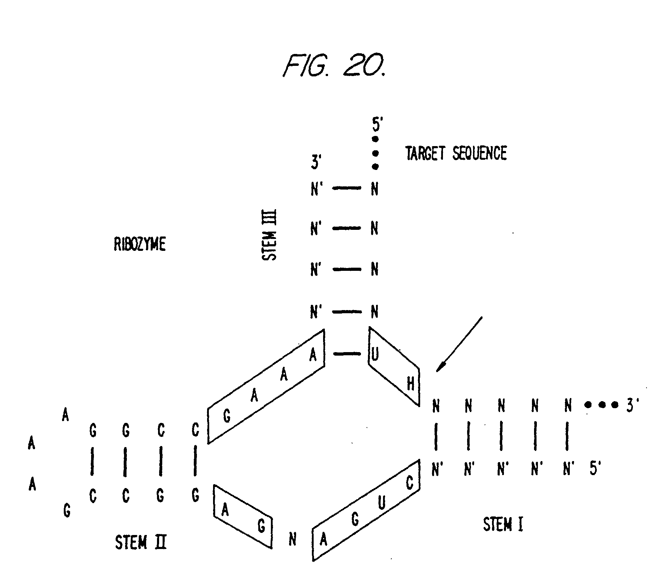

Such juxtapositioning may be achieved by allowing each half-ribozyme to pair at stem II of the ribozyme (see, Fig. 20, which may include between 4 and 7 base pairs, preferably 6 base pairs). Such pairing can be enhanced by increasing the length of stem II. The term "juxtapose" is used merely to indicate that the ribozyme portions are sufficiently close to either be ligated or to have enzymatic activity.

There follows examples of the invention. These examples are not limiting in this invention, and those in the art will recognize that other equivalent procedures can be used. Generally, any ribozyme can be synthesized by the method of this invention, and may have either intra- or inter-molecular cleaving enzymatic activity. Each portion of a ribozyme will generally have only limited or no such activity, and the activity will increase substantially (by at least 5-10 fold) when all portions are ligated (or otherwise juxtaposed) together. The specific examples that follow feature hammerhead ribozyme synthesis, but the invention is equally applicable to hairpin ribozymes, group I intron type ribozymes and hepatitis delta type ribozymes among others.

### Example 40: Synthesis of Hammerhead Ribozyme

Prior to this invention, ribozyme synthesis involved synthesizing a full length hammerhead ribozyme chemically, e.g., one having the structure shown in Fig. 20 where stems I and III are about 5-8 nucleotides in length, and the full length ribozyme includes between 32 and 38 nucleotides.

RNA synthesis is less efficient than DNA synthesis. Stepwise coupling constants for RNA synthesis are only about 96 to 98% (dependent upon the type of phosphoramidite used). Thus, the maximum yield of full length ribozyme for a given synthesis (assuming 97% coupling efficiency) is (0.97)³² or about 38% for a 32 mer, and (0.97)³⁸ or about 31% for a 38 mer.

A method of this invention is shown generally in Fig. 42, and features synthesis of two (or more) shorter "half" ribozymes and ligation of them together using T4 RNA ligase. Referring to Fig. 42, two 17 mers are synthesized, one is phosphorylated, and both are gel purified. These purified 17 mers are annealed to a DNA or RNA or other nucleic acid splint strand complementary to the two 17 mers. This splint has a sequence designed to locate the two portions with one end of each adjacent each other. The juxtaposed RNA molecules are then treated with T4 RNA ligase in the presence of ATP. The 34 mer RNA so formed is then HPLC purified.

The specific steps in this method will now be discussed: Synthesis of RNA 17 mers is practically trivial. Maximum yield for a 17 mer is about (0.97)¹⁷ or 60% (*cf.* about 30% for a 34 mer). Gel purification of a 17 mer can easily be accomplished on a 15% denaturing polyacrylamide gel with single nucleotide resolution, and the separated 17 mer easily removed from a gel slice. HPLC purification of a 17 mer from its failure sequences can be accomplished with much higher resolution than purification of a 34 mer. Resolution of the 34 mer is further reduced by secondary structure inherent to the ribozyme molecule.

After purification of the 3' segment or portion of the ribozyme it is phosphorylated in order to provide the 5' phosphate necessary for ligation. T4 RNA ligase can join the purified RNA molecules after alignment by a third "splint" strand as described by Linne et al., 42 Eur. J. Biochem. 157, 1974, in a manner analogous to T4 DNA ligase (Fareed et al., 246 J. Biol. Chem. 925, 1978). The arrangement shown in Fig. 42 juxtaposes the only 5' phosphate (on the 3' RNA fragment) with the 3' OH of the 5' fragment. The splint strand can be chosen to be generally useful, and can be used for any ribozyme with the desired core sequence, e.g., that shown in Fig. 42 (see, Forster and Symons, 50 Cell 9, 1987). The length of the DNA can be adjusted if necessary. Ligation with T4 RNA ligase can be accomplished in the same buffer as that of T4 polynucleotide kinase (i.e., 50 mM HEPES (pH 7.5), 20 mM MgCl₂, 3 mM DTT, 10 µg/ml nuclease free BSA). This facilitates synthesis since the 3' fragment can be used directly from the phosphorylation reaction (Beckett and Uhlenbeck, In Oligonucleotide Synthesis: A Practical Approach, Chapter 8, pp. 185-197).

After ligation there will be RNA product, DNA splint, RNA starting materials and protein and salt compounds. These can be separated by reversed phase HPLC, or by gel purification. Denaturants may be used to help remove the splint strand.

### Example 41: Synthesis of Several Ribozymes Simultaneously

It is sometimes useful to synthesize ribozymes having varying arm length to one target site. Such ribozymes can be studied to identify the "optimum" arm length at this target site. To vary each arm to include lengths of 5, 6, 7, or 8 nucleotides on each arm (i.e., 16 combinations of arm lengths) would require synthesizing 16 full length ribozymes.

Using the method of this invention, however, one need only synthesize four 5' fragments and four 3' fragments, which produces considerable savings in cost and time over synthesis of 16 different ribozymes. With these two sets of four half ribozymes, one can mix and match to get the desired combinations of arm lengths. One can also combine arms from various ribozymes to test various sequences of the arms to determine which form highly active ribozymes. One scheme for rapid synthesis of four such ribozymes involves ligation of each of four phosphorylated 3' fragments with a chosen 5' fragment using an appropriate DNA splint molecule. The resulting products can be separated by polyacrylamide gel electrophoresis (since each product differs by one nucleotide base in length). This procedure can be repeated with the other three 5' fragments. Thus, in four reactions and one gel, it is possible to purify enough material to provide the desired 16 different ribozymes.

### Example 42: Juxtaposed RNA Portions

Two-part or three-part ribozymes can be formed without a ligation step. For example, such ribozymes can be used to study the activity of a ribozyme as a function of arm length. Referring to Fig. 43, there is shown a ribozyme formed without the stem II loop closed. In this example, it is necessary only to mix and match arm lengths in equimolar amounts. Stem II can be made longer to allow the formation of a significant amount of 5' fragment: 3' fragment complex at 37°C.

### Example 43: No Splint Procedure

Referring to Fig. 45, there is shown an alternative scheme by which a ribozyme can be ligated without use of a splint strand. Specifically, the two half ribozymes are constructed so that they hybridize together to enhance ligation. This procedure is advantageous over that described above since the splint strand need not be later removed. Ligation with such a procedure is almost quantitative and complete within an hour.

### Example 44: Catalytic Activity of Ligated HIV Half-Ribozymes

RNA molecules were chemically synthesized having the nucleotide base sequence shown in Fig. 44 for both the 5' and 3' fragments. Three identical 5' fragments were synthesized except that rather than having all 3 Us at their 5' end they had 0, 1 or 2 such Us. Three identical 3' fragments were also synthesized except that rather than having CUG at their 3' ends, they had only CU, C or nothing. As is evident from the figure the 3' and 5' fragments can hybridize together at stem II, and cannot be ligated because the hybridized complex is blunt-ended. When this structure hybridizes to a substrate, a ribozyme structure is formed.

Forty nanomolar mixtures of the appropriate 3' and 5' fragments were incubated with 1-5 nM substrate for different times in 75 mM Tris/HCl pH 8.0, 10 mM MgCl₂ and shown to -cleave the substrate appropriately with an activity about half that of full length synthesized ribozyme.

### Example 45: Preferred Half Ribozymes

Referring to Fig. 46, there is shown the general structure of two half ribozymes where N is any desired nucleotide base. Thus, both the 3' and 5' fragments have about 21 bases each with stem II having a base paired region of at least 2 bases and preferably between 3 and 5 bases with a loop region containing at least 4 bases (e.g., on the 3' fragment). Stems I and III are designed to have at least 3 base pairs, and preferably between 4 and 8 base pairs.

### Ribozyme Testing

once the desired ribozymes are selected, synthesized and purified, they are tested in kinetic and other experiments to determine their utility. An example of such a procedure is provided below.

### Preparation of Ribozyme

Crude synthetic ribozyme (typically 350 µg at a time) is purified by separation on a 15% denaturing polyacrylamide gel (0.75 mm thick, 40 cm long) and visualized by UV shadowing. Once excised, gel slices containing full length ribozyme are soaked in 5 ml gel elution buffer (0.5 M NH₄OAc, 1 mM EDTA) overnight with shaking at 4°C. The eluent is desalted over a C-18 matrix (Sep-Pak cartridges, Millipore, Milford, MA) and vacuum dried. The dried RNA is resuspended in 50-100 µl TE (TRIS 10 mM, EDTA 1 mM, pH 7.2). An aliquot of this solution is diluted 100-fold into 1 ml TE, half of which is used to spectrophotometrically quantitate the ribozyme solution. The concentration of this dilute stock is typically 150-800 nM. Purity of the ribozyme is confirmed by the presence of a single band on a denaturing polyacrylamide gel.

A ribozyme may advantageously be synthesized in two or more portions. Each portion of a ribozyme will generally have only limited or no enzymatic activity, and the activity will increase substantially (by at least 5-10 fold) when all portions are ligated (or otherwise juxtaposed) together. A specific example of hammerhead ribozyme synthesis is provided below.

The method involves synthesis of two (or more) shorter "half" ribozymes and ligation of them together using T4 RNA ligase. For example, to make a 34 mer ribozyme, two 17 mers are synthesized, one is phosphorylated, and both are gel purified. These purified 17 mers are then annealed to a DNA splint strand complementary to the two 17 mers. This DNA splint has a sequence designed to locate the two 17 mer portions with one end of each adjacent each other. The juxtaposed RNA molecules are then treated with T4 RNA ligase in the presence of ATP. Alternatively, the DNA splint strand may be omitted from the ligation reaction if the complementary binding affects favorable ligation of the two RNA molecules. The 34 mer RNA so formed is then HPLC purified.

### Preparation of Substrates

Approximately 10-30 pmoles of unpurified substrate is radioactively 5' end-labeled with T4 polynucleotide kinase using 25 pmoles of [γ-³²P] ATP. The entire labeling mix is separated on a 20% denaturing polyacrylamide gel and visualized by autoradiography. The full length band is excised and soaked overnight at 4°C in 100 µl of TE (10 mM Tris-HCl pH 7.6, 0.1 mM EDTA).

### Kinetic Reactions

For reactions using short substrates (between 8 and 16 bases) a substrate solution is made 1X in assay buffer (75 mM Tris-HCl, pH 7.6; 0.1 mM EDTA, 10 mM MgCl₂) such that the concentration of substrate is less than 1 nM. A ribozyme solution (typically 20 nM) is made 1X in assay buffer and four dilutions are made using 1X assay buffer. Fifteen µl of each ribozyme dilution (i.e., 20, 16, 12, 8 and 4 nM) is placed in a separate tube. These tubes and the substrate tube are pre-incubated at 37°C for at least five minutes.

The reaction is started by mixing 15 µl of substrate into each ribozyme tube by rapid pipetting (note that final ribozyme concentrations are 10, 8, 6, 4, 2 nM). Five µl aliquots are removed at 15 or 30 second intervals and quenched with 5 µl stop solution (95% formamide, 20 mM EDTA xylene cyanol, and bromphenol blue dyes). Following the final ribozyme time point, an aliquot of the remaining substrate is removed as a zero ribozyme control.

The samples are separated on either 15% or 20% polyacrylamide gels. Each gel is visualized and quantitated with an Ambis beta scanner (Ambis Systems, San Diego, CA).

For the most active ribozymes, kinetic analyses are performed in substrate excess to determine Kₘ and K_{cat} values.

For kinetic reactions with long RNA substrates (greater than 15 bases in length) the substrates are prepared by transcription using T7 RNA polymerase and defined templates containing a T7 promoter, and DNA encoding appropriate nucleotides of the viral RNA. The substrate solution is made 1X in assay buffer (75 mM Tris-HCl, pH 7.6; 0.1 mM EDTA; 10 mM MgCl₂) and contains 58 nanomolar concentration of the long RNA molecules. The reaction is started by addition of gel purified ribozymes to 1 µM concentration. Aliquots are removed at 20, 40, 60, 80 and 100 minutes, then quenched by the addition of 5 µl stop solution. Cleavage products are separated using denaturing PAGE. The bands are visualized and quantitated with an Ambis beta scanner. In one example, a long substrate RNA transcript corresponding to nucleotides 1-493 of the HSV-1 ICP4 mRNA is synthesized *in vitro* with T7 RNA polymerase from a defined template containing T7 promoter and DNA encoding nucleotides 1-493 of ICP4.

### Kinetic Analysis

A simple reaction mechanism for ribozyme-mediated cleavage is: 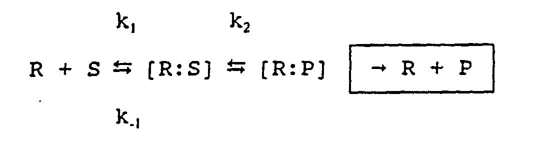 where R = ribozyme, S = substrate, and P = products. The boxed step is important only in substrate excess. Because ribozyme concentration is in excess over substrate concentration, the concentration of the ribozyme-substrate complex ([R:S]) is constant over time except during the very brief time when the complex is being initially formed, i.e.,:$\frac{\text{d[R:S]}}{\text{dt}} \text{= 0}$ where t = time, and thus:${\text{(R) (S)k}}_{\text{1}} {\text{= (RS) (k}}_{\text{2}} {\text{+ k}}_{\text{1}} \text{).}$ The rate of the reaction is the rate of disappearance of substrate with time:$\text{Rate =} \frac{\text{-d(S)}}{\text{dt}} {\text{= k}}_{\text{2}} \text{(RS)}$ Substituting these expressions:${\text{(R) (S)k}}_{\text{1}} {\text{= 1/k}}_{\text{2}} \frac{\text{-d(S)}}{\text{dt}} {\text{(k}}_{\text{2}} {\text{+ k}}_{\text{1}} \text{)}$ or:$\frac{\text{-d(S)}}{\text{S}} \text{=} \frac{{\text{k}}_{\text{1}} {\text{k}}_{\text{2}}}{{\text{(k}}_{\text{2}} {\text{+ k}}_{\text{1}} \text{)}} \text{(R) dt}$ Integrating this expression with respect to time yields:$\text{-ln} \frac{\text{S}}{{\text{S}}_{\text{0}}} \text{=} \frac{{\text{k}}_{\text{1}} {\text{k}}_{\text{2}}}{{\text{(k}}_{\text{2}} {\text{+ k}}_{\text{1}} \text{)}} \text{(R) t}$ where S₀ = initial substrate. Therefore, a plot of the negative log of fraction substrate uncut versus time (in minutes) yields a straight line with slope:$\text{slope =} \frac{{\text{k}}_{\text{1}} {\text{k}}_{\text{2}}}{{\text{(k}}_{\text{2}} {\text{+ k}}_{\text{1}} \text{)}} {\text{(R) = k}}_{\text{obs}}$ where k_{obs} = observed rate constant. A plot of slope (k_{obs}) versus ribozyme concentration yields a straight line with a slope which is:$\text{slope =} \frac{{\text{k}}_{\text{1}} {\text{k}}_{\text{2}}}{{\text{(k}}_{\text{2}} {\text{+ k}}_{\text{1}} \text{)}}$ which is $\frac{{\text{k}}_{\text{cat}}}{{\text{K}}_{\text{m}}}$

Using these equations the data obtained from the kinetic experiments provides the necessary information to determine which ribozyme tested is most useful, or active. Such ribozymes can be selected and tested in *in vivo* or *ex vivo* systems. An example of preparing ribozymes in a liposome delivery vehicle is given below.

### Liposome Preparation

There follows an example of the entrapment of ribozyme molecules within a liposome drug delivery vehicle. Lipid molecules were dissolved in a volatile organic solvent (CHCl₃, methanol, diethylether, ethanol, etc.). The organic solvent was removed by evaporation. The lipid was hydrated into suspension with 0.1x phosphate buffered saline (PBS), then freeze-thawed 3x using liquid nitrogen and incubation at room temperature. The suspension was extruded sequentially through 0.4 µm, 0.2 µm and 0.1 µm polycarbonate filters at maximum pressure of 800 psi. The ribozyme was mixed with the extruded liposome suspension and lyophilized to dryness. The lipid/ribozyme powder was rehydrated with water to one-tenth the original volume. The suspension was diluted to the minimum volume required for extrusion (0.4 ml for 1.5 ml barrel and 1.5 ml for 10 ml barrel) with 1xPBS and re-extruded through 0.4 µm, 0.2 µm, 0.1 µm polycarbonate filters. The liposome entrapped ribozyme was separated from untrapped ribozyme by gel filtration chromatography (SEPHAROSE CL-4B, BIOGEL A5M). The liposome extractions were pooled and sterilized by filtration through a 0.2 µm filter. The free ribozyme was pooled and recovered by ethanol precipitation. The liposome concentration was determined by incorporation of a radioactive lipid. The ribozyme concentration was determined by labeling with ³²P. Rossi et al., 1992, *supra* (and references cited therein), describe other methods suitable for preparation of liposomes.

In experiments with a liposome formulation composed of a synthetic lipid derivative disteraoylphosphatidylethylamidothioacetyl succinimide (DSPE-ATS) co-formulated with dipalmitoylphosphatidyl choline and cholesterol we observed uptake of 100 and 200 nm diameter liposomes with similar kinetics. The larger particles accommodated a larger number of entrapped molecules, or larger molecular weight molecules, such as an expression plasmid. These particles showed a linear relationship between the lipid dose offered and the mean log fluorescence (calcine was used to follow liposome uptake). No cytotoxicity was observed even with a 200 µM dose. These liposomes are particularly useful for delivery to CD4 cell populations.

### In Vivo Assay

The efficacy of action of a chosen ribozyme may be tested *in vivo* by use of cell cultures sensitive to a selected virus, using standard procedures. For example, monolayer cultures of virus-sensitive or virus-expressing cells are grown by established procedures. Cells are grown in 6 or 96 well tissue culture plates or in raft cultures of virus-permissive cells as described previously. Kopan et al., 105 J. Cell Biol. 427, 1987. Prior to infection with virus cultures are treated for 3 to 24 hours with ribozyme-containing liposomes. Cells are then rinsed with phosphate buffered saline (PBS) and virus added at a multiplicity of 1-100 pfu/cell. After a one-hour adsorption, free virus is rinsed away using PBS, and viral RNA is harvested for analysis. Alternatively, the cells are treated for three to five days with appropriate liposome preparations and fresh medium changes. Cells may then be re-fed with fresh medium and re-incubated. Virus is harvested from cells into the overlying medium. Cells are broken by three cycles of incubation at -70°C and 37°C for 30 minutes at each temperature, and viral titers determined by plaque assay using established procedures. These procedures can be modified for each specific virus to be tested. Viral RNA can be harvested using the guanidinium isothiocyanate procedure and the resultant RNA preparation is subjected to analysis using the RNase protection assay.

Alternatively, transformed cell types (e.g., HeLa cells for expression of HPV-18 E6 and E7 mRNA or Caski cells for expression HPV-18 E6 and E7 mRNA / Vero cells) are used to assess the ability of ribozyme preparations to cleave HPV/HSV mRNA and reduce the expression of target proteins. Cell monolayers are treated with ribozyme containing liposome preparations for between 3 and 96 hours. For RNA analyses, the cells are lysed in guanidine isothiocyanate solutions and the RNA are analyzed using the RNase protection assay. For protein quantitation, cell lysates are prepared according to methods known to those in the art and proteins are quantified by immunoanalysis using anti-HPV E6 and E7 / anti-HSV protein specific antibodies.

For HBV, cells are rinsed with phosphate buffered saline (PBS) and HBV DNA is transfected into the cells by the calcium phosphate technique. The cells are treated for three to five days with appropriate liposome preparations in fresh changes of medium. For HCV total cellular RNA is harvested by the guanidine isothiocyanate technique and the amount of virus mRNA is quantified and assessed for ribozyme-mediated cleavage using the ribonuclease protection assay. Alternatively, cellular lysates can be prepared and the core particles of virus could be immunoprecipitated using polyclonal anti-core antiserum adsorbed to protein A-Sepharose according to manufacturers directions. The precipitated cores are treated with ribonuclease to digest any non-encapsidated RNAs and the core protein is digested with proteinase K/phenol extraction. Usually one-half of the total RNA and one-half of the extracted RNA is analyzed using the ribonuclease protection assay.

### Ribozyme Stability

The chosen ribozyme can be tested to determine its stability, and thus its potential utility. Such a test can also be used to determine the effect of various chemical modifications (e.g., addition of a poly(A) tail) on the ribozyme stability and thus aid selection of a more stable ribozyme. For example, a reaction mixture contains 1 to 5 pmoles of 5' (kinased) and/or 3' labeled ribozyme, 15 µg of cytosolic extract and 2.5 mM MgCl₂ in a total volume of 100 µl. The reaction is incubated at 37°C. Eight µl aliquots are taken at timed intervals and mixed with 8 µl of a stop mix (20 mM EDTA, 95% formamide). Samples are separated on a 15% acrylamide sequencing gel, exposed to film, and scanned with an Ambis.

A 3'-labeled ribozyme can be formed by incorporation of the ³²P-labeled cordycepin at the 3' OH using poly(A) polymerase. For example, the poly(A) polymerase reaction contains 40 mM Tris, pH 8, 10 mM MgCl₂, 250 mM NaCl, 2.5 mM MnCl₂,; 3 µl P³² cordycepin, 500 Ci/mM; and 6 units poly(A) polymerase in a total volume of 50 µl. The reaction mixture is incubated for 30 minutes at 37°C.

### Effect of Base Substitution upon Ribozyme Activity

To determine which primary structural characteristics could change ribozyme cleavage of substrate, minor base changes can be made in the substrate cleavage region recognized by a specific ribozyme. For example, the substrate sequences can be changed at the central "C" nucleotide, changing the cleavage site from a GUC to a GUA motif. The K_{cat}/Kₘ values for cleavage using each substrate are then analyzed to determine if such a change increases ribozyme cleavage rates. Similar experiments can be performed to address the effects of changing bases complementary to the ribozyme binding arms. Changes predicted to maintain strong binding to the complementary substrate are preferred. Minor changes in nucleotide content can alter ribozyme/substrate interactions in ways which are unpredictable based upon binding strength alone. Structures in the catalytic core region of the ribozyme recognize trivial changes in either substrate structure or the three dimensional structure of the ribozyme/substrate complex.

To begin optimizing ribozyme design, the cleavage rates of ribozymes containing varied arm lengths, but targeted to the same length of short RNA substrate can be tested. Minimal arm lengths are required and effective cleavage varies with ribozyme/substrate combinations.

The cleavage activity of selected ribozymes can be assessed using virus or virus-homologous RNA substrates or virus genomic RNA. The assays are performed in ribozyme excess and approximate K_{cat}/Kₘᵢₙ values obtained. Comparison of values obtained with short and long substrates indicates utility *in vivo* of a ribozyme.

### Intracellular Stability of Liposome-Delivered Ribozymes

To test the stability of a chosen ribozyme *in vivo* the following test is useful. Ribozymes are ³²P-end labeled, entrapped in liposomes and delivered to virus sensitive cells for three hours. The cells are fractionated and ribozyme is purified by phenol/chloroform extraction. Cells (1x10⁷, T-175 flask) are scraped from the surface of the flask and washed twice with cold PBS. The cells are homogenized by douncing 35 times in 4 ml of TSE (10 mM Tris, pH 7.4, 0.25 M Sucrose, mM EDTA). Nuclei are pelleted at 100xg for 10 minutes. Subcellular organelles (the membrane fraction) are pelleted at 200,000xg for two hours using an SM60 rotor. The pellet is resuspended in 1 ml of H buffer (0.25 M Sucrose, 50 mM HEPES, pH 7.4). The supernatant contains the cytoplasmic fraction (in approximately 3.7 ml). The nuclear pellet is resuspended in 1 ml of 65% sucrose in TM (50 mM Tris, pH 7.4, 2.5 mM MgCl₂) and banded on a sucrose step gradient (1 ml nuclei in 65% sucrose TM, 1 ml 60% sucrose TM, 1 ml 55% sucrose TM, 50% sucrose TM, 300 µl 25% sucrose TM) for one hour at 37,000xg with an SW60 rotor. The nuclear band is harvested and diluted to 10% sucrose with TM buffer. Nuclei are pelleted at 37,000xg using an SW60 rotor for 15 minutes and the pellet resuspended in 1 ml of TM buffer. Aliquots are size fractionated on denaturing polyacrylamide gels and the intracellular localization determined. By comparison to the migration rate of newly synthesized ribozyme, the various fraction containing intact ribozyme can be determined.

To investigate modifications which would lengthen the half-life of ribozyme molecules intracellularly, the cells may be fractioned as above and the purity of each fraction assessed by assaying enzyme activity known to exist in that fraction.

The various cell fractions are frozen at -70°C and used to determine relative nuclease resistances of modified ribozyme molecules. Ribozyme molecules may be synthesized with 5 phosphorothioate (ps), or 2'-O-methyl (2'-OMe) modifications at each end of the molecule. These molecules and a phosphodiester version of the ribozyme are end-labeled with ³²P and ATP using T4 polynucleotide kinase. Equal concentrations are added to the cell cytoplasmic extracts and aliquots of each taken at 10 minute intervals. The samples are size fractionated by denaturing PAGE and relative rates of nuclease resistance analyzed by scanning the gel with an Ambis β-scanner. The results show whether the ribozymes are digested by the cytoplasmic extract, and which versions are relatively more nuclease resistant. Modified ribozymes generally maintain 80-90% of the catalytic activity of the native ribozyme when short RNA substrates are employed.

Unlabeled, 5' end-labeled or 3' end-labeled ribozymes can be used in the assays. These experiments can also be performed with human cell extracts to verify the observations.

### Administration of Ribozyme

Selected ribozymes can be administered prophylactically, or to virus infected patients, e.g., by exogenous delivery of the ribozyme to an infected tissue by means of an appropriate delivery vehicle, e.g., a liposome, a controlled release vehicle, by use of iontophoresis, electroporation or ion paired molecules, or covalently attached adducts, and other pharmacologically approved methods of delivery. Routes of administration include intramuscular, aerosol, oral (tablet or pill form), topical, systemic, ocular, intraperitoneal and/or intrathecal. Expression vectors for immunization with ribozymes and/or delivery of ribozymes are also suitable. Of particular value is the use of the CMV immediate early promoter in expression vectors because this promoter is known to exhibit very good activity in a number of cell types, including epithelial cells.

The specific delivery route of any selected ribozyme will depend on the use of the ribozyme. Generally, a specific delivery program for each ribozyme will focus on naked ribozyme uptake with regard to intracellular localization, followed by demonstration of efficacy. Alternatively, delivery to these same cells in an organ or tissue of an animal can be pursued. Uptake studies will include uptake assays to evaluate cellular oligonucleotide uptake, regardless of the delivery vehicle or strategy. Such assays will also determine the intracellular localization of the ribozyme following uptake, ultimately establishing the requirements for maintenance of steady-state concentrations within the cellular compartment containing the target sequence (nucleus and/or cytoplasm). Efficacy and cytotoxicity can then be tested. Toxicity will not only include cell viability but also cell function.

Some methods of delivery that may be used include:
a. encapsulation in liposomes,
b. transduction by retroviral vectors,
c. conjugation with cholesterol,
d. localization to nuclear compartment utilizing antigen binding or nuclear targeting sites found on most snRNAs or nuclear proteins,
e. neutralization of charge of ribozyme by using nucleotide derivatives, and
f. use of blood stem cells to distribute ribozymes throughout the body.

At least three types of delivery strategies are useful in the present invention, including: ribozyme modifications, particle carrier drug delivery vehicles, and retroviral expression vectors. Unmodified ribozymes like most small molecules, are taken up by cells, albeit slowly. To enhance cellular uptake, the ribozyme may be modified essentially at random, in ways which reduce its charge but maintains specific functional groups. This results in a molecule which is able to diffuse across the cell membrane, thus removing the permeability barrier.

Modification of ribozymes to reduce charge is just one approach to enhance the cellular uptake of these larger molecules. The random approach, however, is not advisable since ribozymes are structurally and functionally more complex than small drug molecules. The structural requirements necessary to maintain ribozyme catalytic activity are well understood by those in the art. These requirements are taken into consideration when designing modifications to enhance cellular delivery. The modifications are also designed to reduce susceptibility to nuclease degradation. Both of these characteristics should greatly improve the efficacy of the ribozyme. Cellular uptake can be increased by several orders of magnitude without having to alter the phosphodiester linkages necessary for ribozyme cleavage activity.

Chemical modifications of the phosphate backbone will reduce the negative charge allowing free diffusion across the membrane. This principle has been successfully demonstrated for antisense DNA technology. The similarities in chemical composition between DNA and RNA make this a feasible approach. In the body, maintenance of an external concentration will be necessary to drive the diffusion of the modified ribozyme into the cells of the tissue. Administration routes which allow the diseased tissue to be exposed to a transient high concentration of the drug, which is slowly dissipated by systemic adsorption are preferred. Intravenous administration with a drug carrier designed to increase the circulation half-life of the ribozyme can be used. The size and composition of the drug carrier restricts rapid clearance from the blood stream. The carrier, made to accumulate at the site of infection, can protect the ribozyme from degradative processes.

Drug delivery vehicles are effective for both systemic and topical administration. They can be designed to serve as a slow release reservoir, or to deliver their contents directly to the target cell. An advantage of using direct delivery drug vehicles is that multiple molecules are delivered per uptake. Such vehicles have been shown to increase the circulation half-life of drugs which would otherwise be rapidly cleared from the blood stream. Some examples of such specialized drug delivery vehicles which fall into this category are liposomes, hydrogels, cyclodextrins, biodegradable nanocapsules, and bioadhesive microspheres.

From this category of delivery systems, liposomes are preferred. Liposomes increase intracellular stability, increase uptake efficiency and improve biological activity.

Liposomes are hollow spherical vesicles composed of lipids arranged in a similar fashion as those lipids which make up the cell membrane. They have an internal aqueous space for entrapping water soluble compounds and range in size from 0.05 to several microns in diameter. Several studies have shown that liposomes can deliver RNA to cells and that the RNA remains biologically active.

For example, a liposome delivery vehicle originally designed as a research tool, Lipofectin, has been shown to deliver intact mRNA molecules to cells yielding production of the corresponding protein. In another study, an antibody targeted liposome delivery system containing an RNA molecule 3,500 nucleotides in length and antisense to a structural protein of HIV, inhibited virus proliferation in a sequence specific manner. Not only did the antibody target the liposomes to the infected cells, but it also triggered the internalization of the liposomes by the infected cells. Triggering the endocytosis is useful for viral inhibition. Finally, liposome delivered synthetic ribozymes have been shown to concentrate in the nucleus of H9 (an example of an HIV-sensitive cell) cells and are functional as evidenced by their intracellular cleavage of the sequence. Liposome delivery to other cell types using smaller ribozymes (less than 142 nucleotides in length) exhibit different intracellular localizations.

Liposomes offer several advantages: They are non-toxic and biodegradable in composition; they display long circulation half-lives; and recognition molecules can be readily attached to their surface for targeting to tissues. Finally, cost effective manufacture of liposome-based pharmaceuticals, either in a liquid suspension or lyophilized product, has demonstrated the viability of this technology as an acceptable drug delivery system.

Other controlled release drug delivery systems, such as nonoparticles and hydrogels may be potential delivery vehicles for a ribozyme. These carriers have been developed for chemotherapeutic agents and protein-based pharmaceuticals, and consequently, can be adapted for ribozyme delivery.

Topical administration of ribozymes is advantageous since it allows localized concentration at the site of administration with minimal systemic adsorption. This simplifies the delivery strategy of the ribozyme to the disease site and reduces the extent of toxicological characterization. Furthermore, the amount of material to be applied is far less than that required for other administration routes. Effective delivery requires the ribozyme to diffuse into the infected cells. Chemical modification of the ribozyme to neutralize negative charge may be all that is required for penetration. However, in the event that charge neutralization is insufficient, the modified ribozyme can be co-formulated with permeability enhancers, such as Azone or oleic acid, in a liposome. The liposomes can either represent a slow release presentation vehicle in which the modified ribozyme and permeability enhancer transfer from the liposome into the infected cell, or the liposome phospholipids can participate directly with the modified ribozyme and permeability enhancer in facilitating cellular delivery. In some cases, both the ribozyme and permeability enhancer can be formulated into a suppository formulation for slow release.

Ribozymes may also be systemically administered. Systemic absorption refers to the accumulation of drugs in the blood stream followed by distribution throughout the entire body. Administration routes which lead to systemic absorption include: intravenous, subcutaneous, intraperitoneal, intranasal, intrathecal and ophthalmic. Each of these administration routes expose the ribozyme to an accessible diseased tissue. Subcutaneous administration drains into a localized lymph node which proceeds through the lymphatic network into the circulation. The rate of entry into the circulation has been shown to be a function of molecular weight or size. The use of a liposome or other drug carrier localizes the ribozyme at the lymph node. The ribozyme can be modified to diffuse into the cell, or the liposome can directly participate in the delivery of either the unmodified or modified ribozyme to the cell. This method is particularly useful for treating AIDS/T-cell leukemia using anti-HIV/HTLV ribozymes of this invention.

A liposome formulation which can deliver ribozymes to lymphocytes and macrophages is also useful for the initial site of enterovirus replication is in lymphoid tissue of the nasopharynx and gut. Coating of lymphocytes with liposomes containing ribozymes will target the ribozymes to infected cells expressing viral surface antigens. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration.

Also preferred in AIDS therapy is the use of a liposome formulation which can deliver oligonucleotides to lymphocytes and macrophages. This oligonucleotide delivery system inhibits HIV proliferation in infected primary immune cells. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration. This formulation offers an excellent delivery vehicle for anti-AIDS ribozymes for two reasons. First, T-helper lymphocytes and macrophages are the primary cells infected by the virus, and second, a subcutaneous administration delivers the ribozymes to the resident HIV-infected lymphocytes and macrophages in the lymph node. The liposomes then exit the lymphatic system, enter the circulation, and accumulate in the spleen, where the ribozyme is delivered to the resident lymphocytes and macrophages.

A liposome formulation which can associate ribozymes with the surface of lymphocytes and macrophages is also useful. This will provide enhanced delivery to HPV-infected cells by taking advantage of the specificity of macrophage and lymphocyte immune recognition of infected cells. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration.

Preferred in EBV therapy is the use of a liposome formulation which can deliver oligonucleotides to lymphocytes and macrophages. This oligonucleotide delivery system inhibits EBV proliferation in infected primary immune cells. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration. This formulation offers an excellent delivery vehicle for anti-EBV ribozymes for two reasons. First, T-helper lymphocytes and macrophages are the primary cells infected by the virus, and second, a subcutaneous administration delivers the ribozymes to the resident EBV-infected lymphocytes and macrophages in the lymph node. The liposomes then exit the lymphatic system, enter the circulation, and accumulate in the spleen, where the ribozyme is delivered to the resident lymphocytes and macrophages.

Preferred in T-cell leukemia therapy is the use of a liposome formulation which can deliver ribozymes to lymphocytes and macrophages. This ribozyme delivery system inhibits HTLV proliferation in infected primary immune cells. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration. This formulation offers an excellent delivery vehicle for anti-HTLV-I or II ribozymes for two reasons. First, T-helper lymphocytes are the primary cells infected by the virus, and second, a subcutaneous administration delivers the ribozymes to the resident HTLV-infected lymphocytes in the lymph node. The liposomes then exit the lymphatic system, enter the circulation, and accumulate in the spleen, where the ribozyme is delivered to the resident lymphocytes and macrophages.

A liposome formulation which can deliver ribozymes to lymphocytes and macrophages is also useful for the initial site of influenza virus replication is in tissues of the nasopharynx and respiratory system. Coating of lymphocytes with liposomes containing ribozymes will target the ribozymes to infected cells expressing viral surface antigens. Whole blood studies show that the formulation is taken up by 90% of the lymphocytes after 8 hours at 37 °C. Preliminary biodistribution and pharmacokinetic studies yielded 70% of the injected dose/gm of tissue in the spleen after one hour following intravenous administration.

A liposome formulation which can associate ribozymes with the surface of lymphocytes and macrophages is also useful. This will provide enhanced delivery to HSV-infected cells by taking advantage of the specificity of macrophage and lymphocyte immune recognition of infected cells. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration.

Intraperitoneal administration also leads to entry into the circulation, with once again, the molecular weight or size of the ribozyme-delivery vehicle complex controlling the rate of entry.

Liposomes injected intravenously show accumulation in the liver, lung and spleen. The composition and size can be adjusted so that this accumulation represents 30% to 40% of the injected dose. The rest is left to circulate in the blood stream for up to 24 hours.

The chosen method of delivery will result in cytoplasmic accumulation in the afflicted cells and molecules should have some nuclease-resistance for optimal dosing. Nuclear delivery may be used but is less preferable. Most preferred delivery methods include liposomes (10-400 nm), hydrogels, controlled-release polymers, microinjection or electroporation (for *ex vivo* treatments) and other pharmaceutically applicable vehicles. The dosage will depend upon the disease indication and the route of administration but should be between 100-200 mg/kg of body weight/day. The duration of treatment will extend through the course of the disease symptoms, usually at least 14-16 days and possibly continuously. Multiple daily doses are anticipated for topical applications, ocular applications and vaginal applications. The number of doses will depend upon disease delivery vehicle and efficacy data from clinical trials.

Establishment of therapeutic levels of ribozyme within the cell is dependent upon the rate of uptake and degradation. Decreasing the degree of degradation will prolong the intracellular half-life of the ribozyme. Thus, chemically modified ribozymes, e.g., with modification of the phosphate backbone, or capping of the 5' and 3' ends of the ribozyme with nucleotide analogues may require different dosaging. Descriptions of useful systems are provided in the art cited above, all of which is hereby incorporated by reference herein.

The claimed ribozymes are also useful as diagnostic tools to specifically or non-specifically detect the presence of a target RNA in a sample. That is, the target RNA, if present in the sample, will be specifically cleaved by the ribozyme, and thus can be readily and specifically detected as smaller RNA species. The presence of such smaller RNA species is indicative of the presence of the target RNA in the sample.

### Example 46: HIV tat Ribozymes

The 5' exon of tat contains a number of potential cleavage sites. Eighteen of these: 2 GUC sites, 3 GUA sites, 5 AUC sites, 3 UCC sites, and 5 CUC sites were examined by computer folding and RNaseH assay to assess their accessibility for ribozyme cleavage.

A measure of the accessibility of each site to binding by a 13-mer oligonucleotide was preferred in the following way:
(a) The first 425 nucleotides of the clone V sequence (this clone was made available by Dr. Rossi [City of Hope Medical Research Center; Duarte, CA] and includes the 5' tat exon at nucleotides 151-366) was folded on RNAFOLD 4.0 (a generally available program) and examined for the presence of folding domains, i.e., self-contained structures closed by a stem.
(b) For each potential cleavage site, the domain containing that site was folded to confirm that it folded as in part (a); then the domain was refolded while forcing the cleavage site and surrounding nucleotides (11-16 nucleotides in all) to remain unpaired. The difference in these two folding free energies was taken as the cost of melting out that region for base-pairing by a ribozyme or DNA oligonucleotide.
(c) The lengths of DNA oligonucleotides were adjusted to give predicted delta-G (binding) of -17 to -18 kcal/mole. Thus, differences in overall binding energy was predicted to be reflected in the free energy differences calculated in part (b). The calculations are shown in Table 5.

**Table 5**

| | | Delta G | | Delta G | | |
|---|---|---|---|---|---|---|
| Total Start Seg | | # Site | Melting Structure | Length | Binding Oligo | Delta G Binding |
| 194 | GUC | 1 (@200) | + 8.6 | 11 | -17.2 | - 8.6 |
| 332 | GUC | 2 (@338) | + 8.2 | 11 | -17.3 | - 9.1 |
| 156 | GUA | 3 (@163) | + 7.5 | 12 | -17.7 | -10.2 |
| 211 | GUA | 4 (@218) | + 8.0 | 13 | -17.5 | - 9.7 |
| 225 | GUA | 5 (@233) | + 6.8 | 15 | -16.3 | - 9.5 |
| 161 | AUC | 6 (@167) | + 5.4 | 13 | -18.0 | -12.6 |
| 186 | AUC | 7 (@192) | + 1.6 | 11 | -17.7 | -16.1 |
| 280 | AUC | 8 (@286) | + 5.5 | 11 | -17.9 | -12.4 |
| 340 | AUC | 9 (@347) | + 7.1 | 13 | -17.0 | - 9.9 |
| 352 | AUC | 10 (@360) | + 1.7 | 14 | -17.4 | -15.7 |
| 240 | UUC | 11 (@248) | +11.7 | 14 | -17.4 | - 5.7 |
| 257 | UUC | 12 (@265) | +10.7 | 16 | -17.4 | - 6.7 |
| 346 | UUC | 13 (@354) | + 9.7 | 14 | -17.2 | - 7.5 |
| 281 | CUC | 14 (@288) | + 5.5 | 12 | -17.6 | -12.1 |
| 319 | CUC | 15 (@326) | +11.9 | 11 | -17.1 | - 5.2 |
| 322 | CUC | 16 (@329) | +13.8 | 11 | -17.5 | - 3.7 |
| 337 | CUC | 17 (@344) | + 6.3 | 12 | -17.2 | -10.9 |
| 349 | CUC | 18 (@356) | +10.6 | 14 | -17.2 | - 6.6 |
| [site refers to nucleotide 5' of cleaved phosphate] | | | | | | |

Eighteen DNA oligonucleotides were made to target the 18 target sites listed in Table 5. RNaseH experiments were performed using approximately 100 nM body labeled RNA transcript (clone V), 0.08 U/µl RNaseH (excess RNaseH) and a 2x dilution of 1 mM, 10 µM or 1 µM DNA oligonucleotide. The results are shown in Table 6. Eight out of 18 sites had greater than 40% cleavage at 5 µM after 10 minutes incubation. Three ribozymes were designed against the three most active sequences (H332, H337b, H352, see Table 6).

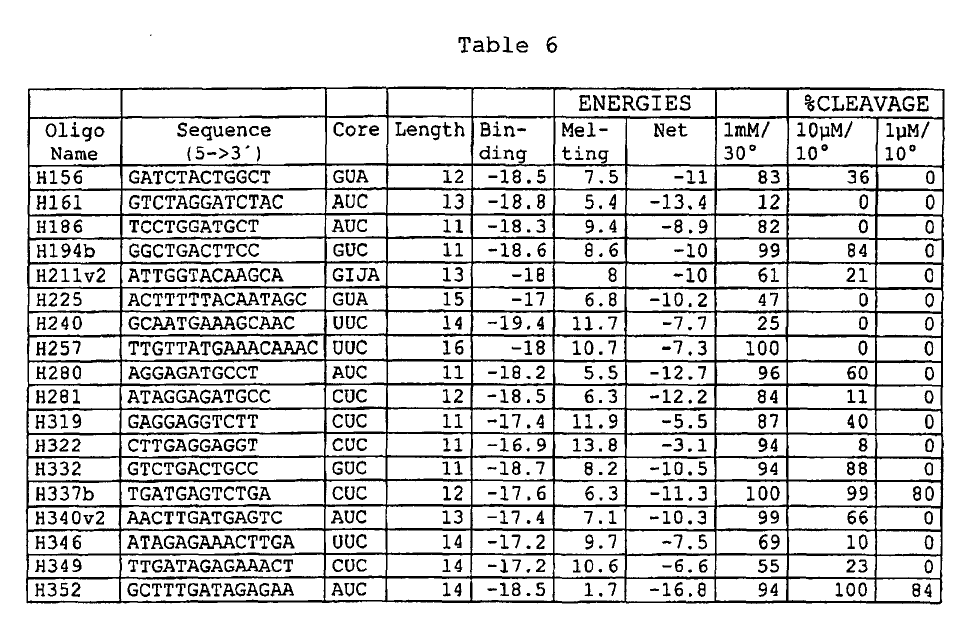

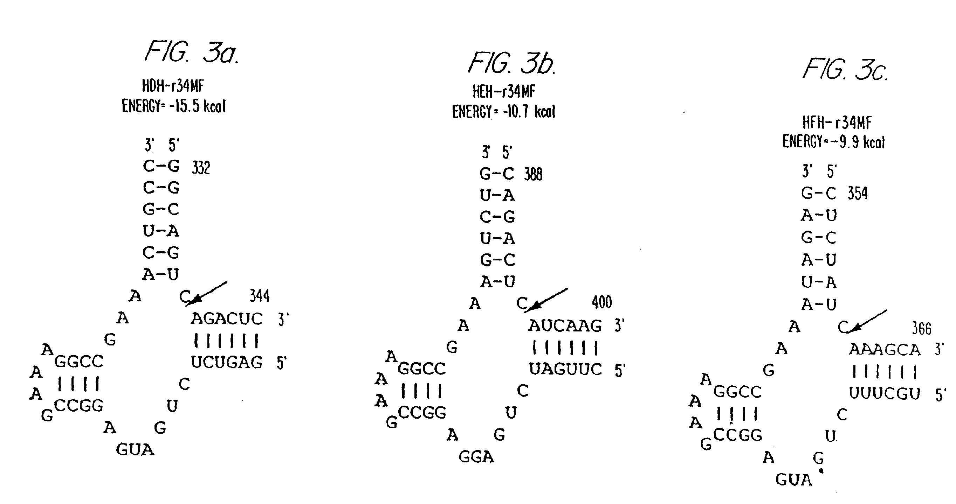

These three ribozymes are shown in Figs. 3A, B and C, labeled respectively HDH, HEH and HFH.

### Example 47: Ribozymes Containing Thiophosphate

The purpose of this example was to evaluate the activity of ribozymes containing substitutions of thiophosphate for phosphate at some backbone positions.

Ribozymes to the HIV-1 tat gene were synthesized on an ABI synthesizer using standard phosphoramidite chemistry, however, at steps where thiophosphate was to be incorporated in the backbone the standard oxidation step (involving Iodine) was replaced with an oxidation step utilizing the Beaucage sulfur transfer reagent. Deprotected and desalted RNA was gel purified, eluted, kinased and sequenced to ensure that the sequence was correct. The end-labeled RNA was also treated with H2O2 which preferentially promotes cleavage at positions containing thiophosphate.

Ribozyme activity was tested against cleavage of a short (12 nucleotides) end-labeled substrate RNA. Substrate concentration was approximately 1 nM; ribozyme concentration was 5-100 nM; incubation was at 37°C in 75 mM Tris (pH 7.5), 0.1 mM EDTA, 10 mM MgCl₂ for 2 - 40 minutes. Cleavage extents were determined by gel electrophoresis (PAGE) followed by quantitation on the AMBIS.

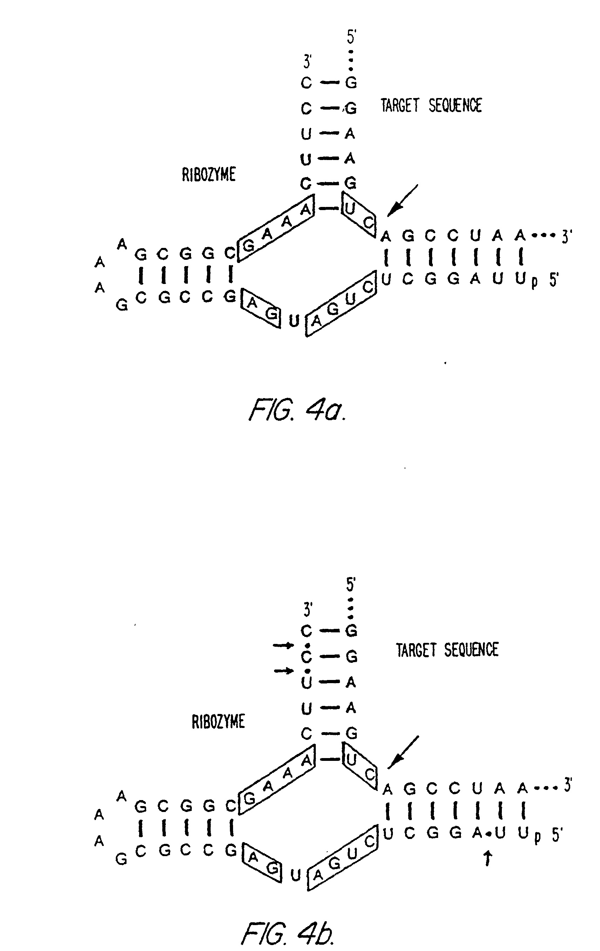

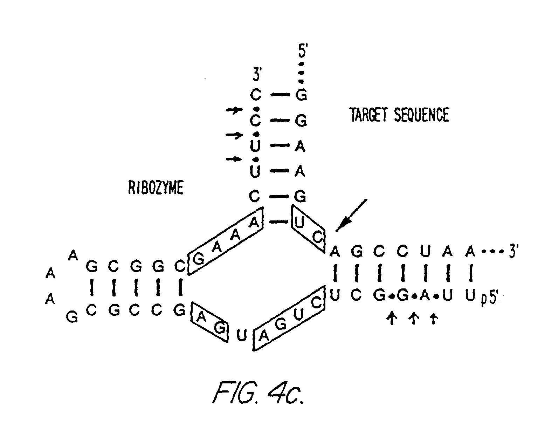

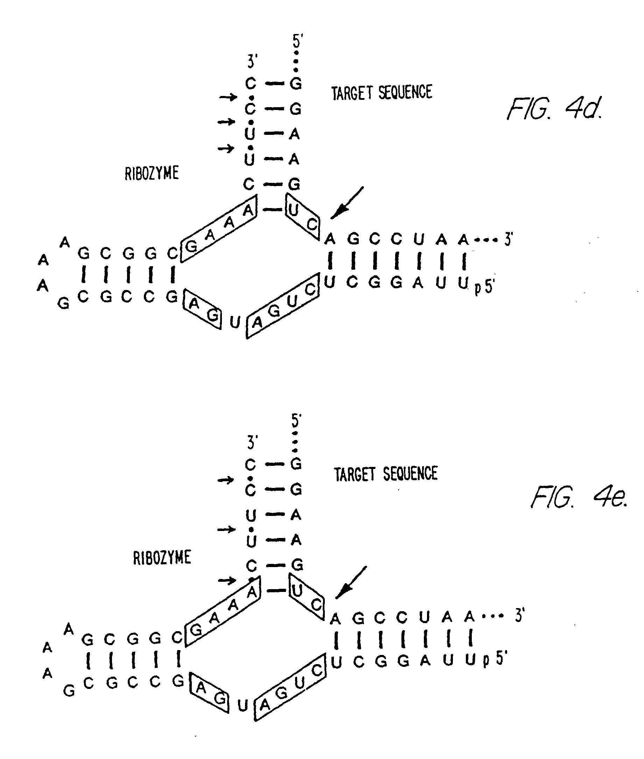

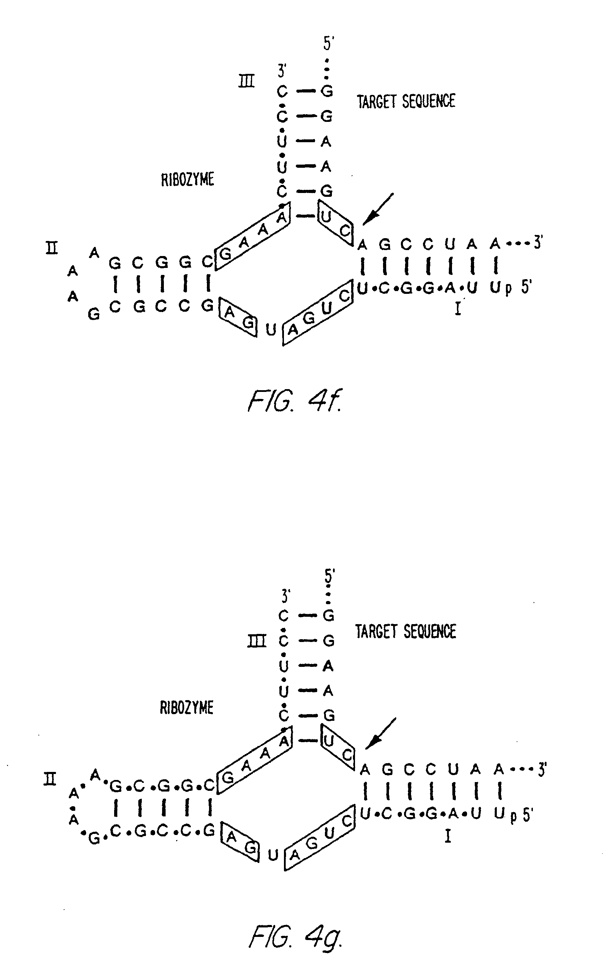

The ribozymes are shown in Figs. 4A - 4G. The following ribozymes showed essentially 100% activity: r37 (unmodified), r37s2 (1 thio on 5' arm, 2 on 3' arm), r37s4 (3 thio modifications on each arm), s37A (3 modifications on 3' arm), s37B (3 modifications alternating on 3' arm).

Ribozymes that are fully modified showed some decrease in activity, for example, s37C (almost completely modified on substrate binding arms) showed a 3x reduction in activity relative to unmodified, s37D (thio modifications in stem I, II and III) showed about a 9x reduction in activity relative to unmodified.

### Example 48: 2'O-methyl-containing Ribozymes

Ribozymes were made on an ABI synthesizer using standard phosphoramidite chemistry with 2'-O-methyl phosphoramidite nucleotides used in place of standard nucleotides. Ribozyme activity was determined in the manner described above with PAGE analysis.

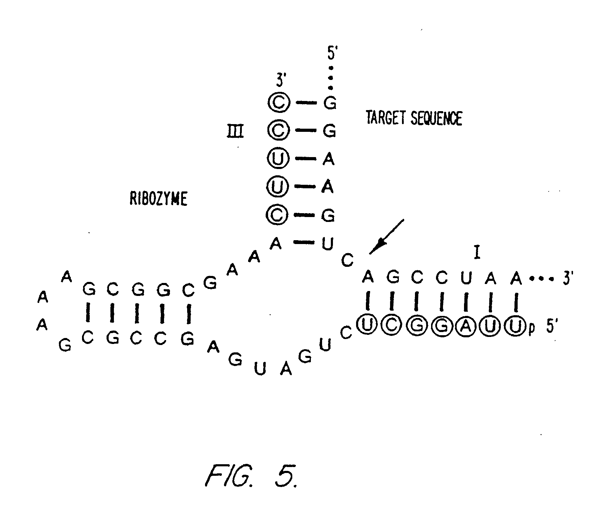

Referring to Fig. 5, a ribozyme containing 2'-O-methyl at all positions that base-pair with substrate (except for the A at the 5' side of stem III) was synthesized. The Kcat/Km for the 2'-O-methyl ribozyme was 44 x 10⁶ M⁻¹ min⁻¹ compared to 41 x 10⁶ m⁻¹ min⁻¹ for the unmodified, and 32 x 10⁶ for a thiophosphate modified ribozyme. Thus, the 2'-O-methyl ribozyme retains 100% activity.

### Example 49: Cleavage of Short Substrate RNAs Corresponding to ICP4 Gene Targets

Substrate/ribozyme pairs were evaluated for predicted structural characteristics as described above. Nine candidate substrate/ribozyme pairs were tested for their capacity to cleave substrate RNAs *in vitro* in a ribozyme cleavage assay described above. Of these nine ribozymes, seven were targeted to the 5' end of the ICP4 mRNA and two ribozymes were targeted to the 3' end of the mRNA. Values for the k_{cat}, kₘ and k_{cat}/kₘ (the "cleavage constant") were experimentally determined for each of these ribozymes based upon the assay reactions. The results are presented in Table 7. The k_{cat}/kₘ values for several of these ribozymes are higher than those recently observed for HIV-specific ribozymes in our laboratory. Ribozyme G was extremely active in this assay, with a calculated k_{cat}/kₘ of approximately 1 x 10⁸ M⁻¹ min⁻¹, the highest level of activity observed using this assay. The generally high level of activity observed suggests that factors other than correct folding and the GUC cleavage region influence the structural characteristics of ribozyme-substrate interaction.

**TABLE 7**

| RIBOZYME CLEAVAGE OF SHORT ICP4 TARGET RNA SUBSTRATES | | | | |
|---|---|---|---|---|
| Ribozyme | ICP4 Cleavage Site¹ | k_{cat}/kₘ | k_{cat} | kₘ |
| A | 66 (1) | 2 x 10⁷ M⁻¹ min⁻¹ | | |
| B | 177 | no activity | | |
| C | 200 | 6 x 10⁷M⁻¹ min⁻¹ | 1.73 min⁻¹ | 34nM |
| D | 231 | no activity | | |
| E | 309 (8) | 5.3 x 10⁷M⁻¹ min⁻¹ | | |
| F | 864,888 | 3.4 x 10^{6 M-1} min⁻¹ | | |
| G | 870,894 | 1.17 x 10^{8 M-1} min⁻¹ | 5.7 min⁻¹ | 127nM |
| H | 3271 | no activity | | |
| I | 3559 | 5 x 10⁷ | 2.2 min⁻¹ | 69nM |

| | | | | |
|---|---|---|---|---|
| 1/ Nucleotide number relative to EMBL HSV-1 ICP4 gene sequence. Numbers in parentheses correspond to target SEQ ID NOS. | | | | |

### Example 50: Cleavage of Long Substrate RNA's Corresponding to ICP4 Gene

The ability of three ribozymes to catalyze the cleavage of an approximately 490 nucleotide long ICP4 RNA substrate (corresponding to nucleotides 1-493 of ICP4 and prepared as described above was determined using the ribozyme cleavage assay described above. The assays were preformed in ribozyme excess and approximate k_{cat}/kₘ values were calculated. The results are presented in Table 8. The cleavage constants for short substrate cleavage by each ribozyme are also included for comparison.

The results indicate that, generally, ribozyme cleavage of long substrates proceeds at a slower rate compared to cleavage activities on short substrates, although cleavage activity is not altogether eliminated by the use of the longer substrate. The effect of using a longer substrate varied among the five ribozymes tested, indicating that the accessibility and structural parameters of different cleavage sites change unpredictably when presented in the context of the longer RNA substrates.

All three of the ribozymes exhibited a low capacity to catalyze the long substrate relative to their activities on the shorter substrates. Two of the ribozymes, ribozymes A and E, were relatively more effective at catalyzing cleavage of the longer substrate RNA.

These results demonstrate that structural characteristics of the substrate RNA may influence ribozvme catalytic activity.

**TABLE 8**

| EFFECT OF SUBSTRATE RNA SIZE ON RIBOZYME ACTIVITY | | | |
|---|---|---|---|
| Ribozyme | Substrate Length¹ | K_{cat}/kₘ (M⁻¹ min⁻¹) | % Activity on Short Substrate |
| A | 17 | 2 x 10⁷ | 100 |
| | 490 | 4 x 10⁴ | 57 |
| | | | |
| C | 17 | 6 x 10⁷ | 100 |
| | 490 | 5 x 10² | 8 |
| | | | |
| E | 17 | 5.3 x 10⁷ | 100 |
| | 490 | 2 x 10³ | 17 |

| | | | |
|---|---|---|---|
| ¹ In nucleotides. | | | |

### Example 51: Delivery of Stable Ribozyme Into Vero Cells

Ribozymes were end-labeled, encapsulated in liposomes and delivered to Vero cells as described above. Intracellular ribozymes were purified from cell fractions by phenol/chloroform extraction, and aliquots were size fractionated along with newly synthesized ribozyme samples on denaturing polyacrylamide gels to determine intracellular location of intact ribozymes. By comparison with the migration rate of newly synthesized ribozymes, only the cytoplasmic fraction was observed to contain intact ribozyme. Although intact ribozymes recovered from the cytoplasmic fraction represented a relatively small percentage of the ribozymes encapsulated into the liposome preparations, these results demonstrate that ribozymes can be delivered to and retain stability within the cell cytoplasm via a liposome delivery vehicle.

### Example 52: Synthesis of Modified Ribozymes with Enhanced Resistance to Nuclease Digestion

Modified ribozymes were synthesized with 5 phosphorothioate (PS) or 2' O-methyl (2' O-Me) modifications at each end of the molecule, and end-labeled with ³²P-γATP using T4 polynucleotide kinase. Unmodified (phosphodiester) ribosomes were synthesized as described above, and similarly end-labeled.

Cultured Vero cells were fractionated as described above. The purity of each fraction was assessed by assaying enzyme activities known to reside in that fraction. As shown in Table 10, all enzyme activities tested were found predominantly in the appropriate cell fractions, indicating an acceptable level of purity. Cell fractions were frozen at -70° C prior to use in a nuclease resistance assay.

**TABLE 9**

| ENZYME | % OF TOTAL ENZYME ACTIVITY | | |
|---|---|---|---|
| | Membrane Fraction | Nuclear Fraction | Cytoplasmic Fraction |
| Lactate Dehydrogenase (cytoplasmic marker) | 3 | 3 | 94 |
| | | | |
| Calcine (membrane marker) | 81 | 3 | 16 |
| | | | |
| Lysosome Hexosaminidase | 75 | 5 | 20 |
| | | | |
| Glucocerebrosidase (membrane marker) | 96 | 0 | 4 |
| | | | |
| Endosome Alkaline Phosphodiesterase (membrane marker) | 97 | 2 | 1 |

Equal concentrations (1-2 pmoles/100 microliters) of unmodified and PS or 2' O-Me modified ribozymes were added to Vero cell cytoplasmic extracts and incubated at 37°C for 0-60 minutes. Aliquots of each were taken at 10 minute intervals, and size fractionated by electrophoresis on denaturing polyacrylamide gels (15%). Relative rates of ribozyme degradation were analyzed by scanning the gels with an Ambis beta scanner as measure of relative nuclease resistance.

We found that the unmodified ribozyme is quickly digested by the cytoplasmic extract, but that the PS and 2' O-Me modified ribozymes are not digested as rapidly and are thus relatively more nuclease resistant. The PS and 2' O-Me modified ribozymes retain 80-90% of the catalytic activity observed with their unmodified ribozyme counterparts, are better able to resist nuclease digestion, and therefore may have increased half-lives *in vivo*. In that connection, PS and 2' O-Me modified ribozymes may have advantages over unmodified ribozymes for use in therapeutic applications.

In addition, we found that nearly equivalent nuclease resistance assay results were obtained for unlabeled, 5' end-labeled and 3' end-labeled unmodified ribozymes indicating that the observed loss of radioactive label in the PS and 2' O-Me modified ribozymes was not due to dephosphorylation of the 5' end labels, but instead reflects degradation by cytoplasmic nuclease. This suggests that a significant component of Vero cell nuclease activity is endonucleolytic in nature, and it may therefore be desirable to introduce similar modifications to the internal regions of the ribozyme molecule in order to best optimize nuclease resistance.

### Example 53: Ribozyme Cleavage of Single Base-Substituted Substrates In Vitro

Unmodified ribozymes I and E, specific for targets within the ICP4 gene of HSV-1, were synthesized and purified as described above.

A single nucleotide change was incorporated into the substrate cleavage site recognized by ribozyme I. Specifically, the substrate sequence CCGGGGGUCUUCGCGCG was changed at the central C nucleotide to become CCGGGGGUAUUCGCGCG, so that the GUC cleavage site became GUA. This modified substrate was synthesized and tested for *in vitro* cleavage by ribozyme I using the ribozyme cleavage assay described above.

Single nucleotide changes were incorporated into the regions complementary to the ribozyme binding arms in the substrate RNAs recognized by ribozymes I and E as indicated in Table 11, *infra.* Computer analysis of ribozyme/substrate folding predicted that these changes would result in the maintenance of ribozyme/substrate binding strength. Four such modified substrates were synthesized and tested alongside their "native" substrate counterparts for *in vitro* cleavage by ribozymes I or E.

Ribozyme I was tested for its ability to cleave two substrate sequences differing by a single base in the substrate cleavage site, one substrate corresponding to nucleotides 3550 - 3566 of the ICP4 mRNA sequence, and the other corresponding to the same sequence with a single base substitution in the cleavage site. The k_{cat}/kₘ values for cleavage of both substrates are 5.3 x 10⁷ M⁻¹ min⁻¹ and 7.3 x 10⁷ M⁻¹ min⁻¹ for the native and modified site respectively. The results indicate that ribozyme I is significantly more efficient at cleaving the modified substrate containing the GUA site. Thus, ribozyme I is not only capable of recognizing and cleaving the "native" ICP4 mRNA target, but is also capable of recognizing and cleaving (more efficiently) that same ICP4 mRNA target with a point mutation in the cleavage site. Natural mutations to particular cleavage sites in viral RNA within infected host cells are possible. Ribozyme I, therefore, may be a particularly good therapeutic ribozyme given its target recognition versatility. The results also suggest that ICP4 mRNA targets containing a GUA cleavage site would be acceptable if not preferred ribozyme substrates.

Ribozymes I and E were also tested for their ability to cleave substrates with single nucleotide modifications in the complementary regions. The results, shown in Table 11, indicate that both ribozymes can recognize and efficiently cleave substrates with nucleotide point mutations. The results also show that relatively minor changes in substrate sequence can influence ribozyme/substrate interactions in ways that were not predicted based on binding strength alone.

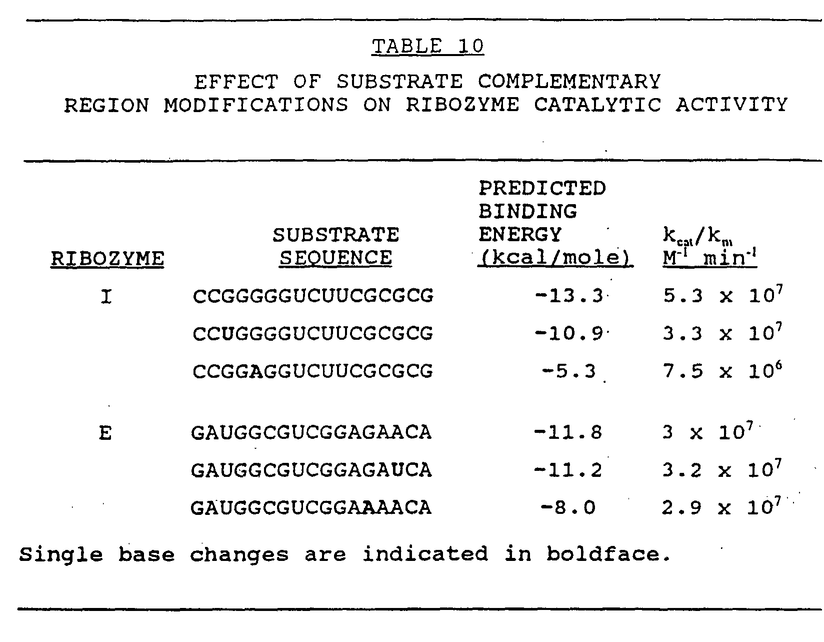

### Example 54: Effect of Ribozyme Arm Length on Cleavage Activity

Variations of ribozymes E and I having longer binding arm lengths were designed, synthesized and tested together with their prototype ribozymes for cleavage of the substrate recognized by the prototypes using a ribozyme cleavage assay.

The results are summarized in Table 11, and indicate that the minimal binding arm lengths required for effective cleavage vary from one ribozyme to another. This indicates that each chosen ribozyme will need to be optimized in its structure on an individual basis.

**TABLE 11**

| EFFECT OF RIBOZYME BINDING STEM LENGTH UPON SUBSTRATE CLEAVAGE | | | |
|---|---|---|---|
| Ribozyme | Hybridizing Stem Length (stem I, stem III) | Binding energy (kcal/mole) | k_{cat}/kₘ |
| E-32 | 5,5 | -9.1 | 3.8 x 10⁷ M⁻¹ min⁻¹ |
| E-33-65 | 6,5 | -9.7 | 2.8 x 10⁷ M⁻¹ min⁻¹ |
| E-33-56 | 5,6 | -11.2 | [NA] |
| E-34 | 6,6 | -11.8 | 3 x 10⁷ M⁻¹ min⁻¹ |
| | | | |
| I-32 | 5,5 | - 9.4 | 1.6 x 10⁷ M⁻¹ min⁻¹ |
| I-33-65 | 6,5 | -10.1 | <<1 x 10⁶ M⁻¹ min⁻¹ |
| I-33-56 | 5,6 | -12.8 | 1 x 10⁶ M⁻¹ min⁻¹ |
| I-34 | 6,6 | -13.3 | 5 x 10⁷ M⁻¹ min⁻¹ |
| NA = not available. | | | |

### Example 55:

We have used the ribonuclease protection assay to determine the localization of the HSV ICP4 and ICP27 mRNAs within infected Vero cells. We found that > 80% of the viral mRNAs were situated in the cytoplasm of the infected cells at 4 hours post infection. We have chosen a site within the HSV ICP4 mRNA which is accessible in RNaseH assays for further study. We have examined the effects of altering ribozyme binding arm lengths on catalytic activity and chosen a ribozyme which has binding arms of 6 nucleotides. We call this molecule RPI 1197. We have checked the ability of RPI 1197 to cleave the ICP4 mRNA in cell lysates from infected cells and found that the amount of ICP4 cleaved by added RPI 1197 increased over time and gave the anticipated cleavage products. Using a liposome formulation which delivers ribozymes to the cytoplasm of cells, we pre-treated cells with two ribozyme molecules (RPI 1197 and RPI 1200 [a 2'-O-Methyl substituted version of 1197]) and tested them for their ability to reduce intracellular levels of the viral ICP4 mRNA and inhibit viral replication. We found that RPI 1197 and RPI 1200 reduced the mRNA levels by 10 and 36%, respectively, and viral load by 6 and 18%, respectively.

Early testing of the accessible sites of the UL5 transcript using the RNaseH assay has demonstrated that a number of regions within the transcript are accessible to binding of ribozymes. We have used a 622 nucleotide region (region D) of the UL5 RNA to check the cleavage ability of ribozymes targeted to both weakly and strongly accessible sites. We have found that the ribozyme activity in this fragment of RNA showed a good correlation between accessibility of sites and the ability of ribozymes to cleave the target RNA at those sites. In preliminary experiments, we have observed that the results of the RNaseH assay can also be mimicked using a gel-binding assay in which pieces of complementary nucleic acid (either RNA or DNA) are bound to target RNA and the mixtures are electrophoresed through gels to observe the formation of oligo/target complexes. By varying the concentration of the oligonucleotide, one can determine the accessible regions of the RNA as well as the binding affinity of the oligonucleotide for that region. We have found a good correlation between the strength of the binding complexes at particular sites and the activity of ribozymes at those sites within the UL5 RNA molecules.

Other embodiments are within the following claims.

## Claims

1. An enzymatic RNA molecule which is active to specifically cleave genomic RNA, or mRNA, or hnRNA of or encoded by a hepatitis B Virus.

2. The enzymatic RNA molecule of claim 1, which cleaves RNA or mRNA encoded by a gene selected from the group consisting of:
the 5' 1500 bases of hepatitis B virus (HBV), and
a region which controls expression of S protein, core protein, X protein or DNA polymerase of HBV.

3. The enzymatic RNA molecule of claim 1, wherein said RNA molecule is in a hammerhead motif.

4. The enzymatic RNA molecule of claim 1, wherein said RNA molecule is in a hairpin, hepatitis Delta virus, group 1 intron, or RnaseP RNA motif.

5. The enzymatic RNA molecule of claim 1, which cleaves the sequence shown as any of SEQ. ID. NOS. 1-33 in Fig. 8.

6. The enzymatic RNA molecule of any of claims 1-5, wherein said ribozyme comprises between 5 and 23 bases complementary to the RNA of said gene or region.

7. The enzymatic RNA molecule of claim 6, wherein said ribozyme comprises between 10 and 18 bases complementary to the RNA of said gene or region.

8. A mammalian cell including an enzymatic RNA molecule of any of claims 1-5.

9. The cell of claim 8, wherein said cell is a human cell.

10. The cell of claim 9, wherein said cell is a T4 lymphocyte having a CD4 receptor molecule on its cell surface.

11. An expression vector comprising nucleic acid encoding the enzymatic RNA molecule of any of claims 1-5, in a manner which allows expression of that enzymatic RNA molecule within a mammalian cell.

12. Use of an enzymatic RNA molecule of any of claims 1-5 for the manufacture of a medicament for treatment of a disease caused by a HBV.

13. Use of a defective viral particle produced by contacting a cell infected with an HBV with an enzymatic RNA molecule active to cleave a gene required for viral replication for the manufacture of a medicament for inducing an immune response or for inducing production of anti-HBV immunoglobulin in a human.
